# EUROPEAN PATENT SPECIFICATION

(11) **EP 2 503 001 B1**
(45) Date of publication and mention of the grant of the patent: **08.03.2017**
(21) Application number: 12172956.0
(22) Date of filing: 01.05.2009
(51) Int. Cl.: C12N 15/82, A01H 5/00

(54) **USE OF RICE POLYPEPTIDES/NUCLEIC ACIDS FOR PLANT IMPROVEMENT**
VERWENDUNG VON REISPOLYPEPTIDEN/NUKEINSÄUREN ZUR VERBESSERUNG VON PFLANZEN
UTILISATION D'ACIDES NUCLÉIQUES/POLYPEPTIDES DE RIZ POUR AMÉLIORATION D'UNE PLANTE

(30) Priority: 01.05.2008 US 49501 P
(43) Date of publication of application: 26.09.2012
(62) Divisional of application: 09739932.3
(73) Proprietor: Academia Sinica, Nan-Kang, Taipei (TW)
(72) Inventor: Yu, Su-May, 115 Taipei (TW); Ko, Swee-Suak, 820 Kaoshiung County (TW); Hsing, Yue-Ie C., 115 Taipei (TW); Ho, Tuan-Hua David, Chesterfield, MO 63017 (US); Lo, Shuen-Fang, 412 Taichung County (TW)
(74) Representative: Chajmowicz, Marion

(56) References cited:
- US-A1- 2004 123 343
- US-A1- 2007 020 621
- US-A1- 2007 039 076
- US-A1- 2007 118 921
- DATABASE UniProt [Online] 24 January 2006 (2006-01-24), "SubName: Full=Expressed protein; SubName: Full=Os11g0497350 protein;", XP002679401, retrieved from EBI accession no. UNIPROT:Q2R3X6 Database accession no. Q2R3X6

### SEQUENCE LISTING

<110> Yu, Su-May
   Ko, Swee-Suak
   Hsing, Yue-Ie C.
<120> Use of Rice Polypeptides/Nucleic Acids for Plant Improvement
<130> B1088EPPC
<140> PCT/US09/42529
   <141> 2009-05-01
<160> 151
<170> PatentIn version 3.5
<210> 1
   <211> 813
   <212> PRT
   <213> Oryza sativa
<400> 1
   Met Lys Lys Ala Lys Phe Pro Gly Ser Ser Ser Ser Ser Ser Ser Ala
   1               5                   10                  15
   Ala Ala Pro Gly Val Thr Glu Lys Asn Gly Leu His Val Asp Pro Thr
               20                  25                  30
   Ala Ala Ala Ala Arg Thr Gly Gly Arg Thr Asn Gly Glu Glu Asp Ala
           35                  40                  45
   Glu Met Val Leu Ala Asp Gln Glu Glu Leu Ala Ala Pro Ser Ala Ser
       50                  55                  60
   Ala Pro Ala Gly Val Ala Ala Asn Leu Phe Arg Lys Lys Ala Thr Leu
   65                  70                  75                  80
   Pro Gln Pro Ser Ala Ala Arg Lys Pro Leu Arg Ile Lys Ile Gly Gln
                   85                  90                  95
   Pro Lys Leu Pro Thr Asn Phe Glu Glu Asp Thr Trp Ala Ile Leu Lys
               100                 105                 110
   Asp Ala Ile Thr Ala Ile Phe Leu Lys Gln Lys Leu Ser Cys Asp Val
           115                 120                 125
   Glu Lys Leu Tyr Gln Ala Ala Gly Asp Leu Cys Leu His Lys Leu Gly
       130                 135                 140
   Ala Asn Leu Tyr Glu Arg Ile Lys Lys Glu Cys Glu Val His Ile Ser
   145                 150                 155                 160
   Ala Lys Ile Ser Ala Leu Val Gly Gln Ser Pro Asp Leu Val Val Phe
   165 170 175
   Leu Ser Leu Val Gln Arg Thr Trp Gln Asp Phe Cys Asp Gln Met Leu
   180 185 190
   Ile Ile Arg Gly Ile Ala Leu Leu Leu Asp Val Lys Tyr Val Lys Asn
   195 200 205
   Val Ala Asn Ile Cys Ser Val Trp Asp Met Gly Leu Lys Leu Phe Arg
   210 215 220
   Lys His Leu Ser Leu Ser Pro Glu Ile Glu His Lys Thr Val Thr Gly
   225 230 235 240
   Leu Leu Arg Leu Ile Glu Ser Glu Arg Leu Gly Glu Ala Ile Asp Arg
   245 250 255
   Thr Leu Leu Ser His Leu Leu Lys Met Phe Thr Ala Leu Gly Met Tyr
   260 265 270
   Ser Glu Ser Phe Glu Lys Pro Phe Leu Glu Cys Thr Ser Glu Phe Tyr
   275 280 285
   Ala Thr Glu Gly Val Lys Tyr Leu Gln Gln Ser Asp Ile Pro Asp Tyr
   290 295 300
   Leu Lys His Val Glu Thr Arg Leu Gln Glu Glu His Glu Arg Cys Ile
   305 310 315 320
   Leu Tyr Leu Glu Ala Asn Thr Arg Lys Pro Leu Ile Thr Ala Thr Glu
   325 330 335
   Lys Gln Leu Leu Gln Arg His Thr Ser Ala Ile Leu Glu Lys Gly Phe
   340 345 350
   Thr Met Leu Met Glu Ala Asn Arg Val Lys Asp Leu Ser Arg Met Tyr
   355 360 365
   Thr Leu Phe Gln Arg Val Asp Ala Ile Glu Leu Leu Lys Gln Ala Leu
   370 375 380
   Ser Ser Tyr Ile Arg Gly Thr Gly Gln Gly Ile Ile Met Asp Glu Glu
   385 390 395 400
   Lys Asp Lys Glu Leu Val Pro Phe Leu Leu Glu Phe Lys Ala Ser Leu
   405 410 415
   Asp Arg Ile Leu Glu Glu Ser Phe Ala Lys Asn Glu Ala Phe Ser Asn
   420 425 430
   Thr Ile Lys Glu Ser Phe Glu His Leu Ile Asn Leu Arg Gln Ile Ser
           435                 440                 445
   Ser Ser Pro Phe Phe Gln Gln Asn Arg Pro Ala Glu Leu Ile Ala Lys
       450                 455                 460
   Phe Leu Asp Glu Lys Leu Arg Ala Gly Asn Lys Gly Thr Ser Glu Glu
   465                 470                 475                 480
   Glu Leu Glu Gly Ile Leu Asp Lys Val Leu Val Leu Phe Arg Phe Ile
                   485                 490                 495
   Gln Gly Lys Asp Val Phe Glu Ala Phe Tyr Lys Lys Asp Leu Ala Lys
               500                 505                 510
   Arg Leu Leu Leu Gly Lys Ser Ala Ser Ile Asp Ala Glu Lys Ser Met
           515                 520                 525
   Ile Thr Lys Leu Lys Thr Glu Cys Gly Ser Gln Phe Thr Asn Lys Leu
       530                 535                 540
   Glu Gly Met Phe Lys Asp Ile Glu Leu Ser Lys Glu Ile Asn Glu Ser
   545                 550                 555                 560
   Phe Lys Gln Ser Ser Gln Ala Arg Thr Lys Leu Pro Ser Gly Ile Glu
                   565                 570                 575
   Met Ser Val His Val Leu Thr Thr Gly Tyr Trp Pro Thr Tyr Pro Pro
               580                 585                 590
   Met Asp Val Lys Leu Pro His Glu Leu Asn Val Tyr Gln Asp Ile Phe
           595                 600                 605
   Lys Glu Phe Tyr Leu Ser Lys Tyr Ser Gly Arg Arg Leu Met Trp Gln
       610                 615                 620
   Asn Ser Leu Gly His Cys Val Leu Lys Ala Glu Phe Pro Lys Gly Lys
   625                 630                 635                 640
   Lys Glu Leu Ala Val Ser Leu Phe Gln Ser Val Val Leu Met Leu Phe
                   645                 650                 655
   Asn Asp Ala Gln Lys Leu Ser Phe Leu Asp Ile Lys Glu Ser Thr Gly
               660                 665                 670
   Ile Glu Asp Lys Glu Leu Arg Arg Thr Leu Gln Ser Leu Ala Cys Gly
           675                 680                 685
   Lys Val Arg Val Leu Gln Lys Met Pro Lys Gly Arg Asp Val Glu Asp
       690                 695                 700
   Lys Asp Glu Phe Val Phe Asn Glu Glu Phe Ser Ala Pro Leu Tyr Arg
   705                 710                 715                 720
   Ile Lys Val Asn Ala Ile Gln Met Lys Glu Thr Val Glu Glu Asn Thr
                   725                 730                 735
   Ser Thr Thr Glu Arg Val Phe Gln Asp Arg Gln Tyr Gln Val Asp Ala
               740                 745                 750
   Ala Ile Val Arg Ile Met Lys Thr Arg Lys Thr Leu Ser His Thr Leu
           755                 760                 765
   Leu Ile Thr Glu Leu Phe Gln Gln Leu Lys Phe Pro Ile Lys Pro Ser
       770                 775                 780
   Asp Ile Lys Lys Arg Ile Glu Ser Leu Ile Asp Arg Glu Tyr Leu Glu
   785                 790                 795                 800
   Arg Asp Arg Ser Asn Pro Gln Ile Tyr Asn Tyr Leu Ala
                   805                 810
<210> 2
   <211> 561
   <212> PRT
   <213> Oryza sativa
<400> 2
   Met Asp Ala Asp Glu Ala Ala Gly Ser Ser Arg Arg Met Asp Leu Asn
   1               5                   10                  15
   Leu Tyr Leu Gly Leu Pro Arg Ala Pro Arg Pro Arg Arg Ser Asp Leu
               20                  25                  30
   Gly Ser Asp Leu Ala Leu Ser Thr Pro Met Pro Ser Ser Pro Ser Ser
           35                  40                  45
   Ser Ala Ala Ser Val Asp Ala Pro Pro Pro Pro Pro Glu Leu Ser His
       50                  55                  60
   Pro Pro Tyr Ser Pro Ser His Ala Asp Leu Ser Pro Pro Leu Gln Glu
   65                  70                  75                  80
   Val Tyr Ser Leu Tyr Asn Pro Asp Asp Pro Pro Ala Ser Glu Thr His
                   85                  90                  95
   Leu Pro Pro Tyr Ala Pro Pro Pro Ala Pro Val Val Ser Glu Leu Pro
   100 105 110
   Asp Asp Leu Glu Phe Gly Leu His Pro Pro Pro Pro Leu Val Arg Ala
   115 120 125
   Ser Glu Leu Leu Gly Trp Glu Asp Arg Pro Ser Ser Ser Thr Ala Ser
   130 135 140
   Ser Ser Phe Leu Pro Asp Thr Ala Ala Arg Tyr Trp Arg Leu Leu Glu
   145 150 155 160
   Gln Thr Gly Ser Arg Trp Leu Arg Ala Arg Arg Phe Arg Ser Asp Leu
   165 170 175
   Pro Pro Leu Ser Ser Glu Ala Tyr Pro Ala Gly Arg Asp Ala Ala Ala
   180 185 190
   Val Pro Val Leu Gln His Glu Pro Met Asn Asp Thr Val Glu His Asn
   195 200 205
   Lys Val Ala Ala Asp Gly Ala Glu Val Gly Ala Ser Glu Glu Ser Glu
   210 215 220
   Glu Gln Gly Arg Ser Ala Ala Thr Phe Glu Cys Asn Ile Cys Phe Asp
   225 230 235 240
   Met Ala Ser Glu Pro Val Val Thr Ser Cys Gly His Leu Phe Cys Trp
   245 250 255
   Pro Cys Leu Tyr Gln Trp Leu Asn Val Tyr Ser Asn His Lys Glu Cys
   260 265 270
   Pro Val Cys Lys Gly Glu Val Thr Glu Ala Asn Ile Thr Pro Ile Tyr
   275 280 285
   Gly Arg Gly Asn Ser Cys Leu Asp Ala Glu Lys Ala Val Glu Gly Gly
   290 295 300
   Lys Gln Thr Gly Pro Thr Ile Pro Pro Arg Pro His Gly Asn Arg Leu
   305 310 315 320
   Glu Ser Phe Arg Gln Gln Phe His His Leu Arg Pro Ile Ser Arg Arg
   325 330 335
   Leu Gly Glu Ala His Gly Leu Leu Ser Ser Trp Arg Arg Leu Leu Asp
   340 345 350
   Gln Gln Ile Met Asn Thr Ala Ser Arg Phe Glu Gly Pro Pro Glu Ser
   355 360 365
   Ala Val Gln Glu Met Val Asp Thr Ala His Ala Gln His Thr Ser Arg
       370                 375                 380
   Leu Ser Arg Leu Ala Ser Arg Met Arg Ala Arg Arg Leu Leu Arg Glu
   385                 390                 395                 400
   Ala Asp Asn Pro Asn Pro Pro Asp Gly Gly Ser Thr Ser Pro Asp Ser
                   405                 410                 415
   Gly Leu Ile Arg Asn Asn Ala Ser Asp Pro Ser Arg Asn Gly Pro Ser
               420                 425                 430
   Ser Leu Leu Pro Asp Gly Ile Asp Trp Leu Arg Gly Leu Thr Leu Leu
           435                 440                 445
   Gly Tyr Glu Asp Thr Glu Arg Phe Ala Ser Ala Met Ser Asp Phe Arg
       450                 455                 460
   Arg Ile Thr Gly Pro Ser Gln Tyr Gly Ala Ser Ala Ser Ser Ser Asn
   465                 470                 475                 480
   Pro Pro Asn Leu Glu Ser Thr Phe Asp Arg Thr His Val Val Ala Ala
                   485                 490                 495
   Pro Ser Ala Asp Gln Ala Ser Asn Ser Ser Thr Ala Ala Val Ile Gln
               500                 505                 510
   Gly Asp Ala Gly Ile Ser Glu Ser Ala Gly Glu Pro Ser Asn Ala Gly
           515                 520                 525
   Ser Ser Arg Ser Leu Arg Arg Arg Gly Arg Ser Ser Ala Leu Gly Ser
       530                 535                 540
   Leu Asp Ala Asp Gly Gly Gly Leu Gln Arg Asn Lys Arg Arg Arg Ile
   545                 550                 555                 560
   Asn
<210> 3
   <211> 173
   <212> PRT
   <213> Arabidopsis thaliana
<400> 3
   Met Cys Pro Arg Ala Thr Gln Thr Cys Glu Ile Cys Glu Lys Val Val
   1               5                   10                  15
   Ser Lys Tyr Lys Cys Pro Ser Cys Leu Val Pro Tyr Cys Ser Leu Gly
               20                  25                  30
   Cys Phe Lys Ile His Lys Glu Thr Pro Cys Ala Lys Pro Ser Asp Pro
           35                  40                  45
   Ser Ser Thr Glu Glu Lys Pro Ala Ala Ser Pro Ala Lys Glu Val Pro
       50                  55                  60
   Val Lys Arg Pro Glu Glu Ala Asn Asp Val Val Glu Lys Thr Gln Gln
   65                  70                  75                  80
   Lys Ala Ser Ala Ala Ser Pro Ala Lys Glu Ile Pro Val Ala Arg Pro
                   85                  90                  95
   Ile Ile Val Glu Glu Glu Lys Tyr Ile Leu Glu Lys Thr Gln Phe Glu
               100                 105                 110
   Ala Ile Ala Ser Ser Ser Glu Ile Arg Glu Ala Leu Lys Asp Glu Pro
           115                 120                 125
   Leu Gln Lys Leu Ile Tyr Ser Ile Asp Ser Ser Ser Asn Pro Leu Gln
       130                 135                 140
   Glu Leu Asp Glu Ala Met Gly Ile Glu Ala Phe Arg Glu Phe Thr Asp
   145                 150                 155                 160
   Lys Ile Leu Ser Asn Ile Ser Lys Ser Asn Asp Glu Gln
                   165                 170
<210> 4
   <211> 519
   <212> PRT
   <213> Oryza sativa
<400> 4
   Met Leu Gly Glu Ala Ala Ser Pro Trp Ser Leu Ala Gly Ala Gly Ala
   1               5                   10                  15
   Ala Val Ala Leu Leu Trp Leu Cys Ala Trp Thr Leu Gln Trp Ala Trp
               20                  25                  30
   Trp Thr Pro Arg Arg Leu Glu Arg Ala Leu Arg Ala Gln Gly Leu Arg
           35                  40                  45
   Gly Thr Arg Tyr Arg Leu Phe Ile Gly Asp Val Ala Glu Asn Gly Arg
       50                  55                  60
   Leu Asn Arg Glu Ala Ala Ser Arg Pro Leu Pro Leu Gly Ser His Asp
   65                  70                  75                  80
   Val Val Pro Arg Val Met Pro Phe Phe Cys Asn Val Leu Lys Glu His
                   85                  90                  95
   Gly Lys Leu Ser Phe Val Trp Thr Gly Pro Lys Pro Phe Val Ile Ile
               100                 105                 110
   Arg Asp Pro Asp Leu Ala Arg Glu Ile Leu Ser Asn Lys Ser Gly Asn
           115                 120                 125
   Phe Ala Lys Gln Thr Thr Ala Gly Ile Ala Lys Phe Val Val Gly Gly
       130                 135                 140
   Val Val Thr Tyr Glu Gly Glu Lys Trp Ala Lys His Arg Arg Ile Leu
   145                 150                 155                 160
   Asn Pro Ala Phe His Gln Glu Lys Ile Lys Arg Met Leu Pro Val Phe
                   165                 170                 175
   Leu Ala Cys Cys Thr Lys Met Ile Thr Arg Trp Val Asn Ser Met Ser
               180                 185                 190
   Ser Glu Gly Ile Ser Glu Leu Asp Val Trp Asp Glu Phe Gln Asn Leu
           195                 200                 205
   Thr Gly Asp Val Ile Ser Arg Thr Ala Phe Gly Ser Ser Tyr Gln Glu
       210                 215                 220
   Gly Trp Arg Ile Phe Gln Leu Gln Glu Glu Gln Ala Lys Arg Val Leu
   225                 230                 235                 240
   Lys Ala Phe Gln Arg Ile Phe Ile Pro Gly Tyr Trp Tyr Leu Pro Ile
                   245                 250                 255
   Glu Asn Asn Arg Arg Ile Arg Glu Ile Asp Gln Glu Ile Arg Thr Ile
               260                 265                 270
   Leu Arg Gly Ile Ile Val Lys Arg Asp Lys Ala Val Arg Asn Gly Glu
           275                 280                 285
   Gly Ser Asn Asp Asp Leu Leu Gly Leu Leu Val Glu Ser Asn Met Arg
       290                 295                 300
   Gln Ser Asn Glu Lys Glu Asp Val Gly Met Ser Ile Glu Asp Met Ile
   305                 310                 315                 320
   Glu Glu Cys Lys Leu Phe Tyr Ala Ala Gly Ser Glu Thr Thr Ser Met
                   325                 330                 335
   Leu Leu Thr Trp Thr Leu Ile Leu Leu Ser Met His Pro Glu Trp Gln
               340                 345                 350
   Glu Gln Ala Arg Glu Glu Val Met His His Phe Gly Arg Thr Thr Pro
           355                 360                 365
   Asp His Asp Gly Leu Ser Arg Leu Lys Ile Val Thr Met Ile Leu His
       370                 375                 380
   Glu Val Leu Arg Leu Tyr Pro Pro Val Val Phe Leu Gln Arg Thr Thr
   385                 390                 395                 400
   His Lys Glu Ile Glu Leu Gly Gly Ile Lys Tyr Pro Glu Gly Val Asn
                   405                 410                 415
   Phe Thr Leu Pro Val Leu Ser Ile His His Asp Pro Ser Ile Trp Gly
               420                 425                 430
   Gln Asp Ala Ile Lys Phe Asn Pro Glu Arg Phe Ala Asn Gly Val Ser
           435                 440                 445
   Lys Ala Thr Lys Phe Gln Thr Ala Phe Phe Ser Phe Ala Trp Gly Pro
       450                 455                 460
   Arg Ile Cys Leu Gly Gln Ser Phe Ala Ile Leu Glu Ala Lys Met Ala
   465                 470                 475                 480
   Leu Ala Thr Ile Leu Gln Ser Phe Ser Phe Glu Leu Ser Pro Ser Tyr
                   485                 490                 495
   Thr His Ala Pro His Thr Val Leu Thr Leu Gln Pro Gln Tyr Gly Ser
               500                 505                 510
   Pro Ile Lys Leu Lys Lys Leu
           515
<210> 5
   <211> 147
   <212> PRT
   <213> Oryza sativa
<400> 5
   Met Ala Lys Asp His Val Lys Ile Val Leu Lys Ala Tyr Ile Leu Gly
   1               5                   10                  15
   Pro Ile Lys Tyr Ile Leu Ser Leu Glu Ser Leu Tyr His Asn Cys Gly
               20                  25                  30
   Gly Leu Val Val Thr Met Ile Leu His Glu Val Ile Arg Leu Tyr Pro
           35                  40                  45
   Ser Gly Ile Phe Leu Gln Arg Thr Thr Arg Lys Glu Ile Glu Leu Gly
       50                  55                  60
   Gly Ile Lys Tyr Pro Glu Gly Ala Asn Phe Thr Leu Pro Val Pro Ser
   65                  70                  75                  80
   Ile His His Asp Pro Ser Ile Trp Gly Gly Asp Ala Ser Glu Phe Asn
                   85                  90                  95
   Leu Glu Arg Phe Ala Asn Gly Val Ser Lys Ala Thr Lys Phe Lys Thr
               100                 105                 110
   Ala Phe Phe Met Phe Gly Trp Gly Phe Ser Asp Leu Pro Trp Thr Glu
           115                 120                 125
   Leu Cys Asn Ala Gly Ser Gln Asp Gly Ala Arg His His Pro Pro Glu
       130                 135                 140
   Leu Leu Leu
   145
<210> 6
   <211> 72
   <212> PRT
   <213> Oryza sativa
<400> 6
   Met Cys Cys Ser Ala Val Ala Val Met Lys Trp Glu Ala Leu Leu Pro
   1               5                   10                  15
   Asn Asp Thr Phe Leu Ile Val Ala Ser Ser Asp Gly Val Phe Glu Lys
               20                  25                  30
   Val Thr Met Gln Asp Val Cys Asp Leu Met Leu Tyr Val Lys Leu Gly
           35                  40                  45
   Val Lys Gln Glu Leu Gly Ser Phe Ala Leu Thr Gln Gln Asn Leu Ala
       50                  55                  60
   Asp Tyr Val Val Asp Leu Ser Leu
   65                  70
<210> 7
   <211> 250
   <212> PRT
   <213> Oryza sativa
<400> 7
   Met Ser Ser Ser Asp Gln Asn Pro Ser Pro Thr Pro Ala Ser Gly Thr
   1               5                   10                  15
   Gly Thr Ser Val Pro Pro Pro Gly Arg Ala Thr Thr Val Ser Ser Gln
               20                  25                  30
   Leu Leu Asp Met Gly Ala Gln Ala Val Gln Ala Leu Lys Pro Val Arg
           35                  40                  45
   Gln Met Lys Gln His Ala Cys Ser Phe Ala Leu Tyr Ala His Asp Leu
       50                  55                  60
   Ser Arg Gln Val Glu Val His His Phe Val Ser Arg Leu Asn Gln Asp
   65                  70                  75                  80
   Val Leu Gln Cys Ala Val Tyr Asp Ser Asp Lys Pro Ser Ala Arg Leu
                   85                  90                  95
   Ile Gly Val Glu Tyr Ile Val Ser Asp Ala Ile Phe Glu Ser Leu Pro
               100                 105                 110
   Pro Glu Glu Gln Lys Leu Trp His Ser His Ala Tyr Glu Val Lys Ala
           115                 120                 125
   Gly Leu Trp Thr Asp Val Gly Val Pro Glu Pro Leu Gln Ser Ser Glu
       130                 135                 140
   Met Ala Arg Met Ala Lys Thr Tyr Gly Lys Leu Trp Cys Thr Trp Gln
   145                 150                 155                 160
   Val Asp Arg Gly Asp Ala Leu Pro Leu Gly Ala Pro Ala Leu Met Val
                   165                 170                 175
   Ser Pro Gln Ala Val Glu Pro Gly Arg Val Arg Ala Glu Leu Val His
               180                 185                 190
   Gly Arg Asp Glu Arg Tyr Lys Ile Asp Ser Ser Ala Gln Gly Leu Lys
           195                 200                 205
   Gly Ala Arg Val Glu Met Asp Glu Pro Glu Trp Ile Asn Pro Asn Ala
       210                 215                 220
   Asp Tyr Trp Arg Leu His Gly Lys Gly Phe Ala Ile Asp Val Thr Ala
   225                 230                 235                 240
   Thr Glu Met Lys Arg His Ala Pro Phe Pro
                   245                 250
<210> 8
   <211> 479
   <212> PRT
   <213> Oryza sativa
<400> 8
   Met Thr Pro Pro Pro Pro Ser Pro Pro His Glu Arg Lys Thr Trp Ala
   1               5                   10                  15
   Glu Ser Val Ala Ser Glu Phe Arg Ala Gln Arg Gly Ile Ala Phe Pro
               20                  25                  30
   Leu Ile Ala Met Asn Leu Thr Trp Phe Ala Lys Leu Ala Val Thr Thr
           35                  40                  45
   Ala Phe Leu Gly Arg Leu Gly Asp Leu Gln Leu Ala Ala Gly Thr Leu
       50                  55                  60
   Gly Phe Ser Phe Ala Asn Val Thr Gly Phe Ala Val Leu Thr Gly Leu
   65                  70                  75                  80
   Cys Ala Ala Met Asp Pro Ile Cys Gly Gln Ala His Gly Ala Ser Asn
                   85                  90                  95
   Gly Lys Leu Leu Arg Lys Thr Leu Val Met Ala Thr Ile Leu Leu Leu
               100                 105                 110
   Gly Ala Ser Ile Pro Ile Ala Phe Leu Trp Leu His Val Asp Ala Val
           115                 120                 125
   Leu Leu Arg Phe Gly Gln Gln Ala Asp Met Ser Ser Asn Ala Arg Ser
       130                 135                 140
   Tyr Val Val Cys Leu Leu Pro Asp Leu Ala Val Thr Ser Phe Val Asn
   145                 150                 155                 160
   Pro Leu Lys Ser Tyr Leu Ser Ala Gln Gly Val Thr Leu Pro Thr Leu
                   165                 170                 175
   Phe Ala Ser Ala Leu Ala Leu Ala Leu His Val Pro Leu Thr Met Trp
               180                 185                 190
   Met Ala Arg Thr Arg Gly Ile Gln Gly Val Ala Thr Ala Val Trp Val
           195                 200                 205
   Ser Asp Leu Ala Val Ala Val Met Leu Ala Gly Tyr Val Leu Val Ser
       210                 215                 220
   Glu Arg Arg Arg Lys Ala Gly Gly Gly Gly Gly Trp Val Glu Gln Thr
   225                 230                 235                 240
   Arg Gly Glu Trp Val Arg Leu Leu Arg Leu Ala Val Pro Ser Cys Leu
                   245                 250                 255
   Asn Thr Cys Leu Glu Trp Trp Cys Tyr Glu Ile Leu Val Leu Leu Thr
               260                 265                 270
   Gly Arg Leu Pro Asp Ala Arg Arg Thr Val Ala Val Met Ala Val Thr
           275                 280                 285
   Leu Asn Phe Asp Tyr Leu Leu Phe Ala Gly Met Leu Ser Leu Ser Val
       290                 295                 300
   Ser Ala Ser Val Arg Val Ser Asn Glu Leu Gly Ala Gly Glu Ala Trp
   305                 310                 315                 320
   Ala Ala Arg Arg Ala Gly Met Val Ser Ile Val Gly Gly Ala Val Gly
                   325                 330                 335
   Gly Val Gly Gly Gly Val Ala Met Val Ala Ala Arg Arg Ala Trp Gly
               340                 345                 350
   Ser Ile Tyr Ser Ser Asp Ala Gly Val Arg Glu Gly Val Gly Arg Ala
           355                 360                 365
   Met Glu Val Met Ala Val Leu Glu Val Val Asn Phe Pro Leu Asn Val
       370                 375                 380
   Cys Gly Gly Ile Val Arg Gly Thr Ala Arg Pro Ala Val Gly Met Tyr
   385                 390                 395                 400
   Ala Val Val Ala Gly Phe Tyr Val Leu Ala Leu Pro Leu Gly Val Ala
                   405                 410                 415
   Leu Ala Phe Lys Ala Arg Leu Gly Ile Gln Gly Leu Leu Leu Gly Phe
               420                 425                 430
   Leu Val Gly Ala Ala Ala Ser Leu Ala Val Leu Leu Thr Phe Ile Ala
           435                 440                 445
   Arg Met Asp Trp Pro Ala Glu Ala Gln Lys Ala Arg Thr Arg Thr Thr
       450                 455                 460
   Ala Thr Val Ala Gln Phe His Gln His Asp Glu Val Val Gln Pro
   465                 470                 475
<210> 9
   <211> 819
   <212> PRT
   <213> Oryza sativa
<400> 9
   Met Pro Glu Ala Ala Ala Ala Ala Ala Gly His Met Asp Pro Val Gly
   1               5                   10                  15
   Asp Glu Ala Ala Glu Arg Arg Glu Met Glu Glu Lys Glu Glu Glu Glu
               20                  25                  30
   Glu Glu Glu Glu Glu Asp Glu Glu Phe Tyr Glu Ser Leu Asp Arg Ile
           35                  40                  45
   Leu Ser Ser Ser Cys Ser Ser Thr Ser Ala Ser Asp Asp Asp Asp Gln
       50                  55                  60
   Gln His Arg Arg Arg Arg Arg His His Pro Gln Pro Gln Gln Leu Ser
   65                  70                  75                  80
   Ser Ser Ala Thr Phe Ser Ala Tyr Glu Val Trp Ile Ser Glu Pro Thr
                   85                  90                  95
   Ser Val Glu Glu Arg Arg Arg Val Leu Leu Arg Arg Leu Gly Leu Ala
               100                 105                 110
   His Asp Ser Glu Pro Leu Pro His Pro Ser Pro Arg Val Ser Ser Ser
           115                 120                 125
   Ser Pro Arg Ser Pro Thr Pro Ser Pro Pro Ser Ser Ser Pro Pro Arg
       130                 135                 140
   Pro Ala Pro Val Val Ala Ala Ala Glu Glu Pro Arg Ser Ser Gly His
   145                 150                 155                 160
   Gly Lys Pro Pro Leu Ala Arg Asn Pro Ser Gly Gly Ala Glu Gln Cys
                   165                 170                 175
   Arg Ile Arg Asn Leu Asp Asp Gly Thr Glu Phe Glu Val Gly Glu Val
               180                 185                 190
   His Asp Glu Val Val Arg Glu Val Gly Thr Gly Arg Gln Leu Thr Phe
           195                 200                 205
   Glu Glu Phe Glu Leu Cys Ile Gly Arg Ser Pro Ile Val Gln Glu Leu
       210                 215                 220
   Met Arg Arg Ala Thr Thr Ala Ala Ser Ser Ser Thr Ser Asp His Ala
   225                 230                 235                 240
   Ala Pro Ala Ser Lys Pro Arg Arg Lys Pro Gly Gly Trp Leu Arg Gly
                   245                 250                 255
   Ile Arg His Leu Ala Gly Ser Val Ala Tyr Gly Arg Ser Ser Thr Asp
               260                 265                 270
   Glu Arg Asp Lys Glu Lys Glu Lys Glu Lys Lys Glu Arg Glu Ala Arg
           275                 280                 285
   Arg Leu Ser Ser Ala Thr Asp Asp Ser Leu Asp Gly Asn Gly Ser Arg
       290                 295                 300
   Asn Ala Gly Arg Val Arg Val Arg Gln Tyr Gly Lys Ala Cys Lys Glu
   305                 310                 315                 320
   Leu Thr Gly Leu Phe Met Thr Gln Glu Leu Ala Ala His Ser Gly Ser
                   325                 330                 335
   Ile Trp Cys Ile Asn Phe Ser Leu Asp Gly Arg Tyr Leu Ala Ser Ala
               340                 345                 350
   Gly Glu Asp Arg Val Ile His Val Trp Glu Val Ser Glu Gly Glu Arg
           355                 360                 365
   Lys Gly Glu Leu Leu Gly Glu Gly Thr Val Ala Arg Glu Asn Gly Gly
       370                 375                 380
   Gly Cys Ser Pro Phe Leu Ala Ala Val Gly Asn Gly Ser Pro Glu Leu
   385                 390                 395                 400
   Ala Thr Leu Ser Leu Ser Cys Ala Asp Gly Gly Phe Val Glu Lys Lys
                   405                 410                 415
   Arg Arg Pro Arg Met Gln Ser Ser Arg Lys Ser Val Gly Ser Asp His
               420                 425                 430
   Leu Val Val Pro Glu Cys Val Phe Gly Phe Arg Asp Lys Pro Val Cys
           435                 440                 445
   Ser Leu Leu Gly His Ala Ala Asp Val Leu Asp Leu Ser Trp Ser Lys
       450                 455                 460
   Ser Gln Tyr Leu Leu Ser Ser Ser Met Asp Lys Thr Val Lys Leu Trp
   465                 470                 475                 480
   Asp Ile Thr Thr Ser Thr Cys Leu Lys Thr Phe Ser His Thr Asp Tyr
                   485                 490                 495
   Val Thr Cys Ile Gln Phe Asn Pro Val Asp Asp Asn Phe Phe Ile Ser
               500                 505                 510
   Gly Ser Leu Asp Glu Lys Val Arg Ile Trp Asn Val His Asp Arg Lys
           515                 520                 525
   Ile Glu Asp Trp Asn Asp Leu His Glu Met Val Thr Ala Ala Cys Tyr
       530                 535                 540
   Ser Pro Asp Gly Gln Val Ala Leu Val Gly Ser His Lys Gly Ser Cys
   545                 550                 555                 560
   His Leu Phe Asp Thr Thr Glu Lys Lys Leu Gln Tyr Lys Ser Gln Ile
                   565                 570                 575
   Glu Leu Arg Ile Arg Lys Lys Lys Ser Gly Gln Lys Lys Ile Thr Gly
               580                 585                 590
   Phe Gln Phe Ala Pro Gly Ser Ser Ser Glu Val Leu Ile Thr Ser Ala
           595                 600                 605
   Asp Ser Arg Ile Arg Val Val Asn Gly Asp Glu Leu Val His Lys Phe
       610                 615                 620
   Lys Gly Phe Arg Asn Thr Ser Ser Gln Ile Ser Ala Ser Val Ala Pro
   625                 630                 635                 640
   Asn Gly Lys Tyr Val Val Cys Ala Ser Glu Asp Ser His Val Tyr Val
                   645                 650                 655
   Trp Arg His Asp Asn Thr Ser His Pro Ser Arg Ser Arg Ser Ala Val
               660                 665                 670
   Asp Val Thr Asn Ser Tyr Glu His Phe His Cys His Asp Val Thr Val
           675                 680                 685
   Ala Ile Thr Trp Pro Gly Ala Glu Ser Arg Gly Ser Phe Gly Ser Arg
       690                 695                 700
   Ser Ser Arg Asn Ser Asp Ser Asp Asp Ala Val Met Asn Thr Gly Arg
   705                 710                 715                 720
   Asp Ala Pro Val Glu Asn Ser Glu His Asp Leu Asn Gly Thr Val Asn
                   725                 730                 735
   Arg Cys Thr Lys Arg Pro Val Cys Glu Gly Val Ala Ser Thr Ser Asn
               740                 745                 750
   Pro Pro Ala Asp Gly Val Ser Thr Ser Trp Pro Asp Glu Lys Gln Ser
           755                 760                 765
   Ser Ala Lys Ser Ser Pro Gly His Cys Ser Ser Asp Leu Cys Ile Gly
       770                 775                 780
   Ala Leu Asp Val Gln Arg Arg Ser Ala Trp Gly Leu Val Ile Val Thr
   785                 790                 795                 800
   Ala Gly Arg Gly Gly Glu Ile Arg Val Phe Gln Asn Phe Gly Phe Pro
                   805                 810                 815
   Val Gln Val
<210> 10
   <211> 206
   <212> PRT
   <213> Oryza sativa
<400> 10
   Met Ala Pro Ala Val Ala Ser Ser Pro Ser Leu Val Leu Ser Ala Ala
   1               5                   10                  15
   Ala Ala Thr Ala Ser Asn Lys Arg Pro Ala Asp Ser Asp Ala Ser Pro
               20                  25                  30
   Pro His Gln Gly Asp Arg Thr Gly Gln Gln Glu Lys Lys Gln Gln Gln
           35                  40                  45
   Leu Glu Cys Pro Arg Cys Arg Ser Thr Asn Thr Lys Phe Cys Tyr Tyr
       50                  55                  60
   Asn Asn Tyr Ser Thr Ser Gln Pro Arg His Phe Cys Arg Ala Cys Arg
   65                  70                  75                  80
   Arg Tyr Trp Thr His Gly Gly Thr Leu Arg Asp Val Pro Val Gly Gly
                   85                  90                  95
   Ala Ser Arg Arg Gly Gly Gly Gly Lys Arg Arg Arg Val Ser Ala Asp
               100                 105                 110
   Ala Asp Pro Ser Ser Ala Ser Pro Pro Pro Pro Thr Thr Ser Thr Thr
           115                 120                 125
   Asp Ala Tyr Ala Asp Leu Pro Ala Gly Phe Pro Phe Leu Ser Asp Gly
       130                 135                 140
   Ala Phe Leu Pro Gln Phe Gly Leu Ala Gly Val Ala Pro Ala Ala Phe
   145                 150                 155                 160
   Ser Trp Ala Ser Ala Val Pro Asp Leu Tyr Asn Cys Gly Ile Ala Pro
                   165                 170                 175
   Trp Asp Asp Gly Thr Ala Val Thr Gly Ala Ala Trp Asp Asn Phe Ala
               180                 185                 190
   Asp Ile Ala Gly Leu Asp Leu Ser Trp Pro Pro Pro Gly Asn
           195                 200                 205
<210> 11
   <211> 583
   <212> PRT
   <213> Oryza sativa
<400> 11
   Met Ala Leu Leu Phe Arg Ile Ser Leu Leu Leu Leu Leu Val Pro Leu
   1               5                   10                  15
   Ile Pro Thr Ala Ala Ala Ser His His His Ser Pro Ala Gly Gly Gly
               20                  25                  30
   Gly Ala Ala Val Pro Leu His Pro Arg Arg His His Arg Ser Val Ala
           35                  40                  45
   Asn Thr Ala Thr Ala Leu Phe Tyr Pro Ala Pro Ser Met His Gln Asn
       50                  55                  60
   His Ile Glu Ala Glu Glu Gly Gln Leu Leu His Val Leu Ala Asp Pro
   65                  70                  75                  80
   Phe Ala Ala Ala Pro Ala Ala Ala Glu Ala Pro Ser Gly Glu Thr Ala
                   85                  90                  95
   Ile Ala Ala Val Gly Ala Ala Ala Glu Glu Ala Thr Pro Thr Leu Ile
               100                 105                 110
   Asp Asp Ser Pro Gln Gln Ala Ala Ala Ala Ser Pro Pro Pro Pro Pro
           115                 120                 125
   Pro Pro Pro Pro Pro Pro Pro Pro Leu Phe Ala Lys Pro Asp Leu Asp
       130                 135                 140
   Ser Thr Ala Pro Pro Gln Pro Lys Glu Glu Gly Val Asp Gly Tyr Gly
   145                 150                 155                 160
   Ser Thr Thr Ala Thr Ala Thr Val Thr Ala Ala Pro Pro Leu Asp Glu
                   165                 170                 175
   Pro Ala Ala Ala Thr Ala Thr Thr Thr Thr Thr Thr Thr Thr Thr Leu
               180                 185                 190
   Pro Leu Pro Arg Tyr Ser His Val Ala Ser Pro Pro Pro Pro Pro Val
           195                 200                 205
   His Ala Gly Val Ala Gly Leu Gly Asp Glu Gln Arg Leu Glu Gln Leu
       210                 215                 220
   Ala Arg Val Leu Ser Ser Leu Gly Tyr Asn Glu Met Ala Ser Ala Ala
   225                 230                 235                 240
   Leu Leu Leu Ala Asn Ser Ala Leu Leu Ala Ala Trp Pro Gly Ser Ile
                   245                 250                 255
   Thr Val Phe Ala Ala Pro Asp Val Phe Leu Arg Ala Ser Cys Pro Met
               260                 265                 270
   Cys Ser Arg Arg His Val Leu Leu Glu His Ile Ala Leu Gly Tyr Phe
           275                 280                 285
   Pro Tyr Thr Glu Leu Ala Ala Ala Ser Thr Ala Lys Leu Pro Ser Ala
       290                 295                 300
   Ser Pro Gly Leu Cys Leu Asn Leu Ala Ser Asp His Gly Pro Phe Ala
   305                 310                 315                 320
   Ile His His Val Arg Leu Tyr Val Asp Gly Val Glu Val Ser His Pro
                   325                 330                 335
   Glu Leu Tyr Asn Asp Gly Arg Tyr Val Val His Gly Leu His Gly Phe
               340                 345                 350
   Leu Pro Pro Leu Ser His Gly Ser Cys Ser His Gly Ser Asn His Arg
           355                 360                 365
   His His Tyr His Tyr Gln Tyr His His His His His His Ile Ile Ala
       370                 375                 380
   Ser Ser Ala Ala Ser Ser Ala Ala Thr Ala Ala Ser Val Val Arg Ile
   385                 390                 395                 400
   Met Ile Arg Glu Ala Ile Ala Arg Leu Arg Asp Ser Gly Tyr Gly Phe
                   405                 410                 415
   Val Ala Leu Ala Met Arg Val Lys Phe Ala Glu Leu Glu Arg Leu Ala
               420                 425                 430
   Asn Met Thr Val Phe Ala Leu Asp Asp Gln Ala Ile Phe Val Gly Gly
           435                 440                 445
   Gly His Asp Tyr Val Ser Ala Val Arg Phe His Val Val Pro Gly His
   450 455 460
   Arg Leu Thr His Ala Asp Leu Gln Arg Leu His Pro Gly Thr Met Leu
   465 470 475 480
   Pro Thr Leu Ala Gly Glu Gly Gln Asn Leu Val Val Thr Gln Gly Ala
   485 490 495
   Ser Gly Ser Gly Ser Gly Pro Arg Asp Val Arg Ile Asn Tyr Ile Pro
   500 505 510
   Ile Lys Asp Pro Asp Val Val Ile Asn Ser Arg Ile Ala Leu His Gly
   515 520 525
   Val Tyr Val Thr Phe Pro Arg Leu His Leu Ala Asn Leu Ala Ala Ala
   530 535 540
   Val Ala Leu Ala Ser Ser Asn Gln Ile Asn Ala Thr Cys Gly Val Phe
   545 550 555 560
   Gly Asp Cys Ala Ser Ala Ala Ala Thr Ser Thr Thr Val Pro Ala Ala
   565 570 575
   His Arg Tyr Gly Glu Gly Gln
   580
<210> 12
   <211> 300
   <212> PRT
   <213> Oryza sativa
<400> 12
   Met Asp Ala Asp Glu Ala Ala Gly Ser Ser Arg Arg Met Asp Leu Asn
   1               5                   10                  15
   Leu Tyr Leu Gly Leu Pro Arg Ala Pro Arg Pro Arg Arg Ser Asp Leu
               20                  25                  30
   Gly Ser Asp Leu Ala Leu Ser Thr Pro Met Pro Ser Ser Pro Ser Ser
           35                  40                  45
   Ser Ala Ala Ser Val Asp Ala Pro Pro Pro Pro Pro Glu Leu Ser His
       50                  55                  60
   Pro Pro Tyr Ser Pro Ser His Ala Asp Leu Ser Pro Pro Leu Gln Glu
   65                  70                  75                  80
   Val Tyr Ser Leu Tyr Asn Pro Asp Asp Pro Pro Ala Ser Glu Thr His
                   85                  90                  95
   Leu Pro Pro Tyr Ala Pro Pro Pro Ala Pro Val Val Ser Glu Leu Pro
               100                 105                 110
   Asp Asp Leu Glu Phe Gly Leu His Pro Pro Pro Pro Leu Pro Val Thr
           115                 120                 125
   Gly Gly Phe Ser Ser Arg Leu Glu Ala Asp Gly Ser Val Arg Gly Gly
       130                 135                 140
   Leu Gly Arg Thr Phe Arg His Ser Val Leu Lys Leu Thr Gln Leu Gly
   145                 150                 155                 160
   Val Met Leu Pro Gln Ser Gln Asn Gly Pro Ser Ser Leu Leu Pro Asp
                   165                 170                 175
   Gly Ile Asp Trp Leu Arg Gly Leu Thr Leu Leu Gly Tyr Glu Asp Thr
               180                 185                 190
   Glu Arg Phe Ala Ser Ala Met Ser Asp Phe Arg Arg Ile Thr Gly Pro
           195                 200                 205
   Ser Gln Tyr Gly Ala Ser Ala Ser Ser Ser Asn Pro Pro Asn Leu Glu
       210                 215                 220
   Ser Thr Phe Asp Arg Thr His Val Val Ala Ala Pro Ser Ala Asp Gln
   225                 230                 235                 240
   Ala Ser Asn Ser Ser Thr Ala Ala Val Ile Gln Gly Asp Ala Gly Ile
                   245                 250                 255
   Ser Glu Ser Ala Gly Glu Pro Ser Asn Ala Gly Ser Ser Arg Ser Leu
               260                 265                 270
   Arg Arg Arg Gly Arg Ser Ser Ala Leu Gly Ser Leu Asp Ala Asp Gly
           275                 280                 285
   Gly Gly Leu Gln Arg Asn Lys Arg Arg Arg Ile Asn
       290                 295                 300
<210> 13
   <211> 480
   <212> PRT
   <213> Oryza sativa
<400> 13
   Met Val Gly Gly Glu Leu Val Leu Ala Ala Leu Val Ile Leu Leu Ala
   1               5                   10                  15
   Leu Leu Leu Thr Leu Val Leu Ser His Phe Leu Pro Leu Leu Leu Asn
               20                  25                  30
   Pro Lys Ala Pro Lys Gly Ser Phe Gly Trp Pro Leu Leu Gly Glu Thr
           35                  40                  45
   Leu Arg Phe Leu Ser Pro His Ala Ser Asn Thr Leu Gly Ser Phe Leu
       50                  55                  60
   Glu Asp His Cys Ser Arg Tyr Gly Arg Val Phe Lys Ser His Leu Phe
   65                  70                  75                  80
   Cys Thr Pro Thr Ile Val Ser Cys Asp Gln Glu Leu Asn His Phe Ile
                   85                  90                  95
   Leu Gln Asn Glu Glu Arg Leu Phe Gln Cys Ser Tyr Pro Arg Pro Ile
               100                 105                 110
   His Gly Ile Leu Gly Lys Ser Ser Met Leu Val Val Leu Gly Glu Asp
           115                 120                 125
   His Lys Arg Leu Arg Asn Leu Ala Leu Ala Leu Val Thr Ser Thr Lys
       130                 135                 140
   Leu Lys Pro Ser Tyr Leu Gly Asp Ile Glu Lys Ile Ala Leu His Ile
   145                 150                 155                 160
   Val Gly Ser Trp His Gly Lys Ser Lys Asp Lys Gly Met Val Asn Val
                   165                 170                 175
   Ile Ala Phe Cys Glu Glu Ala Arg Lys Phe Ala Phe Ser Val Ile Val
               180                 185                 190
   Lys Gln Val Leu Gly Leu Ser Pro Glu Glu Pro Val Thr Ala Met Ile
           195                 200                 205
   Leu Glu Asp Phe Leu Ala Phe Met Lys Gly Leu Ile Ser Phe Pro Leu
       210                 215                 220
   Tyr Ile Pro Gly Thr Pro Tyr Ala Lys Ala Val Gln Ala Arg Ala Arg
   225                 230                 235                 240
   Ile Ser Ser Thr Val Lys Gly Ile Ile Glu Glu Arg Arg Asn Ala Gly
                   245                 250                 255
   Ser Ser Asn Lys Gly Asp Phe Leu Asp Val Leu Leu Ser Ser Asn Glu
               260                 265                 270
   Leu Ser Asp Glu Glu Lys Val Ser Phe Val Leu Asp Ser Leu Leu Gly
           275                 280                 285
   Gly Tyr Glu Thr Thr Ser Leu Leu Ile Ser Met Val Val Tyr Phe Leu
       290                 295                 300
   Gly Gln Ser Ala Gln Asp Leu Glu Leu Val Lys Arg Glu His Glu Gly
   305                 310                 315                 320
   Ile Arg Ser Lys Lys Glu Lys Asp Glu Phe Leu Ser Ser Glu Asp Tyr
                   325                 330                 335
   Lys Lys Met Glu Tyr Thr Gln His Val Ile Asn Glu Ala Leu Arg Cys
               340                 345                 350
   Gly Asn Ile Val Lys Phe Val His Arg Lys Ala Leu Lys Asp Val Arg
           355                 360                 365
   Tyr Lys Glu Tyr Leu Ile Pro Ser Gly Trp Lys Val Leu Pro Val Phe
       370                 375                 380
   Ser Ala Val His Leu Asn Pro Leu Leu His Gly Asn Ala Gln Gln Phe
   385                 390                 395                 400
   Gln Pro Cys Arg Trp Glu Gly Ala Ser Gln Gly Thr Ser Lys Lys Phe
                   405                 410                 415
   Thr Pro Phe Gly Gly Gly Pro Arg Leu Cys Pro Gly Ser Glu Leu Ala
               420                 425                 430
   Lys Val Glu Ala Ala Phe Phe Leu His His Leu Val Leu Asn Tyr Arg
           435                 440                 445
   Trp Arg Ile Asp Gly Asp Asp Ile Pro Met Ala Tyr Pro Tyr Val Glu
       450                 455                 460
   Phe Gln Arg Gly Leu Pro Ile Glu Ile Glu Pro Leu Cys Ser Glu Ser
   465                 470                 475                 480
<210> 14
   <211> 810
   <212> PRT
   <213> Oryza sativa
<400> 14
   Met Ala Thr Leu Pro Asp Leu Gly Val Ser Ala Phe Ile Asn Ile Leu
   1               5                   10                  15
   Gly Ala Phe Val Phe Leu Leu Ile Phe Ala Ala Leu Arg Leu Gln Pro
               20                  25                  30
   Ile Asn Asp Arg Val Tyr Phe Pro Lys Leu Tyr Leu Thr Gly Gln Arg
             35                  40                  45
   Arg His His Pro His Pro His Gly Phe Val Asn Leu Asp Leu Cys Ser
       50                  55                  60
   Tyr Leu Arg Phe Leu Ala Trp Val Pro Gly Ala Leu Arg Met Ser Gln
   65                  70                  75                  80
   Pro Asp Leu Ile His His Ala Gly Leu Asp Ser Ala Val Tyr Leu Arg
                   85                  90                  95
   Ile Tyr Thr Leu Gly Leu Lys Ile Phe Leu Pro Ile Met Thr Val Ala
               100                 105                 110
   Leu Leu Val Leu Ile Pro Val Asn Val Ser Gly Gly Thr Leu Leu Asn
           115                 120                 125
   Leu Arg Lys Glu Ile Val Phe Ser Asp Ile Asp Lys Leu Ser Ile Ser
       130                 135                 140
   Asn Val Asn Pro Gly Ser Asn Arg Phe Phe Ile His Leu Leu Met Ala
   145                 150                 155                 160
   Tyr Val Phe Thr Phe Trp Thr Cys Phe Met Leu Tyr Lys Glu Tyr Ser
                   165                 170                 175
   Asn Val Ala Phe Met Arg Leu His Phe Leu Ala Ser Gln Lys Arg Cys
               180                 185                 190
   Ala Asp Gln Phe Thr Val Ile Val Arg Asn Ile Pro His Val Ser Ser
           195                 200                 205
   His Ser Thr Ser Glu Thr Val Asp Glu Phe Phe Arg Arg Asn His Pro
       210                 215                 220
   Asp His Tyr Leu Gly Gln Gln Ala Val Tyr Asn Ala Asn Arg Tyr Ala
   225                 230                 235                 240
   Lys Leu Val Lys Lys Lys Glu Arg Leu Gln Asn Trp Leu Asp Tyr Tyr
                   245                 250                 255
   Gln Leu Lys Phe Glu Arg His Pro Gly Lys Arg Pro Ile Gly Arg Thr
               260                 265                 270
   Gly Cys Leu Gly Phe Cys Gly Arg Glu Val Asp Gln Ile Asp Tyr Tyr
           275                 280                 285
   Arg Ala Arg Ile Ser Glu Leu Asp Lys Lys Leu Ala Ser Glu Arg Gln
       290                 295                 300
   Arg Val Leu Asn Asp Pro Lys Ala Val Met Pro Val Ala Phe Val Thr
   305                 310                 315                 320
   Phe Asp Ser Arg Trp Gly Ala Ala Val Cys Ala Gln Thr Gln Gln Ser
                   325                 330                 335
   Lys Asn Pro Thr Gln Trp Leu Thr Asp Trp Ala Pro Glu Pro Arg Asp
               340                 345                 350
   Val Tyr Trp Gln Asn Leu Ala Ile Pro Phe Phe Ser Leu Ser Ile Arg
           355                 360                 365
   Lys Phe Leu Ile Ser Ile Ala Val Phe Ala Leu Val Phe Phe Tyr Met
       370                 375                 380
   Ile Pro Ile Ala Phe Val Gln Ser Leu Ala Asn Leu Glu Gly Ile Glu
   385                 390                 395                 400
   Lys Val Ala Pro Phe Leu Arg Pro Val Ile Asp Thr Pro Val Val Lys
                   405                 410                 415
   Ser Phe Leu Gln Gly Phe Leu Pro Gly Leu Ala Leu Lys Ile Phe Leu
               420                 425                 430
   Tyr Ile Leu Pro Thr Val Leu Met Ile Met Ser Lys Val Glu Gly Tyr
           435                 440                 445
   Val Ser Leu Ser Ser Leu Glu Arg Arg Ala Ala Ser Lys Tyr Tyr Tyr
       450                 455                 460
   Phe Met Leu Val Asn Val Phe Leu Gly Ser Ile Ile Ala Gly Thr Ala
   465                 470                 475                 480
   Phe Glu Gln Leu Asn Ala Phe Phe His Gln Pro Pro Ser Gln Ile Pro
                   485                 490                 495
   Arg Thr Ile Gly Val Ala Ile Pro Met Lys Ala Thr Phe Phe Met Thr
               500                 505                 510
   Tyr Ile Met Val Asp Gly Trp Ala Gly Ile Ala Asn Glu Ile Leu Arg
           515                 520                 525
   Val Lys Pro Leu Val Ile Tyr His Leu Lys Asn Met Phe Ile Val Lys
       530                 535                 540
   Thr Glu Arg Asp Arg Glu Arg Ala Met Asp Pro Gly Ser Ile Gly Leu
   545                 550                 555                 560
   Ala Glu Asn Leu Pro Ser Leu Gln Leu Tyr Phe Leu Leu Gly Leu Val
                   565                 570                 575
   Tyr Ala Val Val Thr Pro Ile Leu Leu Pro Phe Ile Ile Ile Phe Phe
               580                 585                 590
   Ala Phe Ala Phe Leu Val Tyr Arg His Gln Ile Ile Asn Val Tyr Asn
           595                 600                 605
   Gln Glu Tyr Glu Ser Ala Ala Ala Phe Trp Pro Gln Val His Ser Arg
       610                 615                 620
   Ile Ile Ala Ser Leu Leu Ile Ser His Val Thr Leu Phe Gly Leu Met
   625                 630                 635                 640
   Ser Thr Met Lys Ala Ala Tyr Ser Thr Pro Leu Leu Ile Phe Leu Pro
                   645                 650                 655
   Leu Leu Thr Ile Trp Phe His Lys Tyr Cys Lys Ser Arg Phe Glu Pro
               660                 665                 670
   Ala Phe Arg Lys Tyr Pro Leu Glu Glu Ala Met Glu Lys Asp Asn Leu
           675                 680                 685
   Glu Arg Thr Ser Glu Pro Asn Leu Asn Leu Lys Ser Tyr Leu Gln Asn
       690                 695                 700
   Ala Tyr Leu His Pro Ile Phe His Met Phe Glu Gln Gln Gln Gln Gln
   705                 710                 715                 720
   Glu Gln Glu Gln Gln Arg Glu Glu Lys Val Glu Val Arg Ile Asp Lys
                   725                 730                 735
   Ala Gln Gln His His His Arg Gln Val Glu Glu Glu Glu Glu Glu Ser
               740                 745                 750
   Lys Ser Ser Gln Ala Thr Thr His Tyr Tyr His His His His Glu Gln
           755                 760                 765
   Thr Thr Thr Thr Thr His His His Tyr His Gln His Glu His Met Ser
       770                 775                 780
   His Tyr His Met Gly Pro Ser Asp Thr Ala Asp Ser Pro Ser Pro Pro
   785                 790                 795                 800
   His Phe Val Tyr His Tyr Gly Val Asp Pro
                   805                 810
<210> 15
   <211> 293
   <212> PRT
   <213> Oryza sativa
<400> 15
   Met Glu Met Thr Arg Ser Leu Thr Leu Val Pro Leu Pro Ala Thr Leu
   1               5                   10                  15
   Arg Pro Ala Ser Ala Ala Cys Arg Arg Arg Arg Arg Arg Arg Gly Leu
               20                  25                  30
   Pro Phe Gly Ala Leu Phe Ser Pro Ser Pro Pro Ser Asn Gln Gln Gln
           35                  40                  45
   Gln Glu Met His Ile Arg Ala Leu Gln Pro Arg Gln Asp Trp Val Gly
       50                  55                  60
   Glu Trp Val Arg Ser Asn Asp Thr Leu Val Arg Gly Leu Pro Ile Leu
   65                  70                  75                  80
   Gly Gly Gly Ala Ser Leu Leu Ala Val Leu Leu Asn Arg Ala Val Ser
                   85                  90                  95
   Gly Ile Ala Ala Val Ala Asp Ala Ser Ser Ser Gln Ser Arg Ala Asp
               100                 105                 110
   Ile Leu Thr Leu Ala Leu Ser Val Thr Asp Ile Leu Ala Gly Leu Val
           115                 120                 125
   Trp Leu Ser Ile Arg Pro Lys Ser Ile Ser Pro Val Val Pro Arg Gly
       130                 135                 140
   Val Glu Cys Lys Arg Val Gly Thr Gly Val Leu Asp Ser Ala Leu Arg
   145                 150                 155                 160
   Glu Leu Leu Trp Thr Trp Asp Ser Leu Thr Thr Ala Thr Cys Cys Lys
                   165                 170                 175
   Ser Leu Val Val Val Tyr Gly Gly Asn Cys Val Leu Gln Ile Gly Val
               180                 185                 190
   Ala Ala Gly Ser Pro Glu Asp Gly Asn Ala Val Met Val Asp Ala Gln
           195                 200                 205
   Lys Phe Met Gln Gly Ser Leu Tyr Arg Ser Ala Met Glu Ser Lys Lys
       210                 215                 220
   Gln Ser Tyr Leu Ala Asn Leu Ala Leu Tyr Pro Gly Arg Thr Glu Leu
   225 230 235 240
   Pro Phe Leu Pro Ala Asn Thr Gln Ala Leu Ile Leu Gln Pro Ile Gly
   245 250 255
   Asp Lys Gly Ile Ala Val Ile Gly Gly Asp Thr Ile Arg Gly Phe Thr
   260 265 270
   Asn Leu Asp Gln Ala Trp Ile Ala Met Ile Ala Asp Lys Leu Asp Ala
   275 280 285
   Thr Leu Ser Lys Ser
   290
<210> 16
   <211> 155
   <212> PRT
   <213> Oryza sativa
<400> 16
   Met Ala Ser Ser Val Ala Gly Ser Val Thr Arg Arg Pro Pro Pro Val
   1               5                   10                  15
   Leu Leu Ala Cys Arg Ser Arg Pro Asn Asn Arg Arg Leu Ile Arg Leu
               20                  25                  30
   Leu Pro Leu Leu Phe Ala Val Val Val Leu Leu Ala Leu Leu Pro Pro
           35                  40                  45
   Cys Val His Gly Ala Arg Ala Leu Asn Asp Ala Lys Glu Ala Lys Val
       50                  55                  60
   Ala Glu Ala Ser Asp Gln Thr Thr Thr Thr Thr His Ala Ala Ala Ala
   65                  70                  75                  80
   Ala Val Ala Arg Trp Ser Val Thr Val Arg Glu Gly Gly Gly Gly Gly
                   85                  90                  95
   Gly His Gly Ser Gly His Ala Gly Ala Gly His Gly His Gly Ser Gly
               100                 105                 110
   His Gly Arg Pro Glu Pro Ala Glu His His Thr Gly Arg Arg Ser Ala
           115                 120                 125
   Ala Ala Gly Ser Val Arg Pro Pro Met Ala Ala Ser Cys Ala Ala Leu
       130                 135                 140
   Leu Val Ala Ala Val Val Ala Leu Leu Arg Phe
   145                 150                 155
<210> 17
   <211> 124
   <212> PRT
   <213> Oryza sativa
<400> 17
   Met Glu Ser Ala Lys Arg Ser Cys Leu Ala Ile Ser Leu Ile Leu Leu
   1               5                   10                  15
   Leu Leu Leu Val Pro Ser Ile His Gly Ala Arg His Val Ala Ala Ala
               20                  25                  30
   Ile Lys Gly Thr Gly Ala Asp Ser Glu Met Val Val Thr Glu Arg Thr
           35                  40                  45
   Ala Gly Gly Gly Gly Gly His Gly Arg Gly Tyr Thr Ser His Arg Ser
       50                  55                  60
   His Asn Pro Asn Asn Pro Asn Asp Gly Gly Ser Gly Thr Pro Val Val
   65                  70                  75                  80
   Asp Pro His Asn Val Ala Thr Arg Gly His His His Arg Gly Ala Ala
                   85                  90                  95
   Thr Arg Thr Ala Ala Gly Gly Asp Pro Arg Leu Ala Ala Cys Met Leu
               100                 105                 110
   Arg Leu Gly Ala Thr Phe Phe Leu Leu Val Leu Gly
           115                 120
<210> 18
   <211> 215
   <212> PRT
   <213> Oryza sativa
<400> 18
   Met Ala Gly Val Gly Phe Val Glu Asp Met Leu Arg Glu Gln Ser Leu
   1               5                   10                  15
   Leu Glu Ala Thr Cys Gly Asp Leu Phe Asp His Ile Asp Asp Leu Leu
               20                  25                  30
   Asp Phe Pro Lys Glu Glu Ser Ala Ala Asp Val Leu Leu Leu Asp Ala
           35                  40                  45
   Pro Ala Pro Gly Ser Pro Leu Ser Ser Arg Ile Ile Gly Gly His Ala
       50                  55                  60
   Thr Met Ala Ala Ala Pro Pro Pro Pro Pro Gln Met Met Ala Leu Pro
   65                  70                  75                  80
   Pro Pro Pro Ala Pro Ala Lys Asp Asp Ala Ser Ala Leu Phe Asp Ala
                   85                  90                  95
   Ala Gly Ala Leu Gly Ala Glu Val Phe Asp Arg Lys Asp Ala His Ile
               100                 105                 110
   Gly Pro Cys Asp Glu Leu Asp Met Asp Met Ala Gln Leu Glu Trp Leu
           115                 120                 125
   Ser Gly Leu Phe Asp Asp Gly Thr Ile Pro His Glu Pro Ser Phe Pro
       130                 135                 140
   Gly Val Asn Cys Ala Ala Pro Ile Lys Ala Ser Ala Leu Thr Ala Asn
   145                 150                 155                 160
   Ala Gly Val Val Leu Pro Asp Lys Ala Glu Glu Ala Leu Phe Arg Ser
                   165                 170                 175
   Ser Ser Pro Ile Ser Val Leu Glu His Ser Gly Phe Asn Val Ala Thr
               180                 185                 190
   Asn Gly Gly Ser Ser Ser Ser Ser Ser Ser Ser Ser Pro Ser Pro Trp
           195                 200                 205
   Thr Trp Thr Trp Thr Ala Val
       210                 215
<210> 19
   <211> 376
   <212> PRT
   <213> Oryza sativa
<400> 19
   Met Ile Ile Val Asp Asp Ala Gly Ala Phe Leu Pro Ala Leu Asn His
   1               5                   10                  15
   Ser Pro Trp Asp Gly Val Thr Ile Ala Asp Phe Val Met Pro Phe Phe
               20                  25                  30
   Leu Phe Met Val Gly Ile Ser Leu Thr Leu Ala Tyr Lys Arg Val Pro
           35                  40                  45
   Asp Lys Leu Glu Ala Thr Lys Lys Ala Val Leu Arg Ala Leu Lys Leu
       50                  55                  60
   Phe Cys Leu Gly Leu Val Leu Gln Gly Gly Phe Phe His Gly Val Arg
   65                  70                  75                  80
   Ser Leu Thr Phe Gly Val Asp Ile Thr Lys Ile Arg Leu Met Gly Ile
                   85                  90                  95
   Leu Gln Arg Ile Ala Ile Ala Tyr Leu Leu Ala Ala Ile Cys Glu Ile
               100                 105                 110
   Trp Leu Lys Gly Asp Asp Asp Val Asp Cys Gly Leu Asp Val Ile Arg
           115                 120                 125
   Arg Tyr Arg Tyr Gln Leu Val Val Ala Leu Leu Leu Ser Thr Met Tyr
       130                 135                 140
   Thr Val Ile Leu Asn Gly Val Tyr Val Pro Asp Trp Glu Tyr Gln Ile
   145                 150                 155                 160
   Ser Gly Pro Gly Ser Thr Glu Lys Ser Phe Ser Val Arg Cys Gly Val
                   165                 170                 175
   Arg Gly Asp Thr Gly Pro Ala Cys Asn Ala Val Gly Met Leu Asp Arg
               180                 185                 190
   Thr Ile Leu Gly Ile Asp His Leu Tyr Arg Arg Pro Val Tyr Ala Arg
           195                 200                 205
   Thr Lys Gln Cys Ser Ile Asn Tyr Pro Gln Asn Gly Pro Leu Pro Pro
       210                 215                 220
   Asp Ala Pro Ser Trp Cys Gln Ala Pro Phe Asp Pro Glu Gly Leu Leu
   225                 230                 235                 240
   Ser Ser Val Met Ala Ile Val Thr Cys Leu Ile Gly Leu Gln Phe Gly
                   245                 250                 255
   His Ile Ile Ile His Phe Glu Lys His Lys Gly Arg Ile Ile Asn Trp
               260                 265                 270
   Leu Ile Pro Ser Phe Ser Met Leu Ala Leu Ala Phe Ser Met Asp Phe
           275                 280                 285
   Ile Gly Ile Arg Met Asn Lys Pro Leu Tyr Thr Ile Ser Tyr Ala Leu
       290                 295                 300
   Ala Thr Ser Gly Ala Ala Gly Leu Leu Phe Ala Gly Ile Tyr Thr Leu
   305                 310                 315                 320
   Val Asp Val Tyr Gly Phe Arg Lys Leu Thr Ile Pro Met Glu Trp Met
                   325                 330                 335
   Gly Lys His Ala Leu Met Ile Tyr Val Leu Val Ala Cys Asn Ile Leu
   340 345 350
   Pro Ile Phe Ile His Gly Phe Tyr Trp Arg Glu Pro Lys Asn Asn Leu
   355 360 365
   Leu Lys Phe Ile Gly Val Gly Ala
   370 375
<210> 20
   <211> 488
   <212> PRT
   <213> Oryza sativa
<400> 20
   Met Ala Ala Thr Gly Gly Ala Ala Gly Glu Lys Thr Ala Ser Ser Leu
   1               5                   10                  15
   Leu Leu Gly Val Arg Gly Tyr Thr Ser Thr Leu Lys Asn Ala Ser Thr
               20                  25                  30
   Ala Ser Cys Arg Leu Ser Ala Gly His Pro Ile Glu Val Thr Leu Trp
           35                  40                  45
   Glu Ala Ser Pro Pro Ala Leu Ser His Phe Ser Val His Cys Pro Asp
       50                  55                  60
   Leu Pro Ser Phe Asn Gly Asn Leu Leu Gly Ala Pro Lys Ala Ile Ala
   65                  70                  75                  80
   Ala Ala Val Asp Asp Ala Asp Gly Gln Leu Leu Leu Leu Leu Arg Val
                   85                  90                  95
   Pro Ile Asp Gln Leu Gly Ala Pro His Asp Asn Asp Tyr Leu Val Tyr
               100                 105                 110
   His Pro Asp Pro Pro Ser Pro Lys Leu Asp Leu Leu Pro Asn Pro Pro
           115                 120                 125
   Pro Pro Thr Leu Gly Asp His Gln Leu Ala Ile Leu Ser Cys Gly Asp
       130                 135                 140
   Asp Arg Tyr Val Val Ala Ala Leu His Val Trp Ser Glu Phe Thr Ser
   145                 150                 155                 160
   Thr Leu Arg Leu Tyr Arg Ser Ser Cys Ser Ser Gly Ser Trp Thr Ser
                   165                 170                 175
   Glu Glu Val Ser Val Glu Glu Pro Val Arg Asp Arg Leu Cys Pro Ile
               180                 185                 190
   Pro Asp Ser Ala Lys Arg Gln Leu Tyr His Val Thr Thr Lys Thr Ile
           195                 200                 205
   Thr Leu Gly Gly Ala Lys Gly Thr Val Gly Trp Val Asp Leu Trp Arg
       210                 215                 220
   Gly Ile Leu Leu Cys Asp Val Leu Asp Glu Met Ser Pro Arg Lys Leu
   225                 230                 235                 240
   Arg Asp Met Pro Leu Pro Trp Pro Ala Lys Gly Asn Trp Arg Met Tyr
                   245                 250                 255
   Leu Asn Gly Asp Val Ser Phe Cys Arg Asp Ile Ala Ile Ser Gln His
               260                 265                 270
   Lys Asp Ser Ile Lys Tyr Leu Glu Met Glu Ile Val Ser Pro Arg Thr
           275                 280                 285
   Val Thr Thr Thr Ile Pro Thr Ser Thr Ser Ala Asp Pro Thr Ser Tyr
       290                 295                 300
   Leu Glu Trp Val Arg Arg Ser Arg Glu Pro Gln Pro Thr Arg Arg Arg
   305                 310                 315                 320
   Ser Val Phe His Pro Gly Ser Trp Arg Ile Thr Thr Trp Ser Met Pro
                   325                 330                 335
   Ile Pro Val Thr Ser Trp Asp Asp Trp Arg Arg Asp Cys Thr Ala Glu
               340                 345                 350
   Ser Arg Glu Val His Leu Asp Thr Asn Pro Ser His His Tyr Glu Leu
           355                 360                 365
   Leu His Ser Leu Met Leu Ser Asn Ser Gly Asp Glu His Arg Glu Glu
       370                 375                 380
   Ala Gln Gly Gln Gly Ala Thr Ser Ser Leu Ser Leu Gly Arg Leu Arg
   385                 390                 395                 400
   Leu Cys Tyr Pro Ala Leu Ser Cys Ile Asp Asp Asp Val Val Tyr Leu
                   405                 410                 415
   Leu Gly Asn Ala Ala Gly Arg Gly Ala Lys Thr Gly Gly Met Met Val
               420                 425                 430
   Ala Val Asp Val Arg Asn Lys Glu Leu Arg Gly Val Ala Lys Leu Asp
           435                 440                 445
   Pro Glu Lys Asn Thr Leu Tyr Ser Met Arg Cys Tyr Leu Ala Thr Gly
   450 455 460
   Ile Ser Lys Arg Leu Asn Thr Thr Thr Asp Thr Arg Val Gly Arg Pro
   465 470 475 480
   Glu Glu Asp Ala Glu Ala Ala Glu
   485
<210> 21
   <211> 167
   <212> PRT
   <213> Oryza sativa
<400> 21
   Met Ala Asn Tyr His His Gln Glu Tyr Tyr Gln Met Ala Ala Ala Ala
   1 5 10 15
   Ala Val Ala Trp Pro Arg Glu Pro Asp Ser Pro Gln Leu Ser Ile Met
   20 25 30
   Ser Gly Cys Ser Ser Leu Phe Ser Ile Ser Thr Leu Arg Asp Asp Asp
   35 40 45
   Asp Gly Gly Gly Val Arg Leu Ala Gly Ala Ala Leu Pro Ala Thr Pro
   50 55 60
   Val Ser Leu Ala Gly Ile Ala Gly Gly Ala Ser Thr Pro Gly Gly Asp
   65 70 75 80
   Glu Val Asp Met Glu Val Arg Gln Gln Ser Gly Gly Ser Gly Asp Asp
   85 90 95
   Arg Arg Thr Ile Arg Met Met Arg Asn Arg Glu Ser Ala Leu Arg Ser
   100 105 110
   Arg Ala Arg Lys Arg Ala Tyr Val Glu Glu Leu Glu Lys Glu Val Arg
   115 120 125
   Arg Leu Val Asp Asp Asn Leu Asn Leu Lys Lys Gln Cys Lys Glu Leu
   130 135 140
   Lys Gln Glu Val Ala Ala Leu Val Met Pro Thr Lys Ser Ser Leu Arg
   145 150 155 160
   Arg Thr Ser Ser Thr Gln Phe
   165
<210> 22
   <211> 310
   <212> PRT
   <213> Oryza sativa
<400> 22
   Met Ala Glu Lys Lys Lys Lys Lys Lys Lys Lys Lys Pro Gln Ser Leu
   1               5                   10                  15
   Leu Val Leu Thr Ser Trp Arg Ser Ile Gly Met Gly Arg Gly Arg Val
               20                  25                  30
   Glu Leu Lys Arg Ile Glu Asn Lys Ile Asn Arg Gln Val Thr Phe Ala
           35                  40                  45
   Lys Arg Arg Asn Gly Leu Leu Lys Lys Ala Tyr Glu Leu Ser Val Leu
       50                  55                  60
   Cys Asp Ala Glu Val Ala Leu Ile Ile Phe Ser Asn Arg Gly Lys Leu
   65                  70                  75                  80
   Tyr Glu Phe Cys Ser Thr Gln Ser Met Thr Lys Thr Leu Glu Lys Tyr
                   85                  90                  95
   Gln Lys Cys Ser Tyr Ala Gly Pro Glu Thr Ala Val Gln Asn Arg Glu
               100                 105                 110
   Ser Glu Gln Leu Lys Ala Ser Arg Asn Glu Tyr Leu Lys Leu Lys Ala
           115                 120                 125
   Arg Val Glu Asn Leu Gln Arg Thr Gln Arg Gln Tyr Tyr Lys Ser Lys
       130                 135                 140
   His Arg Leu Cys Leu Val Arg Ser Lys Val Trp Asn Leu Val Lys Ile
   145                 150                 155                 160
   Arg Asp Asp Val Thr Glu Lys Leu Cys Met Tyr Glu Arg Asn Leu Leu
                   165                 170                 175
   Gly Glu Asp Leu Asp Ser Leu Gly Ile Lys Glu Leu Glu Ser Leu Glu
               180                 185                 190
   Lys Gln Leu Asp Ser Ser Leu Lys His Val Arg Thr Thr Arg Thr Lys
           195                 200                 205
   His Leu Val Asp Gln Leu Thr Glu Leu Gln Arg Lys Glu Gln Met Val
       210                 215                 220
   Ser Glu Ala Asn Arg Cys Leu Arg Arg Lys Leu Glu Glu Ser Asn His
   225                 230                 235                 240
   Val Arg Gly Gln Gln Val Trp Glu Gln Gly Cys Asn Leu Ile Gly Tyr
   245 250 255
   Glu Arg Gln Pro Glu Val Gln Gln Pro Leu His Gly Gly Asn Gly Phe
   260 265 270
   Phe His Pro Leu Asp Ala Ala Gly Glu Pro Thr Leu Gln Ile Gly Tyr
   275 280 285
   Pro Ala Glu His His Glu Ala Met Asn Ser Ala Cys Met Asn Thr Tyr
   290 295 300
   Met Pro Pro Trp Leu Pro
   305 310
<210> 23
   <211> 69
   <212> PRT
   <213> Oryza sativa
<400> 23
   Met Glu Gly Gly Gly Arg Arg Arg Lys Arg Gly Lys Val Glu Leu Arg
   1 5 10 15
   Arg Ile Glu Asp Arg Thr Ser Arg Gln Val Arg Phe Ser Lys Arg Arg
   20 25 30
   Ser Gly Leu Phe Lys Lys Ala Tyr Glu Leu Ser Val Leu Cys Asp Ala
   35 40 45
   Gln Val Ala Leu Leu Val Phe Ser Pro Ala Gly Arg Leu Tyr Glu Phe
   50 55 60
   Ala Ser Ser Thr Ser
   65
<210> 24
   <211> 491
   <212> PRT
   <213> Oryza sativa
<400> 24
   Met Ala Leu Leu Leu Ser Tyr Pro Arg Arg His Pro Ser Ile His Leu
   1               5                   10                  15
   Leu Ile Leu Ser Ala Tyr Ala Leu Phe Leu Leu Pro Ile Leu Asp Gly
               20                  25                  30
   Leu Glu Leu Gly Gly Asp Gly Leu Tyr Arg Glu Ile Leu Arg Asp Glu
           35                  40                  45
   Thr Val Leu Arg Leu Lys Glu Leu Gly Lys Ile Ser Asp Gly Glu Gly
       50                  55                  60
   Tyr Leu Glu Arg Thr Phe Leu Ser Pro Ala Ser Ile Arg Ala Ser Ala
   65                  70                  75                  80
   Val Ile Ile Ser Trp Met Lys Asp Ala Gly Leu Thr Thr Trp Ile Asp
                   85                  90                  95
   Gln Met Gly Asn Ile His Gly Arg Phe Glu Pro Thr Asn Ser Thr Lys
               100                 105                 110
   Glu Ala Leu Leu Ile Gly Ser His Met Asp Thr Val Ile Asp Ala Gly
           115                 120                 125
   Met Tyr Asp Gly Ala Leu Gly Ile Ile Ser Ala Ile Ser Ala Leu Lys
       130                 135                 140
   Val Leu Lys Val Thr Gly Arg Leu Gln Arg Leu Thr Arg Pro Val Glu
   145                 150                 155                 160
   Val Ile Ala Phe Ser Asp Glu Glu Gly Val Arg Phe Gln Thr Thr Phe
                   165                 170                 175
   Leu Gly Ser Ala Ala Val Ala Gly Thr Leu Pro Glu Ser Ile Leu Gln
               180                 185                 190
   Val Ser Asp Lys Ser Gly Thr Thr Val Gln Asp Val Leu Lys Leu Asn
           195                 200                 205
   Ser Leu Glu Gly Thr Ala Asn Ala Leu Gly Glu Val Arg Tyr Ser Pro
       210                 215                 220
   Glu Ser Val Gly Ser Tyr Val Glu Val His Ile Glu Gln Gly Pro Val
   225                 230                 235                 240
   Leu Glu Ala Leu Arg Tyr Pro Leu Gly Val Val Lys Gly Ile Ala Gly
                   245                 250                 255
   Gln Thr Arg Leu Lys Val Ile Ile Asn Gly Ser Gln Gly His Ala Gly
               260                 265                 270
   Thr Val Pro Met Lys Leu Arg Arg Asp Pro Met Val Ala Ala Ala Glu
           275                 280                 285
   Leu Val Leu Thr Leu Glu Thr Leu Cys Lys Glu Pro Asn Lys Phe Leu
       290                 295                 300
   Thr Tyr Asp Glu Glu Cys Gly Cys Phe Thr Glu Glu Ser Leu Ala Gly
   305                 310                 315                 320
   Leu Val Cys Thr Val Gly Glu Leu Leu Thr Trp Pro Ser Ala Ser Asn
                   325                 330                 335
   Val Ile Pro Gly Gln Val Asn Phe Thr Val Asp Ile Arg Ala Met Asp
               340                 345                 350
   Asp Lys Val Arg Glu Thr Ile Val Thr Ser Phe Ser Arg Leu Val Leu
           355                 360                 365
   Gln Arg Cys Asp Asp Arg Leu Val Asp Cys Ala Val Glu Gln Lys His
       370                 375                 380
   Ala Ala Ala Ala Thr Pro Cys Asp Ala Glu Leu Thr Ser Arg Leu Glu
   385                 390                 395                 400
   Arg Ala Thr Arg Ser Thr Ile Ser Ser Met Ala Ala Gly Val Arg Arg
                   405                 410                 415
   Ala Gly Gly Glu Thr Pro Val Leu Met Ser Gly Ala Gly His Asp Ala
               420                 425                 430
   Met Ala Met Ala Arg Leu Thr Lys Val Gly Met Leu Phe Val Arg Cys
           435                 440                 445
   Arg Gly Gly Val Ser His Ser Pro Glu Glu Ser Val Met Asp Asp Asp
       450                 455                 460
   Val Trp Ala Ala Gly Leu Ala Leu Val Asn Phe Ile Asp Gln Asn Ala
   465                 470                 475                 480
   Val Asp Ala Ala Ala Ala Thr Ala Ala Glu Ser
                   485                 490
<210> 25
   <211> 267
   <212> PRT
   <213> Oryza sativa
<400> 25
   Met Ser Phe Ala Asp Leu Glu Ala Gly Ala Val Arg Ala Pro Arg Arg
   1               5                   10                  15
   Ala Arg Gly Pro Asp Ala Thr Arg Ala Leu Val Phe Gln Ile Thr Thr
               20                  25                  30
   Ala Val Ala Ser Tyr Arg Arg Leu Leu Asn Ser Leu Gly Thr Pro Lys
           35                  40                  45
   Asp Thr Pro Ala Leu Arg Asp Gln Leu Gln Lys Thr Ser His Asn Ile
       50                  55                  60
   Leu Gln Leu Ala Lys Asp Ala Lys Glu Lys Leu Arg Arg Ala Ala Glu
   65                  70                  75                  80
   Ala Asp Lys Asn Ala Asp Thr Ser Ala Asp Lys Arg Val Ala Asp Met
                   85                  90                  95
   Lys Leu Ala Lys Asp Phe Ala Thr Thr Met Glu Glu Tyr Gly Lys Leu
               100                 105                 110
   Gln Asn Leu Ala Ile Gln Arg Glu Met Ala Tyr Lys Pro Val Val Pro
           115                 120                 125
   Gln Thr Ser Gln Pro Asn Tyr Thr Thr Gly Gly Ile Glu Ala Arg Asp
       130                 135                 140
   Ser Gly Lys Ile Pro Glu Gln His Ala Leu Leu Ala Glu Ser Lys Arg
   145                 150                 155                 160
   Gln Glu Val Leu Gln Leu Asp Asn Glu Ile Val Phe Asn Glu Ala Ile
                   165                 170                 175
   Ile Glu Glu Arg Glu Gln Ala Ile Gln Asp Ile Gln Gln Gln Ile Gly
               180                 185                 190
   Glu Val His Glu Ala Phe Lys Asp Leu Ala Thr Leu Val His Ile Gln
           195                 200                 205
   Gly Val Thr Ile Glu Glu Ile Asp Thr Asn Ile Glu Asn Ser Ala Ala
       210                 215                 220
   Ala Thr Lys Glu Ala Lys Thr Glu Leu Ala Lys Ala Ser Lys Thr Gln
   225                 230                 235                 240
   Lys Ser Asn Ser Ser Leu Leu Cys Ile Leu Leu Val Ile Phe Gly Val
                   245                 250                 255
   Val Leu Leu Ile Val Ile Ile Val Leu Ala Thr
               260                 265
<210> 26
   <211> 95
   <212> PRT
   <213> Oryza sativa
<400> 26
   Met Gln Leu Tyr Met Thr Tyr Gln Ala Cys Pro Met Gly Asp Leu Gln
   1               5                   10                  15
   Met Gly Asp Ser Val Val Ser Thr Ile Asp Ile Arg Ala Leu Tyr Cys
               20                  25                  30
   Asn Lys Ser Pro Gly Lys Ser Ser Ser Ser Ser Met Tyr Gly Ala Ser
           35                  40                  45
   Ser Ser Ser Lys Arg Lys Ala Cys Glu Leu Asn His Gly Asp Gly Gly
       50                  55                  60
   Ser Ala His Asp Asp Val Arg Asp Tyr Gly Val Asp His Val Asp Asp
   65                  70                  75                  80
   Asn Gly Glu Tyr Tyr Gly Asp Asp His Asp Asp Val Met His Gly
                   85                  90                  95
<210> 27
   <211> 1113
   <212> PRT
   <213> Oryza sativa
<400> 27
   Met Arg Ala Ala Ala Ala Ala Ser Lys Ala Ala Gly Lys Glu Lys Ser
   1               5                   10                  15
   Arg Arg Lys Gly Gly Gly Gly Gly Ala Gly Gly Gly Gly Gly Glu Gln
               20                  25                  30
   Leu Leu Thr Asp Gln Val Leu Ser Leu Arg Ala Arg Leu His Leu Ala
           35                  40                  45
   Leu Ala Leu Gly Leu Ala Lys Ser Asp Gly Gly Pro Lys Lys Trp Gln
       50                  55                  60
   Ser Thr Asp Ala Gly Ile Gln Ser His Val Leu Lys Ala Ala Ser Ala
   65                  70                  75                  80
   Phe Leu Gly Cys Leu Thr Asn Glu Met Leu Arg Leu Pro Pro Ile Lys
                   85                  90                  95
   Glu Ser Ile Ser Asp Ile Leu Ile Ala Leu Glu Gly Ile Leu Gln Ser
               100                 105                 110
   Lys Asn Val Ser Val Leu Ile Gln Ala Thr Asp Val Ser Leu Lys Leu
           115                 120                 125
   Val Ser Ser Val Gly Asn Leu Ala Arg Gln Tyr Pro Val Leu Glu Ile
       130                 135                 140
   Val Thr Cys Leu Ala Ser Gln Leu Ser Ala Asn Gln Ile Thr Ile Ala
   145                 150                 155                 160
   Val Ser Ser Ala Ser Thr Leu Asn Cys Ile Leu Asn Thr Leu Ala Thr
                   165                 170                 175
   Ala Arg Ser Ser Ile His Ala Glu Ile Trp Glu Ala Leu Glu Lys Thr
               180                 185                 190
   Asp Ala Val Thr Ser Val Ile Gly Ala Leu Gln Asn Tyr Ser Pro Asp
           195                 200                 205
   Val His Pro Leu Asn Tyr Leu Met Glu Met Met Ser Leu Leu Arg Ile
       210                 215                 220
   Ile Leu Trp Ile Trp Pro Ser Ser Arg Tyr His Val Trp Ser Asn Cys
   225                 230                 235                 240
   Asn Leu Met Gly Lys Leu Ala Gln Tyr Cys Val Ala Ser Glu Met Asp
                   245                 250                 255
   Val Ala Val Arg Val Leu Lys Leu Tyr Ala Ala Leu Ala Leu Cys Gly
               260                 265                 270
   Asn Gly Ala Met Val Leu Leu Asn Asn Glu Asp Leu Met Ala Lys Val
           275                 280                 285
   Gly Ala Leu Leu Gly Lys Ser Asn Pro Ser Ile Ala Arg Ile Glu Ala
       290                 295                 300
   Leu Lys Phe Tyr Gln Ile Leu Leu Arg Ser Ser Lys Gly Cys Asp Leu
   305                 310                 315                 320
   Leu Met Ala Ala His Tyr Gln His Ile Ile Glu Gly Thr Ile Asn Ala
                   325                 330                 335
   Met Ser Arg Asp Asp Glu Arg Leu Leu Thr Ile Glu Gly Cys Arg Thr
               340                 345                 350
   Ala Leu Leu Val Leu Arg Tyr Ala Gly Asp His His Arg Leu Phe Trp
           355                 360                 365
   Ser His Ala Ile Asp Asp Val Leu Tyr Lys Ile Leu Thr Gly Gly Cys
       370                 375                 380
   Thr Ser Ser His Lys Ala Asn Gln Ile Leu Cys His Asp Lys Leu Phe
   385                 390                 395                 400
   Asn Met Val Ser Glu Asn Phe Met Asp Ile His Ser Tyr Val Trp Asp
   405 410 415
   Ile Leu Gly Asn Leu Ala Val His Cys Lys Asn Glu Tyr Leu Ser Val
   420 425 430
   Arg Lys Gly Gln Asp Ser Ala Leu Gln Ala Leu Ile His Cys Ile Cys
   435 440 445
   Ser Leu Ala Ala Asp Ala Met Gln Lys Ser Asn Thr Met Lys Leu Ser
   450 455 460
   Lys Asp Val His Glu Pro Ala Leu Arg Ala Val Leu Met Met Leu Leu
   465 470 475 480
   Ser Pro Ser Gly Tyr Ile Leu Ser Glu Ala Ser Ser Lys Leu Leu His
   485 490 495
   Val Leu Pro Leu Gly Asp Asp Cys Leu Asn Ile Leu Phe Thr Ser Leu
   500 505 510
   Glu Ser Asn Thr Thr Arg Ser Ile Thr Ala Ser Phe Asp Asn Val Lys
   515 520 525
   Ile Met Ser Asn Leu Met Ser Leu Ala Gly Met Ser Ile Asn Phe Val
   530 535 540
   Cys Ile His Cys Lys Arg Asn Leu Asp Val Gly Ile Val Cys Asn Asp
   545 550 555 560
   Cys Arg Asp His Tyr Ser Glu Gly Leu Ile Arg Val Leu Gln Asn Ala
   565 570 575
   Ser Cys Gln Asn Leu Ser Pro Gly Pro Lys Leu Tyr Ile Ser Arg Ile
   580 585 590
   Leu Ser Leu Phe Gly Leu Cys Gly Phe Pro Ser Lys Leu Gly Gly Lys
   595 600 605
   Met Arg Arg Ala Leu Asp Asp Asn Glu Leu Ala Asp Leu Glu Leu Leu
   610 615 620
   Leu Ser Asn Gly Glu Ser Leu Lys Ala His Thr Ala Ile Ile Ser Val
   625 630 635 640
   Arg Cys Pro Lys Leu Leu Pro Ser Ala Lys Ser Leu Gly Ser Asp Gly
   645 650 655
   Lys Ile Thr Asp Glu Trp Gly Arg Ser Phe Tyr His Val Arg Met Ser
   660 665 670
   Asp Arg Val Asp Ser Cys Gly Leu Lys Lys Ile Leu Glu Tyr Thr Tyr
   675 680 685
   Thr Asn Ser Val Met Val Asp Asp Asp Asn Ile Lys Pro Arg Thr Leu
   690 695 700
   Ala Lys Tyr Cys His Leu Lys Ser Leu Gln Glu Met Leu Gln Lys Glu
   705 710 715 720
   Gln Pro Arg Trp Asn Ser Asp Cys Pro Arg Tyr Asp Leu Thr Ala Ala
   725 730 735
   Leu Glu Pro Val Lys Cys Ser Phe Ser Phe Ser Glu Val Ile Asn Val
   740 745 750
   Pro Leu Gly Trp Gln Ala Leu Asn Lys Leu Ile His Trp Phe Tyr Ser
   755 760 765
   Gly Glu Leu Pro Lys Ile Asp Pro Asp Cys Arg Trp Arg Asn Leu Asn
   770 775 780
   Ser Glu Glu Gln Leu Ser Gln Leu Arg Pro Tyr Ala Glu Leu Ser Ser
   785 790 795 800
   Leu Ser Glu Phe Trp Phe Leu Glu Gly Val Lys Glu Glu Ser Leu Ser
   805 810 815
   Val Val Thr Ser Cys Leu Ser Ser Thr Ser Thr Ala Ala Ser Val Glu
   820 825 830
   Phe Val Val Phe Ala Ala Gln Leu Gly Gln Trp Glu Met Val Glu Ala
   835 840 845
   Ala Val Gly Ser Val Ala His Leu Tyr Pro Lys Leu Arg Asp Ser Gly
   850 855 860
   Gln Leu Glu Gln Leu Asp Asp Asp Val Leu Asn Met Leu Arg Thr Glu
   865 870 875 880
   Tyr Val Arg Arg Thr Gln Arg Thr Gly Val Gly Ser Ala Ala Ala Gln
   885 890 895
   Ala Gly Ala Arg Val Val Thr Ala Val Tyr Arg Arg Gly Gln Arg Ala
   900 905 910
   Asp Tyr Trp Gln Ser Gly Gly Phe Gly Asp Asn Trp Asn Phe Gln Met
   915 920 925
   Val Ile Leu Asn Ala Ser Glu Glu His Cys Arg Glu Ser Lys Phe Asp
   930 935 940
   Thr Ile Gly Val Cys Lys Ala Arg Phe Leu Tyr Gly Lys Val Ser Arg
   945 950 955 960
   Gly Phe Arg Leu Arg Thr Ser Gly Ile Asn Lys Glu Gly Gly Pro Arg
   965 970 975
   Gly Gly Thr Val Ile Tyr Ser Arg Ser Ser Gly Gly Leu Pro Pro Trp
   980 985 990
   Cys Gly Ala Gly Ser His Asp Ala Leu Ala Ala Val Arg Trp Pro Ser
   995 1000 1005
   Leu Pro Gly Leu Glu Ser His Gln Thr Ala Gln Val Ile Arg Arg
   1010 1015 1020
   Gly Ala Gly Arg Arg Gly Glu Gly Arg Asp Val Asn Val Thr Lys
   1025 1030 1035
   Gln Ser Asn Ala Pro Met Arg Pro Pro Glu Thr Met Gln Arg Glu
   1040 1045 1050
   Gln Pro Gln Ser Arg Ala Arg Ala Asn Gly Arg Lys Trp Pro Pro
   1055 1060 1065
   Pro Arg Arg Trp Arg Ser Gly Ile Arg Glu Glu Gln Gly Val Pro
   1070 1075 1080
   Ser Ala Lys Ala Trp Gln Glu Lys Arg Lys Arg Thr Gln Gln Gln
   1085 1090 1095
   Arg Cys Ala Leu Pro Ala Ala Ile Ala Ala Ser Arg Leu Gln Leu
   1100 1105 1110
<210> 28
   <211> 412
   <212> PRT
   <213> Oryza sativa
<400> 28
   Met Ala Ser Ala Val Ala Ser Asn Leu Pro Ala Ala Ala Pro Ala Ala
   1               5                   10                  15
   Val Met Pro Phe Gly Gly Trp His Gly Pro Arg Val Ser Phe Ser Arg
               20                  25                  30
   Asp Ala Ala Gly Ala Glu Glu Ala Ala Ala Val Val Val Cys Ser Ser
           35                  40                  45
   Pro Leu Ala Ala Ala Ala Ala Val Ala Thr Thr Thr Thr Pro Glu Pro
       50                  55                  60
   Ala Ile Ser Lys Asp Phe Ile Asp Phe Glu Phe Ser Leu Gly Gly Ser
   65                  70                  75                  80
   Ala Thr Met Leu Pro Ala Asp Glu Leu Phe Ala Asp Gly Lys Leu Leu
                   85                  90                  95
   Pro Leu Arg Lys Ala Ala Ala Val Pro Glu Met Asp Ala Ala Ala Pro
               100                 105                 110
   Arg Pro Pro Gln Pro Glu Ala Met Pro Ala Pro Ser Glu Pro Met Lys
           115                 120                 125
   Pro Leu Arg Ala Ala Thr Ala Ala Val Asp Ala Ala Asp Pro Tyr Val
       130                 135                 140
   Phe Ser Pro Lys Ala Pro Ser Cys Ser Ser Arg Trp Arg Glu Leu Leu
   145                 150                 155                 160
   Gly Leu Lys Arg Ala Ala Ala Gln Ser Pro Lys Pro Ser Pro Ser Ser
                   165                 170                 175
   Ala Pro Ala Arg Thr Pro Gly Arg Ala Met Asn Ser Thr Ala Ala Arg
               180                 185                 190
   Ser Leu Lys Leu Leu Leu Gln Arg Asn Asn Gly Arg Ser Ser Gly Ala
           195                 200                 205
   Ser Ala Ser Glu Leu Ala Ser Ala Pro Leu Leu Arg Asp Ser Ser Asp
       210                 215                 220
   Ser Glu Ala Ser Leu Ser Leu Ala Ser Ser Arg Phe Ser Leu Ser Ser
   225                 230                 235                 240
   Ser Ser Ser Ser Ser Gly His Asp His Asp Asp Ile Pro Arg Leu Ser
                   245                 250                 255
   Leu Asp Ser Ala Ala Asp Pro Asn Pro Pro Arg Ile Arg Leu Val Arg
               260                 265                 270
   Ser Ser His Arg His Ser Thr Ser Ser Ser Ser Ser Ser Arg Ala Gly
           275                 280                 285
   Arg Ser Pro Ala Arg Arg Arg Pro Ser Pro Pro Pro Pro Pro Arg Cys
       290                 295                 300
   Leu Ser Val Asp Ser Pro Arg Met Asn Ser Ser Gly Lys Ile Val Phe
   305                 310                 315                 320
   Gln Gly Leu Glu Arg Ser Ser Ser Ser Pro Cys Thr Leu His Ala Ala
                   325                 330                 335
   Ala Lys Pro Arg Ser Arg Ala Val Asp Arg Ser Tyr Ser Ser Gly Val
               340                 345                 350
   Arg Val Ala Pro Val Val Leu Asn Val Pro Val Cys Ser Arg Pro Val
           355                 360                 365
   Phe Gly Phe Phe Lys Asp Lys Lys Asp Ala Ala Ala Lys Asp Ala Met
       370                 375                 380
   Ala Ala Arg Thr Arg Ser Ser Leu Gly Arg Lys Thr Thr Ala Ala Pro
   385                 390                 395                 400
   Gln Gly Trp Ser Gly Glu Leu Gly Arg Ser Cys Gly
                   405                 410
<210> 29
   <211> 763
   <212> PRT
   <213> Oryza sativa
<400> 29
   Met Lys Ile Ser Gly Leu Leu Thr Ser Ala Gly Ile Asn Ile Ala Leu
   1               5                   10                  15
   Ser Val Leu Phe Ile Ser Leu Tyr Ser Val Leu Arg Lys Gln Pro Ala
               20                  25                  30
   Asn Val Arg Val Tyr Phe Gly Arg Arg Ile Ala Glu Glu His Asn Arg
           35                  40                  45
   Leu Arg Glu Ala Phe Ile Leu Glu Arg Phe Val Pro Ser Thr Gly Trp
       50                  55                  60
   Ile Val Lys Ala Leu Gln Cys Thr Glu Glu Glu Ile Leu Ala Ala Ala
   65                  70                  75                  80
   Gly Leu Asp Ala Val Val Phe Asn Arg Ile Leu Val Phe Ser Leu Arg
                   85                  90                  95
   Ile Phe Ser Leu Ala Ala Ile Leu Cys Val Phe Gly Ile Leu Pro Leu
               100                 105                 110
   Asn Tyr Phe Gly Gln Asp Ile His His Val Arg Ile Pro Ser Glu Ser
           115                 120                 125
   Leu Asp Ile Phe Thr Ile Gly Asn Val Lys Val Arg Ser Arg Trp Leu
       130                 135                 140
   Trp Val His Cys Val Ala Leu Tyr Ile Ile Ser Gly Val Ala Cys Ile
   145                 150                 155                 160
   Leu Leu Tyr Leu Glu Tyr Lys His Ile Ala Arg Leu Arg Leu Arg His
                   165                 170                 175
   Leu Thr Cys Ala Met Pro Asn Pro Ser His Phe Thr Val Leu Val Arg
               180                 185                 190
   Gly Ile Pro Lys Glu Thr Lys Glu Ser Cys Ser Asn Ala Ile Asp Asp
           195                 200                 205
   Phe Phe Thr Lys Tyr His Gly Ser Ser Tyr Leu Phe His Gln Val Val
       210                 215                 220
   Tyr Lys Val Gly Lys Val Gln Lys Ile Met Thr Gly Ala Lys Lys Ala
   225                 230                 235                 240
   Tyr Arg Lys Phe Lys His Phe Thr Asp Ser Thr Ile Asp Gln Arg Cys
                   245                 250                 255
   Arg Ala Ile Ser Tyr Arg Cys Cys Leu Cys Gly Ala Ser Ser Asn Ser
               260                 265                 270
   Phe Gln Leu Leu Ala Thr Gly Leu Glu Gln Asn Gln Gly Lys Ser Asp
           275                 280                 285
   Leu Gln Asp Ser Ser Leu Lys Leu Asp Asp Gln Glu Cys Ala Ala Ala
       290                 295                 300
   Phe Val Tyr Phe Arg Thr Arg Tyr Ala Ala Leu Val Ala Ser Glu Ile
   305                 310                 315                 320
   Leu Gln Thr Ser Asn Pro Met Lys Trp Val Thr Asp Leu Ala Pro Glu
                   325                 330                 335
   Pro Asp Asp Val Tyr Trp Ser Asn Leu Trp Leu Pro Tyr Lys Gln Leu
               340                 345                 350
   Trp Ile Arg Arg Ile Ala Thr Leu Leu Gly Ser Ile Val Phe Met Leu
           355                 360                 365
   Phe Phe Leu Ile Pro Val Thr Phe Ile Gln Gly Leu Ser Gln Leu Glu
       370                 375                 380
   Gln Leu Gln Gln Arg Leu Pro Phe Leu Lys Gly Ile Leu Glu Lys Lys
   385                 390                 395                 400
   Tyr Met Ser Gln Leu Val Thr Gly Tyr Leu Pro Ser Val Ile Leu Gln
                   405                 410                 415
   Ile Phe Leu Tyr Ala Val Ala Pro Ile Met Ile Leu Phe Ser Thr Leu
               420                 425                 430
   Glu Gly Pro Ile Ser His Ser Glu Arg Lys Arg Ser Ala Cys Cys Lys
           435                 440                 445
   Val Leu Tyr Phe Thr Val Trp Asn Ile Phe Phe Gly Asn Val Leu Ser
       450                 455                 460
   Gly Thr Val Ile Ser Gln Leu Asn Val Leu Ser Ser Pro Lys Asp Ile
   465                 470                 475                 480
   Pro Val Gln Leu Ala Arg Ala Ile Pro Val Gln Ala Thr Phe Phe Ile
                   485                 490                 495
   Thr Tyr Val Leu Thr Ser Gly Trp Ala Ser Leu Ser Ser Glu Leu Met
               500                 505                 510
   Gln Leu Phe Gly Leu Ile Trp Asn Phe Val Arg Lys Tyr Ile Leu Arg
           515                 520                 525
   Met Pro Glu Asp Thr Glu Phe Val Pro Ser Phe Pro Tyr His Thr Glu
       530                 535                 540
   Val Pro Lys Val Leu Leu Phe Gly Leu Leu Gly Phe Thr Cys Ser Val
   545                 550                 555                 560
   Leu Ala Pro Leu Ile Leu Pro Phe Leu Leu Val Tyr Phe Phe Leu Gly
                   565                 570                 575
   Tyr Ile Val Tyr Arg Asn Gln Leu Leu Asn Val Tyr Arg Thr Arg Tyr
               580                 585                 590
   Asp Thr Gly Gly Leu Tyr Trp Pro Ile Ala His Asn Ala Val Ile Phe
           595                 600                 605
   Ser Leu Val Leu Thr Gln Ile Ile Cys Leu Gly Val Phe Gly Leu Lys
       610                 615                 620
   Glu Ser Pro Val Ala Ala Gly Phe Thr Ile Pro Leu Ile Ile Leu Thr
   625                 630                 635                 640
   Leu Leu Phe Asn Gln Tyr Cys Arg Asn Arg Leu Leu Pro Leu Phe Arg
                   645                 650                 655
   Thr Thr Pro Ala Gln Asp Leu Ile Asp Met Asp Arg Glu Asp Glu Arg
               660                 665                 670
   Ser Gly Arg Met Asp Glu Ile His His Arg Leu His Ser Ala Tyr Cys
           675                 680                 685
   Gln Phe His Asp Thr Glu Asp Ile Pro Leu Glu Lys Ile Gln Thr Val
       690                 695                 700
   Gly Ser Asp Glu Glu Gln Gly Cys Ser Ser Asp Lys Ser Asn Gly Lys
   705                 710                 715                 720
   Glu Ser Phe Glu Glu Pro Arg Ala Glu Leu Ser His Pro Thr Leu Asn
                   725                 730                 735
   Gly Leu Pro Val Ser Arg Leu Arg His Ala Val Lys Ser Ile Thr Phe
               740                 745                 750
   Leu Val Arg Leu Gln Lys Arg Gly Leu Ser Glu
           755                 760
<210> 30
   <211> 471
   <212> PRT
   <213> Oryza sativa
<400> 30
   Met Ala Ile Leu Arg Glu Phe Gly Thr Ile Glu Gly Met Glu Asn Leu
   1               5                   10                  15
   Leu Pro Glu Asp Val Leu Ser Asn Ile Ile His Arg Leu Ala Pro Arg
               20                  25                  30
   Tyr Leu Ala Ile Ser Arg Cys Val Cys Lys Thr Trp Cys Thr Ile Ile
           35                  40                  45
   Glu Ala His Asn Leu Leu His Val Asp Leu Leu Pro Arg Pro Leu Cys
       50                  55                  60
   Gly Ile Phe Ile Asn Phe Asn Glu Leu Ser Met Ser Glu Phe Phe Ser
   65                  70                  75                  80
   Arg Pro Ser Lys Gly Pro Thr Val Ser Gly Asn Phe Asp Tyr Leu Pro
                   85                  90                  95
   Cys Ser Ser Cys Ile Ile Asp His Cys Asn Gly Leu Leu Leu Phe His
               100                 105                 110
   Lys Tyr Val Val Asn Pro Ala Thr Arg Gln Ser Ala Pro Leu Pro Pro
           115                 120                 125
   Cys Pro Tyr Met Val Val Glu His Ile Phe His Arg Glu Tyr Leu Val
       130                 135                 140
   Phe Asp Pro Thr Leu Ser Pro His Phe Glu Val Phe Met Ile Pro Glu
   145                 150                 155                 160
   Ile Arg Arg Ser Asn Val Trp Tyr Asn Met Leu Asn Ser Asp Asp Lys
                   165                 170                 175
   Leu Asp Pro Ala Ile Glu Glu Leu Glu Trp Pro Pro Ser Pro Cys Ile
               180                 185                 190
   Leu His Val Phe Ser Ser Arg Thr Lys Val Trp Glu Glu Arg Ser Phe
           195                 200                 205
   Val Arg Glu Gly Glu Ala Ala Gly Asn Val Ser Asp Met Arg Leu Asp
       210                 215                 220
   His Pro Tyr Val Pro Asp Thr Ser Val Tyr Val Pro Asp Thr Ser Val
   225                 230                 235                 240
   Tyr Cys Arg Gly Val Leu Tyr Val Tyr Cys Gln Asn Lys Tyr Val Met
                   245                 250                 255
   Arg Ile Ser Leu Ser Asn Gly Lys Tyr Gln Val Ile Lys Pro Pro Ser
               260                 265                 270
   Asp Cys Glu Gly Met Ala Tyr Thr Asn Leu Tyr Leu Gly Lys Ser Met
           275                 280                 285
   Lys Gly Val Tyr Cys Ala Val Arg His Leu Ala Ser Arg Phe Leu Ile
       290                 295                 300
   Tyr Ile Leu Asp Glu Ser Ser Asp Arg Met Glu Trp Val Cys Lys Asp
   305                 310                 315                 320
   Ser Cys Ser Ile Gln Pro Cys Gln Ile Ile Asp Gly Pro Gly Pro Trp
                   325                 330                 335
   Thr Leu Gln Asp Ile Asn Asn Gln Glu Arg Gly Phe Glu Tyr Glu Asp
               340                 345                 350
   Gly Asn Asn Glu Ala Val Val Glu Asp Arg Phe Glu Trp Asp Ser Asp
   355 360 365
   Asn Asp Asn Val Ile Glu Thr Asn Ser Arg Gly Ser Gly Gly Tyr Ile
   370 375 380
   Asn Phe Leu Val Asp Thr Thr Arg Arg Gly Arg Tyr Asn Ser Gly Gly
   385 390 395 400
   Tyr Ile Asp Phe Leu Gly Phe His Pro Tyr Lys Glu Val Ile Phe Leu
   405 410 415
   Ser Asp Ser Leu Arg Arg Gly Leu Ala Tyr His Leu Asn Ser Ser Lys
   420 425 430
   Ile Gln Asp Leu Gly Ser Leu Arg Pro Thr Asn Tyr Gly Thr Glu Val
   435 440 445
   Gly Ile Gln Pro Phe Ile Gln Lys Phe Phe Pro Tyr Ser Pro Trp Met
   450 455 460
   Gly Arg Phe Pro Glu Asp Asn
   465 470
<210> 31
   <211> 763
   <212> PRT
   <213> Oryza sativa
<400> 31
   Met Lys Ile Ser Gly Leu Leu Thr Ser Ala Gly Ile Asn Ile Ala Leu
   1               5                   10                  15
   Ser Val Leu Phe Ile Ser Leu Tyr Ser Val Leu Arg Lys Gln Pro Ala
               20                  25                  30
   Asn Val Arg Val Tyr Phe Gly Arg Arg Ile Ala Glu Glu His Asn Arg
           35                  40                  45
   Leu Arg Glu Ala Phe Ile Leu Glu Arg Phe Val Pro Ser Thr Gly Trp
       50                  55                  60
   Ile Val Lys Ala Leu Gln Cys Thr Glu Glu Glu Ile Leu Ala Ala Ala
   65                  70                  75                  80
   Gly Leu Asp Ala Val Val Phe Asn Arg Ile Leu Val Phe Ser Leu Arg
                   85                  90                  95
   Ile Phe Ser Leu Ala Ala Ile Leu Cys Val Phe Gly Ile Leu Pro Leu
   100 105 110
   Asn Tyr Phe Gly Gln Asp Ile His His Val Arg Ile Pro Ser Glu Ser
   115 120 125
   Leu Asp Ile Phe Thr Ile Gly Asn Val Lys Val Arg Ser Arg Trp Leu
   130 135 140
   Trp Val His Cys Val Ala Leu Tyr Ile Ile Ser Gly Val Ala Cys Ile
   145 150 155 160
   Leu Leu Tyr Leu Glu Tyr Lys His Ile Ala Arg Leu Arg Leu Arg His
   165 170 175
   Leu Thr Cys Ala Met Pro Asn Pro Ser His Phe Thr Val Leu Val Arg
   180 185 190
   Gly Ile Pro Lys Glu Thr Lys Glu Ser Cys Ser Asn Ala Ile Asp Asp
   195 200 205
   Phe Phe Thr Lys Tyr His Gly Ser Ser Tyr Leu Phe His Gln Val Val
   210 215 220
   Tyr Lys Val Gly Lys Val Gln Lys Ile Met Thr Gly Ala Lys Lys Ala
   225 230 235 240
   Tyr Arg Lys Phe Lys His Phe Thr Asp Ser Thr Ile Asp Gln Arg Cys
   245 250 255
   Arg Ala Ile Ser Tyr Arg Cys Cys Leu Cys Gly Ala Ser Ser Asn Ser
   260 265 270
   Phe Gln Leu Leu Ala Thr Gly Leu Glu Gln Asn Gln Gly Lys Ser Asp
   275 280 285
   Leu Gln Asp Ser Ser Leu Lys Leu Asp Asp Gln Glu Cys Ala Ala Ala
   290 295 300
   Phe Val Tyr Phe Arg Thr Arg Tyr Ala Ala Leu Val Ala Ser Glu Ile
   305 310 315 320
   Leu Gln Thr Ser Asn Pro Met Lys Trp Val Thr Asp Leu Ala Pro Glu
   325 330 335
   Pro Asp Asp Val Tyr Trp Ser Asn Leu Trp Leu Pro Tyr Lys Gln Leu
   340 345 350
   Trp Ile Arg Arg Ile Ala Thr Leu Leu Gly Ser Ile Val Phe Met Leu
   355 360 365
   Phe Phe Leu Ile Pro Val Thr Phe Ile Gln Gly Leu Ser Gln Leu Glu
       370                 375                 380
   Gln Leu Gln Gln Arg Leu Pro Phe Leu Lys Gly Ile Leu Glu Lys Lys
   385                 390                 395                 400
   Tyr Met Ser Gln Leu Val Thr Gly Tyr Leu Pro Ser Val Ile Leu Gln
                   405                 410                 415
   Ile Phe Leu Tyr Ala Val Ala Pro Ile Met Ile Leu Phe Ser Thr Leu
               420                 425                 430
   Glu Gly Pro Ile Ser His Ser Glu Arg Lys Arg Ser Ala Cys Cys Lys
           435                 440                 445
   Val Leu Tyr Phe Thr Val Trp Asn Ile Phe Phe Gly Asn Val Leu Ser
       450                 455                 460
   Gly Thr Val Ile Ser Gln Leu Asn Val Leu Ser Ser Pro Lys Asp Ile
   465                 470                 475                 480
   Pro Val Gln Leu Ala Arg Ala Ile Pro Val Gln Ala Thr Phe Phe Ile
                   485                 490                 495
   Thr Tyr Val Leu Thr Ser Gly Trp Ala Ser Leu Ser Ser Glu Leu Met
               500                 505                 510
   Gln Leu Phe Gly Leu Ile Trp Asn Phe Val Arg Lys Tyr Ile Leu Arg
           515                 520                 525
   Met Pro Glu Asp Thr Glu Phe Val Pro Ser Phe Pro Tyr His Thr Glu
       530                 535                 540
   Val Pro Lys Val Leu Leu Phe Gly Leu Leu Gly Phe Thr Cys Ser Val
   545                 550                 555                 560
   Leu Ala Pro Leu Ile Leu Pro Phe Leu Leu Val Tyr Phe Phe Leu Gly
                   565                 570                 575
   Tyr Ile Val Tyr Arg Asn Gln Leu Leu Asn Val Tyr Arg Thr Arg Tyr
               580                 585                 590
   Asp Thr Gly Gly Leu Tyr Trp Pro Ile Ala His Asn Ala Val Ile Phe
           595                 600                 605
   Ser Leu Val Leu Thr Gln Ile Ile Cys Leu Gly Val Phe Gly Leu Lys
       610                 615                 620
   Glu Ser Pro Val Ala Ala Gly Phe Thr Ile Pro Leu Ile Ile Leu Thr
   625                 630                 635                 640
   Leu Leu Phe Asn Gln Tyr Cys Arg Asn Arg Leu Leu Pro Leu Phe Arg
                   645                 650                 655
   Thr Thr Pro Ala Gln Asp Leu Ile Asp Met Asp Arg Glu Asp Glu Arg
               660                 665                 670
   Ser Gly Arg Met Asp Glu Ile His His Arg Leu His Ser Ala Tyr Cys
           675                 680                 685
   Gln Phe His Asp Thr Glu Asp Ile Pro Leu Glu Lys Ile Gln Thr Val
       690                 695                 700
   Gly Ser Asp Glu Glu Gln Gly Cys Ser Ser Asp Lys Ser Asn Gly Lys
   705                 710                 715                 720
   Glu Ser Phe Glu Glu Pro Arg Ala Glu Leu Ser His Pro Thr Leu Asn
                   725                 730                 735
   Gly Leu Pro Val Ser Arg Leu Arg His Ala Val Lys Ser Ile Thr Phe
               740                 745                 750
   Leu Val Arg Leu Gln Lys Arg Gly Leu Ser Glu
           755                 760
<210> 32
   <211> 247
   <212> PRT
   <213> Oryza sativa
<400> 32
   Met Val Glu Leu Ser Ile Ala Asp Ala Ser Ala Ser Asp Leu Cys Gly
   1               5                   10                  15
   Gly Thr Leu Gly Gln Met Val Glu Leu Val Cys Glu Ala Arg Leu Arg
               20                  25                  30
   Val Arg Glu Glu Tyr Val Arg Ser Thr Val Asp Leu Met Ala Leu Leu
           35                  40                  45
   Arg Gly Arg Gly Met Val Phe Asp Gly Val Tyr Val Val Ser Asn Leu
       50                  55                  60
   Thr Arg Leu Phe Ala Glu Leu Asp Phe Gly Arg Gly Glu Trp Val Val
   65                  70                  75                  80
   Ser Gly Met Ala Gln Pro Met Leu Ala Thr Phe Leu Val Thr Cys Arg
   85 90 95
   Asn Gly Asp Asp Glu Asp Ala Val Ala Ala Ser Met Leu Leu Pro Pro
   100 105 110
   Pro Val Lys Leu Arg Phe Ala Glu Glu Leu Ala Gly Leu Met Met Ser
   115 120 125
   Met Pro His Gly Gly Ala Ala Leu Cys Pro Ala Pro Ala Ser Thr Tyr
   130 135 140
   Leu Pro Leu Ser Met Arg Gly Arg Arg Trp Leu His Ile Pro Glu Gly
   145 150 155 160
   Tyr Tyr Gly Asn Ala Leu Ala Tyr Ser Ile Thr Asp Ala Ser Ala Ser
   165 170 175
   Asp Leu Cys Gly Ala Thr Leu Ala Gln Met Met Glu Leu Val Cys Glu
   180 185 190
   Ala Arg Leu Arg Val Thr Glu Glu Tyr Gly Arg Ser Thr Val Asp Leu
   195 200 205
   Met Ala Ser Leu Arg Gly His Asp Thr Val Phe Asp Gly Val Tyr Val
   210 215 220
   Val Ser Asp Leu Gly Ala Gly Ser Gly Trp Ser Ala Ala Trp Pro Ser
   225 230 235 240
   Arg Cys Trp Arg Arg Ser Trp
   245
<210> 33
   <211> 144
   <212> PRT
   <213> Oryza sativa
<400> 33
   Met Asn Gln Gln His Gln Arg Ser Ile Glu His Cys Ser Ile Gly Cys
   1               5                   10                  15
   Phe Leu Ala Ser Pro Pro Pro Arg Phe Phe Pro Ala Arg Thr Arg Ser
               20                  25                  30
   Ala Pro Gly Glu Leu Arg Met Lys Leu Val Val Phe Leu Ile Arg Gly
           35                  40                  45
   Cys Pro Gly Glu Val Leu Leu Arg Pro Ile Val Pro Ala Lys Glu Gly
       50                  55                  60
   Leu Arg Thr Arg Thr Lys Trp His Ile Leu Gln Arg Phe Cys Lys Leu
   65                  70                  75                  80
   Glu Ile Ile Ser Ile Glu Thr Glu Thr Met Ile Thr Ile Ser Ser Arg
                   85                  90                  95
   Ser Ile Ile Lys Ser Arg Cys Lys Lys Ser Asn Lys Lys Ile Leu Val
               100                 105                 110
   Phe Phe Leu Ser Met Ser Val Lys Phe Leu Leu Ile Thr Thr Arg Arg
           115                 120                 125
   Ser Leu Ser Val Gln Lys Arg Ser Ser Thr Phe Ser Gln Leu Leu His
       130                 135                 140
<210> 34
   <211> 94
   <212> PRT
   <213> Oryza sativa
<400> 34
   Met Cys Met Asp Arg Ala Ala Val Pro Val Lys Arg Val Trp Leu Gly
   1               5                   10                  15
   Leu Ala Ala Arg Leu Gly Leu Arg Arg Thr Ser Gly Leu Gly Lys Leu
               20                  25                  30
   Lys Lys Glu Val Arg Thr Cys Glu Tyr His Asp Val His Ile Met Trp
           35                  40                  45
   Glu Met Leu Arg Lys Thr Asp Ala Pro Val Pro Met Ala Glu Lys Glu
       50                  55                  60
   Ala Ala Ala Ala Ala Ala Val Ala Ala Ala Ala Gly Ala Arg Arg Arg
   65                  70                  75                  80
   Lys Ala Ala Trp Arg Arg Phe Leu Tyr Tyr Cys Cys Ala Phe
                   85                  90
<210> 35
   <211> 303
   <212> PRT
   <213> Oryza sativa
<400> 35
   Met Ala Thr Ser Arg Lys Leu Ala Arg Val Asp Ile Ala Glu Leu Lys
   1               5                   10                  15
   Gln Arg Leu Val Lys Arg Leu Gly Arg Gln Arg Ala Gly Gln Tyr Phe
               20                  25                  30
   Ala His Leu Thr Arg Leu Leu Asn Leu Lys Leu Thr Lys Val Glu Phe
           35                  40                  45
   Asp Lys Leu Cys Tyr Ala Thr Ile Gly Arg Glu Asn Ile Ala Leu His
       50                  55                  60
   Asn Ala Leu Ile Arg Gly Ile Ile Ser Asn Ala Leu Ser Gly Val Pro
   65                  70                  75                  80
   Pro Pro Ser Arg Gln Ala Val Thr Gly Gln Ser Gly Thr Thr Thr Ala
                   85                  90                  95
   Pro Ser Gly Gln Cys Val Gly Ile Ala Leu Gln Ser Ala Arg Asn Val
               100                 105                 110
   Gly Ala Val Val Asp Ser Gly Asp Gly Asp Phe Ala Arg Glu Arg Ala
           115                 120                 125
   Val Ala Gly Lys Val Leu Ser Val Glu Asp Gly Glu Glu Val Glu Gln
       130                 135                 140
   Val Arg Ser Ala Pro Cys Val Gln Ser Arg Ser Pro Ile Thr Ala Pro
   145                 150                 155                 160
   Leu Gly Ile Ser Thr Thr Pro Thr Tyr Gly Ala Arg Thr Trp Arg Leu
                   165                 170                 175
   Asp Asp Pro Met Val Ser Cys Tyr Asp Ser His His Leu Leu Asp Thr
               180                 185                 190
   Gly Ser Leu Phe Lys Gly Leu Gln Arg Arg Leu Glu Ser Asp Gly Ile
           195                 200                 205
   Gly Val Ser Val Gln Gly Val Glu Val Leu Asn Arg Gly Leu Asp Glu
       210                 215                 220
   Phe Leu Arg Arg Leu Ile Lys Pro Cys Met Glu Leu Ser Arg Ser Arg
   225                 230                 235                 240
   Ser Ser Gly Arg Arg Val Thr Lys Gly Asn Ala Met Phe Ala Ala Arg
                   245                 250                 255
   Met Asn Gly Leu Gln Gln Ala Asn His Gly His Cys Thr Thr Leu Gln
               260                 265                 270
   Asp Phe Ala Val Ala Met Glu Ser Asp Pro His Leu Leu Gly Thr Asn
           275                 280                 285
   Trp Pro Thr Gln Leu Glu Lys Ile Gln Ala Thr Ser Phe Gly Glu
   290 295 300
<210> 36
   <211> 186
   <212> PRT
   <213> Oryza sativa
<400> 36
   Met Ala Ser Pro Arg Cys Ala Ala Val Ala Leu Leu His Pro Ala Gly
   1 5 10 15
   Val Ala Ala Gly Gly Gly Ala Arg Arg Arg Val Leu Leu Leu Asp Gln
   20 25 30
   Glu Arg Pro Leu Trp Gly Thr Glu Val Arg Arg Arg Arg Arg Arg Arg
   35 40 45
   Phe Ser Ser Leu Glu Thr Pro Pro Arg Cys Ser Lys Met Tyr Val Pro
   50 55 60
   Gly Phe Gly Glu Gly Ser Pro Glu Lys Lys Ala Ala Arg Asn Leu Gln
   65 70 75 80
   His Phe Phe Asn Tyr Ile Ala Val Arg Val Val Leu Thr Gln Leu Glu
   85 90 95
   Ser Tyr Asn Arg Glu Ala Tyr Gly Glu Leu Met Asp Phe Val Asn Arg
   100 105 110
   Asn Ser Leu Asn Asp Ala Asp Thr Phe Cys Lys Lys Leu Ile Arg Glu
   115 120 125
   Ser Pro Arg His Lys Gln Leu Ala Met Arg Ile Leu Glu Val Arg Ser
   130 135 140
   Ala Tyr Val Lys His Asp Phe Glu Trp Asp Asn Leu Lys Arg Leu Ser
   145 150 155 160
   Phe Lys Met Val Asp Glu Ala Asn Thr Lys Leu Met Arg Asp Tyr Val
   165 170 175
   Leu Glu Thr Ser His Ile Glu Asp Asp Asn
   180 185
<210> 37
   <211> 347
   <212> PRT
   <213> Oryza sativa
<400> 37
   Met Asp Ile Thr Gly Ala Gly Ala Met Gly Gly Gly Ser Thr Ala Ala
   1               5                   10                  15
   Thr Ala Ala Ala Ala Ala Gly Ala Gly Trp Lys Thr Pro Val Ser Met
               20                  25                  30
   Val Leu Val Gln Leu Phe Ile Thr Gly Gln Ile Leu Leu Ser Lys Val
           35                  40                  45
   Ser Ile Gly Gly Gly Met Leu Ile Phe Val Leu Leu Ala Tyr Asn Ser
       50                  55                  60
   Phe Phe Ala Val Val Phe Leu Leu Pro Phe Ala Leu Ile Phe Glu Arg
   65                  70                  75                  80
   Gly Lys Trp Arg Asp Met Asp Trp Gly Ala Phe Gly Trp Ile Phe Leu
                   85                  90                  95
   Asn Ala Phe Ile Gly Tyr Ser Val Pro Met Ser Leu Tyr Tyr Tyr Gly
               100                 105                 110
   Leu Lys Asp Thr Thr Ser Ser Tyr Ser Val Ile Phe Leu Asn Ile Thr
           115                 120                 125
   Pro Leu Phe Thr Phe Ile Leu Ser Leu Met Phe Arg Leu Glu Ala Phe
       130                 135                 140
   Lys Leu Arg Ser Ile Pro Gly Val Leu Lys Ile Ala Ser Ile Leu Leu
   145                 150                 155                 160
   Ser Ile Gly Gly Thr Met Leu Ile Ser Leu Tyr Lys Gly Lys Ser Leu
                   165                 170                 175
   His Leu Trp Asp Ser Ile Ile Gln His Gln Asn Glu His Lys Ser Ala
               180                 185                 190
   Thr Asn Gln Leu Arg Gly Thr Ile Leu Leu Val Gly Ser Ser Phe Thr
           195                 200                 205
   Phe Ala Cys Trp Phe Leu Ile Gln Ser Lys Ile Leu Lys Val Tyr Pro
       210                 215                 220
   Tyr Lys Tyr Trp Ser Ser Met Val Thr Cys Leu Val Gly Val Phe Gln
   225                 230                 235                 240
   Thr Ala Leu Val Gly Ile Ile Leu Arg Arg Asp Lys Ser Ala Trp Glu
                   245                 250                 255
   Leu Gly Trp Asn Leu Asn Leu Val Thr Ile Val Tyr Thr Gly Ala Leu
               260                 265                 270
   Ala Thr Ala Gly Lys Tyr Ile Leu Asn Ser Trp Ala Ile Thr Lys Arg
           275                 280                 285
   Gly Pro Thr Tyr Pro Thr Met Phe Ser Pro Leu Ser Val Val Phe Thr
       290                 295                 300
   Val Val Leu Asp Ser Val Leu Leu Gly Asn Asp Ile Thr Ile Gly Ser
   305                 310                 315                 320
   Leu Leu Gly Thr Ala Leu Val Ile Val Gly Leu Tyr Leu Phe Leu Trp
                   325                 330                 335
   Ala Lys Ala Arg Glu Ile Pro Lys Lys Ser Thr
               340                 345
<210> 38
   <211> 175
   <212> PRT
   <213> Oryza sativa
<400> 38
   Met Ala Arg Arg Ala Ala Glu Lys Glu Ala Ala Leu Arg Gln Gly Leu
   1               5                   10                  15
   Thr Ala Gly Asp Gly Glu Ala Arg Arg Thr Gly Ala Leu Trp Arg Thr
               20                  25                  30
   Asp Ala Trp Arg Gln Arg Ala Ala Ala Ser Ala Ala Ala Ala Ser Val
           35                  40                  45
   Val Arg Thr Trp Pro Ser Ser Ala Pro Trp Leu Arg Phe Glu Leu Asp
       50                  55                  60
   Pro Trp Arg Arg Val Cys Gly Glu Gln Asp Leu Gln Thr Ala Ala Cys
   65                  70                  75                  80
   Gly Gly Gly Asp Gly Ala Val Gly Leu Ser Phe Glu Thr His His Gly
                   85                  90                  95
   Gly Ser Val Ala Pro Ser Pro Glu Phe Ala Ala Cys Ala Ala Ser Ser
               100                 105                 110
   Cys Ser Ala Glu Leu Met Val Leu Leu Val Leu Gln Arg Gly Glu Leu
           115                 120                 125
   Leu Val Arg His Asp Arg Pro Ser His His His Arg Arg Arg Phe Pro
       130                 135                 140
   Thr Pro Gln Pro Ala Glu Ala Ala Ala Ala Val Glu Val Gly Trp Gly
   145                 150                 155                 160
   Phe Gln Asn Pro Arg Asp Ala Met Thr Cys Leu Cys Lys Gly Leu
                   165                 170                 175
<210> 39
   <211> 306
   <212> PRT
   <213> Oryza sativa
<400> 39
   Met Gly Ser Gly Gly Gly Gly Cys Gly Arg Asn Gly Ala Val Arg Gln
   1               5                   10                  15
   Tyr Ile Arg Ser Lys Val Pro Arg Leu Arg Trp Thr Gly Glu Leu His
               20                  25                  30
   Cys Ser Phe Val Gln Ala Ile Glu Phe Leu Gly Gly Gln Asp Lys Ala
           35                  40                  45
   Thr Pro Lys Leu Ile Leu Gln Leu Met Gly Val Lys Gly Leu Thr Ile
       50                  55                  60
   Ser His Val Lys Ser His Leu Gln Met Tyr Arg Cys Ser Arg Leu Gly
   65                  70                  75                  80
   Ser His Gly Thr Gly Arg Arg Ser Glu Met Gln Pro Gln Leu Gln Arg
                   85                  90                  95
   Lys His Ser Cys Gly Ala Asp Glu Gln Val Pro Arg Glu Phe Leu Cys
               100                 105                 110
   Pro Pro Leu Lys Arg Thr Arg Met Gly Thr Glu Ala Thr Tyr Lys Gly
           115                 120                 125
   Met Gln Gly Ser Gln Gly Ile Ser Glu Met Arg Thr Thr Gly Thr Gln
       130                 135                 140
   Tyr Cys Ile Asp Asp Tyr Met Gln Ala Met Ala Met Glu Arg Arg Ile
   145                 150                 155                 160
   Lys Glu Glu Gly Leu Arg Trp Gln Arg Asp Ala Ala Ala Ala Ala Ala
                   165                 170                 175
   Ala Asp Gly Gly Ala Ala Ala Ser Asn Leu Gln Thr Val Gly Cys Ser
               180                 185                 190
   Val Gln Glu Ser Asp Pro Phe Lys Ile Ile Lys Pro Glu Val His His
           195                 200                 205
   Leu Gly Pro Val Leu Lys Leu Gln Cys Ser Lys Val Glu Asn Ser Gly
       210                 215                 220
   Phe Ile Ser Ser Ser Thr Gly Thr Ala Ala Arg Asp Gln Pro Glu Pro
   225                 230                 235                 240
   Pro Pro Leu Glu Lys Cys Ser Leu Ser Leu Ser Leu Gly Pro Asp Pro
                   245                 250                 255
   Lys Cys Met Pro Ala Ile Ala Ser Ser Pro Ser Glu Ser Ser Cys Ile
               260                 265                 270
   Leu Ser Ser Ser Ser Arg Ser Phe Ser Asp Cys Ser Gly Asn Ser Gly
           275                 280                 285
   Cys Leu Val Ala Pro Gly Val Asn Leu Glu Leu Ser Met Ser Ile Cys
       290                 295                 300
   Gly Ser
   305
<210> 40
   <211> 187
   <212> PRT
   <213> Oryza sativa
<400> 40
   Met Ala Ala Ala Asp Gln Pro Ala Tyr Gly Asp Arg Arg Pro Ser Arg
   1               5                   10                  15
   Arg Thr Tyr Lys Pro Asp Gln Pro Glu Gly Leu Thr Ile Ser Phe Arg
               20                  25                  30
   Glu Leu Tyr Asp Leu Pro Thr Ser Pro Glu Phe Leu Phe His Glu Glu
           35                  40                  45
   Ala Leu Arg Ser Arg Arg Thr Cys Gly Glu Asp Leu Thr Phe Tyr Thr
       50                  55                  60
   Gly Cys Gly Tyr Leu Val Gly Arg Ala Ala Gly Ala Ala Ala Gly Leu
   65                  70                  75                  80
   Lys Arg Ala Ala Glu Glu Ala Glu Arg Gly Glu Ser Met Lys Leu Arg
                   85                  90                  95
   Gly Gln Pro Arg Pro Gln Pro Val Arg Leu Pro Arg Ala Arg Val Arg
               100                 105                 110
   Gln Pro Ala Arg Arg Arg Arg Ala Ala Leu Arg Gly Asp Arg Glu His
           115                 120                 125
   Arg Gly Gly Pro Pro Arg Arg Arg Arg Leu Gly Gln His Arg Arg Arg
       130                 135                 140
   Arg Asp Arg Tyr Arg Arg Ala Leu Pro Arg Gly Cys Arg Pro Ala Gly
   145                 150                 155                 160
   Gly Asp Arg Arg Gln Leu Arg Arg Gly Ala His Gly Arg Arg Gly Gly
                   165                 170                 175
   Arg Gly Glu Ala Ser Ala Asp Glu Ile Arg Ala
               180                 185
<210> 41
   <211> 424
   <212> PRT
   <213> Oryza sativa
<400> 41
   Met Asp Met Pro Pro Thr Pro Leu Pro Pro Glu Thr Ala Asn Thr Ser
   1               5                   10                  15
   Pro Ala Pro Asn Gly Ala Thr Ala Gly Ile Arg Val Glu Asn Cys Tyr
               20                  25                  30
   Val Phe Lys Ser Arg Leu Gln Glu Tyr Ala Gln Lys Thr Gly Leu Gln
           35                  40                  45
   Thr Pro Glu Tyr His Thr Phe Lys Glu Gly Pro Ser His Glu Pro Val
       50                  55                  60
   Phe Lys Ser Thr Val Val Ile Asn Asn Thr Ser Tyr Asp Ser Leu Pro
   65                  70                  75                  80
   Gly Phe Phe Asn Arg Lys Ala Ala Glu Gln Ser Ala Ala Glu Val Ala
                   85                  90                  95
   Leu Met Glu Ile Val Lys Ser Ile Pro Ala Asn Ala Asn Ile Pro Ala
               100                 105                 110
   Val Gln Glu Thr Gly Leu Cys Lys Asn Leu Leu Gln Glu Tyr Ala Gln
           115                 120                 125
   Lys Met Asn Tyr Ala Ile Pro Ser Tyr Ile Cys Thr Lys Ser Ala Ser
       130                 135                 140
   Gly Leu Ala Pro Phe Ile Cys Thr Val Glu Ile Gly Gly Ile Gln Tyr
   145                 150                 155                 160
   Ile Gly Ala Ala Ala Arg Thr Lys Lys Asp Ala Glu Ile Lys Ala Ala
                   165                 170                 175
   Arg Thr Ala Leu Leu Ala Ile Gln Gly Gln Ser Glu Gly Ser Ala Asn
               180                 185                 190
   Gly Ala Thr Lys Tyr Ile Val Val Pro Gly Lys Arg Val Gly Lys Glu
           195                 200                 205
   Val Glu Lys Arg Pro Ile Glu Thr Pro Lys Pro Leu Lys Val Lys Lys
       210                 215                 220
   Gly Gly Phe Lys Lys Lys Trp Asn Lys Arg Lys Phe Met Lys Lys Asp
   225                 230                 235                 240
   Gly Gln Ala Val Asp Val Glu Lys Asp Glu Ala Arg Val Ala Gly Asp
                   245                 250                 255
   Ala His Asp Ser Asp Val Leu Met Gln Pro Thr Val Ile Thr Gln Glu
               260                 265                 270
   Ala Ser Cys Gly Thr Leu Phe Leu Gln Pro Cys Glu Glu Ala Lys Arg
           275                 280                 285
   Val Glu Ala Glu Pro Pro Arg Asp Ile Glu Met Val Gln Pro Asp Lys
       290                 295                 300
   Glu Asn Gln His Ser Asp Ala Ala Leu Val Gln Pro Asp Asp Glu Ala
   305                 310                 315                 320
   Arg Val Glu Gln Glu Pro Ser Arg Asp Ile Ser Val Val Gln Pro Asn
                   325                 330                 335
   Glu Glu Ala Ile Ser Gly Lys Gln Glu Pro Ser Ile Asp Ala Ala Ile
               340                 345                 350
   Leu Gln Pro Lys Glu Glu Ala Ser Ser Val Lys Gln Glu Pro Phe Ile
           355                 360                 365
   Asp Thr Ala Met Leu Gln Ala Cys Lys Glu Ala Gly Ser Val Glu Leu
       370                 375                 380
   Gly Pro Ala Arg Asp Thr Val Ile Ser Gln Leu Asn Glu Gln Asp Arg
   385                 390                 395                 400
   Ala Val Lys Gln Glu Pro Ala Gly Asp Ile Val Val Pro Gln Pro Asp
                   405                 410                 415
   Val His Ala Arg Val Val Lys Glu
               420
<210> 42
   <211> 1359
   <212> PRT
   <213> Oryza sativa
<400> 42
   Met Ala Leu Gly Asp Leu Met Ala Ser Arg Leu Val His Ser Ser Ser
   1               5                   10                  15
   Ser Ser Ala Ala Pro Ser Ala Ala Leu Pro Asn His His Thr Asn His
               20                  25                  30
   Leu Val Asp Asp His Leu Pro Val Glu Asn Gly Pro Asp Pro Arg Arg
           35                  40                  45
   Asp Val Pro Asp Glu Glu Pro Pro Pro Pro Pro Pro Pro Gln Val Ala
       50                  55                  60
   Leu Leu Pro Gln Val Val Val Leu Cys Glu Gln Arg His Glu Gly Phe
   65                  70                  75                  80
   Asp Glu Ala Ala Ala Ala Ala Ala Gly Pro Ser Thr Ser Gly Pro Val
                   85                  90                  95
   Ser Lys Trp Arg Pro Lys Asp Arg Met Lys Thr Gly Cys Val Ala Leu
               100                 105                 110
   Val Leu Cys Leu Asn Ile Ser Val Asp Pro Pro Asp Val Ile Lys Ile
           115                 120                 125
   Ser Pro Cys Ala Arg Lys Glu Cys Trp Ile Asp Pro Phe Ser Met Ala
       130                 135                 140
   Pro Pro Lys Ala Leu Glu Thr Ile Gly Lys Thr Leu His Ser Gln Tyr
   145                 150                 155                 160
   Glu Arg Trp Gln Pro Lys Ala Arg Tyr Lys Leu Gln Leu Asp Pro Thr
                   165                 170                 175
   Leu Glu Glu Val Lys Lys Leu Cys Asn Thr Cys Arg Lys Phe Ala Arg
               180                 185                 190
   Thr Glu Arg Val Leu Phe His Tyr Asn Gly His Gly Val Pro Lys Pro
           195                 200                 205
   Thr Ala Asn Gly Glu Ile Trp Val Phe Asn Lys Ser Tyr Thr Gln Tyr
       210                 215                 220
   Ile Pro Leu Pro Ile Thr Asp Leu Asp Ser Trp Leu Lys Thr Pro Ser
   225                 230                 235                 240
   Ile Tyr Val Phe Asp Cys Ser Ala Ala Gly Met Ile Val Lys Ala Phe
                   245                 250                 255
   Leu Glu Arg Leu Asp Trp Ser Ser Ser Ser Ser Ala Ser Ser Ser Lys
               260                 265                 270
   Asp Cys Ile Leu Leu Ala Ala Cys Glu Ala His Gln Thr Leu Pro Gln
           275                 280                 285
   Ser Ala Glu Phe Pro Ala Asp Val Phe Thr Ala Cys Leu Thr Thr Pro
       290                 295                 300
   Ile Lys Met Ala Leu His Trp Phe Cys Asn Arg Ser Leu Leu Arg Asp
   305                 310                 315                 320
   Ser Met Glu His Asn Leu Ile Asp Gln Ile Pro Gly Arg Gln Asn Asp
                   325                 330                 335
   Arg Lys Thr Leu Leu Gly Glu Leu Asn Trp Ile Phe Thr Ala Ile Thr
               340                 345                 350
   Asp Thr Ile Ala Trp Asn Val Leu Pro His Asp Leu Phe Gln Arg Leu
           355                 360                 365
   Phe Arg Gln Asp Leu Leu Val Ala Ser Leu Phe Arg Asn Phe Leu Leu
       370                 375                 380
   Ala Glu Arg Ile Met Arg Ser Ala Asn Cys Ser Pro Ile Ser Tyr Pro
   385                 390                 395                 400
   Leu Leu Pro Pro Thr His Gln His His Met Trp Asp Ala Trp Asp Met
                   405                 410                 415
   Ala Ala Glu Ile Cys Leu Ser Lys Leu Pro Gln Leu Ile Ala Asp Pro
               420                 425                 430
   Asn Ala Glu Phe Gln Pro Ser Pro Phe Phe Thr Glu Gln Leu Thr Ala
           435                 440                 445
   Phe Glu Val Trp Leu Asp His Gly Ser Glu Asp Lys Lys Pro Pro Glu
       450                 455                 460
   Gln Leu Pro Ile Val Leu Gln Val Leu Leu Ser Gln Ser His Arg Phe
   465 470 475 480
   Arg Ala Leu Val Leu Leu Gly Arg Phe Leu Asp Met Gly Pro Trp Ala
   485 490 495
   Val Asp Leu Ala Leu Ser Val Gly Ile Phe Pro Tyr Val Leu Lys Leu
   500 505 510
   Leu Gln Thr Ser Ala Met Glu Leu Arg Gln Ile Leu Val Phe Ile Trp
   515 520 525
   Thr Lys Ile Leu Ser Leu Asp Lys Ser Cys Gln Val Asp Leu Val Lys
   530 535 540
   Asp Gly Gly His Ala Tyr Phe Ile Arg Phe Leu Asp Ser Leu Asp Ala
   545 550 555 560
   Tyr Pro Glu Gln Arg Ala Met Ala Ala Phe Val Leu Ala Val Ile Val
   565 570 575
   Asp Gly His Arg Ile Gly Gln Glu Ala Cys Ala Asn Ala Gly Leu Ile
   580 585 590
   Asp Val Cys Leu Arg His Leu Gln Pro Glu Asn Pro Asn Asp Ala Gln
   595 600 605
   Thr Glu Pro Leu Leu Leu Gln Trp Leu Cys Leu Cys Leu Gly Lys Leu
   610 615 620
   Trp Glu Asp Phe Pro Glu Ala Gln Leu Leu Gly Leu Gln Ser Asn Ala
   625 630 635 640
   Pro Glu Ile Val Ile Cys Leu Leu Ser Glu Pro Gln Pro Glu Val Arg
   645 650 655
   Ala Ser Ala Val Phe Ala Leu Gly Asn Leu Val Asp Ile Gly Ser Pro
   660 665 670
   Ser Leu Asn Gly Ala Asp Asp Asp Ser Asp Asp Asp Glu Lys Val Arg
   675 680 685
   Ala Glu Ile Asn Val Val Arg Ser Leu Leu Gln Ile Ser Ser Asp Gly
   690 695 700
   Ser Pro Leu Val Arg Ser Glu Val Ala Val Ala Leu Thr Arg Phe Ala
   705 710 715 720
   Met Gly His Asn Lys His Ile Lys Ser Val Ala Ala Glu Tyr Trp Lys
   725 730 735
   Pro Gln Thr Asn Ser Leu Leu Lys Ser Leu Pro Ser Leu Ala Asn Ile
   740 745 750
   Asn Ser Ser Asn Val Tyr Ser Pro Ser Ser Leu Ile Gln Gly Ser Ser
   755 760 765
   Gly Leu Ala Ser His Ile Gly Pro Val Leu Arg Val Gly Ser Asp Asn
   770 775 780
   Ser Ala Thr Ala Arg Asp Gly Arg Ile Ser Thr Ser Ser Pro Ile Ala
   785 790 795 800
   Thr Asn Ser Ile Met His Gly Ser Pro Gln Ser Asp Asp Ser Ser Gln
   805 810 815
   His Ser Asp Ser Gly Ile Leu Leu Arg Glu Asn Ala Ser Asn Gly Gly
   820 825 830
   Leu Asn Tyr Ser Arg Ser Arg Pro Ile Asp Asn Gly Ile Tyr Ser Gln
   835 840 845
   Phe Ile Ala Thr Met Cys Asn Val Ala Lys Asp Pro Tyr Pro Arg Ile
   850 855 860
   Ala Ser Ile Gly Lys Arg Ala Leu Ser Leu Ile Gly Val Glu Gln Val
   865 870 875 880
   Ser Met Arg Asn Ser Arg Leu Ser Asn Gly Gly Ala His Pro Gly Glu
   885 890 895
   Thr Ser Val Pro Pro Ser Ser Asn Phe Gly Met Ala Arg Ser Ser Ser
   900 905 910
   Trp Phe Asp Met Asn Ser Gly Asn Phe Ser Val Ala Phe Arg Thr Pro
   915 920 925
   Pro Val Ser Pro Pro Gln His Asp Tyr Leu Thr Gly Leu Arg Arg Val
   930 935 940
   Cys Ser Met Glu Phe Arg Pro His Val Leu Asn Ser Pro Asp Gly Leu
   945 950 955 960
   Ala Asp Pro Leu Leu Ser Ser Ser Ala Ala Pro Ser Asn Met Gly Leu
   965 970 975
   Tyr Ile Leu Pro Gln Ser Leu Ile Tyr Arg Trp Ser Cys Gly His Phe
   980 985 990
   Ser Arg Pro Leu Leu Thr Gly Ser Asp Asp Asn Glu Glu Ala Asn Ala
   995 1000 1005
   Arg Arg Glu Glu Arg Glu Arg Ile Ala Met Asp Cys Ile Ala Lys
   1010 1015 1020
   Cys Gln Arg Ser Ser Cys Lys Met Thr Ser Gln Ile Ala Ser Trp
   1025 1030 1035
   Asp Thr Arg Phe Glu Leu Gly Thr Lys Ala Ser Leu Leu Leu Pro
   1040 1045 1050
   Phe Ser Pro Ile Val Val Ala Ala Asp Glu Asn Glu Gln Ile Arg
   1055 1060 1065
   Val Trp Asn Tyr Asp Asp Ala Leu Pro Val Asn Thr Phe Glu Asn
   1070 1075 1080
   His Lys Leu Ser Asp Arg Gly Leu Ser Lys Leu Leu Leu Ile Asn
   1085 1090 1095
   Glu Leu Asp Asp Ser Leu Leu Leu Val Gly Ser Ser Asp Gly Asn
   1100 1105 1110
   Val Arg Ile Trp Arg Asn Tyr Thr Gln Lys Gly Gly Gln Lys Leu
   1115 1120 1125
   Val Thr Ala Phe Ser Ser Val Gln Gly Tyr Arg Ser Ala Gly Arg
   1130 1135 1140
   Ser Ile Val Phe Asp Trp Gln Gln Gln Ser Gly Tyr Leu Tyr Ala
   1145 1150 1155
   Ser Gly Asp Met Ser Ser Ile Leu Val Trp Asp Leu Asp Lys Glu
   1160 1165 1170
   Gln Val Asn Thr Ile Gln Ser Thr Ala Asp Ser Gly Ile Ser Ala
   1175 1180 1185
   Leu Ser Ala Ser Gln Val Arg Cys Gly Gln Phe Ala Ala Gly Phe
   1190 1195 1200
   Leu Asp Ala Ser Val Arg Ile Phe Asp Val Arg Thr Pro Asp Arg
   1205 1210 1215
   Leu Val Tyr Thr Ala Arg Pro His Ala Pro Arg Ser Glu Lys Val
   1220 1225 1230
   Val Gly  Ile Gly Phe Gln Pro  Gly Phe Asp Pro Tyr  Lys Ile Val
       1235                 1240                 1245
   Ser Ala  Ser Gln Ala Gly Asp  Ile Gln Phe Leu Asp  Val Arg Arg
       1250                 1255                 1260
   Ala Ser  Glu Pro Tyr Leu Thr  Ile Glu Ala His Arg  Gly Ser Leu
       1265                 1270                 1275
   Thr Ala  Leu Ala Val His Arg  His Ala Pro Val Ile  Ala Ser Gly
       1280                 1285                 1290
   Ser Ala  Lys Gln Met Ile Lys  Val Phe Ser Leu Glu  Gly Glu Gln
       1295                 1300                 1305
   Leu Thr  Ile Ile Arg Tyr Gln  Pro Ser Phe Met Gly  Gln Arg Ile
       1310                 1315                 1320
   Gly Ser  Val Asn Cys Leu Ser  Phe His Arg Tyr Lys  Ser Leu Leu
       1325                 1330                 1335
   Ala Ala  Gly Ala Gly Asp Asn  Ala Leu Val Ser Ile  Tyr Ala Glu
       1340                 1345                 1350
   Asp Asn  Tyr Gln Val Arg
       1355
<210> 43
   <211> 233
   <212> PRT
   <213> Oryza sativa
<400> 43
   Met Gly Ala Ser Gly Arg Leu Ile Ser Ile Tyr Pro Glu Asp Leu Thr
   1               5                   10                  15
   Phe Leu Phe Glu Leu Asp Lys Pro Cys Tyr Cys Asn Leu Lys Val Val
               20                  25                  30
   Asn Asn Ser Glu His His Val Ala Phe Lys Val Lys Thr Thr Ser Pro
           35                  40                  45
   Arg Lys Tyr Phe Val Arg Pro Asn Ala Ser Ile Ile Gln Pro Trp Asp
       50                  55                  60
   Ser Cys Thr Ile Thr Ile Thr Leu Gln Ala Gln Lys Glu Tyr Pro Pro
   65                  70                  75                  80
   Asp Met Gln Cys Lys Asp Lys Phe Leu Ile Gln Ser Thr Lys Val Ala
                   85                  90                  95
   Ala Ser Thr Asp Met Asp Glu Ile Pro Pro Asn Thr Phe Asn Lys Glu
               100                 105                 110
   Val Asp Lys Val Ile Glu Glu Met Lys Leu Lys Val Val Tyr Thr Val
           115                 120                 125
   Pro Ser Gly Ser Ser Asp Asp Ser Gly Ile Thr Ser Leu Gly Ser Arg
       130                 135                 140
   Ser Phe Lys Leu Gly Ser Asp Asp Leu Thr Met Leu Lys Asn Ala Ser
   145                 150                 155                 160
   Ile Glu Lys Ile Gln Thr Ile Gln Arg Leu Lys Asp Glu Arg Asp Thr
                   165                 170                 175
   Thr Leu Gln Gln Asn Gln Gln Met Gln Arg Glu Leu Asp Val Ile Arg
               180                 185                 190
   Arg Arg Arg Ser Arg Lys Ser Asp Ala Gly Phe Ser Leu Thr Phe Ala
           195                 200                 205
   Ala Phe Ala Gly Leu Ile Gly Val Leu Ile Gly Leu Leu Met Ser Leu
       210                 215                 220
   Ile Phe Pro Arg Pro Gln Ala Ala Ala
   225                 230
<210> 44
   <211> 939
   <212> PRT
   <213> Oryza sativa
<400> 44
   Met Gly Val Met Asn Pro Leu Met Ala Lys Leu Thr Thr Leu Met Gly
   1               5                   10                  15
   Asp Glu Tyr Lys Lys Leu Lys Gly Leu Arg Lys Gln Val Ser Phe Leu
               20                  25                  30
   Lys Asp Glu Leu Thr Thr Met Ser Ala Phe Leu Glu Lys Leu Ala Leu
           35                  40                  45
   Met Asp Asp Asp Asp Asp Gly Glu Leu Asp Pro Leu Ala Lys Asp Trp
       50                  55                  60
   Arg Asn His Val Arg Glu Met Ala Tyr Asp Met Glu Asp Cys Ile Asp
   65                  70                  75                  80
   Asp Tyr Phe Thr Ser His Leu Asp His Arg Tyr Ser Ser Ser Asp Ala
   85 90 95
   Gly Leu Ile Arg Lys Ile Ala Arg Arg Leu Arg Ala Leu Arg Val Arg
   100 105 110
   His Arg Ile Ala Ser Gln Ile Asn Glu Leu Lys Ala Arg Val Val Glu
   115 120 125
   Ala Asn Glu Arg Arg Val Arg Tyr Arg Leu Asp Asp Cys Asn Asn Lys
   130 135 140
   His Gly Val Ser Ala Asn Pro Ala Ile Asp Pro Arg Ile Thr Ser Leu
   145 150 155 160
   Tyr Gln Asn Ala Gly Ser Leu Val Gly Ile Asp Gly Pro Ser Gln Glu
   165 170 175
   Leu Ile Gln Leu Leu Ser Leu Asp Arg Asp Thr Asp Gln Arg Gln Leu
   180 185 190
   Lys Val Val Ser Val Val Gly Phe Gly Gly Leu Gly Lys Thr Thr Leu
   195 200 205
   Ala Lys Tyr Val Tyr Asp Lys Ile Gly His Gln Phe Asp Cys Thr Ala
   210 215 220
   Phe Val Ser Val Ser His Lys Pro Asp Ile Thr Arg Ile Leu Ser Ser
   225 230 235 240
   Ile Gln Ser Lys Leu Asp Ile Gly Gly Thr Ser Gln Ala Cys Asp Asp
   245 250 255
   Val Gln Gln Leu Ile Asp Asp Ile Arg Ala Tyr Leu Glu His Glu Arg
   260 265 270
   Tyr Ile Ile Ile Val Asp Asp Leu Trp Lys Gln Glu Ala Trp Val Ile
   275 280 285
   Ile Ser Cys Ala Phe Pro Asn Asn Gly Lys Gly Ser Arg Val Ile Val
   290 295 300
   Thr Thr Arg Val Lys Asp Val Ala Arg Leu Ala Cys Gly Lys Asp Gly
   305 310 315 320
   Gln Ile Tyr Lys Ile Gln Pro Leu Asn Asn Lys Asp Ser Arg Lys Leu
   325 330 335
   Phe Phe Asp Arg Val Phe Arg Pro Glu Asp Ser Cys Val Leu Gln Tyr
               340                 345                 350
   Glu Glu Ile Ser Thr Glu Ile Leu Lys Lys Cys Ser Gly Leu Pro Leu
           355                 360                 365
   Ala Ile Val Thr Val Gly Ser Leu Leu Ala Cys Arg Pro Arg Thr Met
       370                 375                 380
   Glu Glu Trp Lys Ser Ile Arg Asp Ser Leu Gly Ala Pro Phe Asp Lys
   385                 390                 395                 400
   Asn Lys Ser Leu Glu Gly Met Arg Asn Ile Leu Asn Leu Ser Tyr Lys
                   405                 410                 415
   Asn Leu Pro Leu His Leu Lys Thr Cys Leu Leu Tyr Ile Gly Lys Tyr
               420                 425                 430
   Pro Glu Asp Tyr Glu Ile Gly Arg Asp Glu Leu Val Thr Glu Trp Ile
           435                 440                 445
   Ala Glu Gly Ile Met Gly Asn Pro His Gly Glu Asn Leu Glu Ala Thr
       450                 455                 460
   Gly Asn Gly Tyr Phe Ser Glu Leu Ile Asn Arg Gly Leu Ile Gln Pro
   465                 470                 475                 480
   Glu Ser Thr Gly Tyr Gly Gly Glu Val Leu Ser Cys Lys Val His Asp
                   485                 490                 495
   Met Met Leu Asp Leu Ile Leu Ile Lys Cys Ala Glu Asp Asn Phe Val
               500                 505                 510
   Ser Val Ala His Ser Cys Lys Asp Tyr Met Arg Met Ala Met His His
           515                 520                 525
   Glu Arg Ser Cys Asn Lys Val Arg Arg Leu Ser Leu Gln Cys Lys Ala
       530                 535                 540
   Ala Arg Ser Asp Cys Ala Ile Glu Gly Ser Val Ile Ser Thr Ser Met
   545                 550                 555                 560
   Ala Arg Ala Arg Ser Val Ser Val Phe Gly Glu Cys Ser Arg Gly Leu
                   565                 570                 575
   Pro Phe Leu Met Leu Ser Lys Tyr Ile Arg Val Val His Ile Glu Leu
               580                 585                 590
   Glu Gly His Gly Gly Gln Val Asp Leu Thr Ala Ile Ser His Val Leu
           595                 600                 605
   Gln Leu Arg Tyr Leu Arg Val Glu Thr Pro Gly Cys Glu Ile Asp Leu
       610                 615                 620
   Pro Ser Lys Ile Cys Gly Leu Val His Leu Glu Thr Leu Ser Ile Phe
   625                 630                 635                 640
   Ser His Lys Ala Val Ser Arg Leu Pro Ser Asp Ile Ser Ser Leu Pro
                   645                 650                 655
   Arg Leu Ser Val Leu Ser Leu Val Val Pro Trp Ala Thr Arg Leu Pro
               660                 665                 670
   Asn Lys Leu Asn Lys Leu Lys Gly Ser Leu Arg Ser Leu Thr Ile Leu
           675                 680                 685
   Phe Asn Pro Pro Asp Ala Leu Gly Met Glu Ala Ile Gly Glu Leu Lys
       690                 695                 700
   Asn Leu Arg Asp Leu Asn Ile Ser Val Asn Arg Trp Arg Asp Asp Glu
   705                 710                 715                 720
   Ile Leu Ser Leu Tyr Ala Leu Gly Ser Ser Ile Gly Lys Leu Asp Glu
                   725                 730                 735
   Leu Arg Ser Leu Gln Ile His Val Pro Pro Ala Thr Leu Gly Asp Val
               740                 745                 750
   Asp Leu Leu Gly Ser Leu Pro Ile Phe Pro Gln Ser Ile Glu Arg Leu
           755                 760                 765
   Ile Leu His Gly Trp Cys Phe Ser Lys Val Pro Arg Trp Ile Asn Gly
       770                 775                 780
   Thr Leu Arg Asn Leu Gln His Val Leu Leu Glu Val Ser Glu Thr Ser
   785                 790                 795                 800
   Ser Ser Glu Val Asp Leu Leu Gly Glu Leu Pro Ser Leu Ala Asp Leu
                   805                 810                 815
   Glu Leu Arg Val Gly Leu Lys Thr Arg Asp Val Ile Ala Phe Gly Gly
               820                 825                 830
   Thr Arg Ala Ser Leu Phe Pro Ala Leu Leu Lys Leu Lys Leu Arg Val
           835                 840                 845
   Gly Glu His Val Ala Ser Arg Leu Gln Phe Gln Ala Gly Val Met Pro
       850                 855                 860
   Lys Leu Gln Ser Leu His Leu Trp Phe Arg Asn Cys Glu Ser Gly Ile
   865                 870                 875                 880
   His Val Thr Pro Glu Gly Met Gln His Leu Leu Ser Leu Gln Ser Ile
                   885                 890                 895
   Cys Val Glu Ile Tyr Leu Arg Asp Glu Glu Leu Lys Ala Thr Tyr Pro
               900                 905                 910
   Trp Asp Ala Met Glu Arg Ala Phe Arg Glu Ile Thr Gly Ala Asn Pro
           915                 920                 925
   Asn Arg Pro Ser Phe Lys Phe Val Lys Gln Val
       930                 935
<210> 45
   <211> 337
   <212> PRT
   <213> Oryza sativa
<400> 45
   Met Glu Cys Glu Pro Glu Glu Leu Gln Phe Leu Gly Met Val Gly Ile
   1               5                   10                  15
   Tyr Arg Glu Ala Ala Ser Ile Leu Arg Ala His Arg Pro Leu Phe Ala
               20                  25                  30
   Arg Ile Ala Ala Ala Phe Val Leu Pro Leu Ser Leu Leu Phe Leu Leu
           35                  40                  45
   His Ile Ala Ile Ser His Ala Leu Phe Ser His Ile Asp Ser Asp Asp
       50                  55                  60
   Ser Ala Leu Asp Ser Ala Ala Pro Gly Thr Pro Ala Gln Arg Arg Leu
   65                  70                  75                  80
   Leu His Arg Leu Ala Asp Asp Trp Leu Ala Leu Leu Leu Phe Lys Ala
                   85                  90                  95
   Ala Tyr Leu Leu Ala Leu Leu Leu Phe Ser Leu Leu Ser Thr Ala Ala
               100                 105                 110
   Ala Val Phe Ser Val Ala Ser Val Tyr Ser Ala Lys His Asp Ala Leu
           115                 120                 125
   Ser Phe Pro Arg Val Leu Ser Val Val Pro Arg Val Trp Arg Arg Leu
       130                 135                 140
   Ala Ala Thr Phe Leu Ala Ala Phe Leu Leu Leu Phe Ala Tyr His Leu
   145 150 155 160
   Leu Phe Val Ala Val Phe Val Ala Leu Leu Val Ala Ala Asp Ser Gly
   165 170 175
   Ser Gly Leu Ala Ala Leu Leu Ala Phe Leu Leu Ala Leu Ala Tyr Ile
   180 185 190
   Ala Gly Leu Val Tyr Leu Ser Val Val Trp His Leu Ala Ser Val Val
   195 200 205
   Ser Val Leu Glu Asp Tyr Lys Gly Phe Glu Ala Met Arg Lys Ser Lys
   210 215 220
   Ala Leu Ile Gln Gly Lys Leu Trp Thr Ala Ser Ala Ile Phe Phe Val
   225 230 235 240
   Leu Asn Val Val Phe Ile Val Val Glu Val Ala Phe Arg Ala Trp Val
   245 250 255
   Val Arg Gly Ala Thr His Gly Leu Gly Ala Gly Ser Arg Leu Leu Leu
   260 265 270
   Gly Leu Ala Met Leu Ala Ala Leu Cys Ala Val Val Met Leu Ala Leu
   275 280 285
   Val Val Gln Thr Val Val Tyr Leu Val Cys Lys Ser Tyr His His Glu
   290 295 300
   Ser Ile Asp Lys Ser Asn Leu Ser Asp His Leu Glu Val Tyr Leu Gly
   305 310 315 320
   Glu Tyr Val Pro Leu Lys Ala Ser Asp Val Gln Met Glu Gln Phe Asn
   325 330 335
   Leu
<210> 46
   <211> 311
   <212> PRT
   <213> Oryza sativa
<400> 46
   Met Ala Ser Ser Ser Ala Leu Ala Ser Ser Pro Phe Leu Pro Pro Leu
   1               5                   10                  15
   Ser Thr Pro Asn Pro Arg Ala Leu Ser Leu Arg Leu Pro Ala Arg Arg
               20                  25                  30
   Leu Pro Val Ala Ser Ser Ala Ala Pro Ser Gly Ala Ala Ala Ala Ala
           35                  40                  45
   Ser Ala Arg Glu Arg Arg Arg Phe Leu Glu Arg Tyr Gly Leu Asn Pro
       50                  55                  60
   Asp Asp Phe Glu Asp Asp Ala Glu Ala Glu Pro Arg Glu Glu Arg Arg
   65                  70                  75                  80
   Arg Asp Arg Arg Asn Arg Arg Ser Gly Arg Gly Glu Ala Glu Asp Ala
                   85                  90                  95
   Pro Ala Lys Ala Ala Ala Glu Pro Arg Glu Thr His Lys Met Leu Gln
               100                 105                 110
   Val Leu Gly Gly Lys Val Arg Arg Arg Lys Leu Leu Ser Pro Lys Asp
           115                 120                 125
   Arg Asn Val Arg Pro Met Met Glu Val Val Arg Gly Ala Ala Phe Asp
       130                 135                 140
   Ile Leu Gln Ser Ala Gly Gly Phe Pro Ala Ser Leu Arg Pro Gly Arg
   145                 150                 155                 160
   Trp Leu Asp Leu Tyr Ser Gly Thr Gly Ser Val Gly Ile Glu Ala Met
                   165                 170                 175
   Ser Arg Gly Cys Ser Glu Ala His Phe Val Glu Met Asp Pro Trp Val
               180                 185                 190
   Val Ser Glu Val Leu Lys Pro Asn Leu Glu Cys Thr Gly Phe Leu Asp
           195                 200                 205
   Val Ser His Ile His Met Ile Arg Val Glu Asn Phe Leu Ala Asn Ala
       210                 215                 220
   Glu Lys Ser Ser Gly Lys Tyr Pro Ser Phe Asp Tyr Ile Ser Val Thr
   225                 230                 235                 240
   Pro Pro Tyr Leu Glu Val Asn Tyr Ser Thr Leu Leu Asp Gln Leu Ala
                   245                 250                 255
   Arg Ser Pro Leu Val Gly Glu Asp Cys Phe Ile Leu Val Glu Tyr Pro
               260                 265                 270
   Leu Lys Thr Asp Met Ala Glu Ser Cys Gly Ser Leu Ile Lys Val Ala
           275                 280                 285
   Asp Arg Arg Phe Gly Arg Thr Asn Leu Leu Ile Tyr Gly Pro Thr Trp
   290 295 300
   Ala Glu Lys Lys Arg Arg Ser
   305 310
<210> 47
   <211> 311
   <212> PRT
   <213> Oryza sativa
<400> 47
   Met Asn Asp Leu Met Thr Lys Ser Phe Met Ser Tyr Val Asp Leu Lys
   1 5 10 15
   Lys Ala Ala Met Lys Asp Leu Glu Ala Gly Gly Asp Gly Val Glu Leu
   20 25 30
   Pro Glu Val Gly Val Thr Asp Glu Arg Leu Lys Gly Phe Phe Gln Glu
   35 40 45
   Thr Glu Ala Val Glu Glu Glu Met Ala Ala Ile Arg Asp Ala Leu Ala
   50 55 60
   Arg Leu Asn Ala Ala Asn Glu Glu Gly Lys Ser Leu His Gln Pro Asp
   65 70 75 80
   Ala Leu Arg Ala Leu Arg Gly Arg Val Asn Ala Asp Ile Ile Ala Val
   85 90 95
   Leu Arg Arg Ala Arg Asp Ile Arg Ala Arg Leu Glu Ala Met Asp Arg
   100 105 110
   Ala Asn Ala Ala Gln Arg Arg Leu Ser Ala Gly Cys Arg Glu Gly Thr
   115 120 125
   Pro Leu Asp Arg Thr Arg Thr Ala Leu Thr Ala Ala Leu Arg Lys Lys
   130 135 140
   Leu Lys Asp Leu Met Leu Asp Phe Gln Ala Leu Arg Gln Arg Ile Met
   145 150 155 160
   Ser Glu Tyr Lys Asp Thr Val Glu Arg Arg Tyr Tyr Thr Leu Thr Gly
   165 170 175
   Glu Val Pro Glu Glu Glu Val Ile Glu Arg Ile Ile Ser Glu Gly Arg
   180 185 190
   Ser Glu Glu Leu Leu Cys Ala Ala Val Ala Glu His Gly Lys Gly Ala
   195 200 205
   Val Leu Ala Thr Val His Glu Ile Gln Asp Arg His Asp Ala Ala Arg
   210 215 220
   Glu Val Glu Arg Ser Leu Leu Glu Leu His Gln Val Phe Leu Asp Met
   225 230 235 240
   Ala Val Val Val Glu Ser Gln Gly Glu Gln Leu Asp Asp Ile Glu Arg
   245 250 255
   His Val Asn Ser Ala Thr Thr Tyr Val Gln Gly Gly Asn Lys Glu Leu
   260 265 270
   Arg Lys Ala Arg Glu His Gln Arg Ser Ser Arg Lys Trp Leu Cys Ile
   275 280 285
   Gly Ile Ile Ile Leu Leu Leu Leu Val Leu Leu Val Ile Val Pro Ile
   290 295 300
   Ala Thr Ser Phe Lys Arg Ser
   305 310
<210> 48
   <211> 187
   <212> PRT
   <213> Oryza sativa
<400> 48
   Met Ala Met Glu Gly Lys Ser Arg Arg Phe Ala Val Ala Cys Gly Val
   1               5                   10                  15
   Leu Ser Gln Tyr Val Arg Ala Glu Gln Lys Met Ala Ala Ala Ala Gly
               20                  25                  30
   Ala Ala Pro Ala Arg Ala Val Thr Thr Leu Ser Leu Met Pro Gly Ala
           35                  40                  45
   Glu Val Val Val Glu Glu Glu Glu Arg Arg Glu Val Gly Glu Glu Glu
       50                  55                  60
   Ala Gly Pro Ala Thr Ala Pro Ala Ala Pro Leu Thr Ile Phe Tyr Gly
   65                  70                  75                  80
   Gly Arg Met Val Val Phe Glu Asp Phe Pro Ala Asp Lys Ala Ala Glu
                   85                  90                  95
   Val Met Arg Met Ala Ser Ser Gly Met Ala Ala Ala Pro Ala Gln Arg
               100                 105                 110
   Glu Gly Ala Ala Leu Ala Asp Met Pro Ile Met Arg Lys Ala Ser Leu
           115                 120                 125
   Gln Arg Phe Phe Ala Lys Arg Lys Asp Arg Leu Ala Ala Thr Thr Pro
       130                 135                 140
   Tyr Ala Arg Pro Ser Pro Ala Glu Thr Lys Ala Ser Glu Pro Glu Glu
   145                 150                 155                 160
   Lys Lys Thr Pro Thr Ser Trp Leu Asp Leu Ala Ala Ser Ala Ser Ala
                   165                 170                 175
   Ala Ala Arg Arg Asp Ser Leu Thr Ile Ala Leu
               180                 185
<210> 49
   <211> 1133
   <212> PRT
   <213> Oryza sativa
<400> 49
   Met Ser Ser Leu Ser Arg Glu Leu Val Phe Leu Ile Leu Gln Phe Leu
   1               5                   10                  15
   Asp Glu Glu Lys Phe Lys Glu Thr Val His Lys Leu Glu Gln Glu Ser
               20                  25                  30
   Gly Phe Tyr Phe Asn Met Lys Tyr Phe Glu Asp Glu Val Ile Asn Gly
           35                  40                  45
   Asn Trp Asp Glu Val Glu Arg Tyr Leu Gly Gly Phe Thr Lys Val Asp
       50                  55                  60
   Asp Asn Arg Tyr Ser Met Lys Ile Phe Phe Glu Ile Arg Lys Gln Lys
   65                  70                  75                  80
   Tyr Leu Glu Ala Leu Asp Lys His Asp Arg Ser Lys Ala Val Glu Ile
                   85                  90                  95
   Leu Val Lys Asp Leu Lys Val Phe Ala Ser Phe Asn Glu Glu Leu Phe
               100                 105                 110
   Lys Glu Ile Thr Gln Leu Leu Thr Leu Glu Asn Phe Arg Glu Asn Glu
           115                 120                 125
   Gln Leu Ser Lys Tyr Gly Asp Thr Lys Ser Ala Arg Ala Ile Met Leu
       130                 135                 140
   Val Glu Leu Lys Lys Leu Ile Glu Ala Asn Pro Leu Phe Arg Asp Lys
   145 150 155 160
   Leu Gln Phe Pro Asn Leu Lys Ser Ser Arg Leu Arg Thr Leu Ile Asn
   165 170 175
   Gln Ser Leu Asn Trp Gln His Gln Leu Cys Lys Asn Pro Arg Pro Asn
   180 185 190
   Pro Asp Ile Lys Thr Leu Phe Val Asp His Ser Cys Gly Gln Pro Asn
   195 200 205
   Gly Ala Arg Ala Pro Ser Pro Ala Asn Asn Pro Leu Leu Gly Ser Ile
   210 215 220
   Pro Lys Pro Gly Gly Phe Pro Pro Leu Gly Ala His Ala Pro Phe Gln
   225 230 235 240
   Pro Ala Pro Thr Pro Val Pro Pro Leu Ala Gly Trp Met Ser Asn Pro
   245 250 255
   Pro Ala Val Thr His Pro Ala Val Ser Gly Gly Ala Ile Gly Phe Gly
   260 265 270
   Thr Pro Thr Asn Pro Ala Ala Ile Leu Lys His Pro Arg Thr Pro Thr
   275 280 285
   Thr Ala Asn Pro Ser Met Asp Tyr Pro Ser Gly Asp Ser Asp His Val
   290 295 300
   Ser Lys Arg Thr Arg Pro Val Gly Met Ser Glu Glu Val Asn Leu Pro
   305 310 315 320
   Val Asn Met Leu Pro Val Thr Tyr Pro Gln Ser His Ser Tyr Pro Gln
   325 330 335
   Asp Asp Phe His Lys Asn Val Ala Arg Thr Leu Ser Gln Gly Ser Thr
   340 345 350
   Pro Met Ser Met Asp Phe His Pro Val Gln Gln Thr Leu Leu Leu Val
   355 360 365
   Gly Thr Asn Val Gly Asp Ile Gly Leu Trp Asp Val Gly Thr Lys Glu
   370 375 380
   Arg Leu Val Leu Arg Asn Phe Lys Val Trp Asp Leu Thr Lys Cys Ser
   385 390 395 400
   Met Ala Leu Gln Ala Ser Leu Val Lys Asp Pro Thr Val Ser Val Asn
                   405                 410                 415
   Arg Ile Ile Trp Ser Pro Asp Gly Thr Leu Phe Gly Val Ala Tyr Ser
               420                 425                 430
   Arg His Ile Val Gln Ile Tyr Ser Tyr His Gly Gly Asp Asp Ile Arg
           435                 440                 445
   Gln His Leu Glu Ile Asp Ala His Val Gly Gly Val Asn Asp Ile Ala
       450                 455                 460
   Phe Ala His Pro Asn Lys Gln Leu Cys Ile Ile Thr Cys Gly Asp Asp
   465                 470                 475                 480
   Lys Thr Ile Lys Val Trp Glu Ala Thr Ser Gly Ala Lys Gln Phe Thr
                   485                 490                 495
   Phe Glu Gly His Glu Ala Pro Val Tyr Ser Val Cys Pro His Tyr Lys
               500                 505                 510
   Glu Asn Ile Gln Phe Ile Phe Ser Thr Ala Leu Asp Gly Lys Ile Lys
           515                 520                 525
   Ala Trp Leu Tyr Asp Asn Leu Gly Ser Arg Val Asp Tyr Asp Ala Pro
       530                 535                 540
   Gly His Trp Cys Thr Thr Met Ala Tyr Ser Ala Asp Gly Ser Arg Leu
   545                 550                 555                 560
   Phe Ser Cys Gly Thr Ser Lys Asp Gly Glu Ser His Leu Val Glu Trp
                   565                 570                 575
   Asn Glu Ser Glu Gly Ala Val Lys Arg Thr Tyr Gln Gly Phe Arg Lys
               580                 585                 590
   Arg Ser Met Gly Val Val Gln Phe Asp Thr Thr Arg Asn Arg Phe Leu
           595                 600                 605
   Ala Ala Gly Asp Glu Phe Leu Ile Lys Ile Trp Asp Met Asp Asn Thr
       610                 615                 620
   Ser Leu Leu Thr Thr Ile Asp Ala Asp Gly Gly Leu Pro Ala Ser Pro
   625                 630                 635                 640
   Arg Val Arg Phe Asn Lys Glu Gly Thr Leu Leu Ala Val Ser Thr His
                   645                 650                 655
   Glu Asn Gly Ile Lys Ile Leu Ala Asn Ala Asp Gly Val Arg Leu Leu
               660                 665                 670
   Arg Thr Leu Glu Asn Arg Ser Phe Asp Ala Ser Arg Ser Ala Ser Glu
   675 680 685
   Thr Val Thr Lys Pro Leu Met Asn Pro Leu Thr Ala Ala Ala Ala Ala
   690 695 700
   Ala Ala Ser Ala Ala Ala Ala Gly Thr Ser Ser Gly Asn Ala Ala Pro
   705 710 715 720
   Pro Ala Ile Thr Ala Leu Asn Gly Asp Ser Arg Ser Leu Val Asp Val
   725 730 735
   Lys Pro Arg Ile Ala Asp Glu Pro Leu Asp Lys Ser Lys Val Trp Lys
   740 745 750
   Leu Met Glu Ile Thr Glu Ser Ser Gln Cys Arg Ser Leu Lys Leu Thr
   755 760 765
   Asp Asn Met Arg Thr Ser Lys Ile Ser Arg Leu Ile Tyr Thr Asn Ser
   770 775 780
   Gly Val Ala Ile Leu Ala Leu Ala Ser Asn Ala Val His Leu Leu Trp
   785 790 795 800
   Lys Trp Pro Arg Asn Asp Arg Asn Ser Ser Gly Lys Ala Thr Ala Ser
   805 810 815
   Val Ser Pro Gln Leu Trp Gln Pro Pro Ser Gly Ile Leu Met Thr Asn
   820 825 830
   Asp Ile Thr Asp Asn Pro Glu Glu Ala Val His Cys Phe Ala Leu Ser
   835 840 845
   Lys Asn Asp Ser Tyr Val Met Ser Ala Ser Gly Gly Lys Ile Ser Leu
   850 855 860
   Phe Asn Met Met Thr Phe Lys Thr Met Thr Thr Phe Met Pro Pro Pro
   865 870 875 880
   Pro Ala Ala Thr Phe Leu Ala Phe His Pro Gln Asp Asn Asn Ile Ile
   885 890 895
   Ala Ile Gly Met Asp Asp Ser Thr Ile Gln Ile Tyr Asn Val Arg Ile
   900 905 910
   Asp Glu Val Lys Ser Lys Leu Arg Gly His Ser Lys Lys Ile Thr Gly
   915 920 925
   Leu Ala Phe Ser Asn Val Leu Asn Val Leu Val Ser Ser Gly Ala Asp
   930 935 940
   Ala Gln Ile Cys Val Trp Ser Thr Asp Gly Trp Asp Lys Leu Lys Ser
   945 950 955 960
   Arg Met Leu Gln Ile Pro Ser Ser Arg Pro Ser Ser Ile Ile Leu Asp
   965 970 975
   Thr Arg Val Gln Phe His Gln Asp Gln Leu His Phe Leu Val Val His
   980 985 990
   Glu Thr Gln Ile Ala Ile Tyr Glu Thr Thr Lys Leu Glu Pro Val Lys
   995 1000 1005
   Gln Trp Pro Val Arg Glu Asn Ser Ser Pro Ile Thr His Ala Met
   1010 1015 1020
   Phe Ser Cys Asp Ser Gln Leu Ile Tyr Ala Ser Phe Leu Asp Ala
   1025 1030 1035
   Thr Val Cys Ile Phe Asn Ala Ser Ser Leu Arg Leu Gln Cys Arg
   1040 1045 1050
   Ile Leu Pro Ala Ser Tyr Leu Pro Gln Asn Ile Ser Ser Asn Val
   1055 1060 1065
   Tyr Pro Val Val Val Ala Ala His Pro Ser Glu Ala Asn Gln Phe
   1070 1075 1080
   Ala Leu Gly Leu Thr Asp Gly Gly Val Tyr Val Leu Glu Pro Leu
   1085 1090 1095
   Glu Ser Glu Arg Lys Trp Gly Asn Pro Pro Pro Ala Glu Asn Gly
   1100 1105 1110
   Ser Thr Ser Ala Leu Ser Thr Pro Pro Asn Gly Ala Ser Ser Ser
   1115 1120 1125
   Asp Gln Pro Glu Arg
   1130
<210> 50
   <211> 2511
   <212> DNA
   <213> Oryza sativa
<400> 50
   atgtctcacc cccacgccac cgcccccaag cgccccggcc acttctcctc ctcctccgcc 60
   gccgcctcct ccccgacctc ccccgcgcag ccgcacatga agaaggccaa gttccccggc 120
   tcctcctcct cctcctcctc cgccgccgcc cccggggtca ccgagaagaa cgggctccac 180
   gtcgatccca cggccgccgc cgcccggacc ggtgggcgca ccaacggcga ggaggatgcg 240
   gagatggtgc tcgccgacca ggaggagctc gccgctccga gcgcatcggc cccggcgggg 300
   gtcgccgcca acctcttccg gaagaaggcc acactccccc agccatccgc cgcccgcaag 360
   cccctccgaa tcaaaatagg tcagccaaaa ttgccaacaa actttgagga ggatacatgg 420
   gctattttga aagatgctat tacagctata tttctaaaac agaaactttc gtgcgatgtt 480
   gaaaaacttt accaggctgc aggtgacctt tgtctacaca agctaggcgc aaatctatac 540
   gaacgcataa agaaagaatg tgaagtacac atatcggcaa aaatatcagc attagtgggt 600
   caaagtccag atttagtagt atttttgtct ctggtgcaaa gaacatggca agatttttgc 660
   gatcagatgt tgattattcg tggtattgct ttacttcttg atgtaaaata tgtcaagaat 720
   gttgcaaaca tttgttcagt gtgggacatg gggttgaagc tattccgcaa gcatctttca 780
   ctgtctccgg agattgaaca caaaactgtt actggtcttc taagattaat tgagagtgag 840
   aggcttggtg aagcaataga caggacatta cttagtcatc ttctgaagat gtttactgct 900
   cttggaatgt attctgagag ttttgaaaag ccctttctgg agtgtacatc tgaattttat 960
   gctactgaag gtgttaaata tttgcagcag tctgatattc cagactatct caagcatgtg 1020
   gagacaaggt tgcaagaaga acatgaaagg tgtattctat atttggaagc taacactagg 1080
   aagccgctta taacagctac agaaaagcaa ttattgcagc ggcacacatc tgcaattctt 1140
   gagaagggat tcacaatgct tatggaagca aatcgtgtaa aagacctctc gaggatgtac 1200
   acactcttcc agagggttga tgccattgag ttgctaaagc aagcacttag ttcatatatt 1260
   cggggcacag gccagggcat tatcatggat gaagaaaagg acaaagaact ggtgcccttt 1320
   cttctggaat ttaaggcatc gcttgataga atattggagg aaagttttgc caaaaatgag 1380
   gctttctcca atacaataaa agagtcattc gaacatctta tcaatttacg ccagaatcga 1440
   cctgctgaat tgattgcgaa gtttcttgat gagaaacttc gagctggaaa taaaggtacc 1500
   tccgaagaag agctggaggg aatattggat aaagttttgg ttctgttccg atttatacaa 1560
   ggaaaagatg tatttgaggc attctacaag aaggatctgg ctaagaggtt gctgctgggg 1620
   aagagtgcat cgatagatgc tgaaaaatca atgataacaa agctcaaaac tgagtgtgga 1680
   agtcaattta ccaacaagct ggagggaatg ttcaaggaca ttgaattatc caaagaaata 1740
   aatgagtctt tcaagcaatc atctcaagca aggacaaagc ttccatctgg cattgaaatg 1800
   agtgttcacg tgcttacaac aggctattgg ccaacatatc caccaatgga tgtgaaactc 1860
   ccccatgaac ttaatgtcta tcaggatata tttaaagaat tctatttgag caagtatagt 1920
   ggaaggcgtt tgatgtggca aaactcattg ggtcactgtg tattaaaagc agagttccca 1980
   aaaggtaaaa aggaacttgc ggtgtcacta tttcagagtg tggttttgat gttgttcaat 2040
   gatgcacaaa aactaagctt cctcgatatc aaggaatcga ctggtattga ggataaagaa 2100
   ttgcgaagaa cgctgcaatc acttgcatgc ggtaaagttc gggttctcca aaagatgcca 2160
   aaagggcgag acgtagaaga taaggacgaa tttgtattta atgaagaatt tagtgcccct 2220
   ctctatcgca taaaggtgaa tgctattcag atgaaggaga cggttgaaga aaacacaagc 2280
   acaactgaga gagtattcca ggacagacag tatcaggtgg atgctgccat agttcgaata 2340
   atgaagacac gtaaaaccct cagccacacg cttctaataa ctgagctttt tcagcagctc 2400
   aagttcccaa tcaagccatc ggatatcaag aaaagaatag agagcctaat cgacagggag 2460
   tacctggaga gagacaggag taacccccag atctacaatt acctggcttg a 2511
<210> 51
   <211> 1686
   <212> DNA
   <213> Oryza sativa
<400> 51
   atggacgccg atgaggccgc ggggagtagc aggaggatgg atctgaacct ctaccttggc      60
   ctcccacgcg ccccgcgccc gcgccgctcc gacctcggct ccgacctcgc cctcagcacc     120
   ccgatgccct cctccccgtc ctcctccgca gcctccgtcg acgcgccgcc gccaccgccc     180
   gagctgtcgc atcccccgta ctccccctct cacgccgacc tttcccctcc gctgcaggag     240
   gtctactccc tgtacaaccc cgacgacccg cctgcttccg agacgcacct gccgccgtat     300
   gcgccgcctc cggctccggt ggtctcggag ctccctgacg acctcgagtt tggcctccac     360
   cccccgccgc cgctggtgcg tgccagcgaa ctgctaggtt gggaggaccg gccgtcttcg     420
   tcgacggcat cgtcctcttt cctccctgac accgcagccc gttactggcg gcttctcgag     480
   cagactggaa gcagatggct ccgtgcgagg cggtttaggt cggaccttcc gccactcagt     540
   tctgaagctt acccagctgg gcgtgatgct gccgcagtcc cagtgctgca gcatgaaccg     600
   atgaatgata ctgttgaaca taataaggta gctgccgatg gcgcggaagt aggcgcctcc     660
   gaggaatcgg aggagcaggg caggagcgct gccacatttg agtgtaatat atgcttcgat     720
   atggccagcg agccggtggt cacctcttgt ggccatctct tctgctggcc ttgcttgtac     780
   caatggctca atgtttattc caatcacaag gaatgcccag tctgcaaagg cgaggtgact     840
   gaggcgaata ttactccgat ctatgggaga gggaattcat gtttggatgc cgagaaggct     900
   gtggaaggtg ggaaacaaac aggtcctact atcccaccaa gaccacatgg aaatcggctc     960
   gaaagcttca ggcagcagtt tcaccatttg cgaccgatct caagaaggct tggtgaggct    1020
   catgggttat tgtcatcatg gaggcgcctt ctggaccaac agattatgaa tactgcgagt    1080
   aggtttgaag gtccgcctga atcagctgtg caggaaatgg ttgacactgc tcacgctcag    1140
   cacaccagtc gcctaagtag attggcgtca aggatgagag caagacggtt gctgagagaa    1200
   gcagacaacc ctaaccctcc cgatggcgga tccacttccc ctgacagtgg tttgatcaga    1260
   aacaatgcat cggatccatc cagaaatggt ccgagctcat tattaccaga tggaattgac    1320
   tggttgcgtg gacttaccct tcttgggtat gaagacacgg aaagatttgc atctgccatg    1380
   agtgatttta gaaggataac tggaccaagc caatatggtg catcggcttc atcatcgaat    1440
   cctccaaatc tcgagtcaac atttgacaga actcatgttg ttgcagcacc ttctgcagac    1500
   caagcatcta actcaagcac tgctgcagtg atacaggggg atgctggtat ctctgagagt    1560
   gcaggagaac caagtaacgc ggggtcatca agatccctga ggaggagagg gaggagcagt    1620
   gccctgggtt ctttggatgc tgatggcggg ggcctccaac ggaacaagag gcgaaggata    1680
   aactga                                                               1686
<210> 52
   <211> 522
   <212> DNA
   <213> Oryza sativa
<400> 52
   atggctggtg ggagctgcga cgtgtgcaag gaggcgccgt ccaagtacaa gtgctccgct      60
   tgccgcacgc catattgctc ggtggcatgc tttaaaaatc acaaagataa attttgccag     120
   aagacaatac ctctggaaga agttagcaag tcatctcttc aggaggaaat ttcaaggaac     180
   tctaggtcac tggaagaagc aacaaattgt cctaatgaca aggatcaaac cccgtcttta     240
   ttatcggaca cgacttgtcc cacacaatat ccaaacacat tgcactctgc aaaatctctt     300
   gaagttgagg atccaagctg gcttgttgac aagaatggat taagatcttt agcggaatct     360
   aatgagatcc gagatgctct gaaagattgt aagcttcagc aaatgctact taagattgat     420
   ggctctgcag agccagaaaa ggaattagag aaattgatgg aaggacaagt ttttcaacag     480
   ttcaccaata agattcttga cattgttagc ccacaacaat ga                        522
<210> 53
   <211> 1560
   <212> DNA
   <213> Oryza sativa
<400> 53
   atgctgggag aggccgcctc accgtggagc ctggccggcg cgggcgcggc cgtggcgctg      60
   ctgtggctgt gcgcctggac gctgcagtgg gcgtggtgga cgccgcggcg gctggagcgg     120
   gccctgcggg cgcagggcct ccggggcacc aggtaccgcc tcttcatcgg cgacgtggcc     180
   gagaacggcc ggctcaacag agaggccgcg tcaaggccgc tgccgctcgg ctcgcacgac     240
   gtcgtcccgc gcgtcatgcc cttcttctgc aacgtcctga aagagcacgg gaaactgtcg     300
   ttcgtttgga ctggcccaaa gccattcgtg attatcagag accctgactt agcgagggag     360
   attttgtcca acaagtctgg caatttcgcc aagcaaacga ccgcgggtat tgctaagttc     420
   gtagttggcg gagttgtaac gtatgaaggt gagaaatggg caaaacatcg gagaattctc     480
   aaccctgcct tccaccagga gaaaataaag cggatgctgc cagtgttttt agcatgttgc 540
   accaaaatga tcactagatg ggtgaattca atgtcttcag aaggaatatc tgagttagac 600
   gtttgggatg aatttcaaaa tcttactgga gatgtcatct caagaacggc atttgggagc 660
   agctaccagg aggggtggag aatttttcag ttacaagaag agcaagctaa acgcgtactt 720
   aaagcttttc agagaatctt tatcccaggc tactggtact taccaatcga aaacaacaga 780
   aggatcaggg aaattgatca agaaatccgc acaattctgc gaggaataat agtaaaaaga 840
   gacaaggcag ttagaaatgg tgaaggtagc aatgatgatt tgttgggatt attggtggaa 900
   tcgaatatga ggcaatcaaa tgaaaaagaa gatgtgggaa tgagtataga agatatgatt 960
   gaggaatgca agttatttta cgctgctggt tcggagacaa catcaatgtt gctcacttgg 1020
   actttaattc tgctaagcat gcaccctgaa tggcaagagc aggcaagaga ggaagtgatg 1080
   caccattttg gaagaaccac accagatcat gatggcttga gtcgtctaaa gattgtaacg 1140
   atgattctcc acgaggttct taggttgtac ccaccggtgg tattcctcca aagaacaaca 1200
   cacaaggaaa tagagcttgg tggcatcaaa taccctgaag gagtgaactt cacattgcct 1260
   gttctatcca ttcaccacga tcctagcatc tggggacaag atgcaatcaa attcaacccg 1320
   gaaaggttcg ccaacggagt ctccaaggca acgaagtttc agaccgcgtt cttttcgttt 1380
   gcatggggtc ctcggatctg ccttggccag agctttgcaa ttctggaagc caagatggcg 1440
   ctcgccacca tcctccagag cttctccttc gagctctcgc cgtcatacac ccacgcacca 1500
   cacaccgtgc taactctcca accacagtac ggttctccaa ttaaattgaa gaagctctag 1560
<210> 54
   <211> 444
   <212> DNA
   <213> Oryza sativa
<400> 54
   atggctaaag atcacgtgaa gattgtgtta aaagcttaca ttttaggacc tataaagtat 60
   attttaagtt tagaatcttt gtaccataat tgtggtggac tggtggtaac aatgattctc 120
   cacgaagtta ttaggttata cccatcgggg atcttcctcc aaagaacaac acgcaaggaa 180
   atagagcttg gtggcatcaa ataccctgaa ggagcaaact tcacattgcc cgttccatct 240
   atccaccatg atcccagcat ctggggagga gatgcaagcg agttcaacct ggagaggttt 300
   gccaacggag tctccaaggc aacgaagttt aagaccgcat tctttatgtt tggatggggg 360
   ttctcggatc tgccttggac agaactttgc aatgctggaa gccaagatgg cgctcgccac 420
   catcctccag agcttctcct ttga 444
<210> 55
   <211> 219
   <212> DNA
   <213> Oryza sativa
<210> 56
   <211> 753
   <212> DNA
   <213> Oryza sativa
<400> 56
   atgtcctcta gcgatcagaa cccatcgcca acaccggcgt ccggcaccgg cacgtccgtg 60
   ccgccgccag gcagggcgac gacggtatcc tcgcagctcc tggacatggg cgcgcaagcg 120
   gtgcaggcgc tgaagcccgt gcgccagatg aagcagcacg cgtgcagctt cgcgctgtac 180
   gctcacgacc tgagccgcca ggtcgaggtc caccacttcg tctcccgcct caaccaggac 240
   gtcctccagt gcgccgtcta cgactccgac aagccctcgg cccgcctcat cggcgtggag 300
   tacatcgtgt cggacgccat cttcgagagc ctgcctccgg aggagcagaa gctgtggcac 360
   tcgcacgcgt acgaggtgaa ggccgggctg tggaccgacg tcggcgtgcc ggagccgctg 420
   cagagctcgg agatggcgag gatggccaag acgtacggca agctctggtg cacctggcag 480
   gtggaccgcg gcgacgcgct gcccctgggc gcgccggcgc tcatggtgtc gccgcaggcc 540
   gtggagcccg ggcgggtgcg cgccgagctc gtgcacggcc gcgacgagag gtacaagatc 600
   gacagctcgg cgcaggggct gaagggggcc agggttgaga tggacgagcc ggagtggatc 660
   aacccgaacg ccgactactg gcgcctacac ggcaaggggt tcgccatcga cgtcaccgcc 720
   accgagatga agcgccacgc gcccttcccg tga 753
<210> 57
   <211> 1440
   <212> DNA
   <213> Oryza sativa
<400> 57
   atgacgcctc caccgccgtc gccgccgcac gagaggaaaa cgtgggcgga gtcggtggcc      60
   agcgagtttc gggcgcagcg cggcatcgcg ttccctctca tcgccatgaa cctcacctgg     120
   ttcgccaagc tggccgtcac caccgccttc ctcggccgcc tcggcgacct ccagctcgcc     180
   gccggcaccc tcggcttcag cttcgccaat gtcaccggct tcgccgtcct caccggcctc     240
   tgcgccgcca tggaccccat ctgcgggcag gcgcacggcg ccagcaacgg gaagctcctc     300
   cgcaagacgc tggtgatggc caccatcctg ctgctgggcg cgtccatccc catcgccttc     360
   ctgtggctgc acgtggacgc cgtcctcctc cggttcggac agcaggcgga catgagcagc     420
   aacgcacgca gctacgtggt gtgcctcctc ccggacctcg ccgtcacctc cttcgtcaac     480
   ccgctcaagt cgtacctgag cgcgcagggg gtgacgctcc ccacgctgtt cgcctccgcc     540
   ctggccctgg cgctccacgt ccccctcacc atgtggatgg ccaggaccag gggcatccag      600
   ggcgtcgcca ccgccgtgtg ggtcagcgac ctggccgtgg ccgtcatgct cgccggctac     660
   gtgctcgtct cggagcgacg acggaaggcg ggagggggcg gcggatgggt ggagcagacg     720
   aggggtgagt gggtccggct cctccggctg gccgttccca gctgcctcaa cacctgcctg     780
   gagtggtggt gctacgagat actggtgctc ctgacgggac gcctcccgga cgcccggcgc     840
   acggtggcgg tgatggccgt gacgctcaac ttcgactacc tgctgttcgc ggggatgctg     900
   tccctgtcgg tgagcgcgtc ggtgcgcgtg tcgaacgagc tgggcgcggg ggaggcgtgg     960
   gcggcgaggc gcgcgggcat ggtgtcgatc gtgggcggcg cggtgggcgg ggtgggcggc    1020
   ggggtggcga tggtggcggc gcggcgggcg tgggggagca tatacagctc agacgccggg    1080
   gtgcgggagg gggtggggag ggcgatggag gtgatggcgg tgctggaggt ggtgaacttc    1140
   ccgctgaacg tgtgcggggg gatagtgcga gggacggcga ggccggcggt ggggatgtac    1200
   gccgtggtgg ccggcttcta cgtgctggcg ctgccgctcg gggtcgcgct cgccttcaag    1260
   gccagacttg ggatccaggg cctcctcctg ggcttcctgg tgggcgccgc ggccagcttg    1320
   gcggtgctcc tcaccttcat cgcgcgcatg gattggcccg ccgaggccca aaaggcgcgg    1380
   actagaacca cagcaaccgt ggcccaattc caccaacacg acgaggtcgt ccagccttga    1440
<210> 58
   <211> 2460
   <212> DNA
   <213> Oryza sativa
<400> 58
   atgccggagg ctgcggcggc ggcggcgggc cacatggatc cggttggcga cgaggcggcg      60
   gagaggaggg agatggagga gaaggaggag gaggaggagg aggaggagga ggatgaggag     120
   ttctacgagt cgctggatcg gatcctgtcg tcgtcgtgct cgtccacgtc cgcctccgac     180
   gacgacgacc agcagcaccg gcggaggcgg cggcaccacc cgcagccgca gcagctgtcg     240
   tcgtccgcga cgttctccgc gtacgaggtc tggatctccg agccgacatc cgtcgaggag     300
   cgccgccgcg tgctgctgcg tcggctcggc ctcgcccacg actccgagcc cctgccgcac     360
   ccgtccccac gcgtatcatc ctcctcccct cgttcgccga ccccttcccc cccgtcctcg     420
   tcgccgcctc ggccggctcc cgtggtggcc gccgcggagg agcccagatc cagcggccac     480
   gggaagccgc cgcttgcgag gaacccgagc ggcggcgcgg agcaatgccg gatccggaac     540
   ctggacgacg gtacggagtt cgaggtcggg gaggttcacg atgaggtggt ccgggaggtc     600
   ggcactggcc ggcagctcac cttcgaggag ttcgagctct gcattggccg ctccccgatc     660
   gtccaagagc tcatgcgccg ggccaccaca gccgcatcat cctccacctc cgaccacgcc     720
   gccccagcat ccaagccacg gaggaagcct ggaggctggc tgcgtggcat ccggcacctg     780
   gcgggaagcg ttgcatacgg gcgcagcagc accgatgaga gggacaagga gaaggagaag 840
   gagaagaagg agagggaagc gcggcgcctg agctccgcca ccgatgacag ccttgacggc 900
   aacggctcgc gcaatgcagg gagggtcagg gtgcggcagt atgggaaggc gtgcaaggag 960
   ctcaccgggc tgttcatgac acaagaattg gctgcccatt cgggctcaat ctggtgtatc 1020
   aacttcagct tggatggacg ataccttgca agcgctggcg aggaccgtgt catccatgtg 1080
   tgggaggtat cagagggaga aagaaaggga gaattgctcg gggaaggtac ggtggcaagg 1140
   gagaacggtg gtggctgcag cccgtttctt gcagctgttg ggaatggatc gccggagctg 1200
   gcaacattgt cattgagctg tgctgacggg ggttttgtgg agaagaagag gaggccaagg 1260
   atgcaaagca gccggaagtc tgttggctct gatcatctag ttgtgcctga atgtgtgttt 1320
   gggttcagag ataaaccagt atgctctcta ttgggtcacg ccgccgatgt tcttgatcta 1380
   tcatggtcca aatctcagta cttgctttca tcctcaatgg acaaaactgt taaactatgg 1440
   gacattacta ctagtacctg tctgaaaaca ttttcacaca cagactatgt gacttgcatc 1500
   cagttcaatc ccgtggatga taacttcttc attagtggat cactggatga aaaagtacgc 1560
   atttggaatg tacatgatcg taagattgag gattggaatg atcttcatga gatggtcact 1620
   gctgcgtgtt actcccctga tggacaggtt gcactggtgg gatcacacaa gggaagctgt 1680
   catttatttg atacaactga aaagaagctt cagtacaaaa gtcagataga actaagaatc 1740
   aggaagaaga agtctggcca gaagaagata actggcttcc agtttgctcc tggaagctcg 1800
   tcggaagtcc tgattacctc tgcagattca agaatccgtg ttgttaatgg tgatgaactc 1860
   gttcacaaat ttaaagggtt ccgaaataca agtagccaaa tatccgcttc tgtagctcca 1920
   aacgggaaat atgtggtctg tgccagtgag gactcccacg tgtatgtctg gaggcatgac 1980
   aatacttccc atccgagcag aagcaggagt gcagttgatg taaccaactc atatgagcat 2040
   ttccattgcc atgatgtcac tgtggctatc acatggcccg gcgctgaatc ccgtggctca 2100
   ttcgggtccc gtagcagcag aaacagtgat tcagacgatg cagtgatgaa cacgggtcgg 2160
   gatgcccctg tagagaacag tgagcatgat ctgaatggca ctgtcaatag atgcaccaag 2220
   cgcccagttt gtgaaggtgt tgcaagcaca agcaatcctc cagcggatgg agtatcaacg 2280
   tcctggcctg acgagaaaca atcgtctgcc aagagcagtc ctggtcactg ctcatccgac 2340
   ctttgcattg gagctttgga tgttcagcgc cggtcagctt ggggattggt gattgtcact 2400
   gcaggaaggg gtggtgaaat tagggtgttc cagaatttcg gcttcccggt tcaagtgtaa 2460
<210> 59
   <211> 621
   <212> DNA
   <213> Oryza sativa
<400> 59
   atggctcctg cggttgcctc ctccccgtca ctcgtgctct ccgccgccgc cgccaccgcc      60
   tccaacaagc gcccggccga ttccgacgcc tcgccgccgc accagggaga tcgcacgggg     120
   cagcaggaga agaagcagca gcagctggag tgcccgcgct gccgatccac caacaccaag     180
   ttctgctact acaacaacta cagcacgtcg cagccgcgcc acttctgccg cgcctgccgc     240
   cgctactgga cgcacggggg cacgctccgc gacgtgccgg tgggcggcgc ctctcgccgc     300
   ggcggcggcg gcaagcgccg cagggtctcc gccgacgccg acccttcctc ggcgtcgccg     360
   ccgccaccca cgacttccac cacggacgcg tacgccgacc tcccagccgg cttcccgttc     420
   ctcagtgacg gcgccttcct gccgcagttc ggcctcgccg gcgttgcgcc ggccgcgttc     480
   tcttgggcat cggctgtccc tgacttgtac aactgcggga tcgcgccgtg ggacgatgga     540
   acggcggtca ccggcgcggc gtgggacaac ttcgccgaca tcgccggcct tgatctcagc     600
   tggccgccgc cgggtaactg a                                               621
<210> 60
   <211> 2511
   <212> DNA
   <213> Oryza sativa
<400> 60
   atgtctcacc cccacgccac cgcccccaag cgccccggcc acttctcctc ctcctccgcc      60
   gccgcctcct ccccgacctc ccccgcgcag ccgcacatga agaaggccaa gttccccggc     120
   tcctcctcct cctcctcctc cgccgccgcc cccggggtca ccgagaagaa cgggctccac     180
   gtcgatccca cggccgccgc cgcccggacc ggtgggcgca ccaacggcga ggaggatgcg     240
   gagatggtgc tcgccgacca ggaggagctc gccgctccga gcgcatcggc cccggcgggg     300
   gtcgccgcca acctcttccg gaagaaggcc acactccccc agccatccgc cgcccgcaag     360
   cccctccgaa tcaaaatagg tcagccaaaa ttgccaacaa actttgagga ggatacatgg     420
   gctattttga aagatgctat tacagctata tttctaaaac agaaactttc gtgcgatgtt     480
   gaaaaacttt accaggctgc aggtgacctt tgtctacaca agctaggcgc aaatctatac     540
   gaacgcataa agaaagaatg tgaagtacac atatcggcaa aaatatcagc attagtgggt     600
   caaagtccag atttagtagt atttttgtct ctggtgcaaa gaacatggca agatttttgc     660
   gatcagatgt tgattattcg tggtattgct ttacttcttg atgtaaaata tgtcaagaat     720
   gttgcaaaca tttgttcagt gtgggacatg gggttgaagc tattccgcaa gcatctttca     780
   ctgtctccgg agattgaaca caaaactgtt actggtcttc taagattaat tgagagtgag     840
   aggcttggtg aagcaataga caggacatta cttagtcatc ttctgaagat gtttactgct     900
   cttggaatgt attctgagag ttttgaaaag ccctttctgg agtgtacatc tgaattttat     960
   gctactgaag gtgttaaata tttgcagcag tctgatattc cagactatct caagcatgtg    1020
   gagacaaggt tgcaagaaga acatgaaagg tgtattctat atttggaagc taacactagg    1080
   aagccgctta taacagctac agaaaagcaa ttattgcagc ggcacacatc tgcaattctt    1140
   gagaagggat tcacaatgct tatggaagca aatcgtgtaa aagacctctc gaggatgtac 1200
   acactcttcc agagggttga tgccattgag ttgctaaagc aagcacttag ttcatatatt 1260
   cggggcacag gccagggcat tatcatggat gaagaaaagg acaaagaact ggtgcccttt 1320
   cttctggaat ttaaggcatc gcttgataga atattggagg aaagttttgc caaaaatgag 1380
   gctttctcca atacaataaa agagtcattc gaacatctta tcaatttacg ccagaatcga 1440
   cctgctgaat tgattgcgaa gtttcttgat gagaaacttc gagctggaaa taaaggtacc 1500
   tccgaagaag agctggaggg aatattggat aaagttttgg ttctgttccg atttatacaa 1560
   ggaaaagatg tatttgaggc attctacaag aaggatctgg ctaagaggtt gctgctgggg 1620
   aagagtgcat cgatagatgc tgaaaaatca atgataacaa agctcaaaac tgagtgtgga 1680
   agtcaattta ccaacaagct ggagggaatg ttcaaggaca ttgaattatc caaagaaata 1740
   aatgagtctt tcaagcaatc atctcaagca aggacaaagc ttccatctgg cattgaaatg 1800
   agtgttcacg tgcttacaac aggctattgg ccaacatatc caccaatgga tgtgaaactc 1860
   ccccatgaac ttaatgtcta tcaggatata tttaaagaat tctatttgag caagtatagt 1920
   ggaaggcgtt tgatgtggca aaactcattg ggtcactgtg tattaaaagc agagttccca 1980
   aaaggtaaaa aggaacttgc ggtgtcacta tttcagagtg tggttttgat gttgttcaat 2040
   gatgcacaaa aactaagctt cctcgatatc aaggaatcga ctggtattga ggataaagaa 2100
   ttgcgaagaa cgctgcaatc acttgcatgc ggtaaagttc gggttctcca aaagatgcca 2160
   aaagggcgag acgtagaaga taaggacgaa tttgtattta atgaagaatt tagtgcccct 2220
   ctctatcgca taaaggtgaa tgctattcag atgaaggaga cggttgaaga aaacacaagc 2280
   acaactgaga gagtattcca ggacagacag tatcaggtgg atgctgccat agttcgaata 2340
   atgaagacac gtaaaaccct cagccacacg cttctaataa ctgagctttt tcagcagctc 2400
   aagttcccaa tcaagccatc ggatatcaag aaaagaatag agagcctaat cgacagggag 2460
   tacctggaga gagacaggag taacccccag atctacaatt acctggcttg a 2511
<210> 61
   <211> 1686
   <212> DNA
   <213> Oryza sativa
<400> 61
   atggacgccg atgaggccgc ggggagtagc aggaggatgg atctgaacct ctaccttggc      60
   ctcccacgcg ccccgcgccc gcgccgctcc gacctcggct ccgacctcgc cctcagcacc     120
   ccgatgccct cctccccgtc ctcctccgca gcctccgtcg acgcgccgcc gccaccgccc     180
   gagctgtcgc atcccccgta ctccccctct cacgccgacc tttcccctcc gctgcaggag     240
   gtctactccc tgtacaaccc cgacgacccg cctgcttccg agacgcacct gccgccgtat     300
   gcgccgcctc cggctccggt ggtctcggag ctccctgacg acctcgagtt tggcctccac     360
   cccccgccgc cgctggtgcg tgccagcgaa ctgctaggtt gggaggaccg gccgtcttcg     420
   tcgacggcat cgtcctcttt cctccctgac accgcagccc gttactggcg gcttctcgag     480
   cagactggaa gcagatggct ccgtgcgagg cggtttaggt cggaccttcc gccactcagt     540
   tctgaagctt acccagctgg gcgtgatgct gccgcagtcc cagtgctgca gcatgaaccg     600
   atgaatgata ctgttgaaca taataaggta gctgccgatg gcgcggaagt aggcgcctcc     660
   gaggaatcgg aggagcaggg caggagcgct gccacatttg agtgtaatat atgcttcgat     720
   atggccagcg agccggtggt cacctcttgt ggccatctct tctgctggcc ttgcttgtac     780
   caatggctca atgtttattc caatcacaag gaatgcccag tctgcaaagg cgaggtgact     840
   gaggcgaata ttactccgat ctatgggaga gggaattcat gtttggatgc cgagaaggct     900
   gtggaaggtg ggaaacaaac aggtcctact atcccaccaa gaccacatgg aaatcggctc     960
   gaaagcttca ggcagcagtt tcaccatttg cgaccgatct caagaaggct tggtgaggct    1020
   catgggttat tgtcatcatg gaggcgcctt ctggaccaac agattatgaa tactgcgagt    1080
   aggtttgaag gtccgcctga atcagctgtg caggaaatgg ttgacactgc tcacgctcag    1140
   cacaccagtc gcctaagtag attggcgtca aggatgagag caagacggtt gctgagagaa    1200
   gcagacaacc ctaaccctcc cgatggcgga tccacttccc ctgacagtgg tttgatcaga    1260
   aacaatgcat cggatccatc cagaaatggt ccgagctcat tattaccaga tggaattgac    1320
   tggttgcgtg gacttaccct tcttgggtat gaagacacgg aaagatttgc atctgccatg    1380
   agtgatttta gaaggataac tggaccaagc caatatggtg catcggcttc atcatcgaat    1440
   cctccaaatc tcgagtcaac atttgacaga actcatgttg ttgcagcacc ttctgcagac    1500
   caagcatcta actcaagcac tgctgcagtg atacaggggg atgctggtat ctctgagagt    1560
   gcaggagaac caagtaacgc ggggtcatca agatccctga ggaggagagg gaggagcagt    1620
   gccctgggtt ctttggatgc tgatggcggg ggcctccaac ggaacaagag gcgaaggata    1680
   aactga                                                               1686
<210> 62
   <211> 1443
   <212> DNA
   <213> Oryza sativa
<400> 62
   atggtgggag gagagcttgt gctggctgct ctggtgatcc tgcttgcttt gctgctgacc      60
   ctggtgctga gccacttcct gcctttgctc ctgaatccca aggctcccaa gggaagcttt     120
   gggtggcctc tccttggtga gacgctgagg ttcctcagtc ctcatgctag caacaccctg     180
   ggcagcttcc tggaggatca ctgctccagg tatgggaggg tgtttaagtc ccatctgttc     240
   tgcaccccca ccatagtgtc ctgtgaccag gagctgaacc acttcatcct tcagaatgag     300
   gagaggctgt ttcagtgcag ctaccccagg ccaattcatg gcattctggg caagtcctcc     360
   atgttagtgg tcctagggga ggaccacaag aggctcagga accttgctct agcactggtc     420
   acctccacaa agctcaagcc cagctacctt ggcgacattg agaagattgc actgcatata     480
   gttgggtcat ggcatggcaa gagcaaggac aaggggatgg tcaatgtcat cgccttctgc     540
   gaggaggcaa gaaagtttgc attcagtgta atagtgaagc aggtgctggg gctatcacca     600
   gaggagccgg tcactgccat gatacttgaa gatttcctcg ccttcatgaa gggtctcatc     660
   tctttccctc tctacatccc agggacgccc tatgccaaag ctgtgcaggc cagagcgagg     720
   atatcaagca ctgtgaaggg tattattgag gagaggagga atgctggctc cagcaacaag     780
   ggtgatttcc ttgatgtgct gctttcaagc aatgagctct ctgatgagga gaaagtgagc     840
   tttgtgctgg attccttact gggaggatat gagaccacct cactcttgat ctccatggtt     900
   gtgtatttcc ttgggcagtc agctcaagat ctggaactag tgaagaggga gcatgaaggc     960
   ataagatcga agaaagagaa ggacgagttc ttgagctctg aagactataa gaagatggaa    1020
   tatacccaac atgttatcaa tgaggcactg agatgtggca acattgtcaa gtttgtccac    1080
   aggaaggctc tcaaagatgt cagatacaaa gagtatctga ttccttctgg ttggaaggtc    1140
   ctacctgttt tcagtgctgt tcatttgaac cccttacttc atggaaatgc ccaacaattt    1200
   cagccctgca gatgggaggg tgcaagccaa gggacaagca agaagtttac gccgttcggc    1260
   ggtggccccc ggctctgccc tggatcagag cttgcaaaag tagaggctgc tttcttcctc    1320
   catcaccttg tgctcaatta tagatggaga atcgatggcg atgacattcc gatggcatac    1380
   ccgtacgtgg agttccagag aggtctgccc atagaaatcg agccactttg ctctgaatcc    1440
   tga                                                                  1443
<210> 63
   <211> 2433
   <212> DNA
   <213> Oryza sativa
<400> 63
   atggcgacgc tgccggacct gggtgtgtcc gccttcatca acatcttggg cgccttcgtc      60
   ttcctcctca tcttcgccgc cctccgcctc cagcccatca acgaccgcgt ctacttcccc     120
   aagctctacc tcactggcca gcgacgccac caccctcacc ctcatggctt cgtcaacctc     180
   gacctctgct cctacctccg cttcctcgcc tgggtccccg gcgccctccg catgtcccag     240
   cccgacctca tccaccacgc cggcctcgac tccgccgtct acctccgaat ctacacgctc     300
   ggcctcaaga tatttttgcc catcatgact gtcgccttgc tggttcttat tccagttaat     360
   gtctctggtg gcacgttact taatttacga aaagaaattg tctttagtga tattgataag     420
   ctttccatat caaatgtcaa ccctggatcc aacaggttct ttatccatct attaatggca     480
   tatgtgttca ctttttggac ttgctttatg ctatacaaag agtatagcaa tgtggcattt     540
   atgagattgc acttcctggc ttctcagaag cgttgtgctg atcagttcac tgtgattgtt     600
   agaaacatac ctcatgtttc aagccattca acatctgaaa cagtggatga attcttccgt 660
   aggaatcatc cagaccacta tcttggtcag caggctgttt ataacgcaaa caggtatgct 720
   aaacttgtga agaaaaaaga gaggcttcaa aactggttgg attactacca gctgaagttt 780
   gaaaggcatc ctggaaaaag accaattgga aggacagggt gccttggttt ctgcggtaga 840
   gaagtggatc aaatcgacta ttaccgtgct agaatcagcg agcttgataa gaagcttgca 900
   tctgagcgtc aaagagttct caatgaccca aaagctgtta tgccagttgc ttttgtgaca 960
   tttgactcga gatggggagc tgctgtatgt gcacagacac aacagtcaaa gaatcctacc 1020
   caatggctaa ctgattgggc tcctgaaccg cgggatgtat attggcagaa tcttgccatt 1080
   ccatttttct ctctcagtat ccgcaagttc ctgatatcca ttgcagtttt tgctctggtg 1140
   ttcttctaca tgatacctat agcttttgtg caatcacttg ccaatcttga gggtattgaa 1200
   aaagttgcac ctttcctaag gcctgtgata gacacaccag tggtgaaatc cttcctgcag 1260
   ggtttccttc cgggtttggc tttgaagatt tttctgtata tcctcccaac ggttttgatg 1320
   attatgtcaa aggttgaagg ttatgtgtct ttatcatctc tggaaaggag ggctgcttca 1380
   aaatattact acttcatgct ggtgaatgta tttcttggaa gcataatcgc tggcacagct 1440
   tttgaacagc taaatgcatt tttccatcag ccaccttcac aaataccaag gaccattgga 1500
   gtagctatac caatgaaagc tacatttttt atgacataca taatggttga cgggtgggct 1560
   ggcatcgcga acgagattct tcgagtgaag ccgctggtga tataccacct gaagaacatg 1620
   tttattgtga agacggagcg ggacagggag agggcaatgg atccgggcag cattgggctt 1680
   gcagagaacc tcccatcact gcagctgtat tttcttcttg ggcttgtgta tgctgtggtc 1740
   acccccattc tcctcccttt cattatcatc ttctttgcct tcgctttcct cgtgtacaga 1800
   caccagatca tcaacgtgta caaccaagaa tacgagagtg ctgctgcgtt ttggcctcag 1860
   gtgcactctc gcataatagc gagcttgctg atctcgcatg taactctgtt tgggctgatg 1920
   agcaccatga aggctgccta ctccaccccg ctgcttatct ttctgccact cctcaccata 1980
   tggttccaca agtactgcaa gagccgtttc gagcctgctt tccgcaagta ccctctagag 2040
   gaagcgatgg agaaggacaa tctggagcgc acgtcggagc caaacctgaa cctcaaatcg 2100
   tacctgcaga acgcttacct gcaccccatt ttccacatgt ttgagcagca gcagcagcag 2160
   gagcaggagc agcaacggga ggagaaggta gaggtgcgaa tcgacaaggc gcagcaacat 2220
   catcatcggc aggtagagga ggaagaggag gagagcaaga gcagccaggc tacaacacac 2280
   tactaccacc atcaccatga gcagaccaca acgacgacac accaccatta ccatcagcat 2340
   gagcatatga gccactacca catgggcccc tccgacacag ctgactcacc ctcgccgccg 2400
   cactttgtct accattatgg cgtcgaccct tga 2433
<210> 64
   <211> 882
   <212> DNA
   <213> Oryza sativa
<400> 64
   atggagatga ccagaagcct tacgctcgtg ccgctcccgg cgacgctccg gccggcatcc      60
   gcggcctgtc gccggcggcg gaggcggcga gggcttccct tcggtgcact cttctcacca     120
   tcgcctcctt cgaaccagca gcagcaggaa atgcacatca gggcgctgca gccgcggcag     180
   gattgggtgg gggagtgggt ccggagcaac gacacgctag tccgcggcct gcccatcctc     240
   ggcggcggcg cctccctgct cgccgtcctc ctcaaccgtg cggtttccgg cattgcagct     300
   gtcgccgacg cctccagttc gcagtcaagg gctgacatac tgactcttgc tctctccgta     360
   actgatattc ttgctggcct tgtttggttg tccatccggc cgaaatccat ttctcctgtt     420
   gttcctcgag gtgtcgagtg caaacgggta ggaacgggtg tattggactc ggctcttcgt     480
   gaactacttt ggacatggga ttcccttaca actgcaactt gttgcaaatc cttggttgtt     540
   gtgtatggag gtaattgtgt tcttcaaatt ggggttgctg ctggctctcc agaggatggt     600
   aatgcagtta tggtggatgc acagaagttc atgcaaggtt ccctttatag aagtgccatg     660
   gaatccaaga agcaatctta cctagcaaat cttgccttat atcctggaag gactgaacta     720
   ccattcttgc cagctaacac gcaggcccta atattgcaac caattggtga taaaggaatt     780
   gcagttattg gtggtgacac tataaggggg ttcactaatc ttgatcaggc atggattgca     840
   atgatagcag ataaactgga tgctacattg tcaaagtcgt aa                        882
<210> 65
   <211> 468
   <212> DNA
   <213> Oryza sativa
<400> 65
   atggcatcat cagttgcagg ctcagtgact cgtcgtcctc ctcccgtgct gctggcttgc      60
   cgatcgcgac caaataatcg tcgcctcatc agattgttac cgctcctctt cgccgtcgtc     120
   gtcttgcttg ctcttcttcc accatgcgtt catggagctc gtgctctgaa tgatgccaaa     180
   gaagccaaag ttgcagaggc cagcgaccag acgacgacga cgacgcacgc cgcggcggcg     240
   gcggtggctc ggtggtctgt caccgtgagg gaaggaggag gtggtggtgg tcacggcagc     300
   ggccatgccg gcgccggcca cggccacggc agcggccacg gcaggccgga gccagccgag     360
   caccacacgg gcaggcgcag cgcggcggcc ggatccgtgc gccctcccat ggcggcctcc     420
   tgcgccgccc tccttgtcgc cgccgtcgtc gctctgcttc gcttctga                  468
<210> 66
   <211> 375
   <212> DNA
   <213> Oryza sativa
<400> 66
   atggagagtg caaagagatc ttgccttgct atctccctca tcctactcct actccttgtt 60
   ccaagtatcc atggagcaag gcatgttgct gcagctatca agggtacagg cgccgacagc 120
   gagatggtgg tgacggagag gacggccggc ggcggcggcg gacatggacg cggttacaca 180
   agccaccggt cgcacaaccc caacaatccc aacgacggtg gctccggcac gccggtggtg 240
   gacccgcaca atgtcgccac caggggccac caccaccgcg gcgcggcgac gaggacggcc 300
   gccggcggcg acccccgcct ggcagcctgc atgcttcgtc tgggagcgac cttcttcctg 360
   ctggttcttg gctga 375
<210> 67
   <211> 1257
   <212> DNA
   <213> Oryza sativa
<400> 67
   atggcgggcg ttggattcgt ggaggacatg ctgcgggagc agagcctcct ggaggcgacc      60
   tgcggcgacc tcttcgacca catcgacgac ctgctcgact tccccaagga ggagtcggcc     120
   gccgacgtgc tcctgctcga cgcgccggcg ccagggagcc cgctgtcctc gcgcatcatc     180
   ggcggccacg ccaccatggc ggcggcgccg ccaccgccgc cgcagatgat ggcgctcccc     240
   ccgccgccgg cccccgcgaa ggacgacgcg tcggcgctgt tcgacgcggc cggcgcgctc     300
   ggcgccgagg tgttcgaccg caaggacgcc cacattggcc cgtgtgatga gctggacatg     360
   gacatggcgc agctggagtg gctgtcgggg ctgttcgacg atggaaccat cccgcacgag     420
   ccgagttttc cgggcgtcaa ctgcgcggcg ccgatcaagg cgtcggcgct gacggcgaac     480
   gccggcgtcg tgctgccgga caaggcggag gaggcgctgt tccgcagctc cagccccatc     540
   tccgtgctgg agcacagcgg cttcaacgtg gcaaccaatg ggggctcctc ctcgtcgtcc     600
   tcctcgtcgg cgtcctcctc gtcggagtcg ttctccggca gcggccgcgc gtggtccgcg     660
   cccgtgtcgc cgcgcccgga gccgcccgtg ctcgtcatcc cggcgcgcgc gcgcagcaaa     720
   cggtctaggc cgtccgcgtt cccggctgtc cgcggcgcgc cggcggcgac ggagaccacc     780
   atcctggtgc cgacgccaat gtactcgtcc acctcgtcgc actcggatcc cgagagcatt     840
   gccgagtcca acccgcaccc gccgccgatg aagaagaaga agaaggccaa gaagccggcc     900
   gctccggccg ccgcctctga cgccgaggcc gacgccgacg cggcggacgc cgactacgag     960
   gaaggcggcg cgctcgcgct cccgccgggc accgtgcggc ggtgcacgca ttgccagatc    1020
   gagaagacgc cgcagtggcg cgcgggcccg ctcggcccca agacgctctg caacgcgtgc    1080
   ggcgtccgct acaagtccgg ccgcctcttc ccggagtacc gcccggcggc gagccccacc    1140
   ttcatgccgt ccatccattc caactcccac aagaaggtgg tggagatgcg ccagaaggca    1200
   acccggaccg ccgacccgtc ctgcgacctc ctgcagtaca tccgccgccg ggattaa        1257
<210> 68
   <211> 1329
   <212> DNA
   <213> Oryza sativa
<400> 68
   atgggcgggt acgagctcgt caggagcgac gacgccgcgg cggccggccc gccagatctc 60
   gagctcggcg gcagcggcag ctgcaacggc ggcggcgtct cggccaagtc ccggcctccg 120
   tcatcgccgc cgtcgcaggg cggcgcgcgg cagcggctcg tctccctcga cgtcttccgc 180
   gggatcaccg tgctgcttat gatcattgtc gatgatgctg gagcttttct cccagcactg 240
   aaccactctc catgggacgg cgtaaccatt gcagatttcg tcatgccatt cttccttttc 300
   atggttggga tctctctaac gctcgcgtac aagagggtgc cggacaaatt ggaggctact 360
   aagaaggctg tactacgtgc cctcaagttg ttctgccttg gccttgttct ccaaggcggt 420
   tttttccatg gtgtccgcag tctcactttt ggtgttgata ttacaaaaat acggttgatg 480
   ggtatacttc agagaattgc tatagcttat cttttggctg caatctgtga aatttggctc 540
   aagggagatg atgatgtaga ttgtggactc gatgtgatac ggagataccg ttaccaattg 600
   gttgtagcat tgctcctgtc aaccatgtat actgttattt taaacggtgt ctacgttcca 660
   gactgggaat accagatatc aggtcctggt tccacagaga aatcattctc tgtgagatgt 720
   ggagtaagag gagacactgg tccagcttgc aatgccgttg gaatgcttga ccgtacaatc 780
   ttggggatcg atcatctcta cagacgaccg gtttatgcgc gtacaaagca atgtagtata 840
   aactatccgc aaaatgggcc ccttccacct gatgctccat catggtgtca ggctccattt 900
   gatcctgaag gcctcctcag ctctgttatg gcaattgtca catgcttgat tgggctgcag 960
   tttggacata taattataca ttttgagaaa cacaagggaa ggataataaa ttggctaatt 1020
   ccttccttca gcatgttagc actggccttc tcaatggact tcattgggat tcgtatgaac 1080
   aagccgctgt acacgataag ttacgccttg gctacctctg gtgctgcagg gcttcttttt 1140
   gctgggatct acacactggt ggacgtgtat ggattcagga aacttaccat ccccatggag 1200
   tggatgggta agcacgcgct gatgatctac gtgctagtgg catgcaacat cctgcccatt 1260
   ttcatccatg gtttctattg gagggagccc aagaacaacc ttttgaagtt catcggagtt 1320
   ggggcatga 1329
<210> 69
   <211> 1467
   <212> DNA
   <213> Oryza sativa
<400> 69
   atggccgcga ctggcggcgc cgccggggag aagacggcca gcagcctcct gctcggcgtc 60
   cggggctaca cgtccaccct caagaacgcc tccaccgcca gctgcaggtt gagcgccggc 120
   catcccatcg aggtgacttt gtgggaggcg tccccgcctg ccctctccca cttctccgtc 180
   cactgccccg atctcccatc cttcaatggc aatctgcttg gcgcgcctaa agccatcgcc 240
   gccgccgtcg acgacgccga cggccagctc ctcctcctcc tccgagtccc catcgatcag 300
   cttggtgccc cgcatgacaa cgactacttg gtctaccatc cggatccccc gtctccgaaa 360
   ctggatctgc tccccaaccc gcctccccct accctcggtg accaccagct cgccatactc 420
   agctgcggcg acgaccgcta cgtcgtggcc gccctccacg tctggagtga gttcacttcc 480
   acgctgcgcc tgtacagatc ttcttgttcg tctgggagtt ggacatcgga ggaggtgtcc 540
   gtggaggagc cggtgaggga caggctgtgc ccgatcccgg actcagccaa gaggcagctg 600
   taccacgtca ccaccaagac catcacgctc ggaggtgcga agggcaccgt gggctgggtt 660
   gatctctggc gcggcatcct cctctgcgac gtgctcgacg aaatgtctcc aaggaagctc 720
   cgcgacatgc cgctgccgtg gccggccaag ggcaattgga ggatgtacct caatggagat 780
   gtgtcctttt gtcgggacat cgccatcagc caacacaagg attccatcaa gtatctggag 840
   atggagatcg tttcaccaag aacggtgacc accaccatac ccacctccac ctctgcagat 900
   cctacttcat accttgaatg ggttcgccgc agcagagaac ctcagccgac acggcgacgc 960
   tccgtgttcc accctggttc gtggagaatc actacatgga gcatgcctat cccggtcact 1020
   tcatgggacg actggcgccg tgactgcact gctgaatcgc gtgaagtcca tcttgacacc 1080
   aacccaagtc accattacga gttgcttcat agcctcatgc tcagcaacag cggtgatgaa 1140
   cacagggagg aggctcaagg tcaaggggca acctcttcct tgtccctagg tcgcctgcgt 1200
   ttgtgttacc cggccttgag ttgcatcgat gatgatgttg tttacctctt gggcaacgct 1260
   gctggcaggg gtgctaagac gggaggaatg atggtcgctg ttgacgtcag gaacaaggag 1320
   ctgcgaggag tggccaagct tgaccccgaa aagaacaccc tctactccat gcgatgctac 1380
   cttgcaactg ggatctccaa acgcctcaac actaccacag acacaagagt tggacgacct 1440
   gaggaggatg cagaagccgc cgagtag 1467
<210> 70
   <211> 504
   <212> DNA
   <213> Oryza sativa
<400> 70
   atggcgaact accaccacca ggagtactac cagatggcgg cggcggcagc ggtggcgtgg 60
   ccgagggagc cggacagccc gcagctgagc atcatgagcg gctgcagctc cctcttctcc 120
   atctccaccc tgagggacga cgacgacggc ggcggcgtcc gcctcgccgg cgccgcgctg 180
   cccgccacgc cggtgtcgct cgccgggatc gccggcggcg ccagtacccc cggcggcgac 240
   gaggtggaca tggaggtgcg gcagcagagc ggcggcagcg gcgacgaccg gaggaccatc 300
   cggatgatga ggaaccggga gtccgcgctt cgctccaggg cgcgcaagag ggcgtacgtt 360
   gaagagctag agaaagaggt tcgccggctg gtggatgaca acttgaatct caagaagcag 420
   tgcaaagagc tgaaacagga ggttgctgca ctggtgatgc ctacaaagag ctcactgcga 480
   cgaacttcat caactcaatt ctga 504
<210> 71
   <211> 933
   <212> DNA
   <213> Oryza sativa
<400> 71
   atggcagaga aaaagaagaa gaagaagaag aagaagccgc aatcactcct agtccttaca 60
   agctggagat cgatcgggat ggggaggggt cgggtggagc tgaagaggat cgagaacaag 120
   atcaaccggc aggtgacgtt cgccaagcgc aggaatggcc tgctcaagaa ggcgtacgag 180
   ctctccgtcc tctgcgacgc cgaggtcgcc ctcatcatct tctccaaccg cggcaagctc 240
   tacgagttct gcagcaccca gagcatgact aaaacgcttg agaagtatca gaaatgcagt 300
   tacgcaggac ccgaaacagc tgtccaaaat agagaaagtg agcaattgaa agctagccgc 360
   aatgaatacc tcaaactgaa ggcaagggtt gaaaatttac aacggactca aaggcaatac 420
   tacaaatcta aacataggct gtgtttagtt cggtccaaag tttggaattt ggttaaaatt 480
   agagacgatg tgactgaaaa gttgtgtatg tatgaaagaa atttgctggg tgaagatctt 540
   gattcattag gcataaaaga gctcgagagc ctagagaagc agcttgattc atccctgaag 600
   cacgtcagaa ctacaaggac aaaacatctg gttgaccaac tgacggagct tcagagaaag 660
   gaacaaatgg tttctgaagc aaatagatgc cttaggagaa aactggagga aagcaaccat 720
   gttcgcgggc agcaagtgtg ggagcagggc tgcaacttaa ttggctatga acgtcagcct 780
   gaagtgcagc agcctcttca cggcggcaat gggttcttcc atccacttga tgctgctggt 840
   gaacccaccc ttcagattgg gtaccctgca gagcatcatg aggcgatgaa cagtgcgtgc 900
   atgaacacct acatgccccc atggctacca tga 933
<210> 72
   <211> 612
   <212> DNA
   <213> Oryza sativa
<400> 72
   atggagggag gagggaggag gaggaagagg gggaaggtgg agctgcggcg gatagaggac      60
   cggacgagcc ggcaggtgcg attctcgaag cggcggagcg ggctgttcaa gaaggcgtac     120
   gagctgtccg tgctctgcga cgcccaggtc gccctcctcg tcttctcccc cgccggccgc     180
   ctctacgagt tcgcctcttc cacctccagc attgatacaa tttttggtcg gtattgggac     240
   cttctggaca caacaattga tctcaatatt gaagcaaggg aatctcgggt tgattgcaat     300
   atacaggtct ggatacggag acaaatcgtc cacggagggc tagctccttt gttggttgga     360
   gatgcagctc ctccatttag gggtgttggt gtggctcctc gcctagccct ttgtatttat     420
   gacgacactc cacctaggat caaggtggca aaagctggtg gcatggaagg tggcatgatg     480
   gacgtcaatg atgggttgca agagtttggg ctgcatctca tgattgcctt agtgtggtat     540
   tttatgcgac aaggctcgtt aggcaacaaa ggatcaatct ctggcacgac gatcatcaaa     600
   gtttctaagt aa                                                         612
<210> 73
   <211> 435
   <212> DNA
   <213> Oryza sativa
<400> 73
   atgggtttga ggtgggacaa ggcggagagg tcaacgagaa gggcaaggag cgcaccgacg      60
   agggagaaga ggccggagaa ggggaagtcc cgccagaggc gtcgcgtggc aacgacgcag     120
   tcggcggcgg cggcactgcc aagcgtgcgc cgcgctggcg gtgagacgcc ggtgctgatg     180
   agcggcacga ggcacgacgc gatggcgatg gtgaggctga caaaggtcag gatgctgttc     240
   gtgcggtgcg gtggcagcgt gaggcactcg tcggaggagt ctgtgttgga cgacgacatc     300
   tgggccgccg cgaggaagaa ggggaaaaga aaagaagaga gagagaggaa gaacagtgtc     360
   aaaggtgggc gggctaaggt gagatcaagg ctaagacgag agtgccatca aacgtcgaat     420
   tcttcgtctg gctag                                                      435
<210> 74
   <211> 804
   <212> DNA
   <213> Oryza sativa
<400> 74
   atgagcttcg cggatctgga ggccggcgcg gtgcgggcgc ccaggagggc gcggggcccc      60
   gacgccacgc gcgcgctcgt cttccagatc accaccgccg tggcctccta ccgccgcctc     120
   ctcaactcgc tcgggacgcc caaggacacc cccgcccttc gtgaccagct gcagaagact     180
   agtcataaca ttcttcaatt ggcaaaggat gcgaaggaga agctcaggag agctgctgag     240
   gcagacaaga acgccgatac tagtgcagac aagagggttg ctgacatgaa gcttgccaag     300
   gattttgcca cgacgatgga ggagtatgga aaacttcaaa atcttgcgat tcaaagggag     360
   atggcatata agccagttgt tccccagaca tctcagccaa actatactac aggtggtata     420
   gaagccaggg attctggtaa aattcctgaa cagcatgcgc tactcgcaga atcaaagagg     480
   caagaggtgc tgcaattgga taatgaaatt gttttcaatg aagctatcat tgaggaaagg     540
   gagcaagcta ttcaagatat tcaacaacag attggtgaag tacatgaagc atttaaggat     600
   cttgctacac ttgtgcatat tcaaggagtt acaatcgagg aaatcgatac aaacattgag     660
   aattctgcag cagcaaccaa agaggcaaag acagaactcg cgaaagcgtc caagactcag     720
   aaatcgaatt catcactgct ctgcattctt ctggtgatct tcggggttgt cttgctaatt     780
   gtgataatag ttttggcaac ttga                                            804
<210> 75
   <211> 318
   <212> DNA
   <213> Oryza sativa
<400> 75
   atgtgtgtgc tcgcacaaga gaaagagaga agaggtaaca atgcacttgc tgcagaatgg      60
   tttccagcaa ggatattatg catgtactgg agtacaccaa gtttcaggaa gatgtcaatg     120
   cgtggcaagg agaatcggtt ggtaggaggt aacactgtat accatcgtag cggatcacgt     180
   ggcttattag ggactcgaca attcctaaag accaaaaatg gagttgatcc tgggcaggca     240
   ggagcatggc atcatcaaca ccgaatggaa catgacgggc caagagggct ctgctgtgag     300
   aaaactgcta ttttttag                                                   318
<210> 76
   <211> 3345
   <212> DNA
   <213> Oryza sativa
<400> 76
   atgcgtgcgg cggcggcggc ctccaaggcg gcggggaagg agaagagcag gaggaagggc      60
   ggcggcggag gagcaggagg aggaggaggc gagcagctgc tcaccgacca ggtcctctcc     120
   ctccgcgccc gcctccacct cgccctcgcg ctcggcctcg ccaagtctga tggaggtcca     180
   aagaaatggc agtctactga tgctggaata cagtctcatg tgctcaaagc agcatcagcc     240
   tttcttggct gtttgaccaa tgagatgctg cggcttcctc ctataaagga gtcaatttcg     300
   gatatactca tagcactgga aggtattctt cagtccaaga atgtgtcggt tctgatccaa     360
   gcaactgatg ttagcttgaa gttagtttcc agtgtaggaa atttagctcg ccaatacccg     420
   gttttagaga tcgttacatg cctcgcgagt cagctttctg caaaccagat aactatagct     480
   gtctcatctg caagtacatt gaactgcata ctgaacaccc tagcaacagc gagaagttcg     540
   attcatgcag aaatttggga agctttggag aaaaccgatg cagttacaag tgtcattgga     600
   gctctgcaga attactcccc tgatgtccat ccattaaact atctgatgga aatgatgtct     660
   ctgctaagaa ttatactgtg gatttggcct tcttcgagat accatgtatg gagtaactgc     720
   aacttgatgg ggaagctagc acaatactgt gttgcctctg aaatggatgt tgctgttaga     780
   gtcctcaagc tatatgctgc tttagcttta tgtgggaatg gtgcaatggt ccttctgaat     840
   aatgaagact tgatggctaa ggttggtgcg cttttgggga agtcaaatcc atctattgct     900
   agaattgaag cattgaaatt ctatcagatt cttttgcgat cttcaaaagg gtgcgatctg     960
   ttaatggctg cacactatca acacattatt gaaggcacaa tcaacgcaat gtctagagat    1020
   gatgaaagat tgttaacaat agagggctgc cgcactgcac tgctggtcct tcgttatgct    1080
   ggggatcatc atcggctctt ttggtctcat gctattgatg atgtattata taagattctt    1140
   actggtggct gcacctcttc acataaagcc aatcagattt tgtgccacga caagcttttt    1200
   aatatggttt ccgagaactt tatggatata cattcttatg tgtgggatat acttggaaat    1260
   ctagcagtac attgcaaaaa tgagtatctc tctgttagga aagggcaaga ctctgccttg 1320
   caggcactaa tacattgtat ttgctcactt gcagcagatg ctatgcagaa aagcaacacc 1380
   atgaaattat ccaaggatgt gcatgagcca gctttgaggg ctgttctgat gatgcttctc 1440
   tcacccagtg gatacatttt gtctgaggca agttctaaac tcttacatgt tttaccttta 1500
   ggtgatgact gtttgaatat tctgttcacg tcgttagaat caaatactac aagaagcatt 1560
   actgcatctt ttgacaatgt caaaattatg tccaacctca tgagcctagc gggcatgagc 1620
   atcaattttg tttgtatcca ctgtaaaagg aatttggatg tggggattgt gtgcaatgat 1680
   tgcagagatc attatagtga aggtctgatt agagttcttc aaaatgcgtc atgtcaaaac 1740
   ttgagcccag gaccgaagtt gtacatttca cgtatactga gtttgtttgg cctatgcggt 1800
   tttccaagca agttgggagg aaagatgaga agggccttag atgataatga gctagctgat 1860
   ctggaactgt tgctttcaaa tggtgaatct ttaaaagctc atacagccat catttcagta 1920
   aggtgtccaa agttgttgcc atctgcaaaa tcccttggta gtgatggaaa aattactgat 1980
   gaatggggca gatcatttta tcatgttcga atgtctgatc gtgttgatag ttgtggcttg 2040
   aagaaaattt tggaatacac atacacaaat tctgtcatgg tagatgatga caacattaag 2100
   ccagtaagga cacttgcgaa gtattgtcac ttgaaatcat tacaagagat gcttcaaaaa 2160
   gagcagccta ggtggaactc tgattgtcct agatatgatc ttactgcagc acttgaacca 2220
   gttaaatgtt cattctcctt ctcagaagtt atcaatgttc cactggggtg gcaagcgctg 2280
   aacaaactga tccactggtt ctactcaggc gagctaccca agatcgaccc cgattgccga 2340
   tggcgaaacc tgaacagcga ggagcagctt tctcagctgc ggccttacgc cgagctgtca 2400
   tccctgtctg aattctggtt cctggaggga gtgaaggagg agagcctgtc agtggtcacc 2460
   tcctgcctga gttccaccag cacggccgcc tccgtcgagt tcgtcgtctt cgcggcgcag 2520
   ctgggtcagt gggagatggt ggaggccgcc gtcggcagcg tcgcccatct gtaccccaag 2580
   ctgcgggact ccggtcagct ggagcagctt gatgatgatg tgctcaacat gctgcggaca 2640
   gagtatgtca gacgcacgca aagaacggga gttggctcgg cggcggcgca ggcgggggcc 2700
   agagtggtga cggcggtgta caggcggggg cagagggcag attattggca aagtggtggt 2760
   tttggagata attggaattt tcaaatggtg attcttaatg cctcggaaga gcattgccgg 2820
   gagtccaaat ttgatacaat tggagtttgt aaagctcgtt tcttgtacgg gaaggtttct 2880
   aggggattta gattgcgtac ctcgggtata aacaaagagg gaggcccaag agggggtaca 2940
   gtaatttata gcaggtcgtc aggagggctg cctccctggt gtggtgcagg aagtcacgac 3000
   gcattggcag ccgtcagatg gccgtcactt ccaggcttgg agtcgcatca gacggcgcag 3060
   gtgataaggc gaggcgcagg gcgcagaggc gaggggagag acgtaaacgt aaccaagcaa 3120
   agcaacgcgc ccatgcggcc gcccgagacg atgcaacgcg agcagccgca gagcagagca 3180
   agagccaacg gtaggaaatg gccaccacca cgacgatgga gatccggaat tcgcgaggag 3240
   cagggggtgc caagtgcaaa ggcgtggcag gagaagagga agaggacaca gcaacagcgt 3300
   tgcgcactgc ctgctgccat tgctgcctcg cgtttacagc tatag 3345
<210> 77
   <211> 1239
   <212> DNA
   <213> Oryza sativa
<400> 77
   atggcctccg ccgtcgcgag taacttgcct gcagctgcgc ccgcggctgt catgccgttc 60
   ggtggatggc atggtccgcg tgtctcgttc agccgcgacg ccgccggggc tgaggaggct 120
   gccgcggtgg tcgtgtgttc ttcgcccctg gccgccgcgg cggcggtggc gacgacgacg 180
   acgccggagc cggcgatatc caaggacttc atcgacttcg agttcagcct cgggggctcc 240
   gccaccatgc tcccggcgga cgagctcttt gccgacggga agctgctccc gcttcggaag 300
   gcggcggctg tgccggagat ggatgcggcg gcgccacggc cgccgcagcc tgaggcaatg 360
   ccggcgcctt cggagccgat gaagccacta cgggcggcta ccgccgcggt tgacgccgcc 420
   gacccgtacg ttttctctcc taaggcgccc agctgctcga gccggtggcg ggagttgctc 480
   gggctgaaga gagcggcagc gcagagcccg aagccatcgc cgtcgtctgc gcccgcgaga 540
   acccccggga gagcgatgaa ctcgacggcg gcgaggtcgc tgaagctgct gctccaacgg 600
   aacaacggcc gctcgtccgg ggcctccgcg tcggagctcg cctctgcgcc gctcctccgc 660
   gacagctccg actcggaggc gtctctctcc ctcgcctcct cccgcttctc cctctcgtcg 720
   tcgtcgtctt cctccggcca cgaccacgac gacatcccgc gcctctccct cgactccgcc 780
   gctgacccca acccgccccg catccgcctc gtccgttcct cccaccgcca ctccacctcc 840
   tcatcctcct catcccgcgc cggccgaagc cccgcgcgcc gccgcccctc cccgccgccg 900
   ccgccgcgct gcctctccgt cgactccccg cgcatgaact cctccggcaa gatcgtgttc 960
   cagggcctgg agcgcagctc cagctcaccg tgcaccctcc acgccgcggc gaagccacgc 1020
   tcccgcgccg tcgaccggtc atactcctcc ggcgtccgcg tggcgccggt ggtgctgaac 1080
   gtgccggtgt gctcgcggcc ggtgttcggg ttcttcaagg acaagaagga cgcggcggcg 1140
   aaggacgcca tggcggcgag gacgaggtcg tcgctggggc ggaagacgac ggcggcgccg 1200
   caagggtgga gcggcgagct ggggagatct tgtgggtaa 1239
<210> 78
   <211> 2292
   <212> DNA
   <213> Oryza sativa
<400> 78
   atgaaaatca gcggacttct gacctctgct ggcatcaata tcgctctttc tgtgctgttt       60
   atatcgctct attctgttct gaggaagcag ccagccaatg tcagggtcta ctttgggagg      120
   aggattgccg aggagcataa tcggctccga gaagctttta tcttggagag atttgtacca      180
   tctactggct ggatagtaaa agccctgcag tgtaccgagg aagagatctt ggctgctgct      240
   gggctagatg ctgttgtttt caatagaatt ctagtattca gcttacgcat cttctctcta      300
   gctgccattc tgtgtgtgtt tggaattcta ccactgaact actttgggca agatatacat      360
   catgttcgga ttccttcaga atcattggat atctttacaa ttgggaatgt gaaagtgaga      420
   tcaagatggc tttgggtcca ttgtgtagcc ttgtacataa tatcaggagt agcttgcatt      480
   ctcctatatc ttgagtacaa gcacattgct aggctgaggc tccgtcatct tacttgtgca      540
   atgcccaatc caagccattt tactgtcctt gttcgtggaa taccaaagga aaccaaagaa      600
   tcatgcagta atgctattga tgatttcttc accaagtacc atggatcaag ctacctgttc      660
   catcaagttg tttacaaagt tggaaaagtt cagaagataa tgactggtgc taagaaggca      720
   tacaggaaat tcaaacattt tacagacagc actattgatc agaggtgtcg agcaatttca      780
   taccggtgct gtctgtgcgg agcctcatct aattctttcc agctgttggc aactgggctt      840
   gagcagaatc aggggaaatc tgaccttcaa gattccagct tgaaactaga tgatcaggaa      900
   tgtgcagctg cttttgtata tttcagaact cggtatgctg ctcttgttgc ctcagaaata      960
   ctccaaacat ctaaccctat gaaatgggtt actgatctag ctccagaacc agatgatgtg     1020
   tattggtcaa atctttggct accttataag cagctttgga ttcgccgaat agctacgctc     1080
   cttggttcaa ttgtttttat gttattcttt ctgataccag tgacatttat acaaggacta     1140
   tctcagctag agcagttgca gcagaggctt cctttcctga aggggatact ggagaagaaa     1200
   tacatgagcc agcttgtaac tgggtacctt cccagtgtca tactgcaaat atttttatat     1260
   gccgttgcac cgataatgat attattttct acattagagg ggcctatatc tcacagtgaa     1320
   aggaagagga gtgcttgctg taaagtgctg tacttcactg tttggaacat attctttgga     1380
   aatgtactat ctggtactgt cataagccaa ttgaatgtgt tatcaagccc aaaggacatc     1440
   cctgtccagc ttgctagagc tatacctgtc caggctacct tctttatcac ctatgttctg     1500
   acatcaggat gggccagttt atcatctgaa cttatgcaat tatttggttt aatatggaac     1560
   tttgtgagga aatatattct acgtatgcca gaagacacag agtttgttcc ctcattccca     1620
   tatcacacag aagtgccaaa agttttgctg ttcggactac tgggcttcac atgctctgta     1680
   ctggcacctt tgatcttacc ttttctgtta gtgtacttct tccttggtta catcgtgtac     1740
   cgcaatcagt tgctcaatgt ttaccgcaca agatatgaca cagggggttt gtactggcca     1800
   atcgcacaca acgcagtgat attctctctc gtgctcacac agattatctg ccttggtgta     1860
   tttggcctga aagaatcacc agtagctgca ggcttcacca tacctcttat catcctcact     1920
   ctgttattca atcagtattg cagaaatcga cttctcccat tattcagaac taccccagca     1980
   caggatttaa ttgacatgga cagggaagac gaacggtcag gaagaatgga tgaaattcac     2040
   caccggcttc attctgccta ttgtcagttc cacgacactg aagatatacc cttggagaaa 2100
   attcagactg tcgggagcga tgaggaacaa gggtgtagct ctgataagtc gaatggaaaa 2160
   gaaagcttcg aggaacccag agcggagttg tctcacccaa cactgaatgg actcccagtt 2220
   agccgtcttc ggcatgctgt gaagtcgatt actttccttg tcagattgca gaaaagaggt 2280
   ttgtcagaat ag 2292
<210> 79
   <211> 2106
   <212> DNA
   <213> Oryza sativa
<400> 79
   atggcggggg cggcggaggg agggagagcg aaccctacca cgaggcgcca ccagaggacg 60
   gcgatcaaca tatctcctcc tccacggcat ggcgtggtgg cgcctcgccg gagtgcgcgg 120
   cggcgcgtag gcgtagcagc tcagcttagg cttcgcccta actctcgatc ccaatcccca 180
   tgcggcgtct ctacctctac caatcaggtc aggctccgat ccccattcgc tcgtgctctc 240
   gtcggcgtgg aacaacaggt gcaagaagct gggcaggctg gccaagatgt ccagcgccga 300
   cgtggaaggg gccgagaagg cttagatggg cctgagtggg aatgcgactc tctcttctgg 360
   ttgggccaga acagtatcaa tgacatacat gcggcccata tttattgggc cggaacggga 420
   ttgctacttc gtgcggcggt cgcgctcctc ccaaaccatc tccgcggcct cttcgtctgc 480
   ctcaatgaat cgtgcctcca tggcttcttc gcccgtccct cgccgccggc caccatcccc 540
   ggcatcgacc tcgactacga cctggacgac gatgccacca tcgaagtcca ctgcaacggc 600
   ctcctcttgc tcgatcgcca catcgtcaac ccggccacac gccagtggat gcgtctacct 660
   cctgtccccc cgtacgcctc actgcccaac ataatgtacg gtgatcgggg cctcgtcttc 720
   gacccagcag cgtcaccaca ctacgacgtc ctctggatgc cctacctgat tcttcatcga 780
   ctccctgctg cgtctctgtc ggatcagtgg ccgccgtcgc cgttcatcct gcacgtcttc 840
   tcctccacga cggggcgatg ggaggagaag tcgtttctcc gggaaggcga cgccaccatg 900
   ggcaccatgg ctgatgtatc cttggcaagg gtaccttacc actgcaagac tcactccgtc 960
   tacctccggg gagcgctcta catgcattgc cagaatgatt gcgtcatcaa gatcacactt 1020
   aacgaccaca agtatcgggt catcagattg cctggcgact ctgcatcaaa cagaaagact 1080
   agggaccctt tcctaggaaa atcaaaagac agagtgtgct acgtattggt caccggtcta 1140
   agtcgactcc aaatttggct cctcaacgaa acttcttctt cttcttcttc ttcttcttca 1200
   tacgacgaca acgagtgggt gctcaagcat ggtgttgacc tagggccaat aatacaaagc 1260
   tacccctgca accatggtcg tcagcaatgg atatggcata atgctgacac taaacaagac 1320
   aaaaccaggg aattaccagc tgtaaatgat atggaggaat ttgaatgggc tatcgataag 1380
   gactctgatg acattattag tggtgccaat gaaagcatcc accataatgg agaatacatc 1440
   tccgctgtac tcggatttca tccttttaaa gacatcgtct tcttgcacga tacaaattta      1500
   agagttgttg catatgacta caacaaggca aaggttcaag acttgggtat gatgttccta      1560
   taccataata cagatagagt gccgtcggat ggaaaggctc agggttcaca gcacgtgagg      1620
   cccatgtcac cttcctcctc ccatctcatt ctcatctcac catcccacgc ttttggactc      1680
   ccaaacgacg ctgttgcctt tcgtcaaccc ccacttctcc ttccccgccg ccgccgctcg      1740
   ccgcccgccc gcccgcccgc catggatgag gagtacgacg tgatcgtgct ggggacgggc      1800
   ctcatggagt gcatcctcag cggcctcctc tccgtcgacg gcctcaagcc gcactcggat      1860
   gagctgctgc tggttgcttt ttttttttta cctcgccatt tgatcgctgc acagagatcg      1920
   aagggagggc ctgccatggc cgctcaacgt actcccgccc cctctccgcc ctccactgcc      1980
   ttctacacat cggctcctcc ctctccactc ccccgctttt gcacccacca ccgcatcccc      2040
   cgcccggaga ccggcaacga cggagggtta gcttcgacga gcgaccgacc acgagatatg      2100
   atatga                                                                 2106
<210> 80
   <211> 909
   <212> DNA
   <213> Oryza sativa
<400> 80
   atggcgccgc ctaatgacgc cggagatggc gacgacgaca agcccgagat gccggtggtg    60
   ctcatcaccg gctgcgccaa cggcggcatc ggctacgagt actgcaaggc cttcgcctcc   120
   ctcggctgcc gcgtcgtcgc caccgacgtc cccgaccgcg tgcccgacct cgccggcctc   180
   gacgccgacc acctcccgct cgacgtcacc tccgacgaga gcgtcgaggg cgccgtggcg   240
   cgcgtcctcg ccgagcacgg gcgcgtcgac gtgctggtga acaacgccgg catcgggtgc   300
   accggcccgc tcgccgagct ccgcggcgag gccgtgcgtc gcgccatgga cgtcaacttc   360
   ctcggccagc tccggatggt gcgcgccgtg gcgccgcaca tggcgtcgcg gcgttccggg   420
   cgcgtggtga acgtgggcag cgtggtgggc acggcggcga cgccgtgggc cggcccctac   480
   tgcgcgtcga aggcggcggt gcacgcggcg acggacgcgc tgcgggtgga gctgcggccg   540
   ttcggcgtgc acgtggtgaa ggtggtgccc cgccgcggcg aggtccgggc tgggccacgc   600
   caacacggcg cagctcgccg gagggcaggc ggagtggcgg ctgtaccggg agttcgcggc   660
   ggcgatcgcg gagagggcgc gggcgtcgca ggcggggggc gcgacggacg gcggcgtgtt    720
   cgcggcgcac gtggctcggc gggtgatgag cgcgcggccg ccgcgggaga tcgtgtacgg   780
   gaacatgacg ttgctgttcg cggcgctggc ggcggcgccg ctgtgggcgc gcgacgcctt    840
   cttcgccaag cgcttcggcc tcgacaagat gctaccaccg cgctagagaa gaagaagaag   900
   ccatcatga                                                            909
<210> 81
   <211> 744
   <212> DNA
   <213> Oryza sativa
<400> 81
   atggtggagc tctctatcgc cgacgccagc gccagtgatc tgtgcggcgg cacgctgggg 60
   cagatggtgg agctcgtctg cgaggcgagg ctacgagtga gggaggagta tgtgagatca 120
   acagtggact tgatggcgtt gctgcgtggg cgcggcatgg tgttcgacgg ggtgtacgtg 180
   gtgtcgaacc tgacgcggct cttcgcggag ctggactttg ggcgcgggga gtgggtggtt 240
   agcggcatgg cacagccgat gctggcaacg ttcctggtga cgtgcaggaa cggcgacgac 300
   gaggacgcgg tggcggcatc gatgctgttg ccgcctccgg tgaagttgag gtttgcagag 360
   gagcttgctg ggctgatgat gagcatgccg cacggcggcg ctgccctatg ccccgcaccg 420
   gcgagtacgt acctccctct tagcatgcgt ggaagacggt ggctacacat cccggagggg 480
   tactacggca acgcactcgc atactccatc accgatgcca gtgccagcga tctgtgcggc 540
   gcgacgctgg cccagatgat ggagctggtc tgcgaggcga ggctacgggt gacggaggag 600
   tacgggagat cgacagtgga cttgatggcg tcgctccgtg ggcacgacac ggtgttcgat 660
   ggggtgtacg tggtgtcgga cctgggtgcg gggagtgggt ggtcagcggc atggcctagc 720
   cgatgctggc gacgttcctg gtga 744
<210> 82
   <211> 435
   <212> DNA
   <213> Oryza sativa
<400> 82
   atgaatcaac aacaccaaag atcaatcgag cactgctcga tcggttgctt tctggcctcg 60
   ccgccgcctc ggttcttccc agctcggact cgatcggctc ccggcgagct tcgaatgaag 120
   ctcgtcgtgt tcttgattcg aggctgtccc ggcgaagttt tgctgagacc aatcgtcccg 180
   gcaaaagagg ggctgcgaac cagaacgaaa tggcacatat tgcagaggtt ttgcaaactt 240
   gaaataataa gtatagagac agaaacgatg atcacgatct cgagtcgatc gatcatcaaa 300
   tcgagatgta aaaagtcaaa taaaaagatt ttggttttct ttttatctat gtcagtgaaa 360
   tttctgctca tcacaaccag aagatctttg tcagtacaga agagatcttc cacgttctcc 420
   caacttttgc attag 435
<210> 83
   <211> 285
   <212> DNA
   <213> Oryza sativa
<400> 83
   atgtgcatgg accgagctgc cgtgccggtg aagagggtgt ggctcggcct cgccgcgcgc 60
   ctcggcctcc ggcgaaccag cgggctgggg aagctgaaga aggaggtgag gacgtgcgag 120
   taccacgacg tgcacatcat gtgggagatg ctgaggaaga cggacgcgcc ggtgcccatg 180
   gcggagaagg aggccgccgc cgccgcggcc gtcgcggcgg ccgccggcgc ccggaggagg 240
   aaggcggcgt ggagacggtt cctctactac tgctgcgcgt tctaa 285
<210> 84
   <211> 912
   <212> DNA
   <213> Oryza sativa
<400> 84
   atggcgacgt cccgcaagct ggcccgcgtc gacatcgccg agctgaagca gcggctggtg 60
   aagcggctgg ggcggcagcg cgccgggcag tacttcgcgc acctcaccag gctgctgaat 120
   ctgaagctca ccaaggtgga gttcgacaag ctctgctacg ccaccatcgg gagggaaaac 180
   atcgctctgc acaacgccct gattaggggg atcatcagca atgcgctgtc cggggtgccc 240
   ccgcccagcc gccaggcggt gacggggcag tccgggacga ccacggctcc cagcgggcag 300
   tgcgtcggca tcgcgctaca gagcgcccga aatgtagggg ccgtggtgga ttcgggcgat 360
   ggggactttg cgagggaacg ggcggttgcc ggcaaggtgt tgtcggtgga ggatggggag 420
   gaggtggagc aggttaggtc tgctccatgt gtgcagagcc gaagcccgat aactgcccca 480
   ttggggattt cgactacgcc aacctatggt gcaaggacat ggaggttgga tgatccaatg 540
   gtgtcgtgtt acgattccca ccatctgctg gacactggtt ctctgttcaa gggtttgcag 600
   cgtcggttgg agagtgatgg cattggagtg tcggtgcagg gtgttgaagt tttaaatcgt 660
   ggattagatg agtttttgcg gaggttgatt aaaccatgca tggaattgtc caggtcaagg 720
   tccagcggta gaagagttac caaaggcaat gctatgtttg cagctaggat gaatggcttg 780
   caacaagcca atcatggtca ttgtacaaca ctacaagatt ttgctgtcgc tatggaatct 840
   gatccacatt tgcttgggac caattggcct acacagcttg aaaagataca ggcaacgtcg 900
   tttggtgaat ga 912
<210> 85
   <211> 561
   <212> DNA
   <213> Oryza sativa
<400> 85
   atggcgtctc ctcgctgcgc cgccgtcgcc ctcctccatc ccgccggagt cgccgccggc    60
   ggcggagctc gccggcgtgt cctcctcctc gaccaagagc ggccgttgtg ggggactgag   120
   gtgcgccggc gccggcgccg gcgtttctcg agcctcgaga cgccgccgcg gtgcagcaag   180
   atgtacgtac ccggcttcgg agagggatcg ccggagaaga aggcagcaag aaacctgcag   240
   cacttcttca actacattgc tgtcagggtt gtgctcacac agcttgagag ttacaaccgg   300
   gaagcatatg gtgagctgat ggatttcgtg aaccgaaact ccctcaacga cgctgatact   360
   ttctgcaaga agttgatccg cgagtctcca aggcacaagc agctagcaat gaggatcttg   420
   gaggtccgat ctgcttatgt caagcatgat ttcgagtggg ataatctgaa aaggttatct   480
   ttcaagatgg ttgatgaggc caacacaaag ctcatgaggg actatgtctt ggagaccagc   540
   cacatcgaag acgataactg a                                             561
<210> 86
   <211> 1044
   <212> DNA
   <213> Oryza sativa
<400> 86
   atggacatca ccggcgcggg cgctatggga ggaggatcca cggccgccac cgccgccgcg 60
   gcggcggggg ccgggtggaa gacgccggtg tcgatggtgc tggtgcagct gttcatcacg 120
   gggcagatcc tgctgtccaa ggtgtccatc ggcggtggca tgttgatctt cgtgctgctt 180
   gcgtacaaca gcttcttcgc cgtcgtcttc ctcctcccct tcgcgctcat cttcgagaga 240
   ggcaagtgga gggacatgga ttggggtgca ttcggatgga tctttctcaa tgcgttcatt 300
   gggtactctg tgccaatgag cctctactac tatggcctca aggataccac atcatcctat 360
   tccgttatct ttcttaatat aactcccctg ttcaccttca tcctctcact tatgttcagg 420
   ttggaggcat tcaaacttag aagcatacct ggagtactga aaatagcgag catactgctt 480
   tccattggag ggacaatgct tataagcctt tacaaaggca agtcattgca tctctgggat 540
   tctatcatac aacaccaaaa tgaacacaag tcagcaacca atcagctaag aggaacaatt 600
   ctattggttg gcagcagctt cacctttgct tgctggtttc ttattcagtc aaagattctc 660
   aaagtgtatc catacaaata ttggtcgtcc atggtgacat gcttggttgg agtatttcaa 720
   accgcattgg tcggaatcat attaaggaga gacaagagtg catgggagct aggatggaat 780
   ctcaaccttg ttaccatcgt gtacacgggg gcacttgcaa cagccgggaa atatatattg 840
   aattcatggg caataactaa gcgaggccca acctatccca caatgttcag tccattatca 900
   gtcgtcttca ctgttgtgtt ggattcagtc cttctaggaa atgatattac aattggaagt 960
   cttctaggca cagcattggt gattgtcggg ctctaccttt ttctctgggc caaagcacga 1020
   gaaataccta agaagtcaac atag 1044
<210> 87
   <211> 528
   <212> DNA
   <213> Oryza sativa
<400> 87
   atggctcggc gcgctgcgga aaaagaggcg gcgctccgcc agggtctcac cgccggtgac 60
   ggcgaggcgc gacggacagg tgcattgtgg cggacagatg cgtggcggca gcgggcagct 120
   gctagtgcgg ctgcagcgtc ggtggtgcgg acttggccga gctctgcgcc gtggctgcgg 180
   tttgagctgg atccatggcg acgggtctgt ggagaacagg acctgcagac cgcggcctgc 240
   ggcggcggcg acggcgccgt ggggctcagc ttcgagacgc accacggcgg cagcgtggcg 300
   ccttcgccgg agttcgcggc gtgcgcggcg agctcttgca gcgcggaact catggtcttg 360
   ctggtcctgc agcgcggcga gctcctggtg cgccatgacc ggccgagcca tcaccaccgc 420
   cgtcgcttcc caacgccgca accagccgaa gccgccgccg cagttgaagt tggatgggga 480
   tttcagaatc ccagagatgc aatgacttgc ctttgtaaag gcttataa 528
<210> 88
   <211> 921
   <212> DNA
   <213> Oryza sativa
<400> 88
   atgggcagcg gcggcggtgg ctgcggcagg aacggcgctg tgaggcagta catcaggtcc      60
   aaggtgccga ggctgaggtg gaccggggag ctccactgca gcttcgtcca agccatcgaa     120
   ttcctcggtg gccaagacaa ggctacacct aagctcattc ttcagctcat gggggtgaag     180
   gggctgacca tatctcatgt caagagccac ctccagatgt acagatgctc caggctcggc     240
   tcccatggca cgggaaggag atcagagatg caaccacagc tgcaaaggaa gcactcatgt     300
   ggtgctgatg agcaagtccc cagagaattc ctgtgccccc ctctgaaaag gaccaggatg     360
   gggacagaag ccacatacaa aggcatgcaa ggaagccaag gaatcagtga gatgaggact     420
   actggcaccc agtactgcat tgatgattac atgcaagcca tggcaatgga gaggagaata     480
   aaggaggagg gcctcagatg gcagagggat gctgctgctg ctgctgctgc agatggtggt     540
   gctgctgctt ccaacctcca aaccgtggga tgttcggtgc aagaatctga cccctttaag     600
   atcatcaaac cagaagtgca ccatcttggt cccgtgttga agctgcaatg ctccaaggtg     660
   gagaacagtg gattcatctc cagcagcacc ggcacggctg caagggatca accggagccg     720
   ccgccgctgg agaaatgttc gctgtcactc tccctcggtc cagaccccaa atgcatgccg     780
   gcgatcgcct cgtcgccgag cgaaagcagc tgcatcctct cgtcgtcgtc caggagcttc     840
   agcgactgct ccgggaactc aggttgtctt gttgccccgg gtgtgaactt ggaactctcc     900
   atgtccatct gtggatctta g                                               921
<210> 89
   <211> 564
   <212> DNA
   <213> Oryza sativa
<400> 89
   atggctgccg ccgaccagcc cgcctacggc gatcgccggc cgtcccggcg cacgtacaag      60
   ccggaccagc cggagggcct caccatctcg ttccgcgagc tctacgacct gccgacctcg     120
   ccggagttcc tgttccacga ggaggcgctc cgcagccgcc gcacctgtgg cgaggacctc     180
   acgttctaca ccggctgcgg ctacctcgtc ggccgggcgg cgggggcggc cgcggggctg     240
   aagcgcgcgg cggaggaggc ggagcgcggc gagtcgatga agctgcgggg gcagccgcgt     300
   cctcaaccag tgcggctccc tcgggcgcgc gtacggcaac cggctcggcg tcgtcgcgct 360
   gctcttcgtg gggatcgaga gcaccgtggg gggcctccgc gacgccgacg gctgggccaa 420
   caccgtcgcc gccgggatcg gtaccggcgc gctctaccgc gcggctgccg gcccgcgggc 480
   ggcgatcgtc ggcagctccg tcggggggct catggccggc gcggtggtcg tggggaggca 540
   agcgctgacg agatacgcgc ctaa 564
<210> 90
   <211> 1275
   <212> DNA
   <213> Oryza sativa
<400> 90
   atggacatgc cgcccacgcc ccttccgccg gagaccgcca acacctcgcc cgctcccaac 60
   ggcgccaccg ccggaatccg ggttgagaac tgctacgtct tcaagagccg gctgcaagag 120
   tatgcacaga aaaccggcct ccagactcca gagtatcata ccttcaagga gggaccttcc 180
   cacgagcctg tcttcaagtc cacagtggtg attaataata ccagctatga ctccctgccc 240
   ggattcttca acagaaaggc tgcagaacag tctgctgctg aagttgccct catggaaatt 300
   gtcaagtcca taccagccaa cgcaaatatc ccagctgttc aagagactgg gctgtgcaag 360
   aatcttcttc aggagtatgc acagaagatg aattatgcca ttccatctta tatttgcacc 420
   aaatcagcct caggcttagc tcctttcata tgcactgtag agattggtgg aatacaatat 480
   attggtgctg cagccaggac aaagaaagat gcagagataa aagctgcccg aactgctctt 540
   ctggcaatcc aaggtcaatc agagggttcg gcaaatggtg caacaaaata tattgtagtt 600
   cctggtaaaa gggtaggtaa ggaggtagag aaaaggccaa ttgaaacacc gaaaccactt 660
   aaagtaaaga aaggtggttt caagaagaaa tggaacaaga ggaaattcat gaagaaggat 720
   ggtcaagctg ttgatgtgga aaaggatgaa gctagagtgg ctggagatgc tcacgattct 780
   gatgtcctaa tgcagccaac agtaataaca caggaggcat cttgtggcac tctgttcctg 840
   caaccttgtg aggaagctaa aagagtagaa gctgagccac ctagagatat tgaaatggta 900
   cagcctgata aggagaacca acacagtgac gctgcattgg tgcaacctga tgatgaagct 960
   agagtagaac aggagccatc cagagatatt tcagtggtgc aacctaatga ggaagctata 1020
   agtggtaagc aggaaccatc catcgatgct gcaattctgc aacctaaaga ggaagcttca 1080
   agtgtaaagc aggagccatt catcgatact gcaatgctgc aagcttgtaa ggaagctgga 1140
   agtgtagaac ttgggccagc cagagatact gtaatttccc aacttaatga gcaagatagg 1200
   gctgtaaagc aggagccagc tggtgacatt gtagtgccac aacctgacgt gcacgctagg 1260
   gtcgtaaagg agtag 1275
<210> 91
   <211> 4080
   <212> DNA
   <213> Oryza sativa
<400> 91
   atggcattgg gggatctcat ggcctccagg ctcgtccact cctcctcctc ctccgccgcg 60
   ccatccgccg ccctgcccaa tcaccatacc aaccacctcg tcgatgacca cctccccgtg 120
   gagaatggac cggaccccag gagggacgtg cccgacgagg agccgccgcc cccgccgccg 180
   ccgcaggtcg ccttgctgcc ccaggtggtc gtgctgtgcg agcagcggca cgaggggttc 240
   gacgaggccg ccgctgcggc ggccgggccc tccaccagcg ggcccgtctc caaatggcgc 300
   cccaaagacc ggatgaaaac tggctgtgtg gcacttgtgt tgtgtttaaa cattagtgtt 360
   gatccgccgg atgtgatcaa aatctcccct tgtgcaagaa aggagtgctg gatagatcca 420
   ttttctatgg cacctccaaa agcccttgaa actattggga aaacattaca ctcacaatat 480
   gagcgctggc agccaaaggc tcgttacaag cttcagctgg atccgacatt agaggaagtt 540
   aagaagctat gtaatacttg ccgtaaattt gctagaacag agagagtcct ttttcattac 600
   aatggtcatg gtgtaccaaa gcctacagct aatggggaga tttgggtatt taacaagagt 660
   tacacacagt atattccgct tcctattact gatcttgatt catggctgaa aacaccctct 720
   atatatgttt ttgactgctc agcagctgga atgatcgtga aagcttttct ggagcgccta 780
   gactggagtt ctagctcgtc tgcatcttca tcgaaggact gcattctcct tgcggcctgt 840
   gaggcacatc aaactctccc acagagcgca gaatttcctg ctgatgtgtt cacagcttgc 900
   ctcaccacac ccatcaaaat ggcactgcac tggttttgta accgatcgtt actccgtgat 960
   tccatggaac acaatcttat cgaccaaatt cctggaaggc aaaatgaccg caaaactctt 1020
   ctaggggagt tgaactggat tttcactgct atcacagaca ctattgcatg gaatgttctt 1080
   cctcatgatc tattccaaag acttttcagg caggatcttt tggttgctag tctctttcgc 1140
   aacttcttac ttgctgagag aatcatgcgg tccgcaaatt gttccccaat ttcataccct 1200
   ttgttgccac caactcatca gcaccatatg tgggatgcat gggacatggc tgcagagatc 1260
   tgcctttcta agcttcctca attaattgct gatcctaatg cagagtttca gccgagtcca 1320
   tttttcacgg agcaattgac agcatttgaa gtttggcttg atcatggctc tgaagacaag 1380
   aaacccccag aacagctacc tattgttctt caggttttgc ttagtcagtc acacagattt 1440
   agagcacttg ttctgcttgg aagatttctt gacatgggac cttgggcagt tgatttggct 1500
   ttgtccgttg gcatcttccc ttatgtactt aaactgcttc aaacaagtgc aatggagttg 1560
   cgccaaattc ttgtgttcat atggacaaaa attctctctc ttgataagtc atgccaggtt 1620
   gacttggtga aagatggagg gcatgcatac tttatcaggt ttcttgacag tttggatgct 1680
   tacccagagc agcgtgcaat ggctgctttc gttttagccg ttattgtgga tgggcatagg 1740
   attggtcaag aggcttgtgc taatgcaggg cttatagatg tctgcctgag acatctgcaa 1800
   cctgaaaatc cgaatgatgc tcagacagag cctttgctct tgcaatggct ttgtttatgc 1860
   cttggcaaac tttgggaaga tttccctgag gctcagttac ttggtctgca atcaaacgca 1920
   ccggaaattg ttatatgctt attgtcagag cctcaacctg aagtcagagc ttctgctgtt 1980
   tttgcacttg gaaatcttgt ggatattgga tctccatcac tgaatggagc tgacgacgat 2040
   tctgatgatg atgaaaaggt gagagctgaa ataaatgttg tccgaagcct tctgcagatc 2100
   tcttcagatg gtagccctct tgttagatct gaggttgccg tagcgcttac ccgctttgca 2160
   atggggcaca ataaacatat caaatctgtt gccgccgagt actggaaacc tcaaaccaat 2220
   tcactgctca agtcattacc atcgttggct aatattaatt cgagcaatgt ttacagtccc 2280
   agcagcttaa tacaaggtag cagtggcctt gcctcacata ttggtcctgt tttaagggtt 2340
   ggcagtgata acagtgccac tgctcgtgat ggaagaatct ctacgagcag cccgattgca 2400
   acaaatagca tcatgcatgg ttctccacag tcagatgatt cttcccaaca ctctgattca 2460
   ggcatattac tgagagagaa tgcaagtaat ggtggtctca actactcaag atcgaggcct 2520
   attgataatg ggatctattc ccaatttata gcaactatgt gcaatgttgc taaagatcct 2580
   tacccaagaa ttgcaagtat tgggaaaagg gcattgtccc tcataggtgt tgagcaagta 2640
   agcatgagaa acagtagact tagcaatgga ggtgcacacc caggagagac atctgtgccc 2700
   ccttcatcaa actttggaat ggcacgctcc tcttcctggt ttgatatgaa ctctggaaat 2760
   ttctcggtgg cctttaggac tcctcctgtt agtccccctc agcatgacta cctcacagga 2820
   ttgcgccgag tgtgctcgat ggagttcaga ccacatgttt tgaactcacc tgatggctta 2880
   gctgatccgc ttttaagctc cagtgcagcc cccagcaaca tggggctcta tatacttccc 2940
   caatcattaa tttacagatg gagttgtggt cacttttcta ggccacttct aactggttct 3000
   gatgataacg aggaagcaaa tgctagaaga gaagagcgag aacgaattgc aatggattgc 3060
   attgctaaat gccaacgatc atcttgcaag atgaccagcc aaattgctag ctgggatacg 3120
   aggtttgagt tgggtacaaa agcatcattg ttgttgccat tttctcctat tgttgttgct 3180
   gcggatgaaa atgagcaaat acgagtatgg aactatgacg atgcgctgcc agtgaatact 3240
   tttgaaaacc acaagttatc tgacagaggc ctatctaaac ttttgctgat caatgagctt 3300
   gatgatagct tgttgttagt tggctcaagt gatggaaatg tccgcatatg gagaaactat 3360
   actcaaaagg gaggacaaaa acttgtaact gctttttcat cagttcaagg ctatcgaagt 3420
   gctggtcgca gtattgtatt tgattggcag caacagtcgg gttatctgta tgcatctggt 3480
   gacatgtcct ctatccttgt atgggatctt gacaaggaac aagtcaacac catccagtca 3540
   actgctgata gcgggatttc agctctttct gcatctcagg ttcgatgtgg ccaattcgct 3600
   gctggttttc ttgatgcatc tgttaggata tttgacgtgc gtacacctga taggctagta 3660
   tatacagcaa gaccacatgc cccaagatca gaaaaggttg ttggtatagg atttcagcct 3720
   gggtttgatc cctacaagat tgtaagtgca tctcaagctg gagacattca gttccttgat 3780
   gttagaaggg catctgaacc ctacctcact attgaagcac ataggggttc attaacggca 3840
   ttagctgttc atcggcatgc cccagttatt gcaagcggct cagccaagca gatgatcaaa 3900
   gtgtttagtc ttgaaggaga acagttgaca ataattcgct accagccatc ttttatgggt 3960
   caacgaatag gcagcgtaaa ctgcctttct ttccaccgat acaaatcact ccttgccgct 4020
   ggtgctggtg ataatgctct tgtttctatc tacgcggagg acaattacca agtacgatga 4080
<210> 92
   <211> 702
   <212> DNA
   <213> Oryza sativa
<400> 92
   atgggtgcca gcggaaggct gatctccatt tacccagagg atctcacttt cctatttgag 60
   ctagataagc catgctattg caatctcaag gtggtgaaca acagcgagca tcatgttgca 120
   tttaaggtca agacgacatc accgaggaag tattttgtcc ggccgaacgc gagcatcatc 180
   cagccatggg attcttgcac aataacaatt acgctccagg cgcagaaaga gtacccacca 240
   gatatgcaat gcaaggataa attcttgatc cagagcacca aggtagctgc cagtactgac 300
   atggacgaga tcccccctaa cacgttcaac aaggaagtcg ataaggtgat tgaggaaatg 360
   aagcttaagg ttgtttatac agttcccagt ggaagttctg acgactctgg tattacatct 420
   ttaggcagca ggagcttcaa attggggtct gacgatctca cgatgctgaa gaatgcaagc 480
   attgaaaaga tacagacaat acaacgccta aaagacgaac gagacaccac cctgcagcaa 540
   aatcagcaaa tgcaacgtga attggatgtg atcaggaggc gtagaagccg caaaagcgat 600
   gcgggtttct ccttaacgtt tgctgctttt gctgggctca taggtgtcct gattgggctc 660
   ttgatgagcc tcatcttccc tcgcccacag gctgctgctt aa 702
<210> 93
   <211> 2820
   <212> DNA
   <213> Oryza sativa
<400> 93
   atgggggtga tgaacccgct gatggcaaag ctcaccacgc tcatgggcga cgagtacaag      60
   aagctcaagg ggctcaggaa gcaggtctcc ttcctcaagg atgagctcac caccatgagc     120
   gctttcctcg agaagctcgc gctcatggat gatgatgatg atggtgagct cgatcctctg     180
   gccaaggact ggaggaacca cgtcagggag atggcctatg acatggaaga ctgcattgat     240
   gattacttca caagtcatct tgatcatcgt tactcttcct cagatgcagg gttaatccgc     300
   aagatagctc gccgtctcag ggcattgcgg gtgcgtcatc gcatagccag ccagatcaat     360
   gagctcaagg ctcgtgtggt cgaggcaaat gagcgtcgcg tgagatacag gcttgatgac     420
   tgtaacaaca aacatggtgt ttctgccaat cctgctatag atccacggat aacatcgctc     480
   taccaaaatg ccgggagtct tgtgggtatt gatggcccaa gccaagaact aatccagctg     540
   ttgtcgttag atcgtgatac cgatcaacga caactcaaag tggtgtccgt cgtgggattt 600
   ggaggtctcg gtaaaacaac tcttgcaaaa tatgtgtacg acaagatcgg gcatcaattc 660
   gattgcacgg cattcgtctc agtatcccac aaacctgaca taacaaggat cctcagtagc 720
   atccaatcca agcttgacat agggggcacg tctcaagctt gcgacgacgt gcaacaactc 780
   atcgacgaca tacgagccta tctggagcat gaaaggtata ttattatagt cgatgacctg 840
   tggaaacaag aagcatgggt tattattagt tgtgcctttc caaacaatgg caaaggtagc 900
   agagtgatag taaccacacg agtgaaagat gtggccaggt tggcatgtgg caaggatgga 960
   caaatttaca aaatacagcc tctgaacaat aaggactcaa gaaagttatt cttcgataga 1020
   gtatttaggc ctgaagatag ttgtgtcctg cagtatgaag aaatttcaac tgaaatctta 1080
   aagaagtgta gtggcttgcc acttgcaatt gttactgtag ggagcctctt agcctgtcga 1140
   ccaagaacaa tggaagaatg gaagagcata cgggattctt tgggtgcccc gtttgacaaa 1200
   aacaagagct tggaaggcat gaggaatatt ttaaacctga gttacaagaa tcttcctctt 1260
   catctcaaaa catgcctcct atatattggt aaatatccag aggactatga gatcgggagg 1320
   gatgaactag ttacggagtg gatagctgaa ggtattatgg gtaaccctca tggggaaaac 1380
   ttggaggcta ctggtaatgg ctacttcagt gagcttatca acaggggctt gattcaacca 1440
   gagagcaccg gctatggtgg ggaggtgttg agttgcaagg tgcatgatat gatgcttgat 1500
   ctgatcctca tcaagtgtgc agaagataat tttgtcagcg ttgcacacag ttgtaaagac 1560
   tacatgcgca tggctatgca ccatgagcgg agttgcaata aggtccgtcg gctatccctg 1620
   cagtgcaagg ctgcaagatc agattgcgca attgagggca gcgtcatttc tacaagcatg 1680
   gctcgtgctc gatcagtttc agtgtttggt gagtgttcac gtgggctccc atttctgatg 1740
   ctatccaagt atatacgggt ggtgcacatc gaattggaag gccatggcgg tcaagtcgac 1800
   ctcactgcca ttagccatgt gcttcaattg aggtatttga gagtggagac gcctggttgt 1860
   gagatagatc tccccagcaa aatatgtggg ctcgtgcatt tggagacatt gtcaatattt 1920
   tcccataaag ctgtaagtcg gctcccttca gatattagca gtcttccccg cttgtcagtc 1980
   ctgtccctgg tggttccatg ggctacaagg ctacccaaca agttaaacaa gctaaaaggg 2040
   tcactacgca gtctcaccat actattcaat cccccggatg cgttaggcat ggaggccatt 2100
   ggtgagctga aaaatctaag ggacctaaac atctctgtta acaggtggcg ggacgatgag 2160
   atccttagcc tttatgctct ggggtcttcc attggaaaac tggatgaact caggagtttg 2220
   caaattcatg tcccacctgc taccttaggt gatgttgacc tgctgggctc attacccatt 2280
   tttcctcaaa gtatcgagag actaatacta cacggttggt gcttctccaa agtacctcga 2340
   tggatcaacg gtactctccg taacctccaa catgtgttgc tggaggtatc ggagacatcg 2400
   agtagcgagg ttgaccttct tggtgaacta ccctccctcg ccgacctcga gctgagagta 2460
   ggactcaaga caagagatgt catcgcgttc ggtggcacta gagcatcatt attccctgct 2520
   ctcctgaaac tcaagctgcg tgtgggtgaa cacgttgcct caaggctgca gttccaggca 2580
   ggggtgatgc ccaagctcca aagcctccat ctgtggttcc ggaattgtga gtcgggcatt 2640
   cacgtaacac cggagggtat gcagcacctc ctgagcctcc aaagcatctg cgtggagata 2700
   tacctccggg atgaggagct gaaagcaact tatccatggg acgccatgga gcgtgcgttc 2760
   agggaaatca ctggagcaaa ccccaaccgg ccttccttca aatttgtcaa gcaagtctga 2820
<210> 94
   <211> 1014
   <212> DNA
   <213> Oryza sativa
<400> 94
   atggagtgcg agccggagga gctgcaattc ctgggcatgg tgggtatcta cagggaggcg 60
   gcgtccatcc tgcgcgccca ccggccgctc ttcgcccgca tcgccgccgc cttcgtcctc 120
   ccgctctccc tcctcttcct cctccacatc gccatctccc acgccctctt ctcccacatc 180
   gactccgacg actccgccct cgactccgcc gccccgggca cccccgccca gcgccgcctc 240
   ctccaccgcc tcgccgacga ctggctcgcc ctcctcctct tcaaggccgc ctacctcctc 300
   gccctcctcc tcttctccct cctctccacc gccgccgccg tcttctccgt cgcctccgtc 360
   tactccgcca agcacgacgc cctctccttc cccagggtcc tctccgtcgt cccccgcgtc 420
   tggcgccgcc tcgccgccac cttcctcgcc gccttcctcc tcctcttcgc ctaccacctc 480
   ctcttcgtcg ccgtcttcgt cgccctcctc gtcgccgccg actccggatc gggcctcgcc 540
   gcgctgctcg ccttcctcct cgccctcgcc tacatcgcgg gcctcgtcta cctcagcgtc 600
   gtctggcacc tcgccagcgt cgtctccgtc ctcgaggact acaagggatt cgaggccatg 660
   cgcaagagca aggcgctcat acagggcaag ctctggaccg cctccgccat cttcttcgtc 720
   ctcaacgtcg tcttcatcgt cgtcgaggtc gccttccggg cgtgggtggt gcgcggggcc 780
   acccacggcc tcggcgccgg ctcaaggctc ctcctgggcc tcgccatgct cgccgcgctc 840
   tgcgctgtcg tgatgctggc gctcgtggtg cagacggtgg tgtacctggt gtgcaagagc 900
   taccaccacg agagcatcga caagagcaac ctctccgacc acctcgaggt ctacctcggc 960
   gagtacgtcc cgctcaaggc cagcgacgtc cagatggagc aattcaacct ctga 1014
<210> 95
   <211> 936
   <212> DNA
   <213> Oryza sativa
<400> 95
   atggcgtcct cctccgccct cgcttcctcc cccttcctcc cgcccctctc aaccccaaac      60
   cctagggccc tctccctccg cctccccgct cgccgcctcc ccgtggcgtc ctccgcggct     120
   ccctcgggcg ctgccgctgc ggcgtcggcg agggagcgcc gccgcttcct ggagcggtac     180
   ggcctcaacc ccgacgactt cgaggacgat gccgaggcgg aacccaggga agagaggaga     240
   agggataggc ggaaccggcg gtcgggtaga ggggaggcgg aggatgctcc ggcgaaggcg     300
   gcggctgagc ctcgggagac gcataaaatg cttcaggtgt taggaggaaa agtacgcaga     360
   agaaaattac tttcaccaaa agataggaat gttcgtccaa tgatggaagt tgtacgaggg     420
   gcagcctttg acattttaca gtcagctggt ggttttccgg cttcgcttag acctggtcga     480
   tggttagact tgtatagtgg tactggatct gttggaattg aggctatgag ccgtggatgt     540
   tcagaggcac attttgttga gatggatcct tgggttgttt ctgaggtcct taaaccgaat     600
   ctggagtgta ctggatttct tgatgtttcg cacatacata tgatccgcgt cgaaaacttc     660
   ttggccaatg ctgaaaaatc tagtggtaaa tatccttctt ttgattatat tagtgtaaca     720
   ccgccatatc ttgaggtaaa ctacagtaca ctactcgatc aacttgcaag gtcaccattg     780
   gttggagaag attgcttcat tctcgttgaa tacccactga aaacagacat ggccgaatca     840
   tgtggaagcc ttataaaagt agctgacagg aggtttggta ggacaaactt gctaatttat     900
   gggccaacct gggctgagaa gaagaggaga tcttga                               936
<210> 96
   <211> 936
   <212> DNA
   <213> Oryza sativa
<400> 96
   atgaacgacc tcatgaccaa gtcgttcatg agctacgtcg acctgaagaa ggcggcgatg      60
   aaggacctgg aggcgggcgg ggatggcgtg gagctccccg aggtgggcgt caccgacgag     120
   cgcctcaagg ggttcttcca ggagacggag gcggtggagg aggagatggc cgccatccgc     180
   gacgcgctgg cgaggctcaa cgccgccaac gaggagggca agtcgctgca ccagcccgac     240
   gccctccgcg cgctccgcgg ccgcgtcaac gccgacatca tcgccgtgct ccgccgcgcg     300
   cgcgacatcc gcgccaggct cgaggccatg gaccgcgcca acgcggcgca gcgcaggctc     360
   tccgcgggct gccgcgaggg caccccgctc gaccgcaccc gcaccgcgct caccgccgcg     420
   ctccggaaga agctcaagga cctcatgctc gacttccagg ccctgcggca gcggatcatg     480
   tccgagtaca aggacaccgt cgagcgccgc tactacaccc tcaccggcga ggtccccgag     540
   gaggaggtga tcgagcgcat catctccgag ggacgcagcg aggagctcct gtgcgccgcc     600
   gtggcggagc acggcaaggg cgcggtgctg gccacggtgc acgagatcca ggaccgccac     660
   gacgccgccc gcgaggtgga gcgcagcctc ctggagctcc accaggtgtt cctcgacatg     720
   gccgtggtgg tggagtccca gggggagcag ctcgacgaca tcgagcgcca cgtcaacagc     780
   gccaccacct acgtccaggg cggcaacaag gagctacgca aggcccgcga gcaccagcgc     840
   agcagccgca agtggctctg catcggcatc atcatcctgc tgctcctcgt cctcctcgtc     900
   atcgtgccca tcgccaccag cttcaagaga tcgtga                               936
<210> 97
   <211> 564
   <212> DNA
   <213> Oryza sativa
<400> 97
   atggcgatgg aggggaagag caggaggttc gcggtggcgt gcggggtgct cagccagtac      60
   gtgagggcgg agcagaagat ggcggcggcg gcgggggcgg caccggcgag ggcggtgacg     120
   acgctgagcc tgatgcctgg ggcggaggtg gtcgtcgagg aggaggagcg gagggaggtt     180
   ggggaggagg aggcggggcc agcgacggcg ccggccgcgc cgctgaccat cttctacggt     240
   gggaggatgg tcgtcttcga ggacttcccc gcggacaagg cggcggaggt gatgcgcatg     300
   gcctcctccg ggatggcggc ggcgccggct cagcgggagg gcgccgcgct cgcggacatg     360
   cccatcatga ggaaggcgtc gctgcagcgg ttcttcgcca agcgcaagga ccgcctcgcg     420
   gcgaccaccc cctacgcccg cccgtcgccg gcggagacca aggcctccga gccggaggag     480
   aagaagacgc ccacctcatg gctggacctc gccgcctccg cctccgccgc cgcgcgccgt     540
   gacagcctca ccatcgcgct gtga                                            564
<210> 98
   <211> 3402
   <212> DNA
   <213> Oryza sativa
<400> 98
   atgtcgtcgc tgagccggga gctggtattc ctcatcctgc agttcctcga tgaggagaag      60
   ttcaaggaga ctgttcacaa gcttgagcag gagtctgggt tctacttcaa catgaagtac     120
   ttcgaagacg aggtgatcaa tgggaattgg gatgaggttg agcgctacct cggtggcttt     180
   accaaggttg atgacaaccg ctactcgatg aagatattct ttgagatccg caaacagaag     240
   tatcttgagg cccttgataa gcatgatcgt tcgaaggcgg ttgaaatctt ggtcaaggac     300
   ctgaaggtct tcgcgtcctt taacgaggag ttgtttaagg agatcacaca gcttttgacg     360
   ttggaaaact ttagggaaaa tgagcaactc tccaaatacg gtgatacaaa atctgccaga     420
   gcaataatgc ttgttgaact aaagaagctg attgaagcta atcccttgtt ccgtgacaag     480
   cttcagtttc caaatctgaa gagctccaga ttgcggacac ttataaacca gagcttaaac     540
   tggcagcacc agctttgcaa aaatcctaga cctaaccctg atatcaagac tctgtttgtt     600
   gatcattctt gtggacaacc aaatggtgct cgtgctccat cgccagcaaa caatccatta     660
   cttggatcta taccaaaacc tggaggtttc cccccattgg gtgctcacgc gccatttcaa     720
   cctgcaccta cacctgtccc acctctggct ggctggatgt caaaccctcc agcagtaaca     780
   catcctgctg tgtctggtgg agctattgga tttggtactc ctacaaatcc tgctgctata     840
   ttaaaacatc ctagaacacc aacaactgcc aatccttcta tggattatcc atcaggagat     900
   tctgatcacg tctccaagag aacgagacca gttgggatgt ctgaggaggt gaatcttcca 960
   gtgaatatgt tacctgtgac atatccacag agtcatagtt acccgcaaga tgattttcat 1020
   aaaaatgttg cacggacatt gagccaagga tcaactccaa tgagcatgga cttccatcca 1080
   gttcagcaaa ctctccttct tgttggtacc aatgttggtg acattggatt atgggatgtc 1140
   ggtaccaagg aacgacttgt tttaagaaac ttcaaggttt gggatcttac aaaatgctca 1200
   atggccctcc aggcatcact tgtcaaagac cctactgtct cagttaaccg cataatatgg 1260
   agtcctgatg gaaccttgtt tggtgttgct tattcaaggc atattgtaca gatctattca 1320
   taccatggcg gtgatgatat caggcagcac ttggagattg atgcgcatgt cggtggtgta 1380
   aatgacattg catttgccca tccaaataag cagctatgta taataacctg cggagatgac 1440
   aagacaatta aggtctggga ggccactagt ggagcaaagc aatttacatt tgaaggtcat 1500
   gaagctcctg tttactctgt ttgtccacat tataaggaaa atattcagtt catcttctca 1560
   actgctttgg atggaaagat aaaggcttgg ctatatgata atttgggatc cagagttgac 1620
   tatgatgcgc caggacattg gtgcacaaca atggcatata gtgcagatgg ttcaaggtta 1680
   ttttcttgtg ggactagcaa ggatggcgaa tcacatctag tggaatggaa tgaaagtgaa 1740
   ggagctgtca agagaactta ccagggattt cgcaagcgat cgatgggtgt tgtccaattt 1800
   gataccacac ggaacaggtt tttggctgct ggagatgaat tcttgattaa gatatgggac 1860
   atggacaaca caagtcttct gactaccatt gatgccgatg gtggtcttcc tgcaagtcca 1920
   cgggtccgat tcaacaagga aggtactctg ctggctgttt ctacccatga aaatggtatc 1980
   aagatcttag caaatgctga tggagtacgg ttgctgcgca cattggaaaa tcgttcattt 2040
   gatgcttctc ggagtgcgtc tgagactgta acaaagcccc taatgaatcc attgactgct 2100
   gctgctgctg cggcggcgtc agctgctgct gccgggacta gttcaggaaa tgctgctcca 2160
   ccggcaataa ctgcactgaa tggggatagc cgaagcttgg ttgatgtaaa gcctagaata 2220
   gctgatgagc cattggataa atcaaaagtc tggaaactta tggagataac cgagtcaagt 2280
   cagtgcagat cattgaaatt aacagataat atgaggacaa gcaagatttc aagacttatt 2340
   tacacaaatt ctggtgtcgc tatcttggct ttagcttcaa atgctgttca tctgctctgg 2400
   aaatggcctc gcaatgaccg aaactcaagt ggaaaggcta ctgcaagtgt ttctcctcaa 2460
   ttatggcaac ctccaagcgg catcctcatg actaatgaca taactgacaa ccctgaagaa 2520
   gctgtccatt gctttgcttt gtcaaagaat gattcatatg tcatgtctgc atctggaggg 2580
   aaaatatctc tgttcaacat gatgactttt aagacgatga caacttttat gcctccgccg 2640
   ccggcggcaa cgtttcttgc tttccatcct caagataaca acattatagc aattggaatg 2700
   gatgactcaa ccatccaaat ctacaatgtt cgaattgatg aggtcaaaag caaacttcga 2760
   gggcactcta agaaaattac tggacttgct ttttcaaatg tattaaatgt gttagtctct 2820
   tctggagctg atgcgcagat atgtgtgtgg agcacagatg ggtgggataa attaaagagc 2880
   agaatgttac agataccatc aagtcgtcca tcatctataa tcttagacac acgtgttcag     2940
   ttccatcagg atcaattgca ctttcttgtt gtgcatgaga cccagattgc catatatgaa     3000
   actacaaaat tagaacccgt gaagcagtgg cctgtccggg agaactcttc tccaataacg     3060
   catgccatgt tctcctgcga tagtcaattg atttatgcaa gctttctgga tgccactgtt     3120
   tgcatattta atgcatcgag tttgagactc caatgtcgaa ttcttccagc atcctatctt     3180
   cctcagaata tcagctcaaa tgtttatcct gtcgttgtgg cggcacatcc ttcggaagca     3240
   aatcagtttg ctctaggcct gactgatggt ggtgtttatg tattggaacc cttggaatct     3300
   gagagaaaat ggggaaatcc tccaccagca gagaatggat caaccagcgc tttgtccaca     3360
   cctcctaatg gagcatcaag ttctgatcaa ccagaaagat aa                        3402
<210> 99
   <211> 158
   <212> PRT
   <213> Oryza sativa
<400> 99
   Met Pro Arg His Ala Cys Leu Leu Gln Ser Asp Arg Ile Phe Phe Ile
   1               5                   10                  15
   Ala Thr Glu Glu Cys Arg Arg Arg Arg Arg Gln Gly Gly Ala Asp Ala
               20                  25                  30
   Gly Ala Gly Gly Arg Gly Gly Gly Arg Arg Cys Trp Ala Glu Ala Ala
           35                  40                  45
   Glu Ala Ala His Met Ala Ala Ala Ala Ala His Arg Ala Ala Ala Val
       50                  55                  60
   His Arg Ala Ala Cys Gly Ser Ser Thr Val Ala Val Gly Leu Arg Glu
   65                  70                  75                  80
   Leu His Arg Arg Arg Leu Cys Ser Thr Cys Thr Gly Asp Gly Asp Gly
                   85                  90                  95
   Asp Arg Met Leu Met Gln Arg Asn Asp Ser Ser Asn Ser Lys Trp Met
               100                 105                 110
   Leu Ser Arg Arg Ala Phe Ser Pro Phe His Val Ser Ala Lys Phe Ala
           115                 120                 125
   Trp Glu Val Gln Glu Ser Leu Leu Asp Gly Gly Ser Thr Trp Phe Cys
       130                 135                 140
   Leu Gly Ser Ser Ala Tyr Phe Val Ala Val Lys Tyr Asp Trp
   145                 150                 155
<210> 100
   <211> 117
   <212> PRT
   <213> Oryza sativa
<400> 100
   Met Gly Thr Tyr Lys Cys Cys Ile Phe Phe Thr Arg Arg Phe Ala Leu
   1               5                   10                  15
   Ser Asp Ala Ser Thr Pro Gly Asp Val Arg Met Leu Phe Thr Arg His
               20                  25                  30
   Ala Gly Gly Ala Pro Tyr Met Gly Ile Asp Glu Leu Arg Arg Tyr Leu
           35                  40                  45
   Ala Ala Ser Gly Glu Ala His Val Asp Ala Asp Thr Ala Glu Arg Ile
       50                  55                  60
   Ile Asp Arg Val Leu Gln Glu Arg Ser Arg Thr Pro Arg Phe Gly Lys
   65                  70                  75                  80
   Pro Ser Leu Thr Ile Asp Asp Phe Gln Tyr Phe Leu Phe Ser Glu Asp
                   85                  90                  95
   Leu Asn Pro Pro Ile Cys His Ser Lys Glu Glu Ser Phe Asp Ala Met
               100                 105                 110
   Glu Lys Leu Glu Val
           115
<210> 101
   <211> 490
   <212> PRT
   <213> Oryza sativa
<400> 101
   Met Gln Val His His Asp Met Asn Ala Pro Leu Ser His Tyr Phe Ile
   1               5                   10                  15
   Tyr Thr Gly His Asn Ser Tyr Leu Thr Gly Asn Gln Leu Ser Ser Asp
               20                  25                  30
   Cys Ser Asp Ile Pro Ile Ile Lys Ala Leu Gln Ile Gly Val Arg Val
           35                  40                  45
   Ile Glu Leu Asp Met Trp Pro Asn Ser Ser Lys Asp Asp Val Asp Ile
       50                  55                  60
   Leu His Gly Arg Thr Leu Thr Ala Pro Val Ser Leu Ile Lys Cys Leu
   65 70 75 80
   Lys Ser Ile Lys Glu Tyr Ala Phe Val Ala Ser Pro Tyr Pro Val Ile
   85 90 95
   Ile Thr Leu Glu Asp His Leu Thr Ser Asp Leu Gln Ala Lys Val Ala
   100 105 110
   Lys Met Val Leu Glu Val Phe Gly Asp Thr Leu Tyr Tyr Pro Glu Ser
   115 120 125
   Lys His Leu Gln Glu Phe Pro Ser Pro Glu Ala Leu Arg Gly Arg Val
   130 135 140
   Ile Leu Ser Thr Lys Pro Pro Lys Glu Tyr Leu Glu Ser Lys Gly Gly
   145 150 155 160
   Thr Met Lys Asp Arg Asp Ile Glu Pro Gln Phe Ser Lys Gly Gln Asn
   165 170 175
   Glu Glu Ala Val Trp Gly Thr Glu Val Pro Asp Ile Gln Asp Glu Met
   180 185 190
   Gln Thr Ala Asp Lys Gln His Glu Asn Asp Ile Leu Tyr Thr Gln Arg
   195 200 205
   Asp Val Glu Glu Asp Asp Glu Lys Lys Met Cys Gln His His Pro Leu
   210 215 220
   Glu Tyr Lys His Leu Ile Thr Ile Lys Ala Gly Lys Pro Lys Gly Ala
   225 230 235 240
   Val Val Asp Ala Leu Lys Gly Asp Pro Asp Lys Val Arg Arg Leu Ser
   245 250 255
   Leu Ser Glu Gln Glu Leu Ala Lys Val Ala Ala His His Gly Arg Asn
   260 265 270
   Ile Val Ser Phe Thr His Lys Asn Leu Leu Arg Ile Tyr Pro Lys Gly
   275 280 285
   Thr Arg Phe Asn Ser Ser Asn Tyr Asn Pro Phe Leu Gly Trp Val His
   290 295 300
   Gly Ala Gln Met Val Ala Phe Asn Met Gln Gly Tyr Gly Arg Ser Leu
   305 310 315 320
   Trp Leu Met His Gly Phe Tyr Lys Ala Asn Gly Gly Cys Gly Tyr Val
   325 330 335
   Lys Lys Pro Asp Phe Met Met Gln Thr Cys Pro Asp Gly Asn Val Phe
   340 345 350
   Asp Pro Lys Ala Asp Leu Pro Val Lys Lys Thr Leu Lys Val Lys Val
   355 360 365
   Tyr Met Gly Glu Gly Trp Gln Ser Asp Phe Lys Gln Thr Tyr Phe Asp
   370 375 380
   Thr Tyr Ser Pro Pro Asp Phe Tyr Ala Lys Val Gly Ile Ala Gly Val
   385 390 395 400
   Pro Ser Asp Ser Val Met Gln Lys Thr Lys Ala Val Glu Asp Ser Trp
   405 410 415
   Val Pro Val Trp Glu Glu Glu Phe Val Phe Pro Leu Thr Val Pro Glu
   420 425 430
   Ile Ala Leu Leu Arg Val Glu Val His Glu Tyr Asp Val Ser Glu Asp
   435 440 445
   Asp Phe Gly Gly Gln Thr Ala Leu Pro Val Ser Glu Leu Arg Pro Gly
   450 455 460
   Ile Arg Thr Val Pro Leu Phe Asp His Lys Gly Leu Lys Phe Lys Ser
   465 470 475 480
   Val Lys Leu Leu Met Arg Phe Glu Phe Val
   485 490
<210> 102
   <211> 211
   <212> PRT
   <213> Oryza sativa
<400> 102
   Met Ala Met Gly Ala Ala Ala Ala Pro Trp Tyr Gly Ala Ile Gly Gly
   1 5 10 15
   Gly Gly Ser Arg Arg Ala Arg Val Arg Ala Gln Ala Ala Ala Pro Trp
   20 25 30
   Ala Gly Gly Ala Glu Glu Leu Val Arg Ser Gly Ala Val Arg Ala Val
   35 40 45
   Arg Ala Arg Glu Ala Ala Gly Ala Met Ser Ala Glu Gly Phe Arg Leu
   50 55 60
   Leu Asp Val Arg Pro Glu Trp Glu Arg Ala Arg Ala Ala Val Arg Gly
   65 70 75 80
   Ser Ala His Ala Pro Leu Phe Val Gly Asp Asp Asp Thr Gly Pro Val
   85 90 95
   Thr Leu Leu Lys Lys Trp Val His Phe Gly Tyr Ile Gly Leu Trp Thr
   100 105 110
   Gly Gln Ser Phe Thr Lys Met Asn Asp Arg Phe Leu Asp Asp Val Ala
   115 120 125
   Ala Ala Ala Gly Glu Gly Lys Asp Ala Lys Leu Leu Val Ala Cys Gly
   130 135 140
   Glu Gly Leu Arg Ser Leu Ile Ala Val Arg Met Leu Tyr Asp Asp Gly
   145 150 155 160
   Tyr Lys Asn Leu Ala Trp Leu Ala Gly Gly Phe Ser Lys Cys Val Asp
   165 170 175
   Gly Asp Phe Ala Asp Val Glu Gly Glu Ser Lys Leu Gln Tyr Ala Thr
   180 185 190
   Val Gly Gly Val Ser Tyr Ile Phe Leu Gln Ile Leu Leu Leu Leu Arg
   195 200 205
   Val Val Lys
   210
<210> 103
   <211> 315
   <212> PRT
   <213> Oryza sativa
<400> 103
   Met Ala Ala Ala Ala Gln Arg Arg Arg Ser Ser Ser Ala Ser Pro Glu
   1               5                   10                  15
   Phe Arg Phe Trp Pro Leu Asp Ala Asp Pro Ala Ala Ser Pro Ser Cys
               20                  25                  30
   Ala Asp Glu Leu Phe Ser Gly Gly Val Leu Leu Pro Leu Gln Pro Leu
           35                  40                  45
   Pro Tyr Pro Arg Arg Asp Ala Asp Leu Ser Met Ser Leu Ala Val Ala
       50                  55                  60
   Asp Asp Asp Asp Asp Glu Asp Glu Glu Glu Glu Glu Val Gln Pro Gly
   65                  70                  75                      80
   Ala Ala Val Ala Ser Arg Ala Pro Pro Thr Ala Ala Val Ala Ala Ser
                   85                  90                  95
   Gly Gly Gly Gly Gly Gly Ser Lys Arg Trp Thr Asp Ile Phe Ala Lys
               100                 105                 110
   Lys Gln Gln Gln Pro Ala Ala Glu Glu Lys Glu Lys Asp Gln Pro Thr
           115                 120                 125
   Arg Arg Arg Arg Pro Ala Gly Gly Gly Gly Gly Ser Glu Leu Asn Ile
       130                 135                 140
   Asn Ile Trp Pro Phe Ser Arg Ser Arg Ser Ala Gly Gly Gly Gly Val
   145                 150                 155                 160
   Gly Ser Ser Lys Pro Arg Pro Pro Pro Arg Lys Ala Ser Ser Ala Pro
                   165                 170                 175
   Cys Ser Arg Ser Asn Ser Arg Gly Glu Ala Ala Ala Val Ala Ser Ser
               180                 185                 190
   Leu Pro Pro Pro Pro Arg Arg Trp Ala Ala Ser Pro Gly Arg Ala Gly
           195                 200                 205
   Gly Gly Val Pro Val Gly Arg Ser Ser Pro Val Trp Gln Ile Arg Arg
       210                 215                 220
   Pro Pro Ser Pro Ala Ala Lys His Ala Ala Ala Asp Arg Arg Pro Pro
   225                 230                 235                 240
   His His Lys Asp Lys Pro Thr Gly Gly Ala Lys Lys Pro His Thr Thr
                   245                 250                 255
   Ser Ala Thr Gly Gly Gly Gly Ile Arg Gly Ile Asn Leu Ser Ile Asn
               260                 265                 270
   Ser Cys Ile Gly Tyr Arg His Gln Val Ser Cys Arg Arg Ala Asp Ala
           275                 280                 285
   Gly Val Ala Arg Ala Ser Ala Gly Gly Gly Gly Gly Gly Gly Leu Phe
       290                 295                 300
   Gly Ile Lys Gly Phe Phe Ser Lys Lys Val His
   305                 310                 315
<210> 104
   <211> 306
   <212> PRT
   <213> Oryza sativa
<400> 104
   Met Gln Gln Lys Pro Ala Ala Glu Ala Met Glu Glu Glu Leu Lys Gly
   1               5                   10                  15
   Glu Ala Val Gly Pro Arg Arg Pro Gly Leu Gly Leu Trp Leu Ala Ala
               20                  25                  30
   Arg Arg Arg Leu Ala Pro Asp Asp Pro Phe Phe Ala Ala Gly Asp Met
           35                  40                  45
   Glu Arg Glu Leu Leu Ala Lys Gln Val Ala Leu Asp Leu Ser Glu Asp
       50                  55                  60
   Glu Arg Tyr Gln Leu Glu Arg Met Glu Val Ala Ser Ala Asn Ala Leu
   65                  70                  75                  80
   Leu Cys Pro Ile Ser Gly Cys Gly Ala His Leu Asp Cys Leu Glu Asn
                   85                  90                  95
   Phe Glu Asp His Tyr Arg Thr Arg His Thr Ala Ser Cys Ser Val Cys
               100                 105                 110
   Trp Arg Val Tyr Pro Thr Ser Arg Leu Leu Ser Ile His Ile Ser Glu
           115                 120                 125
   Ala His Asp Ser Phe Phe Gln Ala Lys Val Ala Arg Gly Phe Pro Met
       130                 135                 140
   Tyr Glu Cys Leu Val Glu Gly Cys Gly Val Lys Leu Lys Ser Tyr Lys
   145                 150                 155                 160
   Ser Arg Gln Gln His Leu Leu Asp Lys His Gln Phe Pro Lys Ser Phe
                   165                 170                 175
   Glu Phe Phe Lys Lys Ala Arg Pro Ser Gln Arg Gln Arg Asn Lys Asn
               180                 185                 190
   Gln Lys Gln Arg Gln Thr Val His Lys Gly Asp Glu Thr Ser Glu Thr
           195                 200                 205
   Leu Met Asp Val Asp Gly Lys Lys Ser Ser Arg Tyr Met Asn Ser Arg
       210                 215                 220
   Tyr Arg Pro Lys Gln His Asp Gly Lys Glu Ser Lys Glu Asn Glu His
   225                 230                 235                 240
   Ser Ser Cys Lys Glu Ala Lys Asn Asn Glu Met Glu Val Asp Lys Gln
   245 250 255
   Val Asp Glu Leu Ala Ser Ala Val Ser Arg Leu Ser Thr Ala Asp Ser
   260 265 270
   Thr Pro Ser Ser Ile Ser Phe Gly His Arg Arg Ser Arg Gly Leu Ala
   275 280 285
   Phe Val Pro Arg Ser Ile Arg Gln Asn Lys Gln Val Ser Gln Thr Glu
   290 295 300
   Pro Lys
   305
<210> 105
   <211> 328
   <212> PRT
   <213> Oryza sativa
<400> 105
   Met Gly Gly Gly Gly Gly Ala Glu Glu Glu Leu Thr Ala Gln Glu Thr
   1               5                   10                  15
   Ala Leu Tyr Asp Arg Gln Ile Arg Val Trp Gly Val Asp Ala Gln Lys
               20                  25                  30
   Arg Leu Ser Lys Ala His Val Leu Val Cys Gly Met Asn Gly Thr Thr
           35                  40                  45
   Thr Glu Phe Cys Lys Asn Ile Val Leu Ala Gly Val Gly Ser Leu Ser
       50                  55                  60
   Leu Met Asp Asp His Leu Val Thr Glu Asp Asp Leu Asn Ala Asn Phe
   65                  70                  75                  80
   Leu Ile Pro His Asp Glu Ser Ile Tyr Gly Gly Arg Ser Arg Ala Glu
                   85                  90                  95
   Val Cys Cys Glu Ser Leu Lys Asp Phe Asn Pro Met Val Arg Val Ala
               100                 105                 110
   Val Glu Lys Gly Asp Pro Ser Leu Ile Asp Gly Glu Phe Leu Asp Lys
           115                 120                 125
   Phe Asp Ile Ile Val Val Ser Cys Ala Pro Ile Lys Thr Lys Leu Leu
       130                 135                 140
   Ile Asn Asp Asn Cys Arg Lys Arg Ser Lys His Ile Ala Phe Tyr Ala
   145                 150                 155                 160
   Ile Glu Cys Lys Asp Ser Cys Gly Glu Ile Phe Val Asp Leu Gln Asn
                   165                 170                 175
   His Ser Tyr Val Gln Lys Val Gly Gly Glu Pro Lys Pro Lys Glu Leu
               180                 185                 190
   Ala Tyr Pro Ser Leu Gln Glu Ala Ile Ser Val Pro Trp Lys Asn Leu
           195                 200                 205
   Pro Arg Lys Thr Thr Lys Leu Tyr Phe Ala Met Arg Val Leu Glu Asn
       210                 215                 220
   Tyr Glu Ser Ser Glu Gly Arg Asn Ala Cys Glu Ala Ser Leu Ser Asp
   225                 230                 235                 240
   Arg Pro Ala Val Leu Ala Leu Arg Lys Asp Met Cys Asp Lys Met Ser
                   245                 250                 255
   Leu Ser Glu Ser Gln Ile Pro Thr Ala Leu Leu Glu Arg Leu Leu Ala
               260                 265                 270
   Ala Gly Lys Lys Gln His Pro Pro Val Cys Ala Ile Leu Gly Gly Ile
           275                 280                 285
   Leu Gly Gln Glu Val Ile Lys Ser Ile Ser Gly Lys Gly Asp Pro Ile
       290                 295                 300
   Lys Asn Phe Phe Tyr Tyr Asp Ala Ala Asp Gly Lys Gly Ile Ala Glu
   305                 310                 315                 320
   Asp Ile Pro Pro Leu Ser Ser Asp
                   325
<210> 106
   <211> 301
   <212> PRT
   <213> Oryza sativa
<400> 106
   Met Ala Met Pro Leu Pro Pro Pro Pro Pro Pro Arg Pro Pro Leu Gly
   1               5                   10                  15
   Arg Gly Arg Leu Val Gly Val Gly Pro Ala Pro Ala Pro Ala Thr Ala
               20                  25                  30
   Ser Gln Ser Asn Arg Pro Val Pro Pro Leu Gln Leu Pro Arg Cys Arg
           35                  40                  45
   Cys His Arg Ser Glu Gly Pro Trp Arg Thr Thr Ala Ala Ala Asn Gly
       50                  55                  60
   Arg Arg Arg Trp Trp Ser Asp Glu Asp Met Glu Glu Glu Asp Asp Glu
   65                  70                  75                  80
   Glu Gly Tyr Gly Tyr Asp Asp Gly Gly Ala Pro Gly Gly Ser Ala Gln
                   85                  90                  95
   Glu Leu Phe Gly Glu Pro Trp Phe Ser Lys Leu Phe Arg Ala Tyr Gly
               100                 105                 110
   Tyr Val Leu Pro Leu Leu Leu Ala Ser Met Leu Val Ala Thr Gly Pro
           115                 120                 125
   Arg Ala Phe Leu Met Ala Met Ala Leu Pro Leu Ala Gln Ser Ala Ile
       130                 135                 140
   Ser Trp Val Val Ser Phe Phe Thr Thr Arg Ser Arg Arg Gln Gln Glu
   145                 150                 155                 160
   Glu Glu Glu Ser Tyr Gly Tyr Asp Tyr Asp Asp Asp Pro Ala Phe Gln
                   165                 170                 175
   Arg Arg Glu Glu Asp Asp Asp Asp Gly Asp Tyr Tyr Asp Ala Gly Ala
               180                 185                 190
   Trp Gln Trp Arg Ser Arg Ser His Gln Gln Ser Thr Glu Ser Gly Ser
           195                 200                 205
   Gly Phe Gly Gly Trp Asp Asp Leu Leu Tyr Asp Asp Glu Glu Lys Lys
       210                 215                 220
   Glu Gln Glu Ser Ser Gly Lys Lys Arg Thr Pro Pro Glu Pro Asp Thr
   225                 230                 235                 240
   Ala Ala Ala Ala Ala Ala Ser Asp Leu Gly Leu Gly Leu Arg Ala Arg
                   245                 250                 255
   Arg Gly Pro Arg Arg Ser Asn Gly Gly Met Ser Arg Gly Arg Ser Ser
               260                 265                 270
   Ser Ser Met Arg Tyr Asn Gln Ala Pro Leu Leu Thr Arg Leu Leu Val
           275                 280                 285
   Ala Leu Phe Pro Phe Leu Gly Ser Trp Phe Arg Ile Leu
       290                 295                 300
<210> 107
   <211> 406
   <212> PRT
   <213> Oryza sativa
<400> 107
   Met Arg Gly Ser Leu Glu Val His Ala Ile Gly Arg His Ala Ala Ser
   1               5                   10                  15
   Pro Cys Ala Leu Arg Leu Lys Ala Leu Pro Ala Leu Asp Met Met Arg
               20                  25                  30
   Tyr Gln Arg Leu Ser Pro Asp Cys Leu Pro Leu Ala Asn Gly Gly Gly
           35                  40                  45
   Gly Gly Ser Gly Ser Val Thr Arg Lys Pro Ala Ser Arg Ser Cys Lys
       50                  55                  60
   Asp Asp Asp Gly Gly Met Ala Val Ala Ala Asp Ser Ser Arg Leu Ser
   65                  70                  75                  80
   Ser Tyr Leu Pro Ser Ser Gln Leu Asp Ser Lys Pro Leu Arg Ala Arg
                   85                  90                  95
   Ala Pro Gln Pro Ser Ser Ser Ser Ala Ala Ala Trp Ser Pro Ala Arg
               100                 105                 110
   Asp His Ala His Ala His His Asn His His His His His His Pro Ser
           115                 120                 125
   Asp Ser Ser Asp Thr Ala Ser Pro Ser Ser Asn Gly Ala Gly Thr Gly
       130                 135                 140
   Gly Asp Val Leu Leu Gln Trp Gly His Asn Lys Arg Ser Arg Cys Arg
   145                 150                 155                 160
   Arg Asp Ala Ser Ser Ser Ala Asn Ala Ala Pro Ser Ser Ser Gln Arg
                   165                 170                 175
   Arg Gln Thr Ala Ser Ala Ala Gly Lys Ile Leu Arg Arg Ser Ser Ala
               180                 185                 190
   Pro Ala Glu Lys Leu Met Pro Pro Pro Pro Pro Ser Thr Thr Thr Gly
           195                 200                 205
   Ser Tyr Thr Arg Gly Ser Asn Leu Arg Ser Ala Ser Ser Phe Pro Thr
       210                 215                 220
   Arg Ser Ala Ala Ala Ala Ala Val Gly Asp Ala His His His Arg Ser
   225                 230                 235                 240
   Ala Val Glu Glu Arg Ser Gly Gly Gly Tyr Lys Arg Ser Pro Asp Lys
                   245                 250                 255
   Ala His Lys Ser Ala Leu Asp Ala Ala Leu His Met Asp Ser Lys Asn
               260                 265                 270
   Asn His His His His His His Asp Ser Ser Val Thr Ala Asn Gly Gly
           275                 280                 285
   Ala Gly Ala Gly Glu Lys Ile Gly Ser Glu Arg Phe Glu Leu Pro Arg
       290                 295                 300
   Ile Tyr Ile Ser Leu Ser Arg Lys Glu Lys Glu Asp Asp Phe Leu Ile
   305                 310                 315                 320
   Met Lys Gly Thr Lys Leu Pro Gln Arg Pro Lys Lys Arg Ala Lys Asn
                   325                 330                 335
   Val Asp Lys Thr Leu Gln Tyr Val Phe Pro Gly Met Trp Leu Ser Asp
               340                 345                 350
   Leu Thr Arg Gly Arg Tyr Glu Val Arg Glu Lys Lys Cys Val Lys Lys
           355                 360                 365
   Val Tyr Ser Thr Leu His Leu Ala Phe Ser Val His Ala Phe Cys Val
       370                 375                 380
   Phe Leu Gln Thr Arg Arg Gly Lys Leu Pro Arg Phe Arg Ala Ser Val
   385                 390                 395                 400
   Thr Gln Ile Gly Trp Val
                   405
<210> 108
   <211> 203
   <212> PRT
   <213> Oryza sativa
<400> 108
   Met Ala Thr Thr Ala Ser Leu Leu Pro Pro Leu Leu Pro Ala Pro Ser
   1               5                   10                  15
   Ser Ser Pro Arg His Leu His Pro Ser Pro Arg His Leu Arg Pro Leu
               20                  25                  30
   Pro Pro Ile Arg Leu Leu Arg Ala Ala Arg Arg Arg His Pro Asp Ala
           35                  40                  45
   Val Val Val Val Pro Asp Ala Arg Pro Trp Val Gly Asp Leu Ser Gly
       50                  55                  60
   Ala Ala Ala Ser Tyr Arg Asp Gly Arg Glu Glu Asp Asp Asp Asp Ala
   65                  70                  75                  80
   Gly Glu Glu Asp Asp Glu Asn Asp Asp Asp Asp Glu Asp Arg Ser Leu
                   85                  90                  95
   Asp Leu Leu Val Arg Phe Leu His Ser Val Phe Arg Lys Val Ser Arg
               100                 105                 110
   Arg Ala Arg Arg Ala Ala Arg Ser Val Leu Pro Pro Ser Val Pro Ala
           115                 120                 125
   Glu Leu Val Lys Phe Ser Val Asn Gly Val Leu Val Leu Thr Phe Leu
       130                 135                 140
   Trp Val Leu Lys Gly Leu Leu Glu Val Val Cys Thr Phe Gly Ser Met
   145                 150                 155                 160
   Val Phe Val Thr Ile Leu Leu Val Arg Gly Ile Trp Ser Gly Val Thr
                   165                 170                 175
   Tyr Ile Arg Glu Asn Arg Tyr Ser Tyr Ile Arg Gln Ile Asp Asn Asp
               180                 185                 190
   Asp Asn Arg Trp Ser Arg Val Gln Thr Ala Gly
           195                 200
<210> 109
   <211> 809
   <212> PRT
   <213> Oryza sativa
<400> 109
   Met Lys Leu Arg Leu Arg Ser Met Asp Gln Arg Gly Gly Ala Gly Gly
   1               5                   10                  15
   Ala Ala Glu Thr His Arg Val Gln Leu Pro Asp Thr Ala Thr Leu Ser
               20                  25                  30
   Asp Val Lys Ala Phe Leu Ala Thr Lys Leu Ser Ala Ala Gln Pro Val
           35                  40                  45
   Pro Ala Glu Ser Val Arg Leu Thr Leu Asn Arg Ser Glu Glu Leu Leu
       50                  55                  60
   Thr Pro Asp Pro Ser Ala Thr Leu Pro Ala Leu Gly Leu Ala Ser Gly
   65                  70                  75                  80
   Asp Leu Leu Tyr Phe Thr Leu Ser Pro Leu Pro Ser Pro Ser Pro Pro
                   85                  90                  95
   Pro Gln Pro Gln Pro Gln Ala Gln Pro Leu Pro Arg Asn Pro Asn Pro
               100                 105                 110
   Asp Val Pro Ser Ile Ala Gly Ala Ala Asp Pro Thr Lys Ser Pro Val
           115                 120                 125
   Glu Ser Gly Ser Ser Ser Ser Met Pro Gln Ala Leu Cys Thr Asn Pro
       130                 135                 140
   Gly Leu Pro Val Ala Ser Asp Pro His His Pro Pro Pro Asp Val Val
   145                 150                 155                 160
   Met Ala Glu Ala Phe Ala Val Ile Lys Ser Lys Ser Ser Leu Val Val
                   165                 170                 175
   Gly Asp Thr Arg Glu Met Glu Asn Val Gly Gly Ala Asp Gly Thr Val
               180                 185                 190
   Ile Cys Arg Leu Val Val Ala Leu His Ala Ala Leu Leu Asp Ala Gly
           195                 200                 205
   Phe Leu Tyr Ala Asn Pro Val Gly Ser Cys Leu Gln Leu Pro Gln Asn
       210                 215                 220
   Trp Ala Ser Gly Ser Phe Val Pro Val Ser Met Lys Tyr Thr Leu Pro
   225                 230                 235                 240
   Glu Leu Val Glu Ala Leu Pro Val Val Glu Glu Gly Met Val Ala Val
                   245                 250                 255
   Leu Asn Tyr Ser Leu Met Gly Asn Phe Met Met Val Tyr Gly His Val
               260                 265                 270
   Pro Gly Ala Thr Ser Gly Val Arg Arg Leu Cys Leu Glu Leu Pro Glu
           275                 280                 285
   Leu Ala Pro Leu Leu Tyr Leu Asp Ser Asp Glu Val Ser Thr Ala Glu
       290                 295                 300
   Glu Arg Glu Ile His Glu Leu Trp Arg Val Leu Lys Asp Glu Met Cys
   305                 310                 315                 320
   Leu Pro Leu Met Ile Ser Leu Cys Gln Leu Asn Asn Leu Ser Leu Pro
                   325                 330                 335
   Pro Cys Leu Met Ala Leu Pro Gly Asp Val Lys Ala Lys Val Leu Glu
   340 345 350
   Phe Val Pro Gly Val Asp Leu Ala Arg Val Gln Cys Thr Cys Lys Glu
   355 360 365
   Leu Arg Asp Leu Ala Ala Asp Asp Asn Leu Trp Lys Lys Lys Cys Glu
   370 375 380
   Met Glu Phe Asn Thr Gln Asp Thr Cys Gly Cys Met Met Cys Lys Cys
   385 390 395 400
   Ile Tyr Ser Asp Gln Arg Lys Asp Ile Val Leu Ala Asp Lys Tyr Thr
   405 410 415
   Cys Gly Asn Tyr Met Gln Lys Pro Val Thr Gln Pro Gly Arg Trp Leu
   420 425 430
   Ile Ile Leu Val Tyr His Ser Leu Leu Cys Gln Tyr Ile Thr Ile Gly
   435 440 445
   Leu Ser Leu Leu Trp Tyr His Leu Val Asp Leu Val Gln Asp Ala Pro
   450 455 460
   Ala Ala Gly Ile His Phe Asp Cys Ile Ile Pro Leu Pro Ile Asn Pro
   465 470 475 480
   Tyr Gln Leu Pro Pro Ser Ala Gly Ala Cys Cys Ser Thr Thr Gln Ala
   485 490 495
   Ser Ala Ser Ala Lys Asp Gly Gly Asn Met Tyr Ser Pro Pro Cys Ser
   500 505 510
   Ala Ala Ala Ser Ser Gln Gly His Cys Phe Ala Val Gly Ala Asn Gln
   515 520 525
   Leu Ala Ser Leu Asp Leu Ala Met Asp Phe Asp Glu Pro Ile Leu Phe
   530 535 540
   Pro Val His Asn Ala Ser Leu Gln Glu Gly Ile Gln Phe Tyr Asn Pro
   545 550 555 560
   Thr Gly Asp Thr Gln Leu Ser Arg Asn Met Ser Ile Asp Lys Cys Leu
   565 570 575
   Lys Gly Ser Lys Arg Lys Gly Ser Gly Glu Gly Ser Ser Ser Leu His
   580 585 590
   Ser Gln Glu Glu Thr Gly Glu Met Pro Gln Arg Glu Leu Ser Met Glu
   595 600 605
   His Ala Gly Glu Lys Ala Gly Asp Ala Asp Ala Ser Arg Glu Glu Tyr
       610                 615                 620
   Val His Val Arg Ala Lys Arg Gly Gln Ala Thr Asn Ser His Ser Leu
   625                 630                 635                 640
   Ala Glu Arg Phe Arg Arg Glu Lys Ile Asn Glu Arg Met Lys Leu Leu
                   645                 650                 655
   Gln Asp Leu Val Pro Gly Cys Asn Lys Ile Thr Gly Lys Ala Met Met
               660                 665                 670
   Leu Asp Glu Ile Ile Asn Tyr Val Gln Ser Leu Gln Arg Gln Val Glu
           675                 680                 685
   Phe Leu Ser Met Lys Leu Ser Thr Ile Ser Pro Glu Leu Asn Ser Asp
       690                 695                 700
   Leu Asp Leu Gln Asp Ile Leu Cys Ser Gln Asp Ala Arg Ser Ala Phe
   705                 710                 715                 720
   Leu Gly Cys Ser Pro Gln Leu Ser Asn Ala His Pro Asn Leu Tyr Arg
                   725                 730                 735
   Ala Ala Gln Gln Cys Leu Ser Pro Pro Gly Leu Tyr Gly Ser Val Cys
               740                 745                 750
   Val Pro Asn Pro Ala Asp Val His Leu Ala Arg Ala Gly His Leu Ala
           755                 760                 765
   Ser Phe Pro Gln Gln Arg Gly Leu Ile Trp Asn Glu Glu Leu Arg Asn
       770                 775                 780
   Ile Ala Pro Ala Gly Phe Ala Ser Asp Ala Ala Gly Thr Ser Ser Leu
   785                 790                 795                 800
   Glu Asn Ser Asp Ser Met Lys Val Glu
                   805
<210> 110
   <211> 467
   <212> PRT
   <213> Oryza sativa
<400> 110
   Met Ala Ala Ala Ala Gly Ala Gly Glu Pro Ser Pro Tyr Ala Glu Ala
   1               5                   10                  15
   Ala Gly Ser Asp Leu Ala Asn Ala Arg Ala Pro Ser Pro Val Val Gly
               20                  25                  30
   Lys His Leu Pro Ser Gly Ala Val Pro Arg His Ala Tyr Val Phe Asp
           35                  40                  45
   Gly Glu Gly Gly Phe Ala Asp Ala Ala Trp Asp Val Ala Ala Ala Ala
       50                  55                  60
   Pro Gly Ala Phe Thr Trp His His Ile Glu Leu Pro Arg Gln Gln Pro
   65                  70                  75                  80
   Gly Gly Ala Ala Ala Lys Pro Leu His His Ala Gln Ala Leu Ile Glu
                   85                  90                  95
   Leu Leu Cys Pro Pro Leu Thr Leu Gln Glu Ile Leu Ala Phe Val Ala
               100                 105                 110
   Thr Gly Pro His Cys Gly Val Val Asp Gly Gly Gly Gly Gly Gly Ala
           115                 120                 125
   Gly Ala Leu Leu Leu Arg Val Ser Ser Pro Gly Pro Val Gly Ser Ala
       130                 135                 140
   Phe Ala Leu Arg Leu Ala Ala Arg Val Thr Asp Ser Ser Val Val Thr
   145                 150                 155                 160
   Val Ser Val Gly Gly Val Pro Arg Leu Ala Phe Gly Thr Thr Gln Ala
                   165                 170                 175
   Ser Leu Leu Ser Glu Val Pro Leu Gly Val Thr Ala Ser Leu Ser Asp
               180                 185                 190
   Glu Gly His Gly Gly Gly Arg Ala Val Glu Gly Gly Val Val Ile Glu
           195                 200                 205
   Glu Arg Leu Leu Glu Ser Leu Leu Ala Met Asn His Ala Asp Gly Ala
       210                 215                 220
   His Thr Asp Asn Pro Val Pro Arg Thr Val Ser Asn Leu Leu Val His
   225                 230                 235                 240
   Val Leu Gly Thr His Val Asp His Val His Asp Ile Val Thr Arg Leu
                   245                 250                 255
   Glu Met Glu Leu Asp Ser Ile Glu Leu His Leu Asp Lys Gly Gly His
               260                 265                 270
   Phe Met Arg Lys Leu Leu Leu Asp Gly Arg Arg Phe Pro Lys Met His
               275                 280                 285
   Leu Asp Leu Gln Arg Leu Leu Gln Val Val Ser His Gly Asp Gln Val
       290                 295                 300
   Phe Pro Arg Val Lys Glu Lys Cys Ala Ser Lys Ser Trp Phe Ala Ser
   305                 310                 315                 320
   Glu Asp Ile Val Ala Leu Glu Asp Leu Ile Gly Arg Leu Arg Arg Leu
                   325                 330                 335
   Lys Glu Asn Leu Gly Phe Ile Thr Asn Arg Val Thr Thr Leu Gln Ala
               340                 345                 350
   Ser Leu Asp Ser Trp Gln Ser Glu Gln Ile Asn Lys Ser Leu Tyr Tyr
           355                 360                 365
   Leu Ser Phe Leu Ser Ile Ile Phe Leu Pro Leu Ser Ile Val Thr Gly
       370                 375                 380
   Val Phe Gly Met Asn Val Gly Gly Val Pro Trp Thr Glu Gln Lys Asn
   385                 390                 395                 400
   Pro Ala Asn Leu Asp Gly Phe Phe Asn Val Met Leu Ile Cys Val Val
                   405                 410                 415
   Ile Leu Leu Ile Leu Leu Leu Cys Phe Leu Phe Pro Ser Leu Tyr Ser
               420                 425                 430
   His Val Ser Ala Trp Arg Thr Arg Arg Ala Leu Ala Arg Ser Ser Ser
           435                 440                 445
   Gln Asn Lys Arg His Leu Lys Leu Phe Lys Gly His Lys Asp Gly Tyr
       450                 455                 460
   Met Arg Leu
   465
<210> 111
   <211> 147
   <212> PRT
   <213> Oryza sativa
<400> 111
   Met Gln Asp Gln Leu Ile Cys Ser Gly Cys Arg Arg Val Val Gln Tyr
   1               5                   10                  15
   Arg Arg Gly Val Ala Gly Val Cys Cys Pro Gly Cys Asn Thr Leu Thr
               20                  25                  30
   Ala Val Asn Pro Ser Ala Val Ala Asp Met Ser Glu Leu Ile Cys Ser
           35                  40                  45
   Gly Cys Pro Thr Leu Leu Phe Tyr Asn Arg Gly Ala Ser Asn Ile Arg
       50                  55                  60
   Cys Pro Ser Cys Asn Arg Leu Asn Ser Thr Arg Ser Ala Asn Gln Ile
   65                  70                  75                  80
   Ala His Leu Thr Cys Gly Gln Cys Arg Thr Thr Leu Met His Pro Pro
                   85                  90                  95
   Gly Ala Ser Thr Val Gln Cys Ala Thr Cys Arg Tyr Val Asn His Val
               100                 105                 110
   Arg Asp Ala Arg Pro Gln Thr Val Leu Val Glu Asn Pro Lys Thr Leu
           115                 120                 125
   Asp Asp Lys Gly Lys Leu Val Ser Asn Val Val Val Gly Val Thr Ser
       130                 135                 140
   Trp Lys Arg
   145
<210> 112
   <211> 266
   <212> PRT
   <213> Oryza sativa
<400> 112
   Met Arg Gly Ala Val Ala Ile Phe Asn Glu Phe Lys Arg Arg Gly Leu
   1               5                   10                  15
   Asn Ile Ser Ile Thr Gly Ile Pro Lys Thr Val Asp Asn Asp Ile Gly
               20                  25                  30
   Ile Ile Asp Arg Ser Phe Gly Phe Gln Thr Ala Val Glu Ile Ala Gln
           35                  40                  45
   Gln Ala Ile Asp Ala Ala His Val Glu Ala Val Ser Ala Val Asn Gly
       50                  55                  60
   Ile Gly Leu Val Lys Leu Met Gly Arg Ser Thr Gly His Ile Ala Leu
   65                  70                  75                  80
   His Ala Thr Leu Ser Ser Arg Asp Val Asp Cys Cys Leu Ile Pro Glu
                   85                  90                  95
   Val Asp Phe Tyr Leu Glu Gly Lys Gly Gly Leu Phe Glu Phe Leu Tyr
   100 105 110
   Glu Arg Ile Lys Gln Lys Gly His Ala Val Val Val Val Ala Glu Gly
   115 120 125
   Ala Gly Gln Glu Leu Ile Pro Arg Thr Asp Asp Gln Lys Arg Glu Gln
   130 135 140
   Asp Glu Ser Gly Asn Ile Val Phe Leu Asp Val Gly Pro Trp Leu Lys
   145 150 155 160
   Ser Glu Leu Gly Lys Trp Trp Lys Arg Glu His Pro Ser Glu Leu Phe
   165 170 175
   Thr Val Lys Tyr Ile Asp Pro Thr Tyr Met Ile Arg Ala Val Pro Ala
   180 185 190
   Asn Ala Thr Asp Asn Leu Tyr Cys Thr Leu Leu Ala His Ser Ala Ile
   195 200 205
   His Gly Ile Met Ala Gly Tyr Thr Gly Phe Val Pro Gly Pro Ile Asn
   210 215 220
   Gly Asn Tyr Ser Tyr Ile Pro Leu Glu Asp Val Ala Val Ala Lys Asn
   225 230 235 240
   Pro Val Asp Val Asn Asp His Lys Trp Ala Trp Val Arg Ser Val Thr
   245 250 255
   Asn Gln Pro Asp Phe Met Lys Pro Lys Tyr
   260 265
<210> 113
   <211> 595
   <212> PRT
   <213> Oryza sativa
<400> 113
   Met Val Glu Ser Ala Ser Met Val Asn Glu Asn Ser Glu Asn Pro Tyr
   1               5                   10                  15
   Trp Lys Ala Ile Gly Tyr Arg Val Glu Glu Pro Arg Arg Asp Arg Ala
               20                  25                  30
   Glu Ser Met Pro Ser Pro Ser Pro Ser Pro Val Ser Arg Arg Pro Leu
           35                  40                  45
   Asp Asn Gly Val Val Glu Thr Arg Ala Leu Thr Asp Thr Thr Leu Leu
       50                  55                  60
   Arg Ser Leu Ala Ala Lys Gly Leu Ala Val Arg Pro Gly Ala Ser Asp
   65                  70                  75                  80
   Glu His His Thr Val Arg Cys Asp Ala Val Ile Val Gly Ser Gly Cys
                   85                  90                  95
   Gly Gly Gly Val Ala Ala Ala Val Leu Ala Ser Ala Gly Tyr Lys Val
               100                 105                 110
   Val Val Val Glu Lys Gly Asp Tyr Phe Thr Lys Glu Asp Tyr Ser Ser
           115                 120                 125
   Ile Glu Gly Pro Ser Met Glu Arg Leu Phe Glu Arg Gly Gly Val Phe
       130                 135                 140
   Cys Thr Ser Asn Val Thr Thr Met Ile Phe Thr Gly Ala Thr Val Gly
   145                 150                 155                 160
   Gly Gly Ser Ala Val Asn Trp Ser Ala Ser Ile Arg Thr Pro Ala Gly
                   165                 170                 175
   Val Met Gln Glu Trp Ser Arg Glu His Gly Leu Ala Val Phe Ala Ser
               180                 185                 190
   Pro Gly Tyr Ala Arg Ala Met Asp Ala Val Cys Glu Arg Leu Gly Val
           195                 200                 205
   Thr Asp Ala Cys Arg Glu Glu Gly Phe Gln Asn Lys Val Val Arg Arg
       210                 215                 220
   Gly Cys Asp Ala Leu Gly Leu Arg Ala Asp Ala Val Pro Arg Asn Ser
   225                 230                 235                 240
   Ser Glu Gly His Phe Cys Gly Ser Cys Asn Phe Gly Cys Pro Thr Gly
                   245                 250                 255
   Asp Lys Lys Gly Thr Asp Thr Thr Trp Leu Val Asp Ala Val Glu Arg
               260                 265                 270
   Gly Ala Val Ile Leu Thr Gly Cys Lys Ala Glu His Phe Ile Val Glu
           275                 280                 285
   Ser Asn Gly Gly Gly Gly Gly Arg Ser Lys Arg Cys Val Gly Leu Val
       290                 295                 300
   Ala Thr Cys Met Ser Asn Gly Ile Thr Lys Lys Leu Arg Val Glu Ala
   305                 310                 315                 320
   Lys Val Ser Ile Ser Ala Ser Gly Ala Leu Met Thr Pro Pro Leu Leu
   325 330 335
   Arg Asn Ser Gly Leu Lys Asn Arg His Ile Gly Arg Asn Leu His Leu
   340 345 350
   His Pro Val Ser Met Ala Trp Gly Tyr Phe Pro Asp Asn Thr Pro Glu
   355 360 365
   Pro His Ile Pro Gly Lys Cys Tyr Glu Gly Gly Ile Ile Thr Ser Met
   370 375 380
   His Arg Val Thr Glu Arg Thr Ile Ile Glu Thr Pro Ala Leu Gly Pro
   385 390 395 400
   Gly Ala Phe Ala Ala Leu Val Pro Trp Glu Ser Gly Arg Asp Met Lys
   405 410 415
   Glu Arg Met Arg Arg Tyr Ala Arg Thr Ala His Ala Phe Ala Leu Val
   420 425 430
   Arg Asp Arg Gly Ala Gly Ser Val Asp Gly Glu Gly Arg Val Arg Tyr
   435 440 445
   Ala Pro Ser Arg Asp Asp Ala Glu Glu Leu Arg Ala Gly Leu Arg Arg
   450 455 460
   Ala Leu Arg Ile Leu Val Ala Ala Gly Ala Ala Glu Val Gly Thr His
   465 470 475 480
   Arg Ser Asp Gly Ala Arg Leu Arg Cys Lys Gly Ala Arg Asp Ala Asp
   485 490 495
   Val Glu Ala Phe Leu Asp Glu Val Thr Val Glu Lys Gly Pro Met His
   500 505 510
   Ser Thr Thr Asp Lys Trp Ser Val Leu Cys Ser Ala His Gln Met Gly
   515 520 525
   Ser Cys Arg Met Gly Ala Ser Pro Arg Asp Gly Ala Val Asp Val Ala
   530 535 540
   Gly Glu Ser Trp Glu Ala Glu Gly Leu Tyr Val Cys Asp Gly Ser Leu
   545 550 555 560
   Leu Pro Thr Ala Val Gly Val Asn Pro Met Ile Thr Ile Gln Ser Ile
   565 570 575
   Ala Tyr Cys Val Ala Lys Gly Ile Ala Asp Ser Met Ala His Gly Lys
   580 585 590
   Glu Gln Arg
   595
<210> 114
   <211> 177
   <212> PRT
   <213> Oryza sativa
<400> 114
   Met Ala Pro His Pro Leu Leu Arg Gly Gly Ala Arg Arg Gly Arg Lys
   1 5 10 15
   Tyr Ala His Gly Met His Pro Ala Gln Met Glu Ala Leu Arg Ala Met
   20 25 30
   Cys Gly Ala Leu Ile Pro Ser Leu Pro Val Asp Ala Asp Gly Gly Asp
   35 40 45
   Gly Gly Arg Arg Pro Gly Asp Lys Asp Leu Glu Arg Phe Tyr Leu Ala
   50 55 60
   Ser Ala Ala Asp Ser Ser Ile Pro Asp Glu Val Ala Glu Leu Leu Val
   65 70 75 80
   Thr Arg Cys Ile Trp Glu Ala Val Ala Leu Thr Trp Val Val Leu Trp
   85 90 95
   Ala Leu Ser Thr Arg Ala Gly Thr Leu Leu Leu Cys Gly Arg Asp Ser
   100 105 110
   Val Ala Ala Val Asp Gly Gly Gly Phe Pro Phe Val Ser Val Arg Arg
   115 120 125
   Phe Ala Asp Met Pro Ala Ala Arg Arg Glu Ala Ala Leu Trp Arg Trp
   130 135 140
   Ser Gly Ala Arg Trp Leu Phe Phe Pro Leu Arg Ile Ala Phe Ala Ile
   145 150 155 160
   Ala Lys Ile Leu Cys His Tyr Val Phe Tyr Ser Met Ile Leu Val Ser
   165 170 175
   Ala
<210> 115
   <211> 477
   <212> DNA
   <213> Oryza sativa
<400> 115
   atgcccaggc acgcatgtct gctgcagtct gaccgaattt ttttcatagc cactgaagag     60
   tgcagacgga ggcgacgcca aggaggtgcc gacgccggcg ctggaggcag aggcggaggg    120
   cgccggtgct gggcggaggc ggcggaggcg gcgcacatgg cggcggcggc ggcgcatagg    180
   gcggcggcgg tgcacagggc ggcctgcggt agctccaccg tcgccgtcgg gctgcgggag    240
   ctccaccggc gtcgtctctg cagcacctgc acgggggatg gggatgggga taggatgctt    300
   atgcagagaa atgacagtag caatagcaaa tggatgttgt cacggagggc attttcgcct    360
   tttcacgtga gtgctaaatt tgcatgggag gtacaggaat cgcttctgga tggagggagt    420
   acctggttct gtttaggatc tagtgcgtac tttgtcgcag tcaaatacga ttggtga       477
<210> 116
   <211> 354
   <212> DNA
   <213> Oryza sativa
<400> 116
   atggggacgt acaagtgctg catcttcttc acccgcaggt tcgcgctgag cgacgcgtcc     60
   acgccgggcg acgtgcgcat gctgttcacc cgccacgccg gcggcgcgcc ctacatgggc    120
   atcgacgagc tccggcgcta cctcgccgcc agcggggagg cccacgtcga cgccgacacg    180
   gcggagcgga tcatcgaccg ggtcctgcag gagcgcagcc gcaccccgcg cttcgggaag    240
   ccgtcgctca ccatcgacga tttccagtac ttcctcttct ccgaggacct caacccgccc    300
   atctgccatt ccaaggaaga aagttttgat gcgatggaaa agttggaagt ttga          354
<210> 117
   <211> 1473
   <212> DNA
   <213> Oryza sativa
<400> 117
   atgcaggtcc atcacgacat gaatgcacca ttatcgcact acttcatata cactggacac 60
   aactcgtatc tgacgggcaa tcaacttagc agtgactgca gtgatattcc catcattaag 120
   gcactgcaaa taggcgtccg tgtaattgaa ctggacatgt ggccaaattc ttctaaagat 180
   gatgttgata ttctccatgg aaggacactg actgccccag tatcacttat caaatgcttg 240
   aaatccatca aagaatatgc ctttgttgcg tctccctacc ctgttattat aacattagaa 300
   gaccacctta catctgatct tcaggcgaaa gtagctaaga tggttcttga agtatttgga 360
   gataccctat attatcccga gtcaaaacat cttcaagaat ttccttcacc cgaagcactg 420
   aggggacgtg tcatcctctc aacaaaaccc ccaaaggagt accttgaatc aaaaggtggt 480
   actatgaaag acagagacat tgagcctcag tttagcaaag gacaaaatga agaagctgtc 540
   tggggaacag aagtcccaga tattcaggat gagatgcaaa ccgccgacaa gcagcatgag 600
   aatgatatac tatacaccca aagagatgtg gaagaagatg atgagaagaa aatgtgccag 660
   catcacccac tagagtataa acaccttatt actattaagg caggaaagcc aaagggtgct 720
   gtagttgatg ccttaaaggg tgatccagat aaagttagac gcctcagttt gagtgagcag 780
   gaacttgcaa aagtggcagc gcatcatggt cgtaacatcg tgagctttac acataaaaat 840
   cttctgagaa tatacccaaa gggcactcgc ttcaattctt cgaactataa tccgtttctt 900
   ggttgggtgc atggtgcaca aatggtggca tttaatatgc aggggtatgg aagatctctt 960
   tggctaatgc acggattcta caaggccaac ggtggctgcg gttatgtgaa gaagccagat 1020
   ttcatgatgc aaacttgtcc agatggaaat gtttttgacc cgaaagcaga tttacctgtg 1080
   aagaaaacac tcaaggtcaa agtatacatg ggcgaaggtt ggcagagcga cttcaagcag 1140
   acatacttcg acacgtattc ccctccagac ttctacgcaa aggtgggcat tgccggggtt 1200
   ccgtcggact cggtgatgca gaagacgaaa gccgtggagg acagctgggt tcccgtgtgg 1260
   gaggaggagt tcgtgttccc gctgaccgtc ccggagatcg cgctgctccg cgtggaggtg 1320
   cacgagtacg acgtgagcga ggacgacttc ggcgggcaga cggcgctccc ggtgtcggag 1380
   ctgcggccgg ggatccgcac cgtgccgctc ttcgaccaca aggggctcaa gttcaagagc 1440
   gtcaagctcc tcatgcggtt cgagttcgtc tag 1473
<210> 118
   <211> 636
   <212> DNA
   <213> Oryza sativa
<400> 118
   atggcgatgg gagccgcggc ggcgccatgg tacggcgcca tcggcggcgg tggctcgcgg 60
   cgcgcgcggg tgagggcgca ggcggcggcg ccgtgggcag gaggcgcgga ggagctggtg 120
   cggtcgggcg cggtgcgggc ggtgcgggcg agggaggcgg cgggggcgat gtccgcggag 180
   gggttccggc tgctggacgt ccggccggag tgggagcgcg cgcgcgccgc cgtgcggggc 240
   tcggcgcacg cgccgctgtt cgtcggggac gacgacacgg gccccgtcac gctgctcaag 300
   aagtgggtcc acttcggcta catcggcctc tggaccggcc agtccttcac caagatgaac 360
   gaccgcttcc tcgacgacgt cgccgccgcc gccggcgaag gcaaggacgc caagctgctc 420
   gtcgcctgcg gcgaaggcct ccggtcgttg atcgcggtga ggatgctgta cgacgacggg 480
   tacaagaacc tggcgtggct cgccggaggg ttcagcaagt gcgtcgacgg cgacttcgcc 540
   gacgtggagg gggagagcaa gctgcagtat gccaccgtgg gtggggtgtc ctacatcttc 600
   ctccagatcc tgcttctgct gcgggtagtc aagtga 636
<210> 119
   <211> 948
   <212> DNA
   <213> Oryza sativa
<400> 119
   atggccgcag cagcgcagag gcggcggagc agcagcgcct ccccggagtt ccgcttctgg 60
   cccctcgacg ccgaccccgc cgcatccccc tcctgcgccg acgagctctt ctccggcggc 120
   gtcctcctcc ccctccaacc cctcccctac ccccgccgcg acgccgacct ctccatgtcc 180
   ctcgccgtcg cggatgatga tgatgatgag gacgaggagg aggaggaggt gcagcctggt 240
   gcggccgtcg cgtccagggc gccgcccact gctgcggtgg cggcgtcggg tggtggtggt 300
   ggtgggtcga agaggtggac ggatatattc gccaagaagc agcagcagcc ggcggcggag 360
   gagaaggaga aggatcagcc gacgaggcgg cggagaccgg cgggaggcgg aggcggatcg 420
   gagctgaaca ttaacatctg gccgttctcc cggagccgct ccgccggcgg gggcggcgtg 480
   gggtcgtcga agccccgccc gccgccgcgg aaggccagta gcgccccgtg ctcccgcagc 540
   aactcccgcg gcgaggcggc ggcggtggcg tcgtcccttc ctcctcctcc tcgccgctgg 600
   gccgccagcc ccggccgcgc aggcggcggc gtgccggtgg gccggtctag cccggtctgg 660
   cagatcaggc gcccgccatc gccggcggcg aagcacgccg ccgcggacag gaggccgccg 720
   caccacaagg acaagccaac cggcggcgcc aagaaacccc acaccacctc cgccaccggc 780
   ggcggcggga tacgcggcat caacctgagc atcaactcct gcatcgggta ccgccaccag 840
   gtgagctgcc gccgcgccga cgccggagtc gcccgcgcct ccgccggcgg cggcggcggc 900
   ggcgggctct tcggcatcaa ggggttcttc tccaagaagg tgcattga 948
<210> 120
   <211> 921
   <212> DNA
   <213> Oryza sativa
<400> 120
   atgcagcaga agcccgcggc ggaggccatg gaggaggagt tgaaggggga ggccgtgggg      60
   ccccgccgcc ccgggctagg gttatggttg gcggcgcggc ggcggctggc ccccgacgac      120
   cccttcttcg ccgccgggga catggagcgc gagctcctcg ccaagcaagt tgctctggat     180
   ctctccgaag atgaacggta ccagcttgag aggatggaag tggcgagtgc caatgccctt     240
   ttatgcccaa tttctggctg tggtgctcat ctagattgcc tggagaactt tgaggaccac     300
   tatcgcaccc gtcatactgc ttcatgctct gtatgttgga gagtgtatcc aacttcaagg     360
   ctgctgagta ttcatatttc tgaggcacat gattcctttt ttcaagcaaa agttgcccgt     420
   ggttttccaa tgtatgagtg tttggtggag ggttgtgggg tgaagttgaa gagctacaaa     480
   agtcggcagc agcatcttct tgataagcac cagtttccca agtcatttga attcttcaaa     540
   aaagcacgcc cttcgcaacg ccagcggaac aagaaccaga agcaacggca aacagttcac     600
   aagggagacg agacaagcga aacactaatg gatgttgatg ggaagaagag ctcaaggtac     660
   atgaattcca gatatcggcc aaagcaacat gatggaaaag agtcaaaaga aaatgagcat     720
   agtagctgta aggaggccaa gaacaacgaa atggaggttg acaagcaggt tgatgagctt     780
   gcttcggccg tatcaagact gagcacagcg gattcaactc cttctagcat aagctttggt     840
   catcgtcgct ctcgcggtct tgcttttgtc cctaggtcga ttcggcaaaa caagcaggtt     900
   tctcagacag aaccaaaatg a                                               921
<210> 121
   <211> 987
   <212> DNA
   <213> Oryza sativa
<400> 121
   atgggcggcg gcggcggcgc ggaggaggag ctgacggcgc aggagacggc gctctacgac      60
   cgccagatcc gcgtctgggg cgttgacgcc cagaagaggc taagtaaagc tcatgtgctc     120
   gtgtgcggca tgaatggtac tactactgag ttctgcaaga atattgttct agcaggagtt     180
   ggcagtttat ccttgatgga tgatcattta gtcacagagg atgatctcaa tgcaaatttc     240
   ttaattcctc atgatgagag catatatggt ggtagatcac gagctgaggt ttgctgtgag     300
   tccctgaaag atttcaatcc aatggtccga gttgcagtcg aaaagggtga tccatcatta     360
   attgatggag aattccttga caagtttgac ataattgtag ttagctgtgc gcctattaaa     420
   acaaagttgt taattaacga caactgccgg aagagaagca agcatattgc attctacgcc     480
   attgagtgca aggattcctg tggtgaaata tttgttgatt tgcagaacca tagctatgtt     540
   cagaaggttg gaggtgaacc caaaccaaag gagttggcat atccaagtct ccaggaagct     600
   atctccgtac cctggaagaa tttaccaaga aaaacaacta aactgtactt tgccatgaga     660
   gtactggaga attatgagtc atctgaaggc cgcaatgctt gtgaggcatc actttctgat     720
   cgacctgcag ttttggctct gaggaaggac atgtgtgata aaatgtcttt aagtgagtct     780
   caaattccta ctgctctcct ggaacggctt ttagcagctg gaaagaagca acatcctcct     840
   gtatgtgcaa tccttggcgg cattcttggt caggaggtga ttaagtcaat atctggtaag     900
   ggtgatccga tcaagaattt cttctattac gacgccgctg atggtaaagg gatcgctgaa     960
   gacattcctc ccctttcttc agactga                                         987
<210> 122
   <211> 906
   <212> DNA
   <213> Oryza sativa
<400> 122
   atggctatgc cgctgccgcc gccgccgccg cctcgtcctc ctcttggacg gggacggctc      60
   gtcggagtag gaccagctcc agcaccagca acggcctccc aatccaaccg cccagtgccc     120
   cccctgcagc tgcctcgctg ccgctgccat cgctcggagg gaccctggag gacgacggcg     180
   gcggcgaacg ggaggaggcg gtggtggtcc gacgaggaca tggaggaaga ggacgacgag     240
   gagggatacg gatacgacga cggcggcgcg ccaggcgggt cagcgcagga gctgttcggc     300
   gagccatggt tttccaagct cttccgtgcg tacggctacg tgctgccgct gctgctggcg 360
   tccatgctgg tggccacggg gcccagagct ttcctcatgg ccatggcgct gccgctcgcc 420
   cagtccgcca tctcctgggt cgtctccttc ttcaccacca ggagtcgtcg gcagcaggag 480
   gaggaggagt cgtacggata cgactacgat gacgatcccg ccttccaacg ccgagaggaa 540
   gacgacgacg acggcgacta ctatgatgcc ggggcatggc aatggcggag caggagccac 600
   cagcaatcga ccgaatccgg ctccggtttt ggaggatggg atgacctcct ctacgacgat 660
   gaggagaaga aggagcagga gagctcaggg aagaagagga cgccaccgga gcccgacacg 720
   gcggcggctg ctgccgcctc cgatctggga ctgggattgc gggcgaggag aggtccacga 780
   cgcagcaatg gcggcatgtc gcgaggaaga agcagcagca gcatgaggta taaccaggcg 840
   ccactgctga cgcgccttct cgtggcactc ttccccttcc tcggctcatg gttcaggata 900
   ctctaa 906
<210> 123
   <211> 1221
   <212> DNA
   <213> Oryza sativa
<400> 123
   atgcgtggct ccctggaggt ccacgcgatt ggcagacacg ccgcgtcgcc gtgcgccctg      60
   agactgaaag ccctcccggc attggacatg atgaggtacc aaaggcttag cccggactgc     120
   ctcccgctag ccaacggcgg cggcggagga agcggtagcg tgacacggaa gccggcgtcg     180
   agatcctgca aggacgacga tggcggcatg gccgtcgccg cggacagctc ccgcctctcg     240
   tcgtacctcc cgtcgtcaca gctcgattcc aagccgctgc gcgctcgggc gccgcagccg     300
   tcgtcctcgt cggccgccgc ctggagcccg gcgcgcgacc acgcgcacgc ccaccacaac     360
   caccaccacc accaccaccc gtccgactcc tccgacacgg cctcgccgag ctccaacggc     420
   gcgggcaccg gtggcgacgt gctgctgcag tgggggcaca acaagcggtc ccgctgccgg     480
   cgcgacgcgt cctcctcggc caacgcggct ccctcctcct cgcagcgccg ccagaccgcc     540
   tccgccgccg gcaagatcct gcgccgctcg tcggcgccgg cggagaagct catgccgccg     600
   ccgcccccat ccaccaccac cgggtcgtac acgcgcgggt ccaacctgag gtccgcttcg     660
   tccttcccga cgcggtccgc cgccgccgcc gccgtcggag acgcacacca ccacaggtcc     720
   gccgtggagg agcgatcagg cggcgggtac aagcggtcgc cggacaaggc gcacaagtcc     780
   gccctggacg cggcgctgca catggattcc aagaacaacc accatcacca ccaccacgac     840
   tcgtcggtga ccgcaaacgg cggcgccggc gccggcgaga agatcggctc cgagcggttt     900
   gagctgcccc ggatctacat ctcgctgtcg cgcaaggaga aggaggacga cttcttgatc     960
   atgaagggca ccaagctgcc tcagaggccc aagaagaggg ccaagaacgt ggacaagacc    1020
   ctccaatatg tattccctgg gatgtggctt tcagacttga cgagaggacg gtatgaggtg    1080
   cgagagaaga aatgtgtgaa gaaggtatac tccactctgc acctggcatt ttcagttcat    1140
   gcattctgtg tatttttaca gacaagaaga ggaaaattac ctagatttag ggcatcagtg    1200
   acccagattg gttgggtcta a                                              1221
<210> 124
   <211> 612
   <212> DNA
   <213> Oryza sativa
<400> 124
   atggcgacca ccgcctccct cctccctcct ctcctcccgg ccccttcctc ctccccccgc 60
   cacctccacc cctcccctcg ccacctccgc cccttgccac cgatccgcct cctccgcgcc 120
   gcccgccgcc gccaccccga cgccgtcgtc gtcgtcccgg acgcccgccc ctgggtcggc 180
   gacctctcgg gcgccgccgc gtcctaccgg gacggcaggg aagaggacga cgacgacgcg 240
   ggggaggagg atgacgaaaa cgacgacgac gacgaggacc gcagcctgga cctcctggtc 300
   cgcttcctgc actcggtgtt caggaaggtc tcccgccgcg cgcgccgcgc cgccaggtcc 360
   gtgctgccgc cttccgtccc cgctgagctg gtgaagttct cggtcaacgg cgtgcttgtt 420
   ctcacgtttc tatgggtcct aaaggggcta cttgaggtgg tgtgcacatt tggaagtatg 480
   gtgttcgtga ccatccttct tgttcgtgga atatggtctg gagtgactta cataagagaa 540
   aaccgatata gctatattcg ccagattgat aatgatgaca accgatggag cagagtacag 600
   actgctggct aa 612
<210> 125
   <211> 2431
   <212> DNA
   <213> Oryza sativa
<400> 125
   atgaagcttc ggttgcgatc catggaccag cgcggcggcg ccggcggcgc cgccgagacc 60
   caccgcgtgc agctgccgga cacggccacg ctctccgacg tcaaggcctt cctcgccacc 120
   aagctgtccg cggcgcagcc cgtgcccgcc gagtcggtgc gcctcaccct caaccgctcc 180
   gaggagctcc tcacccccga cccctccgct accctcccgg ccctcgggct cgcgtccggt 240
   gatctcctct acttcacgct ctcccccctc ccgtcgccct cgcctccgcc gcagccgcag 300
   ccacaggccc aaccctgccc cgtaacccta accctgatgt cccctcgatc gcgggagctg 360
   ctgacccgac caaatctcct gtggagtctg gtagtcctcg tcgatgccgc aagctttgtg 420
   cacgaatcct ggcttacctg tcgcatccga tccgcatcat cctccaccgg atgtggtgat 480
   ggcggaggcc ttcgccgtga tcaagagcaa gtcgagtctc gtcgtcgggg atacgaagag 540
   agagatggag aatgtcggtg gtgcggatgg aaccgtcatc tgtcgccttg tcgtggcgct 600
   gcatgcggcc ttgctcgatg ccggcttcct ctatgcaaac ccggtggggt cttgccttca 660
   gctgccacag aattgggcgt caggttcttt tgtccccgta tcgatgaagt acaccctgcc 720
   agagcttgta gaagcgttac ctgtggttga ggaggggatg gtggcagtgc tgaactactc 780
   cttgatgggg aattttatga tggtgtatgg gcatgtgcct ggggcaacat cgggggtgcg 840
   aaggttgtgc ttggagctgc cggagcttgc gcctttgttg tacttggata gtgatgaggt 900
   gagcacagca gaggagaggg aaattcatga gctgtggagg gtcctgaagg atgagatgtg 960
   cttgcctctg atgatatcgt tgtgtcaact gaacaatttg agcttgccac cgtgcttgat 1020
   ggcgctgcca ggtgatgtca aggcaaaggt cctggagttt gttcctgggg tggatcttgc 1080
   aagggttcaa tgcacgtgca aggaattgag ggatcttgct gcagatgata atctttggaa 1140
   gaagaagtgt gagatggagt tcaatactca agatacatgc ggttgtatga tgtgtaaatg 1200
   catttactct gaccaaagga aggatatcgt actagctgat aagtatacct gtggtaatta 1260
   tatgcagaag cccgtcacac aacctggtag gtggcttatt atattagtct accattccct 1320
   actttgccag tacatcacta ttgggttgag tttgctgtgg tatcatttgg ttgatttggt 1380
   tcaggatgct cctgcagcag gcattcactt tgactgtatt attccactgc caatcaatcc 1440
   ttaccagctt cccccatctg ctggtgcctg ctgctcaaca actcaagctt cagcatcagc 1500
   aaaagatggt ggcaatatgt attcccctcc ctgcagtgct gctgcaagca gccaagggca 1560
   ttgtttcgcg gtcggagcta accagcttgc ttcgcttgac cttgccatgg acttcgacga 1620
   gcctatcctt tttcctgtgc ataatgcaag tttgcaagag gggattcagt tttacaatcc 1680
   taccggcgat actcagctaa gtagaaacat gagcattgac aagtgtttga agggcagtaa 1740
   aaggaagggc tcaggcgagg gcagttcatc gctacattcc caagaggaaa ccggtgaaat 1800
   gcctcagaga gaactcagca tggagcatgc cggagagaag gcgggtgatg ctgacgctag 1860
   cagggaggag tacgtgcatg tccgggcaaa acgcggccag gcgaccaaca gccacagcct 1920
   tgcagaaaga tttcgaaggg agaagataaa cgaaaggatg aagcttctgc aggacctcgt 1980
   cccaggatgc aacaagatta cagggaaggc catgatgctc gacgagatca taaactacgt 2040
   ccagtctctg cagcgacagg tggagttcct ctcgatgaag ctctcgacaa tcagtcctga 2100
   gttgaactct gacctcgacc tgcaagatat cctttgttca caagatgctc gctccgcatt 2160
   tctgggatgc agcccgcaat tgagcaatgc ccatcctaac ctttacaggg cggctcagca 2220
   atgcctctca cctcctggct tgtacgggag tgtgtgtgtc ccaaatcccg cagatgttca 2280
   tttggcaagg gccggtcact tggcttcgtt tcctcagcag agaggcctca tctggaacga 2340
   ggaacttcgc aacattgctc cggccggttt cgcttcagac gccgctggca ccagtagctt 2400
   agagaactct gattcgatga aagtggagta g 2431
<210> 126
   <211> 1404
   <212> DNA
   <213> Oryza sativa
<400> 126
   atggcggccg cggccggcgc cggcgagccg tcgccgtacg cggaggcggc gggatccgac      60
   ctcgcgaatg cgcgggcgcc gtctcccgtg gtcggcaagc acctcccgtc gggcgccgtg     120
   ccgcgccacg cgtacgtgtt cgacggcgag ggggggttcg ccgacgcggc gtgggacgtc      180
   gcggcggcgg cgccgggggc gttcacgtgg caccacatcg agctcccgcg gcagcagccc     240
   gggggcgccg ccgcgaagcc gctccaccac gcgcaggcgc tgatcgagct gctctgcccg     300
   ccgctcacgc tgcaggagat cctcgcgttc gtcgccacgg gcccgcactg cggcgtcgtg     360
   gacggcggcg gcggcggcgg ggcgggcgcg ctccttctcc gcgtgagctc gccggggccg     420
   gtggggagcg cgttcgcgct ccgcctcgcc gcgcgcgtca cggacagctc cgtggtgacc     480
   gtgtccgtgg gcggcgtccc gcgcctcgcg ttcgggacca cgcaggcgtc gctcctctcc     540
   gaggtgccgc tcggggtgac cgcgtcgctc tccgacgagg gccacggcgg cgggcgcgcc     600
   gtcgagggcg gggtggtgat cgaggagcgg ctgctcgagt cgctgctcgc catgaaccac     660
   gccgacggcg cgcacaccga caaccccgtg ccgcggaccg tgtccaacct cctcgtgcac      720
   gtcctgggaa cgcacgtaga ccacgtccac gacatcgtca cgcgcctcga gatggagctc     780
   gacagcatcg agctgcatct cgacaagggt ggtcacttta tgaggaaact tttgttggat     840
   ggaaggagat tccccaaaat gcatcttgat ctacagcgcc tgcttcaggt tgtttctcat     900
   ggtgaccaag tattcccccg tgtaaaggaa aaatgtgcga gcaagagttg gtttgcgagt      960
   gaagatattg ttgctcttga agatctgata ggccgtctta ggaggctgaa ggaaaatctt    1020
   ggatttataa cgaatagggt gactacactt caagctagtc tagatagctg gcaatctgag    1080
   cagataaaca aaagcttgta ctatctttca tttttgtcca taatattcct tcctctatcc    1140
   attgtcactg gagtttttgg gatgaatgtt ggtggtgtgc catggactga gcagaaaaac    1200
   cctgcaaatc tagatggctt cttcaatgtc atgttaatat gcgtcgtgat cttgttgatc    1260
   ctgctgcttt gtttcttatt tccttcattg tattcacacg tgtcggcatg gagaacccgc    1320
   cgtgcactgg cccggagcag ttctcagaac aagagacatc tgaaactctt taagggtcac    1380
   aaagatggtt acatgcgcct ctga                                           1404
<210> 127
   <211> 444
   <212> DNA
   <213> Oryza sativa
<400> 127
   atgcaggacc agctgatctg cagcggctgc aggcgcgtcg tccagtacag gagaggggtc 60
   gccggcgtct gctgcccggg ctgcaacacg ctcaccgccg tcaacccgtc agcggtggcc 120
   gacatgtcgg agctcatctg cagcggctgc cccacgctgc tgttctacaa ccgcggcgcc 180
   tccaacatcc gctgccccag ctgcaacagg ctcaactcca ccagatcagc caaccagatt 240
   gcacacctga catgcgggca gtgccggacg actctgatgc acccacctgg agcctcaact 300
   gtgcagtgtg caacctgcag atatgttaac catgtcaggg atgctcggcc tcaaactgtc 360
   cttgtagaga atcctaagac actggatgat aagggcaagc tggtgagcaa tgtggttgtt 420
   ggtgtcacct catggaaaag atga 444
<210> 128
   <211> 801
   <212> DNA
   <213> Oryza sativa
<400> 128
   atgagaggag ctgtggccat cttcaacgag tttaagcgcc gtggtttgaa catttctatt 60
   acagggatcc cgaaaactgt ggacaatgat atcggcatca tagacaggtc atttgggttc 120
   caaaccgcag tggagattgc tcagcaggca atcgacgcag cacatgtcga ggctgtgagc 180
   gccgtgaatg gcattggact tgtcaaactt atgggcagga gcacaggcca cattgctctt 240
   catgccaccc tgagcagccg cgatgttgac tgctgtttga ttcctgaggt tgatttctat 300
   cttgaaggaa aggggggcct gtttgagttc ttgtatgaaa ggataaaaca gaagggacat 360
   gctgttgtcg ttgttgctga aggtgctggt caggaattga ttccaaggac tgacgatcaa 420
   aagcgggagc aggacgagtc cggcaacatt gtgttccttg atgtgggtcc ctggttaaaa 480
   tctgagctgg gtaaatggtg gaagagagaa cacccaagcg agttgttcac tgtgaagtat 540
   atcgatccca cttacatgat acgagctgtt ccagcaaatg ccactgacaa tctgtactgt 600
   acattgttgg cacattcggc gatccatggg atcatggctg ggtacactgg cttcgtccct 660
   ggcccgatta atggaaacta tagctacata ccgctggaag atgttgctgt ggcgaagaac 720
   ccggtggatg tgaatgatca caaatgggca tgggttagat cagtcacaaa ccaaccagat 780
   ttcatgaagc caaaatacta a 801
<210> 129
   <211> 1788
   <212> DNA
   <213> Oryza sativa
<400> 129
   atggttgaaa gtgcaagcat ggtgaacgag aactcggaga atccatactg gaaagcaata 60
   ggatacagag tggaagagcc ccgacgtgat cgagcagagt cgatgccgtc gccgtcgcca 120
   tcgccggtat cgcggcggcc actggacaac ggcgtcgtgg agacgagggc gctgacggac 180
   accaccctcc tccggtcgct cgcggcgaag ggcctcgccg tgaggcccgg cgcgtcggac 240
   gagcaccaca cggtgcggtg cgacgccgtc atcgtcggct ccggctgcgg cggcggcgtg 300
   gccgccgcgg tgctcgcgtc cgccgggtac aaggtggtcg tcgtcgagaa gggcgactac 360
   ttcaccaagg aggattacag ctcgatcgag ggcccgtcca tggagcgcct cttcgagagg 420
   ggcggcgtct tctgcacgtc caacgtcacg acgatgatat tcaccggcgc gacggtcggc 480
   ggcgggtcgg cggtgaactg gtcggcgagc atccgcacgc cggcgggcgt gatgcaggag 540
   tggtcgcgcg agcacgggct ggcggtgttc gcgagccccg ggtacgcgcg ggccatggac 600
   gcggtgtgcg agcgcctcgg tgtgaccgac gcgtgccggg aggaagggtt ccagaacaag 660
   gtggtgcgcc gcgggtgcga cgcgctcggg ctgcgcgccg acgccgtgcc gcgcaactcg 720
   tcggaggggc acttctgcgg cagctgcaac ttcgggtgcc ccaccggcga caagaagggc 780
   accgacacga cgtggctcgt cgacgccgtc gagcgcggtg cggtcatcct gaccgggtgc 840
   aaggccgaac acttcatcgt cgagagcaac ggcggtggcg gcggccggag caagaggtgc 900
   gtcggcctgg tggcgacgtg catgagcaac ggcatcacca agaagctccg cgtcgaggcg 960
   aaggtgtcca tctcggcgag cggcgcgctc atgacgccgc cgctgctgcg caacagcggg 1020
   ctcaagaacc gccacatcgg ccggaacctg cacctccacc cggtgtccat ggcgtggggc 1080
   tacttcccgg acaacacgcc ggagccgcac atcccgggga agtgctacga gggcggcatc 1140
   atcaccagca tgcaccgcgt cacggagcgc accatcatcg agacgccagc gctcggcccg 1200
   ggcgccttcg ccgccctggt gccctgggag tccggccgcg acatgaagga gcggatgcgc 1260
   cggtacgcgc gcacggcgca cgcgttcgcg ctggtgcgcg accgcggcgc cgggtccgtc 1320
   gacggcgagg gccgcgtccg ctacgccccg agccgcgacg acgccgagga gctccgcgcc 1380
   ggcctccgcc gcgcgctgcg catcctggtg gccgccggcg ccgccgaggt gggcacgcac 1440
   cgcagcgacg gggcccgcct ccgatgcaag ggcgcgcgcg acgcggacgt ggaggcgttc 1500
   ctcgacgagg tgaccgtgga gaaggggccg atgcactcga cgacggacaa gtggtcggtg 1560
   ctctgctcgg cgcaccagat ggggagctgc cggatgggcg cgagcccccg cgacggcgcc 1620
   gtcgacgtcg ccggcgagag ctgggaggcg gaggggctct acgtctgcga cggcagcctg 1680
   ctcccgacgg cggtgggcgt gaacccgatg atcaccatac agtccatcgc ctactgcgtc 1740
   gccaagggca tagccgactc gatggcacac ggcaaggagc agcgctag 1788
<210> 130
   <211> 534
   <212> DNA
   <213> Oryza sativa
<400> 130
   atggcgccgc acccgctgct gaggggaggg gcgaggcggg ggaggaagta cgcgcacggg      60
   atgcaccccg cgcagatgga ggcgctgcgc gccatgtgcg gcgcgctcat cccgtcgctg     120
   cccgtggacg cggacggcgg cgacggcggg cgccgccccg gcgacaagga cctcgagcgg     180
   ttctacctcg cctccgccgc cgactcctcc atccccgacg aggtggcgga gctgctggtg     240
   acgcgttgca tatgggaggc ggtggcgctg acgtgggtgg tgctgtgggc gctgagcacg     300
   cgggcgggca cgctgctgct gtgcggccgg gacagcgtcg ccgccgtcga cggcggcggg     360
   ttcccgttcg tgtccgtgcg ccgcttcgcc gacatgccgg cggcgaggcg ggaggcggcg     420
   ctgtggcggt ggagcggcgc gcggtggctc ttcttcccgc tccgcatcgc cttcgccatc     480
   gccaagatcc tctgccacta cgtcttctac tccatgatac tcgtatcagc ctaa           534
<210> 131
   <211> 4029
   <212> DNA
   <213> Oryza sativa
<400> 131
   attgctgcta ctctctctcc ccttctcttc tccggttctc cctctcctcc tcccacctca 60
   gccgccgctt accggcggcg ccgcctgcta atctgccggc gtgagcaccg gcggagatgt 120
   gttactgcta cccaacctat aacgcagctc tcccgcgcag tgcctctgtc tctagctagg 180
   gttttcctct tgggggagac gacctactat ggtaaggtgg ggagctgaag ccggcgacct 240
   ggtgctgtcc ggaggaagct ggagctcgtc gccgtcggat ggacgccgat gaggccgcgg 300
   ggagtagcag gaggatggat ctgaacctct accttggcct cccacgcgcc ccgcgcccgc 360
   gccgctccga cctcggctcc gacctcgccc tcagcacccc gatgccctcc tccccgtcct 420
   cctccgcagc ctccgtcgac gcgccgccgc caccgcccga gctgtcgcat cccccgtact 480
   ccccctctca cgccgacctt tcccctccgc tgcaggaggt ctactccctg tacaaccccg 540
   acgacccgcc tgcttccgag acgcacctgc cgccgtatgc gccgcctccg gctccggtgg 600
   tctcggagct ccctgacgac ctcgagtttg gcctccaccc cccgccgccg ctggtgcgtg 660
   ccagcgaact gctaggttgg gaggaccggc cgtcttcgtc gacggcatcg tcctctttcc 720
   tccctgacac cgcagcccgt tactggcggc ttctcgagca gactggaagc agatggctcc 780
   gtgcgaggcg gtttaggtcg gaccttccgc cactcagttc tgaagcttac ccagctgggc 840
   gtgatgctgc cgcagtccca gtgctgcagc atgaaccgat gaatgatact gttgaacata 900
   ataaggtagc tgccgatggc gcggaagtag gcgcctccga ggaatcggag gagcagggca 960
   ggagcgctgc cacatttgag tgtaatatat gcttcgatat ggccagcgag ccggtggtca 1020
   cctcttgtgg ccatctcttc tgctggcctt gcttgtacca atggctcaat gtttattcca 1080
   atcacaagga atgcccagtc tgcaaaggcg aggtgactga ggcgaatatt actccgatct 1140
   atgggagagg gaattcatgt ttggatgccg agaaggctgt ggaaggtggg aaacaaacag 1200
   gtcctactat cccaccaaga ccacatggaa atcggctcga aagcttcagg cagcagtttc 1260
   accatttgcg accgatctca agaaggcttg gtgaggctca tgggttattg tcatcatgga 1320
   ggcgccttct ggaccaacag attatgaata ctgcgagtag gtttgaaggt ccgcctgaat 1380
   cagctgtgca ggaaatggtt gacactgctc acgctcagca caccagtcgc ctaagtagat 1440
   tggcgtcaag gatgagagca agacggttgc tgagagaagc agacaaccct aaccctcccg 1500
   atggcggatc cacttcccct gacagtggtt tgatcagaaa caatgcatcg gatccatcca 1560
   gaaatggtcc gagctcatta ttaccagatg gaattgactg gttgcgtgga cttacccttc 1620
   ttgggtatga agacacggaa agatttgcat ctgccatgag tgattttaga aggataactg 1680
   gaccaagcca atatggtgca tcggcttcat catcgaatcc tccaaatctc gagtcaacat 1740
   ttgacagaac tcatgttgtt gcagcacctt ctgcagacca agcatctaac tcaagcactg 1800
   ctgcagtgat acagggggat gctggtatct ctgagagtgc aggagaacca agtaacgcgg 1860
   ggtcatcaag atccctgagg aggagaggga ggagcagtgc cctgggttct ttggatgctg 1920
   atggcggggg cctccaacgg aacaagaggc gaaggataaa ctgaacattc tgtgttgtgg 1980
   tgttgatcta aactctgcat gccatgctcg ctgattttca actattgcat ttcatttctt 2040
   cgggtgatgt ctcctgtgtt gtagtgtaac attttttctt ctcttttcat tttcccccgt 2100
   aggttgcact gaaatgttta tctgtttagt tctcatgtag cctgtacctg tttaatttat 2160
   ggaaagttat tgatcaagac atttttgcat tcgaaaggta atgaatggtt caactgcatt 2220
   tccatgacaa taaattggat gctgaaatgt gcatccaaca caatggtatt cttgtgcatc 2280
   aaataatagg cataaacatt gtgtttttat ttgtgtcaat aagctccttc agacatatag 2340
   aaaactaaca ggtcaataat gtagtatatt gaagttggaa ggaataccca gagaatggat 2400
   ccatggacac aattgtcttt tgttgcttgg gagaaggtac atagcctgat ctttagtcct 2460
   tgtttatcct ccaatgaaaa tactcacgca ttgattgttt caatagacaa ggtaaacttt 2520
   gccatcgccg tgaattttat gatccatgga agctgtttca ttgagcagtg gtgggtgtaa 2580
   ctgtgataac ctttatatta cttgcttgct tttaaggaat agcacacttt ttgtggggat 2640
   gggattactc ccctttttgg aactacatat tgaggaacgg atttaacata gaaagagctt 2700
   aatacattta agtactgatt gtgtgcatag aaaggggttt attatttgga acaaaattgc 2760
   ttagctcgta gtaataagga agttcatagt attgaacttt atatgacatg tgagttatat 2820
   atatcatgtt tgtctttggt cacttctatt ttatgaccat gtaacattta tttcatgtgg 2880
   aacttggcag aagagaattt gaagagattt cttctgattg atttccattt cggacaaaat 2940
   acaaagctcc cagaccagct gtcacaaaga ggtctggttg attttccaga tttgtatgcc 3000
   tgtctctagc cgagagtaac aggtattttt ctgcatctat gactggcatg gatagtagat 3060
   tggcatatgg aaacaaactc ggaagggttt gggtggtgct taggtgctct tggcagggaa 3120
   gggagacaac tctgtgtttt tgggttttca gtacatctat cctacatatc ttccaaaagc 3180
   tcacacataa gccagttaat tgtttttttt tttggcttgc agaattacct caataacttc 3240
   catgtaaata ttttactctt ttgcagtatt acatgaacag attcaatgct atttttctgc 3300
   agaactgccc caagatgtaa aatgaaaatg cattgagtct gttcaattag aaaacacaag 3360
   atcactgtgt atactgttca aagaatgtgc taaacatatt atagaaccaa acacagttca 3420
   tctcaacatt gttgcttttt gtcttagttg tcacaactat ttaaacggat gaaacagtgg 3480
   aatctctaag caaagctgtg aagagcttca gggctacctc ctggcgcgaa atgaaggtgc 3540
   tattgcaagc tgctgcagct ctactgggtg aacaattact cacatgtcac cagaactcta 3600
   gcaatgaagc agcatattgg aacctgaaaa gatttggatc tcaccaatgt ggacaacgag 3660
   gctcatgagt tatgactgct atgatgatga tgatgaagat ctgcttcagc aagctcatgt 3720
   tatggatgta ctactttagt atgctttgct ttgttgattg ggagctgcat cagatcctgt 3780
   ttgactgctc aatcaattgc accatcttac tctcacctac tgttggatga gggtggggct 3840
   ttgctgtatg ctcaatgttc ttgaactctt gcatatgaca gtaatgatgt tccgtcctta 3900
   agaaattgta cttatgtagt gcagcagttg atcatccagg atttaaccca agttctaggt 3960
   gatacaatat aggttgtaca tgacctaata aatttctaaa tagaaggtaa atccatgttg 4020
   attttcagg 4029
<210> 132
   <211> 5036
   <212> DNA
   <213> Oryza sativa
<400> 132
   tcgcatatcc cgttaccttt gccgccgcgg cgccgccgcc ctcttccgct cgccgccggc 60
   cgagggcgcc cgtcgcgcgc cgtggagcga gcctagccgc acgagctgaa agcacctaaa 120
   aggtcagcgc gtcccctccc cctttcctct cctttcgccg ctcccgcagc agccacagga 180
   cacctgagac gggtctgggg gtgcaggtcg cgcctggagg aatccctagg ggctagggga 240
   ggtggctgga gatggctggt gggagctgcg acgtgtgcaa ggaggcgccg tccaagtaca 300
   agtgctccgc ttgccgcacg ccatagtaag tccagccacc ggatatctcc acccttctgt 360
   gcttacagct tccatgggta gcgtcgtcgc ataccactgt ccttaaaatt tgggaaatcg 420
   cttcgatcat ggtgctcagt aatggcctac tagctagcat aacaattgaa ttgaagaaca 480
   cttgttacgg ttttagtgca atgtcctact tgctgtgttc acatcgatta cttcactgct 540
   tactgcttga aaagagagta cgggtttgct aggtcaatag agtagattga tgattaatgg 600
   ttggaagcat tagcttcttt ttagaatatg agcctacatc agaattagct tgatatattt 660
   tgggtttgac tcggtagctt gctgctggag tggttttgca tgagtaaaaa aaaaaaaagg 720
   cagcagcaaa acaagacatg taaacctttc tcctgatatc tggtgtcagt gttggttttg 780
   gtactaagct gtattgaatt agtattcaga ggcaaacgtc catcattcaa taatctgtaa 840
   tactttgctt atttttcttt gaatacgcaa gagaattaca tatcatactt tatgacttct 900
   cttgcacaat tgtttcaagg atgtaacctt aggattgcca aatatgatta tctaaacaaa 960
   ctgtttttca agattccctg aaactaaatg gactactgaa ctaacatacc tgttcagcga 1020
   tgcaatcacg gttggacatc ttctatgtgt cctgtagatt tgtttggtaa aattgctctt 1080
   ttgccctaaa gttgaatggc attggctcat ttgccacagt ccggtgtatg acaactgggg 1140
   ccagggctac atgttataca cacatatgtg gcaaaagagc acatgtcata ttgtagacca 1200
   attaagagca tggtccgttc aatttctctg gcttcttttt ctctattgtg taatgcagtt 1260
   gggccttaca aacggtttca ttatcccttg cagttgctcg gtggcatgct ttaaaaatca 1320
   caaaggtact cagtgctatc ctttggtagt ttctaaatat agctctagtg ctgcgatctg 1380
   aaagtagctc aagtagcgcg agggggctgt agcaaactgc cgtgttccct ctgttgcttc 1440
   ttagttgtgt attttaaatc aacaagcttc cctgtactgc cttttctctc tttggccagc 1500
   gttctttgcc agccgcattt ttaaacatag ctcaataaac caaacgtctt aaaaaaatat 1560
   agcaagtgat aaactgctta tcacactcca agtgtccaag tctgcggggg accttttgtt 1620
   ttgaaatttt ggcaatttta ctattgtact tggtttgaga ttagggaaag gctgaacagt 1680
   actaaacaca gcacaaatta taactatcat ttatgcattg cctttatgca aacaaaaatg 1740
   gccaccaaat atcttcatct ccctttttta gaggaatata tttattatct tgatagccag 1800
   tatttgtgct aaatatcgtt ttgccaacag ataaattttg ccagaagaca atacctctgg 1860
   aagaagttag caagtcatct cttcaggagg aaatttgtaa gtgctctagc ttttggtgac 1920
   tcaatacagt tgtttgcaac agctatatag tcctgctata tatggaaatt gagttgaaac 1980
   catgatcatt aaaatagagg ggtcctacat aaaattacat caatatactg taaatgagca 2040
   tttgactgtg tacacctaaa agtcctgcag ggcatgtact tgcccatata taagggcctg 2100
   aattatctta tcatatagtc attcattaaa ttgtcaattg ttctgtccta ctaacatgtt 2160
   tctaaaataa tctactggaa tttacagttg tgccttctat tgttttcagc aaggaactct 2220
   aggtcactgg aagaagcaac aaattgtcct aatgacaagg atcaaacccc gtctttatgt 2280
   aaaaatgcac catacttatc tctgttgtta actctccatt tcttctattt ggttttcact 2340
   aataattgat tgaactcaaa actttataga tcatgactac cttcagttgt aaacaatgta 2400
   accatatgct atcctcgcaa aaaaagaaga agaagtaacc atatgctatg ctttttaatc 2460
   aataatttaa attagtttct ctagaattta ttataagggc tataatatta ttcattgtga 2520
   atcttgaagg ccaagaaaat ttatgttaag ctcaattttt tgtctgtagt atcggacacg 2580
   acttgtccca cacaatatcc aaacacattg cactctgcaa aatctcttga agttgaggat 2640
   ccaagctggc ttgttgacaa gaatggatta agatctttag gtattgccta tgtttgacta 2700
   tttcatacca tgatttgtgt tttttaaaaa ggaatccttt gcttttactt gatttctggc 2760
   tttatttgtt agctgaaaca aatgtctcca tgttagtata gtccagcatt tcaacccaag 2820
   aatatcgact tcaatttcat aattatctga acctaatccc aatcctacag acctacagta 2880
   taggagtaaa atattggcat gcacatttag taatggggaa tttaactatt tgccactttt 2940
   ataaatggct actctccgtt tgccattctt acatttctct acggatttgc caccagagag 3000
   aaggtttctt aatcatttgc cactttcacc tattaagcat gctagtgagg acagccaatg 3060
   tggacaagag acacaaaaag tccattttac ccatggccct tcccttgtgt tcccccctct 3120
   ctccctttcc ttttcagatt tgagctcacc tccgccgctg tttctcgtcc atcttctgca 3180
   ctgctgtgta gtgacaagcc attaggtagc agtaacactt aggtgcgtgc atggatgttc 3240
   tttggctgct attgattgcg gatgtacctt ctccttgtgg ctgtcgatga caaccttcaa 3300
   ccttgcggat gtaccttctc cttgtacctc aacgtgctcc tcacttgccg gccttgccat 3360
   caatgaaccg catgacgacc tgcaccgggg tgctccgcca tcctcccagt gagtttgagg 3420
   gcctcgcggt tatctggcct gccgaagaag atgatgcaga tgcagtgcac aaccttcttg 3480
   ccgctgacac atgattgacg aggcttgatg agacagcgtg gctggtgtgg ccgcggatgg 3540
   tctcaatgag gaggtgaccc cgcaagcagg aagccagaag catacgtgca ccgctgtgga 3600
   tgatctcaat gagatgaagg ggagtggcag gggcaaaaag gacattcacg tcctcagtcc 3660
   atgctggtat gccacatcgg tgaaaggggc aaattgttaa gaaaatttct ctccggtggc 3720
   aaaaatgtaa ggacaatcgt aagtggcatt tggagagtgg ttattcgtaa gagttgacaa 3780
   atagttagta aggatagtac tgtaagcaga atatgggctt catgctagtc aattttgtat 3840
   attggttcca catccctccc tgtacaaatt tcataacctg ctagcatttc taccttattt 3900
   aattaatcat agtaatactc atcgtcttgt ctcgcaaaaa gaaataaaca tcatcaatta 3960
   actcattgct tgatttttgt atccatgcca gcggaatcta atgagatccg agatgctctg 4020
   aaagattgta agcttcagca aatgctactt aagattgatg gctctgcaga gccagaaaag 4080
   gtaattctct actcttgtga taactgcaag tttatttaac aagttaacta tgtttgcaat 4140
   ttctgtccgg ctgctcaaat aacatgcaca taatatgcca atacctattg aggacaatgg 4200
   tttgcagtaa tgaacattgt cactgttata gtacaacact tctgtttctt attccaccat 4260
   catgacttca ttttcctacc agtagttgag aagcttcaaa tgggcacgtt cgatgaatta 4320
   attcctttta ctatagtctg ctgaatactg atacgaatat aacttttctc acttacattt 4380
   atttttttcc tctctctacc acctttattt tatgatgccc actccctcat taattcatcc 4440
   tcaggaatta gagaaattga tggaaggaca agtttttcaa cagttcacca ataaggtttc 4500
   tctttccgtt gcatttgctc ttctatccac aagatattcc ttgttaactc taagttaatt 4560
   aaccagacac ttgcacgttt ttgtttcctc tgtgtttgca gattcttgac attgttagcc 4620
   cacaacaatg aacaccaact ttctggtgga gctattcagc agaagagtgc acgcatcggg 4680
   gcaaatgaca acatgtagag caacttacct cagatgctgt tttcctacca tgatgagatt 4740
   gcctataccc gtgacatttc cttctactac tttgagaaag ttttgtcgtc caatatggtg 4800
   ctagtatttt accatggcat ctctggctca atcaacaact gttcaatttg tctacatccg 4860
   tgacatcttc tttggctaat ggctactgtg agagttttgt tgtccattat gttttttttg 4920
   tggtatactc cctccagttt taggttttgt tgttcagttt tgtggtttac caaagtcaaa 4980
   ctactttaag tttgactaac gttatagaca aatacaataa tattcacatt accaaa 5036
<210> 133
   <211> 2503
   <212> DNA
   <213> Oryza sativa
<400> 133
   atgcccaggc acgcatgtct gctgcaggta gccaaaactg aaacttagaa atccgtgtac 60
   gcgtcgctta agtgcttcgg cttaaggatg ggcattcggt caggctgaaa aattcggtct 120
   cggtttttag ttttttggtt agttcggttt ttgaaaactc aggaccgaat ttcatcacaa 180
   aaatctcata actgataaat tcggtctcgg tttcggtctc agtctgaccg aatttttttc 240
   atagccactg aagagtgcag acggaggcga cgccaaggag gtgccgacgc cggcgctgga 300
   ggcagaggcg gagggcgccg gtgctgggcg gaggcggcgg aggcggcgca catggcggcg 360
   gcggcggcgc atagggcggc ggcggtgcac agggcggcct gcggtagctc caccgtcgcc 420
   gtcgggctgc gggagctcca ccggcgtcgt ctctgcagca cctgcacggg ggatggggat 480
   ggggataggg tatggatgga gagatgcttg tggtggtggg tggataaggt ttttcttttg 540
   ctgtggtctg tggaggtatt gggcctaatg ggccaatgca atatttcggt tttttgtcaa 600
   ttcggttaac cgagccaaaa aaccgaattg accgaactaa atttggttag ctgaaactgc 660
   tgaccgaatt tgtgaccgaa tttttcagtc tcggttagtt cggtttcggt ctcggttatt 720
   ttggttcggt ttttcggttc ggcctttttt cccaccccta ctgcggagaa tgcagcgcga 780
   tgggcctcca atctttgtcc tgcctagctc gcggcggatg tgtagttgca tgatccgagc 840
   agatttgtcg tggcggcgtg cggtcaatat cgggatcagc gcagtggccg acggcgcgga 900
   caccggcaga acgtagcggc cagcgggttc gcggttgatc aaacggctgg gggtgcccgg 960
   tccacgtctc tttgacggta tacgtacata ttcataatat catatatttc atttgatatt 1020
   tttccctctg tttcatatta taattctatt tgactctttt tttaaagtca tttttttcta 1080
   aatttgatta agtttatgta aaaatttagt aacatttaca acacgaaatt aatttcattt 1140
   aaactaacat ttaatatatt tgataatatt tatgttttat gtcaaaaata ttgtcaaatt 1200
   tttctataaa tttaatcgtt cttaaaaaaa attaattaga aaaaaaatca aaacaactta 1260
   aaacgaagtt tgaaactgag gaagtacatt atcatatttt aagacggtaa tagttactac 1320
   gaaagagcag cccgattaac ggaaaagcaa tagagaagaa cgtacgaacg tcgcgtgcgc 1380
   gaaaggagga cagaaagaaa gatcgatcga tcgatggatc ggccaacgcg agcgaaacgt 1440
   cgtacacatg tacacacaga tcacgagttc acggctcatg ggtgtcggcc cttcttttgt 1500
   cgaaaagaaa aattgttgcg tgactgaatg gagatttcag atttctgcgt gctcggttag 1560
   ttgattacaa gtactagtta ctagtatacg caacagagac gtacgtcgtc tgctgcgatc 1620
   tcaatatctc atgctcacgt ttggtggtgg ttgctgttcg atcgtccagg tattaattgt 1680
   cgagccatgc atccacgtgc acagggtatc atgtttaatt cgtgacttac atgtccttta 1740
   tggttgatgt ctcattggat tgatcatttt cctgggagat aattaattac ttactcctac 1800
   tccctcggtc ccagaaagag acgatttctg gaggggagga tttgtccaaa aaaaagcaat 1860
   tcctctacag aaatcaagaa aacttcaagt atatcgtatc attatgggcc caagtggata    1920
   gcgaattctt tttctctcgt tcacattcac cccacaagaa tcttatcgcc tgcccgtctc    1980
   tcgcagtctc gcatttctct ctttttctct cacgtttctc tcccgttccc aaatcgattg    2040
   catcgatgtg gcggcggcgg aggcgacggg gagacgcggc ttcgtcggcg gcggcggaga    2100
   cgcggaggcc acgaggagac gtggcgtcgt cggaggcgga ggcaacaaat ggatgctgct    2160
   tcgtcggcgg cgggggcttg tatgcctaac tgcttatcat ccccggaaga agatctgatg    2220
   cggttgcctg atgcacgagc tgcgttgttc ttgtgttcat tgtgttcatg cactgcattt    2280
   ctttttcttt gggttcttgc catgttcatg cgtgcgtcag atgcttatgc agagaaatga    2340
   cagtagcaat agcaaatgga tgttgtcacg gagggcattt tcgccttttc acgtgagtgc    2400
   taaatttgca tgggaggtac aggaatcgct tctggatgga gggagtacct ggttctgttt    2460
   aggatctagt gcgtactttg tcgcagtcaa atacgattgg tga                      2503
<210> 134
   <211> 567
   <212> DNA
   <213> Oryza sativa
<400> 134
   atggggacgt acaagtgctg catcttcttc acccgcaggt tcgcgctgag cgacgcgtcc      60
   acgccgggcg acgtgcgcat gctgttcacc cgccacgccg gcggcgcgcc ctacatgggc     120
   atcgacgagc tccggcgcta cctcgccgcc agcggggagg cccacgtcga cgccgacacg     180
   gcggagcgga tcatcgaccg ggtcctgcag gagcgcagcc gcaccccgcg cttcgggaag     240
   ccgtcgctca ccatcgacga tttccagtac ttcctcttct ccgaggacct caacccgccc     300
   atctgccatt ccaaggaagt aagcaaacta cccgctcgat ccccaatttc ccaaatgctg     360
   ttagattcat cgtcattccg tgataatcct gccgttgcac aatgcggtga aatggcgtaa     420
   tttgctagga ttcagaaggg gattcttggg gtttgtttag ttcacattaa aattaaaagt     480
   ttggttaaaa ttggaatgat gtgacgaaaa gttagaagtt tgtgtgtgca ggaaagtttt     540
   gatgcgatgg aaaagttgga agtttga                                         567
<210> 135
   <211> 2684
   <212> DNA
   <213> Oryza sativa
<400> 135
   atgcaggtaa tgaattgaat ttccatacaa cattctgctc tcctaagaaa ttacgcttac        60
   aagttcactt ggatattgct aaactccatt ttgatattac ttagtgtgta ctgaatgatc       120
   taagatgtga gttgatggta gatctcgtgc tctcaggtcc atcacgacat gaatgcacca       180
   ttatcgcact acttcatata cactggacac aactcgtatc tgacgggcaa tcaacttagc       240
   agtgactgca gtgatattcc catcattaag gcactgcaaa taggcgtccg tgtaattgaa 300
   ctggacatgt ggccaaattc ttctaaagat gatgttgata ttctccatgg aaggtatgca 360
   tgagaattgc tcacttgaag acatttttgt tctgcactgg aggccattcg atatgctatg 420
   accttattcc aaactatttg cttctttggt aggacactga ctgccccagt atcacttatc 480
   aaatgcttga aatccatcaa agaatatgcc tttgttgcgt ctccctaccc tgttattata 540
   acattagaag accaccttac atctgatctt caggcgaaag tagctaaggt aattgcattt 600
   tcctcgtatg atcaataatt tggtgcagtt gattctgttg tagctagtta tgaaattttc 660
   tttagatggt tcttgaagta tttggagata ccctatatta tcccgagtca aaacatcttc 720
   aagaatttcc ttcacccgaa gcactgaggg gacgtgtcat cctctcaaca aaacccccaa 780
   aggagtacct tgaatcaaaa ggtggtacta tgaaagacag agacattgag cctcagttta 840
   gcaaaggaca aaatgaagaa gctgtctggg gaacagaagt cccagatatt caggatgaga 900
   tgcaaaccgc cgacaaggtt ctactggttt taacatttgt tgtttcttgt ttcttagcat 960
   atggtgtatg tccatcactg ttgtattggc tttattccct agcagcatga gaatgatata 1020
   ctatacaccc aaagagatgt ggaagaagat gatgagaaga aaatgtgcca gcatcaccca 1080
   ctagagtata aacaccttat tactattaag gcaggaaagc caaagggtgc tgtagttgat 1140
   gccttaaagg gtgatccaga taaagttaga cgcctcagtt tgagtgagca ggaacttgca 1200
   aaagtggcag cgcatcatgg tcgtaacatc gtgaggttcg tttagcaaat atactgaatt 1260
   tcgtagcaaa gtattttcta tcattgcacc agagctctct atgtccattg accttaactt 1320
   cattctgttt attcaaagca gctttacaca taaaaatctt ctgagaatat acccaaaggg 1380
   cactcgcttc aattcttcga actataatcc gtttcttggt tgggtgcatg gtgcacaaat 1440
   ggtggcattt aatatgcagg tacatttcta acatgacact cctctgctac atcatattgg 1500
   cctgaatgcc tgatacattt ttcttcgcag gggtatggaa gatctctttg gctaatgcac 1560
   ggattctaca aggccaacgg tggctgcggt tatgtgaaga agccagattt catgatgcaa 1620
   acttgtccag atggaaatgt ttttgacccg aaagcagatt tacctgtgaa gaaaacactc 1680
   aaggtaggtt tgtggcatat gtttcttcct ttcattttca tctctgaaat tcaggaatcg 1740
   agctacttac agcttgcctg tttgtctacc aggtcaaagt atacatgggc gaaggttggc 1800
   agagcgactt caagcagaca tacttcgaca cgtattcccc tccagacttc tacgcaaagg 1860
   tacatcgaat tttacgctga tgccaaacgc caacaaattt gcaaatgcaa aacggagctt 1920
   tgaaaaaaca tgtatatatg tataactttt acatatggag tgagatgaag acaaacttta 1980
   tatcaaaatt gtagagctcc atgagttcta cgacgttctt attgactagt ccatcgttcc 2040
   atcatcataa caggtgggca ttgccggggt tccgtcggac tcggtgatgc agaagacgaa 2100
   agccgtggag gacagctggg ttcccgtgtg ggaggaggag ttcgtgttcc cgctgaccgt 2160
   cccggagatc gcgctgctcc gcgtggaggt gcacgagtac gacgtgagcg aggacgactt 2220
   cggcgggcag acggcgctcc cggtgtcgga gctgcggccg gggatccgca ccgtgccgct 2280
   cttcgaccac aaggggctca agttcaagag cgtcaagctc ctcatgcggt tcgagttcgt 2340
   ctagcaaatt cagtaggcat atcactcgct catgtgtgtt gtatacttag catgatgatc 2400
   tatttctcta gtagcaagat tagattttta cttatgtgtg ttgtatacgt agtatgatga 2460
   tattttctag caagatcaga attttggact acctgttttt ctaggaaaaa acagattatt 2520
   tggacatcgg tgaccagaat tttggactag caagatagat ttggactgct ttgatctgca 2580
   gatcggtgga catttttcta gcaagattag aatattagat tatggtttga ttagatttaa 2640
   gaacttgttt tggtctctat gtagatcgga gaatcagttc catc 2684
<210> 136
   <211> 1012
   <212> DNA
   <213> Oryza sativa
<400> 136
   atggcgatgg gagccgcggc ggcgccatgg tacggcgcca tcggcggcgg tggctcgcgg 60
   cgcgcgcggg tgagggcgca ggcggcggcg ccgtgggcag gaggcgcgga ggagctggtg 120
   cggtcgggcg cggtgcgggc ggtgcgggcg agggaggcgg cgggggcgat gtccgcggag 180
   gggttccggc tgctggacgt ccggccggag tgggagcgcg cgcgcgccgc cgtgcggggc 240
   tcggcgcacg cgccgctgtt cgtcggggac gacgacacgg gccccgtcac gctgctcaag 300
   aagtgggtcc acttcggcta catcggcctc tggaccggcc agtccttcac caagatgaac 360
   gaccgcttcc tcgacgacgt cgccgccgcc gccggcgaag gcaaggacgc caagctgctc 420
   gtcgcctgcg gcgaaggcct ccggtaatta atctaatcac actgaagcta ctgagaattt 480
   ttatctgttt agtgtgtaat acaacgtggc aattaagctt ctggatcggt tgcatgcatc 540
   cgaactaggg ctgctttccg aactactaaa cggtgtgttt tttgtaaaaa aattctatag 600
   gaaagttgtt ttaaaaaatc atattaatcc atttttaaag tttaaaataa ttaatactca 660
   attaatcatg tactaatggc tcacctcgtt ttacgtatct tcccaatctc ctctatctcc 720
   tcctcctcaa acacaggtcg ttgatcgcgg tgaggatgct gtacgacgac gggtacaaga 780
   acctggcgtg gctcgccgga gggttcagca agtgcgtcga cggcgacttc gccgacgtgg 840
   agggggagag caagctgcag tatgccaccg tgggtggggt gtcctacatc ttcctccaga 900
   tcctgcttct gctgcgggta gtcaagtgat gatcatgtaa catcaggaca tgcatccgag 960
   tatccgacca atgttgcagt ggaatatgct gccaagtccc aaatattctc cc 1012
<210> 137
   <211> 1186
   <212> DNA
   <213> Oryza sativa
<400> 137
   atctccaaga agcagtctcc acgccgatcc gagatggccg cagcagcgca gaggcggcgg      60
   agcagcagcg cctccccgga gttccgcttc tggcccctcg acgccgaccc cgccgcatcc     120
   ccctcctgcg ccgacgagct cttctccggc ggcgtcctcc tccccctcca acccctcccc     180
   tacccccgcc gcgacgccga cctctccatg tccctcgccg tcgcggatga tgatgatgat     240
   gaggacgagg aggaggagga ggtgcagcct ggtgcggccg tcgcgtccag ggcgccgccc     300
   actgctgcgg tggcggcgtc gggtggtggt ggtggtgggt cgaagaggtg gacggatata     360
   ttcgccaaga agcagcagca gccggcggcg gaggagaagg agaaggatca gccgacgagg     420
   cggcggagac cggcgggagg cggaggcgga tcggagctga acattaacat ctggccgttc     480
   tcccggagcc gctccgccgg cgggggcggc gtggggtcgt cgaagccccg cccgccgccg     540
   cggaaggcca gtagcgcccc gtgctcccgc agcaactccc gcggcgaggc ggcggcggtg     600
   gcgtcgtccc ttcctcctcc tcctcgccgc tgggccgcca gccccggccg cgcaggcggc     660
   ggcgtgccgg tgggccggtc tagcccggtc tggcagatca ggcgcccgcc atcgccggcg     720
   gcgaagcacg ccgccgcgga caggaggccg ccgcaccaca aggacaagcc aaccggcggc     780
   gccaagaaac cccacaccac ctccgccacc ggcggcggcg ggatacgcgg catcaacctg     840
   agcatcaact cctgcatcgg gtaccgccac caggtgagct gccgccgcgc cgacgccgga     900
   gtcgcccgcg cctccgccgg cggcggcggc ggcggcgggc tcttcggcat caaggggttc     960
   ttctccaaga aggtgcattg agccatggaa gcctttcttt caccttagct agagatccaa    1020
   ataactttta attttctcct ctctttttta ccctcctttt tttacttttc tttttttttt    1080
   taccttttgt aacttttttg tttaaccttt ggggtgcttg tgatcatgat gatgatgatg    1140
   atggctgtta attacatgta attaagccaa taacctgttt ttgtaa                   1186
<210> 138
   <211> 3363
   <212> DNA
   <213> Oryza sativa
<400> 138
   aaaaacgaaa aaaaaaaatc aaaatccttc cccctttcaa attcgaaatt tcgaatcaaa      60
   cacgcgcacg ctcctctctc ctctcccctc tcgatcgagg cacgcttctc cgcggcggcg     120
   gcgcgcgacc ggatcacggc ggcgggggag gggaggggag gggaggatgc agcagaagcc     180
   cgcggcggag gccatggagg aggagttgaa gggggaggcc gtggggcccc gccgccccgg     240
   gctagggtta tggttggcgg cgcggcggcg gctggccccc gacgacccct tcttcgccgc     300
   cggggacatg gagcgcgagc tcctcgccaa gcaagtgcgt tctcttcccc tttcacctcc     360
   cttccttctg ctcgcttaat ttgggcgctt tagcttcttc gattttacgg ggattttttt     420
   tgttagctgt attgagctgg aacccgtgga aaagatgaaa tagagatagt aaaatcagcc     480
   atgataacct ttgattttgc agaaattcag ctgtactgta cataagagga aaagcttttg     540
   cccttctttg aaaaaaaaaa gagagaagaa attcagctgc acttagcatc agttaggtgg 600
   gaattcctta atgctctgca atacttgttt ctttgatttc ttgaacggat catggccgat 660
   tagctcagat accctgccct gatgtgaaaa gtgtagatga actgtgcttt tactcaacaa 720
   gtcccctata ctccactgtt tttttaggtg tctgatgttt tcttgtttag gttgctctgg 780
   atctctccga agatgaacgg taccagcttg agaggatgga agtggcgagt gccaagtaag 840
   agattgatct ttacttatgc ttactctttg gattgatgat tatttatcct ttccgtttca 900
   cgccgaatta gggggggggg ggggttagtc cctgtcacat cagatgtttg gacactaatt 960
   tgaagtatta aacgtagact attgacaaaa cccactccat aaccttggac taattcgcga 1020
   gacgaatcta ttgagccaaa ttaatccatg attagcctat gtgatgctac agtcaacatg 1080
   tgctaattat ggattaatta ggcttaaaaa aaatttcacg tgaattagct ctcatttatg 1140
   taattagttt tgtaagtagt ctatgtttaa tactctaagg gcccctttga atcgtagaaa 1200
   tgaaaaaacg gaggaatagg aaaaacatag gattcgacag gaatataagt gtaaaacaga 1260
   ggattgcaaa acacagggaa aacacatgaa tgatcgtttg attggaccag aggaaaaaca 1320
   caagaatcgg atgagagaga tagactcaaa ggattttttc caagaggttg gacctcttgc 1380
   taagtttcct ccaaaaccta tatgccataa gccattccat aggaattttg taggatttgg 1440
   aaaacttcaa tcctttcaat caaagagcta tataggaaaa tttcctacag gatttcaatt 1500
   ctatgaaatt ccttcataat ttcatttgat tcaaaggggc ccttaattag tgtctaaaca 1560
   tccgatgtgc cagggactac agtttagtcc ctagatccaa acacccccta acataccctc 1620
   tctacagatt taaaacagca tgttaatgta ctgtttttct gcatccccat aacaccataa 1680
   atttcttcgc tgtagctaag gaagtccctg aaccagtggt ccacacccca cagtgactaa 1740
   agtctgagaa aatgacaact ggatgtcttg aactaaatta ttgtacgagt tctctagaac 1800
   ctataattca aaatgattgg cgagtattcc atcctgacac ctgctaaatc atgtcacctt 1860
   ttgcagttgt atgttctctg tgttgtgtta gtactgcaag ttttggtctc attctgttta 1920
   tttgatcata tatactactg caagtttatg tcaccttttg cagttgtatg ttctttgtgt 1980
   tgtgttagta ctgcaagttt tggtctcatt ctgtttattt gatcatatat actactgcaa 2040
   gttttggtct tatctgttcg atttatttga tcatatgtct taagctcttg cagtgccctt 2100
   ttatgcccaa tttctggctg tggtgctcat ctagattgcc tggagaactt tgaggaccac 2160
   tatcgcaccc gtcatactgc ttcatgctct gtatgttgga gagtgtatcc aacttcaagg 2220
   ctgctgagta ttcatatttc tgaggcacat gattcctttt ttcaagcaaa agttgcccgt 2280
   ggttttccaa tggtaataaa ttcataatct atccatgccc ccagttcttt aattggttta 2340
   attttgtggc atcatgcaca ctagccaatt ttatgcaaac ccagaacttc cagtggactg 2400
   tgcctgctgg ccagtttaaa attactactc aatgtcttag cattagttag cattacatgg 2460
   ttttctggtc ccgctaatga agctttccaa tttccacatg tcacctttac tggcatctga 2520
   ttcttgtata aatttacata gtagtaagtg atacttttat attccctcaa ctttctatag 2580
   caattcacat tgtgtatgct atttttgaaa cagtatgagt gtttggtgga gggttgtggg 2640
   gtgaagttga agagctacaa aagtcggcag cagcatcttc ttgataagca ccagtttccc 2700
   aagtcatttg aattcttcaa aaaagcacgc ccttcgcaac gccagcggaa caagaaccag 2760
   aagcaacggc aaacagttca caagggagac gagacaagcg aaacactaat ggatgttgat 2820
   gggaagaaga gctcaaggta catgaattcc agatatcggc caaagcaaca tgatggaaaa 2880
   gagtcaaaag aaaatgagca tagtagctgt aaggaggcca agaacaacga aatggaggtt 2940
   gacaagcagg ttgatgagct tgcttcggcc gtatcaagac tgagcacagc ggattcaact 3000
   ccttctagca taagctttgg tcatcgtcgc tctcgcggtc ttgcttttgt ccctaggtcg 3060
   attcggcaaa acaagcaggt ttctcagaca gaaccaaaat gacagcattt gataccatct 3120
   ttctcttcat tgctgatctc ggatgcatca acaatcctga atgtgctgtt tcctgtacct 3180
   ggacattcac cgaatccact aatatacatt cttgtagttg tatcacgaga tatgtcttct 3240
   agctggttat ccttgcttat ggatgtactg agcttcctgt tactgccatg tagtagaaca 3300
   atttttctga agcggcaaat atgaagtgca aaatacaaga cagttcttgt ggttgatcga 3360
   ttt 3363
<210> 139
   <211> 6316
   <212> DNA
   <213> Oryza sativa
<400> 139
   gaaagccatc atctactagc agcagcgaaa gccaagaacg ccaaaaaccc tcgctttgct      60
   gctgctcctc ccccttctcc ttcggctgct gatcggattc gcggcgtccc aggctaggga     120
   tccgggcggt ggcggcgatg ggcggcggcg gcggcgcgga ggaggagctg acggcgcagg     180
   agacggcgct ctacgaccgc cagatccgcg tctggggcgt tgacgcccag aagaggtatc     240
   cccggctccg cctctctctc tctctctctc tctctctctc tctctctctc tctctctctt     300
   cccctaaaag tcccgggttt tctttcgctc ttcgcttatg tggaggaaat tttgttgagt     360
   tgatgcttct tctttttctt ctcttttaat cgcaggttgt ttcttgcaga aaaggaagaa     420
   aagaaaatca ttcttttggg ggatttcatg gtttatgtta gaacgaatgg ctgttgtttg     480
   tttctttaaa agaaaaactt gagcccgaag tgtttgtatt tttgaagtga agacagagat     540
   atccgctgtt ttcgtatctt gtgtctgcta ttgaattttc ctaaatcttt ttttttgagc     600
   ttgtttggtg ctttccttga atttctcgta actttcaccc ccaaattatt cttccattcc     660
   atcgtatttg ttagcttcgg aaccaaaact tttgatgtct ttcatctgtt tttgactact     720
   gaatctgtgc tgcttggaac aggctaagta aagctcatgt gctcgtgtgc ggcatgaatg     780
   gtactactac tgaggtaggt attttaagat tttcctctcc aattgttgcg ttttgtttgt     840
   agtttacacc gcgtgggcat tcaaagcatt gaaacgaagt tgcattgagt tatggagtaa 900
   taggggttag tttcatgggc caaattgccc atgtttacct gttgctttta gttcgttatt 960
   ataactcatt agttttctgg gcatttgatt attgctctgt tgttcttgta gactttctag 1020
   tgctatggat gcattgcctg cgagataaaa tctcaatgtt cttatttact ttttaggctg 1080
   gctttttctc ttttgtagga cccctgttgt accatctgtt aaagttaatt gcttttagtt 1140
   ctcaacgttt aggctcaatt ttctgtagat aaattctttc atttatttat gctagcttac 1200
   gttgttccaa tatatgtcta tttatccttt tcttttacag tcatatccca tgattatctg 1260
   atcaatgcac ttcgctttgt taagaatatg attgctatta tactatttac tacttccttc 1320
   gtttcatatt ataagacttt ctggcattgc ctacattcat atatatgtta atgaatctag 1380
   acatatatgt gtgcctagat tcattaacat ctatatgaat atgggcaatg ctagaaagtc 1440
   ttataacctg aaacggaggt agtagttaaa gtatatgcct tagaaactag atttctgtag 1500
   tcatagagta tcataagcag ctgcatcctg actcaattgt atattcatct aggtacttaa 1560
   gtacctcagt tcttctggat atattgataa agcttaactc ataatcgaag gcagccttat 1620
   acactaaatg ccatttctct cttaccctgt agttctgcaa gaatattgtt ctagcaggag 1680
   ttggcagttt atccttgatg gatgatcatt tagtcacaga ggatgatctc aatgcaaatt 1740
   tcttaattcc tcatgatgag agcatatatg gtggtagatc acgagctgag gtttgctgtg 1800
   agtccctgaa agatttcaat ccaatggtcc gagttgcagt cgaaaagggt gagcctagtc 1860
   tggttttctt cattggtttg ggtgtcagaa ttagaagtac ataaagtagg atgccttctt 1920
   gcattcttga attgctctcg tcctaatggt taatctttga ataaaatgtt aaagaagaga 1980
   ttctatttca atttgtaatc ttacactttt agctgcactt taacaaggaa agttcttgta 2040
   agaaatcatt taattttagt aacaggcaca tgttactata gtgtatcatt gtaccaagaa 2100
   atcagcatat atgttactga gagaattctg atatatctgt ggccataaca atgactaatt 2160
   ctttggatct tcacagcata ttctaaaaac aaatttctta ctgtttctgc tatttccttt 2220
   aggtgatcca tcattaattg atggagaatt ccttgacaag tttgacataa ttgtagttag 2280
   ctgtgcgcct attaaaacaa aggtgtgtat tcttctcccg tttagttctt catcctattg 2340
   caagttccaa gtcactcagt gtcttttaaa ctaaaattct gcaacaaatt tctctttatt 2400
   gcgctcagtt ctcacaacat ttctgcatgt gttgtactgc tttagttgtt aattaacgac 2460
   aactgccgga agagaagcaa gcatattgca ttctacgcca ttgagtgcaa ggattcctgt 2520
   ggtgaaatat ttgttgattt gcagaaccat agctatgttc aggtatgggc atatatgaca 2580
   tgctatatgt tctgttgatt tttcagttta atttttttcc agcagtccac atatatatct 2640
   gtaagcacta agcatcgcgc agatgtctta aattggttta ttttctccat aatgtttctc 2700
   atatactgct tccaatattc cttcctgtgt acatgcatgt ggtttgtgta cttgttagta 2760
   cttttcttca tgtactctag aggcactcat tttttttcta tgatatgttt gaactcaaaa 2820
   ttgatgtcat tatgtcagac tgttcatgtt aacatattaa atctgatgta cttagcctga 2880
   tgaaggtgcc attaaggatg agaattgcaa aggagcacgt actttttttt ggtaataaaa 2940
   tgtagtgtac taatctgctc tcactgactg gttttcaacc aagttattgt atctgttcct 3000
   tttctgcatc attgaaatgg tgatacaaag aaaggttact tagttttggt gcttaaaata 3060
   tggtaatgtg acactttcta gcatattaag ttttgtaatt tgtaccaagg attcatcttg 3120
   caatgctagt tttggttcaa tcgctatgaa tatataagca cttatgcttt ggaaatcatg 3180
   atcactagaa tgtttcagga attattttct ttgttcagta gtttgggccc tggatacttt 3240
   tttgaagggg tatgtgtttt tttttttctc agaaggttgg aggtgaaccc aaaccaaagg 3300
   agttggcata tccaagtctc caggtaaatg acttgttaag ctgatctttt ttttatatgt 3360
   ttgagaggaa gctgatccaa cttgagataa catctttcat ttaatattac atgttttgta 3420
   ttgcccctgt agtttgttct ctcttggctg ccataaatgt agctgtggaa tatttatgat 3480
   gcaccttata caagctacat ggtatatata catgaaaacc atttaacctg aaaaaataaa 3540
   caatttgaat agtgtcttca tgttttaata atagtaaact tatgttttcc tctgaaaaaa 3600
   atgattttct gtaagacaaa aaaataaata gacaaaatgc ttactctgct gtttttttgt 3660
   tttgactatc agaatgccag agtgcttatt tattcgaata aatgtgtatc atatattata 3720
   tttttacaat actaagaaca gtcatcttca atctaaaaca aagtaaaacc atatcagccg 3780
   gcaatctaca tgtatacatg tgccaatttg cagttaatca aatcctaaaa aggacatact 3840
   tatttacata gatattgacg gagatcagtg tagcatacca acaccatctc cttactcctt 3900
   ttaaggagta ggtaaactaa gtatgattga caatctggat aagacttgaa tacgtagata 3960
   catctacaga agattgatgt agagcacagc aacactatta ccaaatcctc ttttcacggc 4020
   atagaaaaat acacttattt atgctcatgt agcccagcct tactattgga ggtcttcatt 4080
   tcatgtggta attaaattgg ttccacagaa atcccctttc tgggttatag gtgtttttgg 4140
   aataattttg taagagttca aatctacact agacaatgtg ctgatgcttt ctggtttgtt 4200
   cattttgcat acatccctag aagaatggag tagcgtagca tgttgacctt gactttttag 4260
   ctttctttat gtgtatcatc acttaaatat gtgttcaatt tgttgccata tgccaccata 4320
   tatcaccatt ttggatgctt cagtttggaa tgttacagtt tgtttatcat tggaaatgat 4380
   cttttataca ggaagctatc tccgtaccct ggaagaattt accaagaaaa acaactaaac 4440
   tgtactttgc catgagaggt gaggatgtca gacttttgca gcactgttga agattcatta 4500
   gtgcagtttt atctccccaa atactcaaac aaatcagcca gctttctaac ttgtttttcc 4560
   ctgcccctgc gcatatgtcc tcttgaaatg tccttttgca gtactggaga attatgagtc 4620
   atctgaaggc cgcaatgctt gtgaggcatc actttctgat cgacctgcag ttttggctct 4680
   gaggaaggac atgtgtgata aaatggtata taagtttttg tgttccttaa tttcaatcat 4740
   gttctttaag atttttttca tggtgtggta aataaactgc agtctttaag tgagtctcaa 4800
   attcctactg ctctcctgga acggctttta gcagctggaa agaagcaaca tcctcctgta 4860
   tgtgcaatcc ttggcggcat tcttggtcag gtaggtacca atgttccatt acttgaatgt 4920
   gaaaattgag tatatgtggt tgaactagat ggcaattaag gatttaccct ctagtagatg 4980
   gtaattgaag taaactacta acttcacaat aaccatttcc cccaaaagaa aaaaataaac 5040
   tcctcaacaa atactatgca tccacatttt actcctgcag taattcactt aaaccacatt 5100
   acttgggtcc atgaacttag gaactacata ttaaggtcct tagtgggtct tttcggacat 5160
   attgaaagac aacttaattt ataatcatga atgatggccc ctggctgagg gtgccttgct 5220
   tgtcctggtt gtgggtctca cagcctccag gcccaattcc catgacagac actttgccca 5280
   gacatgtgct gaagtttgac tatccttttg ctacgtaaga tcgatgtaaa gctagcagaa 5340
   gtgctgtgtc ctctaattat cagttatgat tccctagctc agccatacaa ttttgggagc 5400
   atgaatgtgt agttctcagt ccaggggccc aagatgcata cttggatatt gtgttgccac 5460
   aattagatga tagttacaca tggaattgga tcggtactct cactggacac cttcaaagat 5520
   gtaaatattt tatctacagc cttttggtgg aatacggatt ttgtattgat ggttcaagga 5580
   attatacaca atgaaagaac tagcacaaga aagatttgaa cattctgtag caatgtaaaa 5640
   ttttctgaat tatttagcaa cctcaactca gtgatgtctg aaaggagtcc aaaagtagta 5700
   atattccttt ttttgccaag aaaagtattc tgtactttgt caaatgttaa tcttcaaaag 5760
   cagcaggaac ttaattttgt tctgttccaa acaggaattt catctgtgtt tatgcacagt 5820
   tctcattgtt ttgcactctg tttttgccta acaggaggtg attaagtcaa tatctggtaa 5880
   gggtgatccg atcaagaatt tcttctatta cgacgccgct gatggtaaag ggatcgctga 5940
   agacattcct cccctttctt cagactgaac cagttaactg ctcgactccc attcagcctg 6000
   gcttcactaa tccctgtacc cattaattag cttcaaatta gattagcagt caatttaagt 6060
   ctgagagata cttagaactc tactatttgt tatttaagtt gtgccagctt agaaatggta 6120
   tcagatagaa attttacatt ttgtgccaac aatttcaaag ctgaaaccag gaaaggtttt 6180
   gtgctgattg aaagattaaa tgtgttgctc tgtattctct tccaatgatg tgcctacaac 6240
   tatgccatgc ccgtgtacta atctacccct ttgttctaaa atataatcat ttatgtgcgt 6300
   tttccaatat ccagat 6316
<210> 140
   <211> 1614
   <212> DNA
   <213> Oryza sativa
<400> 140
   cttcaaactt ccaacttttc catcacatca aaactttcct acatacataa acttctaaca 60
   ttttcgtcac atcgttccaa tttcaatcaa actttcaatt ttaacgtgaa ctaaacacac 120
   cagttgtgtt tgtttgtaga ccctgttaca gctgctgcat tcttggttca aactattgga 180
   agggaaaaat gtcaagctga tgcaaggttt gctatatttc gttgtgatga attagagcag 240
   cgaaaagacc ttactaacct cctgcttgtc ttgtccactc cagtccacac agccactcga 300
   tcgttatccc ttgtccgtct tgccttgcct tgtcgtaagc taggcaatgg ctatgccgct 360
   gccgccgccg ccgccgcctc gtcctcctct tggacgggga cggctcgtcg gagtaggacc 420
   agctccagca ccagcaacgg cctcccaatc caaccgccca gtgccccccc tgcagctgcc 480
   tcgctgccgc tgccatcgct cggagggacc ctggaggacg acggcggcgg cgaacgggag 540
   gaggcggtgg tggtccgacg aggacatgga ggaagaggac gacgaggagg gatacggata 600
   cgacgacggc ggcgcgccag gcgggtcagc gcaggagctg ttcggcgagc catggttttc 660
   caagctcttc cgtgcgtacg gctacgtgct gccgctgctg ctggcgtcca tgctggtggc 720
   cacggggccc agagctttcc tcatggccat ggcgctgccg ctcgcccagt ccgccatctc 780
   ctgggtcgtc tccttcttca ccaccaggag tcgtcggcag caggaggagg aggagtcgta 840
   cggatacgac tacgatgacg atcccgcctt ccaacgccga gaggaagacg acgacgacgg 900
   cgactactat gatgccgggg catggcaatg gcggagcagg agccaccagc aatcgaccga 960
   atccggctcc ggttttggag gatgggatga cctcctctac gacgatgagg agaagaagga 1020
   gcaggagagc tcagggaaga agaggacgcc accggagccc gacacggcgg cggctgctgc 1080
   cgcctccgat ctgggactgg gattgcgggc gaggagaggt ccacgacgca gcaatggcgg 1140
   catgtcgcga ggaagaagca gcagcagcat gaggtataac caggcgccac tgctgacgcg 1200
   ccttctcgtg gcactcttcc ccttcctcgg ctcatggttc aggatactct aaatttgaga 1260
   agaagaagaa aaactgagag atttcagcat tcagaatgga ttgattcatc gtcagttcgt 1320
   cagactctta tcacaatttc cttctcccgg tcaggtgcac tttgcctctt ttttgtcttg 1380
   gtcatgttca cctgacaatc acaactcaca acttcatgca aatcaaaaca aaaaaaaaat 1440
   cacttggttt cttcaggaac caaaccatga aaattgagat gaaatttctg gccttgttgt 1500
   ctactgatag caagaagcat cagacgctga tgtggacagg cagaagaact gaccttcttc 1560
   tccttctcct tctattcctt cgccgtgctg gcgctgttgt tctgcctact cttt 1614
<210> 141
   <211> 6053
   <212> DNA
   <213> Oryza sativa
<400> 141
   atcgccattg ctgccctcct ctccgtcttc ctcctccctc ctctgtcgcc tgttcttctt      60
   cttctcacga tttttttccc ctgtaaattt cccggcggct tctcgcatct cgttcatcgt     120
   cgtcgtctgc tcgcgacttg acggacgagg aggaggaaga agggagcgat tggtttggac     180
   ggtaagaaag ggagggacgc gcgggcggcc ggagccggtg gccgacggcc attttttcat     240
   gcgtggctcc ctggaggtcc acgcgattgg cagacacgcc gcgtcgccgt gcgccctgag 300
   actgaaagcc ctcccggcat tggacatgat gaggtagacg gcagatctcc aggccctcga 360
   gcctctcgtc cttgttcggt tgttcctgcg gttcctcgtt tcttgctctc ttctgtgtca 420
   gatcgctatg ttgtttctat ggttcgcgtt cggttttgtt ttcctctttc ggttttttgt 480
   tcggctggtg aagcgatttg gttgatttgc ttgcctgccg gccggtgatg gccggcctcc 540
   ttgtcgaatt gctgtccttt gtgattctac ttgttaatct gtctgatggt tttgttctgt 600
   tcgtttgatt tttggatttc atggtgttgc tgctgttgcg gttcgattga tctctacttg 660
   actgtttcta tgccggtttc gctaattcgc gtggaattcg tgcgtctcgt tttgacccga 720
   tttcattctc tccgtatttc gcagtcttct gctcataatc tcagtataat cgtcttttgc 780
   ttttcttggt tcctctctgg ctgttttaga gtctgggtac aaaagcatcg gatcgtttgg 840
   tcgttttgca accatgggta ctctttctgg gatgatgaga gccatagttc atgtcagttt 900
   gttcatgttc tttgtggccg tgttcccttt caagaactgg ttggtttgtt gtcaaatttt 960
   cggtcatttt ggtgcctcct gttcaaccct gcttctgttg catctgcaga gttcgatctg 1020
   ttcgttttat ttccattcca tgtgatggtc tgagaaggcc cgatttgcga ctcgcatttt 1080
   gtagcagatc tctttcctcc gatcaaatcg ctaatcggcg cgttttgatt cactgcaggt 1140
   accaaaggct tagcccggac tgcctcccgc tagccaacgg cggcggcgga ggaagcggta 1200
   gcgtgacacg gaagccggcg tcgagatcct gcaaggacga cgatggcggc atggccgtcg 1260
   ccgcggacag ctcccgcctc tcgtcgtacc tcccgtcgtc acagctcgat tccaagccgc 1320
   tgcgcgctcg ggcgccgcag ccgtcgtcct cgtcggccgc cgcctggagc ccggcgcgcg 1380
   accacgcgca cgcccaccac aaccaccacc accaccacca cccgtccgac tcctccgaca 1440
   cggcctcgcc gagctccaac ggcgcgggca ccggtggcga cgtgctgctg cagtgggggc 1500
   acaacaagcg gtcccgctgc cggcgcgacg cgtcctcctc ggccaacgcg gctccctcct 1560
   cctcgcagcg ccgccagacc gcctccgccg ccggcaagat cctgcgccgc tcgtcggcgc 1620
   cggcggagaa gctcatgccg ccgccgcccc catccaccac caccgggtcg tacacgcgcg 1680
   ggtccaacct gaggtccgct tcgtccttcc cgacgcggtc cgccgccgcc gccgccgtcg 1740
   gagacgcaca ccaccacagg taacacacgc ctcctacctc ctcctctccg tctcgtctca 1800
   tatcgatgtc gtggggaaga tgacgtcctt cctttttcct cgcctgcatt tcgctcggcg 1860
   actttttttc tgtttccgct tttgcccctc cgcggaaact accgggccgc gcgctctcat 1920
   attcctcgtt ggagtcgtgg tggctcttgc gtttgttttg ttttgttggg ttcgtgagtt 1980
   cgtgtcttcg ccgcgttatt tattcgcttc gggttttttt ttgggtcttc gggtttgggt 2040
   ctcttttgtt tctgagctgt accgctccgt agagagaagg gtgggttgag tttgataaaa 2100
   gattcttttc tcctttttac ttcacacact tttgtttcta ttaaaaaatt ggagcaggtt 2160
   ctcacctgat ctagatctct tcgtctctcc ccgaattttc gtggtattta taaccctttt 2220
   aatttttatt tacaatattt ttcggaggat gttgtgtgtc cggatctggt gtgtggtaca 2280
   ttgaggtggt aactttacct tcctcttcct tgactaatct gtaacccaag tcgtaaggtc 2340
   ataggcagca gcacccgtat ttgtcacacg ttgcatgatg catggggacc cccatcgaga 2400
   tctccatatc tcgctgaaat tcgttgatgg tcatggtggt actccttggt tagggcagct 2460
   ttaatttggg aggatcgttt ggtcgatctg gttaatttgg ggtgttggga tgatgatgat 2520
   catcatcata tgaatttgca gcatcaagag gagggagagc ttgtagtact gcttgtaggg 2580
   tagatggtta taaatatatc cacatcacag ctcccctctc ttttctttct gggcatcaac 2640
   aatagtagta tagcttttgc tcatagtgca tccatcttgt tgtggtagga gtctccagca 2700
   gaccagttgt actactagtg agtaaccaat ccaccctacc caccccttct tcccctttac 2760
   cctttctctc aacctcccaa ccacctccaa gtccatcatc accaccacta gcacctgcaa 2820
   caatccccat gattaacgtc ttgttttctt tctctggtgg tatgatcagg tccgccgtgg 2880
   aggagcgatc aggcggcggg tacaagcggt cgccggacaa ggcgcacaag tccgccctgg 2940
   acgcggcgct gcacatggat tccaagaaca accaccatca ccaccaccac gactcgtcgg 3000
   tgaccgcaaa cggcggcgcc ggcgccggcg agaagatcgg ctccgagcgg tttgagctgc 3060
   cccggatcta catctcgctg tcgcgcaagg agaaggagga cgacttcttg atcatgaagg 3120
   gcaccaagct gcctcagagg cccaagaaga gggccaagaa cgtggacaag accctccaag 3180
   tatgccaatc tttgctccgc aaaccggcct gatcttatct gctccgatcc attcttgcat 3240
   ctgttctgat cacagagcta acatgtgtct cgtgttgccc ccgatgtgat gtgcagtatg 3300
   tattccctgg gatgtggctt tcagacttga cgagaggacg gtatgaggtg cgagagaaga 3360
   aatgtgtgaa gaaggtatac tccactctgc acctggcatt ttcagttcat gcattctgtg 3420
   tatttttaca gacaagaaga ggaaaattac ctagatttag ggcatcagtg acccagattg 3480
   gttgggtcta atgcaaaaat aaagccgaag agtaggtgaa ttacttgccc ttttctttta 3540
   gtttggcccc ttctccctca catggggccc atgactcgtg atgctccgtg tcctcattga 3600
   tgacggcacg gcactgattt atttggctca gctttattgc tgtggacgtg gagatgcaag 3660
   cgaacgatca ttctattgcc cttacctaaa ggagtcttct tgttctacgt ttagttggat 3720
   ccattcgttc atctttctac atttgtcatg tgctttccca ggcaaatctc attcaagaac 3780
   actacagtca caactcacaa caaaccagag tgtgccacat gtcatagcag aaagaacaaa 3840
   aaaaagtctt ggcctctttc agtatggggc ccagctgtca gcctcttatc gtccttagct 3900
   aagcatgaga aagaactgta gcattattac tacttaggtg gtgttagttc agtgcttagc 3960
   tgcgggtgcg agattgttga caacttgatt attccggacc ttgaaggctt gaatgcaagt 4020
   cgtcgtcgct gacaagtagg gaagggccaa cgtggagaca tgtatctgtg tcttttgtta 4080
   ggaccacatg ataatgtctg gatagagctt ggaatcataa ccatcgaaaa agagaagaaa 4140
   aagaaatggt acaaagggtt ggaaaagaaa ttgtggtgct gtcttttgat ctactagtct 4200
   gtttttcacc ttttccgtga tagggtcttc aactcattcc ctttgttatt aggagcattc 4260
   aagcatgctg tagtttaatg agctcctgta acatactaac atgcatatat tttccaatag 4320
   taacaaggcc agctcgaatc ttgtaacatg aactctacta gtattgttta gctgactggc 4380
   agctattaaa aacccctgat ggttaggttg tgcttgaggg gtgctagatt ctccacaaga 4440
   attaggctca gcacatgcac ctaatcacct agtagtatct ccatccatca aggtttctgc 4500
   ttaatctcgg gcactaaccg aaaactcctg tttctgacta tgcagaggcg tagagggctg 4560
   aaagggatgg agagcatgga cagtgactcg gagtgacggc agctggaggt caagcgaggc 4620
   acccaaagcg aaaggagcaa taagattgga gtggatggag attgggccat tgcaatcaaa 4680
   gtggatgggg ggagaagaag taagggcaag ccaaagggaa ggcgaaggcg aagagtgttt 4740
   tttgtgtaaa tggagaggaa aaagaagagg aggatgtgcc gagggcagga ggtgcacgcg 4800
   taacgcgttt gcctcacaca ccctcctcga ggcgcccggc ccagcggaag tggtggcttg 4860
   agacgacgac gacgactatg ccccggggtg aatttttttc gtttctttcg ggttttgtca 4920
   gagccggcca cctgctcgca cgtcgtcgca gcggcgcaca acttggtgcc gcgtttttct 4980
   ccccgcgaga ggccattttg tggtgtaatt ttttgggtgg gctccgttgg cccggccggc 5040
   ctcgccttcc tgtgagcttt tgcggtttgg tttggttgga atggttaccc ggtgactctt 5100
   gtgcatagtt ctttactatt agtactagta ccattttttt tcttcttctt tctcccattc 5160
   attcacgttg ggcaagtctt cggaacacca aaagtattct gaaaaggtta aaaaaaaaag 5220
   aaagaatcat tgtgcagtcg caatgaggca ggagattctg aatggattca gtatgggcca 5280
   gggccagggc tgatggaggc tgcttcgatg ggcctggcgc gtcgcggtca cctgcaaagt 5340
   gcggtagtcc cttgtgacga gctgacaaac gttcggcatg ccggacggac gggggctgag 5400
   atatctaacg aggatcttct acgtcatata tgatggaaga atctgttaca gaagtttggc 5460
   atggctcgtc tatcagccgt gcgattacac cgaatattgg acacgtgtcg gcatctcgcg 5520
   tgatcaactt gaccactatt tcctttggct tcttccttcg tccatcagtt gtgtgtacag 5580
   tactgttgtg gaagaggcga tgattaaact ctccaatcat gtattcatgt gctgatgcgt 5640
   atatgtatgt gcacgtcaca tgtgggcgag tatgggaggg ccgtgagggc gaggaaagcg 5700
   tggttgaaaa acgccagcga ccacgatttt ccatacgcaa cgccggccat tgctggtgaa 5760
   gtagctcttt tttccccttt tctttggaaa accctgtact actctttacc cagtttgcaa 5820
   tattttaggg tggaggagta ctccgtattt agtttagggg cggagaattt gaggctttgt 5880
   tgttcggaca tggtacgagt ggcgaactgg cggcgatggc agccataaac aggaacacaa 5940
   ttgtctgtct gtctgtatgt ttactacaga ctgtttacgg tctcatgtcc agctgtttga 6000
   attttgtacc tgaatgtttt tttctttgga caaaaagatg gagtatttga ata 6053
<210> 142
   <211> 3348
   <212> DNA
   <213> Oryza sativa
<400> 142
   acacctgctt tccaatcgca gctgcctccc atggcgacca ccgcctccct cctccctcct      60
   ctcctcccgg ccccttcctc ctccccccgc cacctccacc cctcccctcg ccacctccgc     120
   cccttgccac cgatccgcct cctccgcgcc gcccgccgcc gccaccccga cgccgtcgtc     180
   gtcgtcccgg acgcccgccc ctgggtcggc gacctctcgg gcgccgccgc gtcctaccgg     240
   gacggcaggg aagaggacga cgacgacgcg ggggaggagg atgacgaaaa cgacgacgac     300
   gacgaggacc gcagcctgga cctcctggtc cgcttcctgc actcggtgtt caggaaggtc     360
   tcccgccgcg cgcgccgcgc cgccaggtcc gtgctgccgc cttccgtccc cgctgagctg     420
   gtccgttcaa cccttccgtt tctccccttt ggtttcaatc ttcttcttgt tcaaattgag     480
   cggatccttc tctgtattgt gtgatctggc cttgatatgt ttagtttatg cttgtgcagg     540
   tgaagttctc ggtcaacggc gtgcttgttc tcacgtttct atgggtccta aaggggctac      600
   ttgaggtgag caattcattt gtctgaggct tgtttcaaaa ttgtgagctg attgtgatca     660
   tgcccttgat atgaactact aagtgttaca ctgtgatact atccagtgga ggattcagga     720
   attttcttga gcctggtcaa acctagtgat gtataacaag tacataaaaa atcagtatag     780
   ctccatactt ccatagacca tatactatga acgcaagcaa aaccgacagc tgccaagttg     840
   tgatttagga agagcaaaac cagtacgtac gagagattat atcgttgaga tgatggctgt     900
   ctattgccaa tgggctagac tatggggcta ttatcatcct gacaaatgtg gtgatgagtg     960
   ctagcttaga gttgtgtcgg tttgaccctc gtgcagcaaa cattcaaacc ggcatatcaa    1020
   gtagtttgct tggttggttt ggctcccatt cgaaccagca gatcaagtag taattgatgg    1080
   agtgctgtct tgctcatgtg ctgatcgcag agcctggaga ccagccacag ttgctggggc    1140
   ttggctcctc cgctgatact aacagtgaca tataatggaa taacattata tgtccagaga    1200
   acaaacattg tgcatgcttc tgaatttcat gtatgtccag agaacaaatg ggtgttctac    1260
   cttcgttgtc attcgcttag agagtgaaga agacaatcag tgtctgatta catgctgata    1320
   tgacactttt aaatgtttga tttgagtttt ttcccttcat tctgatgtct cgtaaatttg    1380
   gttttcaaat tttggtcaac caagtttgat gaacatggat gagaaccaga caagtgtata    1440
   tgataccaca aaaaagattg tccttttctc taactgtcac gtgtagatga gaatcctcac    1500
   tttgttgaag tgatgccctc ttttcccttg tactagatgc ttgctcccag ttttgagcat    1560
   aaaacaaaaa catgttctag tacagagtat ataataatgt gcatatatac tcagagtagg    1620
   gaatggtgca catcagcaca tggttcaatc attccttatt gattgtctac tctttgattg    1680
   gagtcatgct tatgatcaat aaacagttat gctttcaatt cagaaaatta taaaaaaaaa      1740
   ttgtgatgaa ataacaaata ctccacatgc attacaagta caagtttgtt tctgggagac 1800
   atacaagtag tttagaatca tgagattact taatgcttat aatggcgttt tcgatggatg 1860
   aattcagacc aaatgagaat gaatcttgta ttcttttgca ctgacacttt cagcaaattt 1920
   catttcttag cattctggta gtttggtcat gtgatttacc tctggtgctt ttggaatacg 1980
   agttttttat tcgttgtaac atttcctatt gataatagat ggaaatggtt cgtcttatta 2040
   agatgattct ctttagtgaa attatgaatt ctgtttagta gagaaagagc ctactgctac 2100
   tgctgtcaag tttgatccca tacccacccc aacccccagc gtctggcggg cttccccttc 2160
   tggatgagct cggtccaccg ctatcctcca caaggtcgtg ccattgtcct ccgtcgcttc 2220
   ccctccctcc actcctgtcg cttgccttcc ccttccttcc gtcgatctga aggcagtgag 2280
   cagagaggcc aaggtggtgg ctgcaaggag tagatggcga tgtcgcctgg atccaagaga 2340
   gagagaaggg gtaagaagga atatatgctg gcaaatgggg acagtcggat tttgtaaaat 2400
   tatttcataa tcttgctgag tggattgcca tgtgaccaaa ttaacaccat gtagtatcaa 2460
   aaccactccg ttttttgcca ggggggtaat ttgtccagat tcaatagctt ggaggtgtca 2520
   aatgtccggt attgtagtgt agttcgcgca gggcgggtgg gtgtggggtg taaatcgtac 2580
   ggtcctaacc cttataattg catacaagct taaactacac cctggttctc aagcagggct 2640
   actgagtact gactgttact ctcttcaact gacaccccta cacactgtct tctagtgcca 2700
   tgcctttcct tcagtaactg tttatattgt ttgtgctcag gtggtgtgca catttggaag 2760
   tatggtgttc gtgaccatcc ttcttgttcg tggaatatgg tctggagtga cttacataag 2820
   agaaaaccga tatagctata ttcgccagat tgataatgat gacaaccgat ggagcagagt 2880
   acagactgct ggctaatcat ttgatttcct tgactacata catacacttt gcaccaaagg 2940
   gatcatcagt aaacctttcc tcactgttaa gacagcatgc taccagttca gcaccaactg 3000
   ccaacagctg ttgctataca gcagagttga acaagaacaa aaggaatccc atgtattcgt 3060
   cgaataagtg gaattttctt cctctgcaca aatgcaggag ggatggttga gctgaacaag 3120
   attaatacgc gtgtaaaaca atccagcaca ctgatgaatg agagctgctg ctgtgatgca 3180
   ttttctgcta ctcttttctt gaagagatgt atcggtcctt attagtgtat gtattgttcc 3240
   atgctgttac aactagccgg tgtagaaaaa ctcatgtttg ttgtattgaa aggtggtgca 3300
   cgataccttt ttggttaaaa agtgaatatc atttcgattt catttgaa 3348
<210> 143
   <211> 8998
   <212> DNA
   <213> Oryza sativa
<400> 143
   ggtagacacc gcttcagcct ctgcccatcc aactcgcaaa aattccccac gattccacga 60
   aagtaggaac catgaagctt cggttgcgat ccatggacca gcgcggcggc gccggcggcg 120
   ccgccgagac ccaccgcgtg cagctgccgg acacggccac gctctccgac gtcaaggcct 180
   tcctcgccac caagctgtcc gcggcgcagc ccgtgcccgc cgagtcggtg cgcctcaccc 240
   tcaaccgctc cgaggagctc ctcacccccg acccctccgc taccctcccg gccctcgggc 300
   tcgcgtccgg tgatctcctc tacttcacgc tctcccccct cccgtcgccc tcgcctccgc 360
   cgcagccgca gccacaggcc caacccctgc cccgtaaccc taaccctgat gtcccctcga 420
   tcgcgggagc tgctgacccg accaaatctc ctgtggagtc tggtagctcc tcgtcgatgc 480
   cgcaagcttt gtgcacgaat cctggcttac ctgtcgcatc cgatccgcat catcctccac 540
   cggatgtggt gatggcggag gccttcgccg tgatcaagag caagtcgagt ctcgtcgtcg 600
   gggatacgaa gagagagatg gagaatgtcg gtggtgcgga tggaaccgtc atctgtcgcc 660
   ttgtcgtggc gctgcatgcg gccttgctcg atgccggctt cctctatgca aacccggtgg 720
   ggtcttgcct tcagctgcca cagaattggg cgtcaggttc ttttgtcccc gtatcgatga 780
   agtacaccct gccagagctt gtagaagcgt tacctgtggt tgaggagggg atggtggcag 840
   tgctgaacta ctccttgatg gggaatttta tgatggtgta tgggcatgtg cctggggcaa 900
   catcgggggt gcgaaggttg tgcttggagc tgccggagct tgcgcctttg ttgtacttgg 960
   atagtgatga ggtgagcaca gcagaggaga gggaaattca tgagctgtgg agggtcctga 1020
   aggatgagat gtgcttgcct ctgatgatat cgttgtgtca actgaacaat ttgagcttgc 1080
   caccgtgctt gatggcgctg ccaggtgatg tcaaggcaaa ggtcctggag tttgttcctg 1140
   gggtggatct tgcaagggtt caatgcacgt gcaaggaatt gagggatctt gctgcagatg 1200
   ataatctttg gaagaagaag tgtgagatgg agttcaatac tcaaggtgag agttctcagg 1260
   tgggcaggaa ctggaaggaa aggtttggag cagcctggaa ggtttctaac aataagggcc 1320
   agaagaggcc cagtcctttt tttaactatg gctggggtaa tccttatagt ccacatggct 1380
   ttccggtgat tggtggggat tcagacatgc tcccgtttat cgggcatccc aatctccttg 1440
   ggcgcagctt tggaaatcag cgcaggaaca tctcacccag ctgcagtttt ggtggacacc 1500
   atcgcaactt tcttggttaa gtcatttcgt gggttttgct agtatgttaa gaatatttca 1560
   tctgaaaagc tacatataac atattgtaca tattttatag ttggcacttt atgcatgttc 1620
   agttgttaac tgtattactg tactcgtaat cttttctttc tttgttgata tatcctatat 1680
   tttcttgtag taccagtgtt atgcatgcct taatcatggt aaagtatcgt ctgtttaatt 1740
   ctctgtgcta caatatgcat ttcaaacact tgtaacttgt aagtctcatt tgttggatgc 1800
   ctttagtcaa tctgattatt tcatccatca acggagaaac aagatactgg tcatgttata 1860
   taccatcatg atctgctgat gagattgaaa ctgtcacttg tttctaaagt ttgcgtgaaa 1920
   taactggaag caggtggtgt ctttctttgg taaaagaaaa gtattgtcct tatcatctct 1980
   ttgttctttt cgttttatat gctatgaaaa gatatattca tcccatattc cgataatttg 2040
   gaatacttgc ttgccttttg tgctatggca acttatgcat attattttgt tatttttatg 2100
   ttcgtggggg gttgtagcct cacaggttgt agcctccata ctgaatcgtg caaaactgct 2160
   atcctacaaa gaaggacaaa caaactggat aggctgtact cattaatcaa tgtctaagct 2220
   agtgcgatta acttgggcag catatggtcc gaaaacaaag aaggaaaagg tgaacatata 2280
   tcaggaacag atcaatagac ttatcacgag actataacca ctggtgccaa acgaattagc 2340
   aaacagataa taccttagaa tttttgtatt tggcaataaa atctagtaag aatttgttga 2400
   gctgcactac aaacatgtat agataagaaa tagcatccaa ggcgaggatg atatgttgtt 2460
   aagacatact atcgagcaaa tcctgtggca ggtttctctt acaccaggtt ttacctatgg 2520
   tttgtaagtt tctacctgat tttcattgta tatattattt tgtgattaca cgaatcaatt 2580
   gtttccttct atatattgct gaaaccgagc tgccctgttt aaatgcatta gttaatgtta 2640
   tacgttatct gtgtttgata aaaagcttct atgaaactat gaccactgtt tgcttttgtt 2700
   ttgatcaagc tttcagtgca aggacttttg gttgtgcaca cgtatgtgac atttagtgga 2760
   ttttttaaaa tcaaatacat tatcagtact tggggctgga gcaatctgtt ccctggggat 2820
   acttttagca ggaacatgac tgaaacatta tcagtttaaa acaatatgac tgattgtcat 2880
   ttccttatta ttgtaattgt atttagcagg aacatgattc tgaaacttgt gtcttgatga 2940
   tcagatacat gcggttgtat gatgtgtaaa tgcatttact ctgaccaaag gaaggatatc 3000
   gtactagctg ataagtatac ctgtggtaat tatatgcaga agcccgtcac acaacctggt 3060
   aggtgagtaa tatatataag cactctgggg aactatttat ttctttctag aaatattctg 3120
   aatagttgtt atgttacctg catgcctaag ttaatttctt attccctttg tgtccttttt 3180
   gtgtttgtct gttactttat tttgtacaat gtttcgcaga tcgtcaatat tctcgtggct 3240
   tgcatctcaa ttggatttct ccaactgatg cttcctccta acatatccat ttttggttgc 3300
   gcgtacttgt tttatgataa aggagaataa aggagtcatc cttttttttt tcacttcgac 3360
   ttacgaatat ggtttatttt cttggttgtc gatgcaccac tttatgaatc tgactgtagt 3420
   atttgctttt acttttattt ttccttcgca ataggtggct tattatatta gtctaccatt 3480
   ccctactttg ccagtacatc actattgggt tgagtttgct gtggtatcat ttggttgatt 3540
   tggttcaggt ataatttttt aagagatttt agtcttttgt cctaagtgaa tatgggttgc 3600
   aggatctata tgacaataaa gttcttgatt ttatacagaa gcttcacatt tacactgcag 3660
   tcactacttg aattatcaac atttctcact atacatatat aatcagctga acgcctgaac 3720
   cttttgagat atttgagtta tgactagagg caaaaatgga tagtttcttt gtaaaacgat 3780
   atataacaat caataatggt ttttcatgga cttctgaagc aactcgacat tgatgttccc 3840
   ataccatatt tttcttgagg ctatgatggt tgagtgaacc atatagctct tctctctcca 3900
   tagtccattg gagtcttaga cctggggggc caaagattgc tccattttct taaagtgggc 3960
   tttatattga ccgcagggag aaatatcact tttttggtgt aggcgtgcat ctatctactt 4020
   tgcctacaca tgttctattg actattggac tcatctgtct ttatgttgca taattaaacc 4080
   atgaaatatc tttcatgaga tttaactttt tgatcacttc tctttggact gagactgaac 4140
   caccgttacg atactcaaat gggagctgta cggagtgtca cggagtccaa gaaaagctac 4200
   aactttcagt aaggggagta ctctttgctt gtggcttggt gcactgaaaa gattgtgggg 4260
   gaaggagtat gggaagaaag agtttataaa tccaaatggg taagaatttg agtgttttac 4320
   tgccaggata tctcaatgct atgattggtg atctaaatta tggttaaacg ttactctgtg 4380
   gttccatgaa ctttggctgc tctatgaaaa gtatttagtt tcagttccgt gccaaataca 4440
   gcatttgagt ttcagttatg tggcaactac cgttcatacg cagccttata tattttcctc 4500
   attgttcctt ttaccaatag tcctgtaaac ccgaattctt ctgtttcaca ttcaactttg 4560
   tcctgcatac agtatgtttt acgttctcag cctgtgctat tattgaaagg ctattgcatt 4620
   gcagtggagg acctgaagcg atactgcatc ccagcgaccc agctcaattc acgcatccag 4680
   ttctgttagc ctcggaacaa tagtactcct acagatagct ggctgatact gcacaagcta 4740
   caggcagcct cagcggagta agtacaagaa tccaattcgc tgccaacaca cgtctgcctg 4800
   ccgctggcag gatgctcctg cagcaggcat tcactttgac tgtattattc cactgccaat 4860
   caatccttac cagcttcccc catctgctgg tgcctgctgc tcaacaactc aagcttcagc 4920
   atcagcaaaa gatggtggca atgtactcca gattccaaag cctcttgaag tgaaacagca 4980
   cagtgatgaa cttctatgat tgacacttgg gcaccctgct ttgagctttg ccttttgctc 5040
   tctcatctgc tactagtagc atgctggacc ttatccttat gcaacacaag taatatacta 5100
   acaggtattg cttgttggag aaggcctaac caggaccgat ttttaagcca aggtggatag 5160
   gataatcttg tggcaattga aatctgcaaa tgtgcaacta gtcttcttca tgaagggaag 5220
   ttgtacttct gctatgctta caccgaggtg taatcaaata aagacactgg gaagctggtg 5280
   gaagcagcag tggtggcctt ctagtatctt ttatttcacc cctcctgtcc tagccacatg 5340
   tctctgcatg cagccactac atggtgaaca ctattcgttc taccataggc tggtgagtaa 5400
   ctaacacctc tgatcaagag aggtggagca gagaaagtgg cagcagccct cacccccgac 5460
   tggtaataag aactctcccc ttccatccta aatatatatc ttgttcaata ttttctacat 5520
   caattttatg cattttggca gaatagtttc tttgtagaca gtgcattgtt ttttcccttg 5580
   atgaaactac agcacaagaa cattattagc tgtttgctca ttaagtgcca acagcctttt 5640
   tactgaacgg tttctgtgct ccatccaagt ccttttgcct ctcctcaatc tacacattaa 5700
   agaaagggga gaagtttcaa cgttgtacta acccttgtcc ttgcatctgg gatcaatcaa 5760
   tttctccctt ctgaatttcg agatagccct taaactgtca tggtagaagc tctgaattgg 5820
   tgagtagtac gaagtgtcga cagcctgtgt aaaatcgggc agtcattgtc gtgcttgaca 5880
   gatcatttac agtgccagca ccaaattcgg atgatggtat gtacgatact cactgttgag 5940
   agccgaagaa tccctctgct ttgctactga taacaatcag ctctcttttt aacttttatc 6000
   gatcatagaa cctaatcact tccctggttt ctctgatgat ttcatcgaag ctttgcacat 6060
   tcttagctgt tgctgtcttt gttgttctgt ggatctgatt ctacagaacg aacttctgac 6120
   atttccattc agatttcaga gcgacagttt gaactgtgta acaactaacc ttctgtcctt 6180
   gttacctcta gcctcacatc caccccagtg aatacgcaat ctgagtcttt gtgttggaga 6240
   tttcgtttaa ttacaaatta aaaagagagg actaaggttt agtctgtaac attaattacc 6300
   acacttgaaa cgacgcctta catctaggca ctgccactga aaggtgggtt cccttttctc 6360
   ctcttatgca agaattgttg aacatgttaa gaataagact ttgaaactaa aaacttgtaa 6420
   gttgggttta tcagaaaaaa atggtgaaga agggtattaa tccagtagta caaaatttaa 6480
   gagggtttaa ggctttaagc aaagatggat ctggttcatt aattaatcat taaccttatt 6540
   ctgggctggc ccatacagtg gatgacaata gcatctgttc tttggtttgg tcttcatttt 6600
   acagtaccac ctgcaattta tcttaattca gagaatttta ttctgattca tggatgtgat 6660
   ccagctggtg catggttgtt agcagtaccg acaattctat tccaggactg tggtttccac 6720
   ctttgccctt gcgtttgtct attgcattag gcttacttaa cttttcactt tggacaatct 6780
   ttatgtaagg ctgcaagggt tagttgttcc ttgttgagcc ttgcaagaaa ttgactgcca 6840
   cagctcccga tctaccctac cctttaagta aagcccattc acttgtcaaa gctgacaatt 6900
   tagaaggcca tcacgcattt cttaaaatga ttgcaatatc accctgagat caagtatcag 6960
   gcacaaggtt ggtggcttgt ttaatttctt catatgtatg ttcttgggag ttgggaacta 7020
   gcatctatct aatctagtac acactagatg acttatctca gagagttgtg atataatggt 7080
   catcatgtga ttgatcatcg tttcttctgc agatgtattc ccctccctgc agtgctgctg 7140
   caagcagcca agggcattgt ttcgcggtcg gagctaacca gcttgcttcg cttgaccttg 7200
   ccatggactt cgacgagcct atcctttttc ctgtgcataa tgcaagtttg caagagggga 7260
   ttcagtttta caatcctacc ggcggtatgt ctctctcgtt acctatgttc tattttcaag 7320
   gataaccaca gtatcctcct ctcttttttt ttttcaatta gataaccaca gtttcttaat 7380
   ttgtgaagtt cctaactatt acagtttccg tgttccaact ccccagatac tcagctaagt 7440
   agaaacatga gcattgacaa gtgtttgaag ggcagtaaaa ggaagggctc aggcgagggc 7500
   agttcatcgc tacattccca agtaacaagt taattagaag ctctctttgc ttagcttcat 7560
   cgggtgggag cacgtttcat cgtgaaaatc gtactactgc aggaggaaac cggtgaaatg 7620
   cctcagagag aactcagcat ggagcatgcc ggagagaagg cgggtgatgc tgacgctagc 7680
   agggaggagt acgtgcatgt ccgggcaaaa cgcggccagg cgaccaacag ccacagcctt 7740
   gcagaaagag taattgatct ctccaacatt aatggaagat ctttctgtgt atagattttc 7800
   ttgctcacac agcttcacca tctgaatgca gtttcgaagg gagaagataa acgaaaggat 7860
   gaagcttctg caggacctcg tcccaggatg caacaaggta gcaacgaaat caataactct 7920
   ttgagtctgt gatggtgtgg tgtgctctaa cctgtgtgaa catgttgctc ttgacaaagc 7980
   agattacagg gaaggccatg atgctcgacg agatcataaa ctacgtccag tctctgcagc 8040
   gacaggtgga ggtaagtgtc ccgaaattac acatcttgtc aacaagaatt tacacttctc 8100
   aatgccaatc actgactgaa ctatccatga agtgcttatc cgtgccgggt tttgcagttc 8160
   ctctcgatga agctctcgac aatcagtcct gagttgaact ctgacctcga cctgcaagat 8220
   gtaagatgaa aaaactccaa ctctgaagaa caaataactc atctatcacc attgctacac 8280
   cttgatcctt tctttttcac tgccatacag atcctttgtt cacaagatgc tcgctccgca 8340
   tttctgggat gcagcccgca attgagcaat gcccatccta acctttacag ggcggctcag 8400
   caatgcctct cacctcctgg cttgtacggg agtgtgtgtg tcccaaatcc cgcagatgtt 8460
   catttggcaa gggccggtca cttggcttcg tttcctcagg tctacatcta actccagtga 8520
   atacagtagt tcaaatcctt cagaacagcc gagagttatt catgttttct ttgctgcagc 8580
   agagaggcct catctggaac gaggaacttc gcaacattgc tccggccggt ttcgcttcag 8640
   acgccgctgg caccagtagc ttagagaact ctggtatttt tcagagctcc actgccctac 8700
   ttgctttttt taaatacatt tcttctgcag ctgaaattct ggcgatcgtg atgctgcaga 8760
   ttcgatgaaa gtggagtagc tagtcagcag ctggtgatga acaattgaca cgcctgaaag 8820
   tcctgaaatg atcgcgcgtt ggactgctaa tggagggatg cactctttca ggtttgcaaa 8880
   ggctgcacac aggtttccat tggggtgagc gaatttggtg gtcgtcgaag ttctcgagga 8940
   aaactctgta gcctaatcat tgtacagttt gactaatcga aaagatgaaa gtttgaga 8998
<210> 144
   <211> 2611
   <212> DNA
   <213> Oryza sativa
<400> 144
   atggcggccg cggccggcgc cggcgagccg tcgccgtacg cggaggcggc gggatccgac 60
   ctcgcgaatg cgcgggcgcc gtctcccgtg gtcggcaagc acctcccgtc gggcgccgtg 120
   ccgcgccacg cgtacgtgtt cgacggcgag ggggggttcg ccgacgcggc gtgggacgtc 180
   gcggcggcgg cgccgggggc gttcacgtgg caccacatcg agctcccgcg gcagcagccc 240
   gggggcgccg ccgcgaagcc gctccaccac gcgcaggcgc tgatcgagct gctctgcccg 300
   ccgctcacgc tgcaggagat cctcgcgttc gtcgccacgg gcccgcactg cggcgtcgtg 360
   gacggcggcg gcggcggcgg ggcgggcgcg ctccttctcc gcgtgagctc gccggggccg 420
   gtggggagcg cgttcgcgct ccgcctcgcc gcgcgcgtca cggacagctc cgtggtgacc 480
   gtgtccgtgg gcggcgtccc gcgcctcgcg ttcgggacca cgcaggcgtc gctcctctcc 540
   gaggtgccgc tcggggtgac cgcgtcgctc tccgacgagg gccacggcgg cgggcgcgcc 600
   gtcgagggcg gggtggtgat cgaggagcgg ctgctcgagt cgctgctcgc catgaaccac 660
   gccgacggcg cgcacaccga caaccccgtg ccgcggaccg tgtccaacct cctcgtgcac 720
   gtcctgggaa cgcacgtaga ccacgtccac gacatcgtca cgcgcctcga gatggagctc 780
   gacagcatcg agctgcatct cgacaagggt aggtggaggt tgctttcttg actagtaatc 840
   gcatataaac ataaatcaaa ttattagggt tctaagaaca tctccattcg ttagacaaag 900
   ttggtgccct ttttttccct tttctggtga tgttgttagg taggacgaac tagcttcttc 960
   gttacgtctg ttgttcatga ttatgcacga gagaaattta ttgtgctttg ctttgttcta 1020
   tgggctcatt ggattacact aaactggaag tctaaaaatt gggaatttgt cgaaaattat 1080
   cgattctgtt gtgattgtcc atccgcattg gggcggcaac ttgatgaaaa actcctatga 1140
   ttggaaggag gttgctgatg ctattgaatt gattcttgat tgttactgtt ccatggagaa 1200
   gttccaattc catattcgtg cttaaattga ttctgaagcg gtgtatttcc ttttagaggt 1260
   gatttcgggt gtgtttagtt cccaccaaaa ttggaagttt ggttgaaatt ggaacgatgt 1320
   gacggaaaag ttggaagttt gtatgtgtag gaaagttttg atgtgatgga aaagatggaa 1380
   gtttgaagaa aaactttgga actaaaccca gccaataaat tataaatttg gggtgaagta 1440
   aggtcagaga agaaggaaag gtcatcaatt tatagtttgt tatgtatggt ggaatgaaat 1500
   ttctgaatgt catgttggca gcaaccgtat ttcctgaaat gccatgatta tattgctagc 1560
   tgtgattcca gatgggattc atttcacatg atcagtatgt gcaacagaat attttctgag 1620
   atggatttct cacgtagtct atggatatct gttctacttc tacataggtt aatgtcaatg 1680
   gaagtcaatg tgaagtctag tttatttact actagccaaa cttatgctca aatttgttat 1740
   agcattctgc agatgttatt ctttctctta atgggctttg aaggtattta tgatatttct 1800
   gtgaatcttg cttgcaggtg gtcactttat gaggaaactt ttgttggatg gaaggagatt 1860
   ccccaaaatg catcttgatc tacagcgcct gcttcaggta ttttctgaga tttatctcaa 1920
   attgctaaaa tggagttcaa tgtatgagta tgttattgtt cactggatcg ggaacgcata 1980
   atagaagtca tgggctttaa tgatttcttg cctaggttgt ttctcatggt gaccaagtat 2040
   tcccccgtgt aaaggaaaaa tgtgcgagca agagttggtt tgcgagtgaa gatattgttg 2100
   ctcttgaaga tctgataggc cgtcttagga ggctgaagga aaatcttgga tttataacga 2160
   atagggtgac tacacttcaa gctagtctag atagctggca atctgagcag ataaacaaaa 2220
   gcttgtacta tctttcattt ttgtccataa tattccttcc tctatccatt gtcactggag 2280
   gtatgttccc atgcgtattt ctgatgccat ttattgctta aggtctccaa tttacatgat 2340
   ctgctgcaat gtttgtgcag tttttgggat gaatgttggt ggtgtgccat ggactgagca 2400
   gaaaaaccct gcaaatctag atggcttctt caatgtcatg ttaatatgcg tcgtgatctt 2460
   gttgatcctg ctgctttgtt tcttatttcc ttcattgtat tcacacgtgt cggcatggag 2520
   aacccgccgt gcactggccc ggagcagttc tcagaacaag agacatctga aactctttaa 2580
   gggtcacaaa gatggttaca tgcgcctctg a 2611
<210> 145
   <211> 2561
   <212> DNA
   <213> Oryza sativa
<400> 145
   accgtggagc actcgatctc caagctcatc tagctcttct tgcttcagct tcttcctctc 60
   tctcacagtt catcacactt ggctttttga gtcaggtgct tgttcttcct gctgttcttg 120
   ccttggtaat gctcttgatc tcttcttaga tgtaatcttg ggttagctag catgcagttc 180
   ttggggttta tctcatcttt ctgtagttta gtgtgtcagg tttagtagta attgttcgtc 240
   gagaaaacaa aatgatatat gggttggatg aatggagaag aatgtggttc agttcagtgt 300
   gatcatcttc ttgttgtggc tctgaatcga atgagctgtg aatttggtga atttgcaggc 360
   atatgcagga ccagctgatc tgcagcggct gcaggcgcgt cgtccagtac aggagagggg 420
   tcgccggcgt ctgctgcccg ggctgcaaca cgctcaccgc cgtcaacccg tcagcggtgg 480
   ccgacatgtc ggagctcatc tgcagcggct gccccacgct gctgttctac aaccgcggcg 540
   cctccaacat ccgctgcccc agctgcaaca ggctcaactc caccagatca ggtgaatgat 600
   catatttttg cacatactat atctttctct gaaaagatca tatttgcagc tgattcagag 660
   ctgcaaattg ggatgaaatt aatctactga aatctgagct gatcatggat ggttttattt 720
   ggtggttcag ccaaccagat tgcacacctg acatgcgggc agtgccggac gactctgatg 780
   cacccacctg gagcctcaac tgtgcagtgt gcaacctgca gatatgttaa ccatgtcagg 840
   gtatgttctc atctctgaat gtttctaccc tctatatttc tcgcatcgcg cattcgctct 900
   ccggtttcgc tctctggtag taatcatcgt cggctcatcg gctcggtgat aaccagggtt 960
   ggaaattccg aaacgaaatt tccgaaattt cggacatttt agacctctct gatatgatat 1020
   tatttcggcc aaattttttt atttttttaa ttttttcgtg aactttggta atatttgttc 1080
   aaattcaact aaattttatt caaaatttcg gaaatttcag accgaaattt caaaaaattt 1140
   ggcatttcca tgaggaccga tcaaatcggc taaaccgaaa ggtttaaccc tggtgataaa 1200
   tagttagtgt gttcttgaat gatctttcca caactgaaca gcaacctgag ttgatcaact 1260
   ggaaagatgg aataaccttt taactttgcc tagaatatca gtagtcggca caggtttttc 1320
   tgaagctgaa aatatggaat tttcaattag attttctcat ttctgtgaac aaaatttcat 1380
   gggaccattg actgaacaaa ccagcacttg cttttatctt taattttgca tcaactaggg 1440
   gatgcatatg gttctcctct tcttaaaaaa gatgacaact ttggtaggtt cagcttgctc 1500
   catggttttt catggaatca gttctcaaag taggcatgta cctatgatta gccagcagaa 1560
   ctcgatgcct cggccaagat ttttatcttc caattccgtt atcaggacaa tgaaactaaa 1620
   tgatgatggt ccattgggac aaatgaaaca atctgaatct tatgtgatgt caaatgttcc 1680
   atgatgacga tgctagcttt gttctctgaa tttccttttc tttctcctac ttgcaggatg 1740
   ctcggcctca aactgtcctt gtagagaatc ctaagacact ggatgataag ggcaagctgg 1800
   taagcctcat ctctaggcta tatatctcta ctatctacta ctttaaaaga cgcagtcctc 1860
   ccatcccacc ctaccgcacg cagcacgaga aaatcttgta ataaaccgaa ccggcccacc 1920
   caccgcaccc tccttcccgc accgcgtgag agaaaaaaaa agtgcacgtc cgacttccat 1980
   gtcgtcttct cgctagaaaa accgagtttc agtatcagac aaaaatatga tagttgtttt 2040
   tatgggtcca ttgcaacgcc tagacattta actagtagta ccctaaaacc aaaatttcct 2100
   tctgaatttt tttcttggaa tagcagaaag tttcacttct gaaatgatag ctgaagagta 2160
   catgtatttc agctctgaaa tgtccagttg attgactgtt ctgttttgtt atggtatagg 2220
   tgagcaatgt ggttgttggt gtcacctcat ggaaaagatg atgaacaggg gctcatctag 2280
   ggtttaatcc caagggtcta tggatgattg atacccttgg tggatttgta tcattactac 2340
   aaagctattg ttaaatcaag tgtgttttga agcttgatat aaacaagaga aaaacaatag 2400
   ggcaaaaaat gaagaaaaaa atatatccga aatgtgccat gtttgtgctt cttttgtgag 2460
   aaaaaaaaat gtaaatatgc tggctatggt gctagcctat gtgcttttta gcaaaaagga 2520
   atggaaatga gatgagcagc attgtgtcct ggaattttta t 2561
<210> 146
   <211> 2377
   <212> DNA
   <213> Oryza sativa
<400> 146
   ccccttcatt ttctcgaagc gcttcccttc ccttcgcttc cccgcacaat ctgcaaacgc      60
   gccacccgtc gctcataaac ccctctccct ctctctctgg ccgacacatg gaccccaccg     120
   ctcccaactc cggcgacgcc aacggcggcg cggccgccga cgccaccgcc ccgtccaaca     180
   ccaccgtcac cctcccgccg ctcaccctcc gagacgtgcc cctgctcccg tcggcggcgg     240
   cggcgaccga cacgatcccc aacccgatct cccgccaccc ctacttccac ccgccggcca     300
   ccttctacat ctccccgggt gacgtctccc tccgccacgc cttcttcgac ctcgcctccg     360
   cgtcgccgtc cccgctcgtc gcctaccgcc cgcgcggggc cgcgcgcggg ggtcgccgtg     420
   gaccccgccc gcgcgcgcgc cgcggtcgtc acctgcgggg ggctctgccc gggcctcaac     480
   accgtcctca gggagctcgt cgtcggcctc cgggagctct acggcgtccg cgacgtcttc     540
   ggcgtcgccg ccggctaccg cgggttctac ggccccgacg ccgaccacgc ccgcctggac     600
   ctggccgccg tcgacgactg gcacaagaag ggcggcaccg tgctcaagac cacccgtggt     660
   ggctttgatc tcaacaagat cgttgacggc atcgtcgcgc gcgggtatac gcaggtgaga     720
   gcaaacccct accgttttta gagttttcag gttttatgta tttttttggc aaatagctaa     780
   agtttaaact gatctcacaa atcatgtgca cgtggaaacc cctaagcttc tttttacgcc     840
   aagctggcaa atgaaagctg gccaattttg ttaagattag aagcacaaga aacatgttac     900
   aggtatttgc aagtgtacaa tgtgtcatct gaattggcaa ggatactatc ttggtgctac      960
   aaatttgctt tagacatttt agtatgttgg tgtacaatca tgggggttgt tctgaattga     1020
   catggagtag tacttattat gttaggtgtc attatggaag aattatctca aagaatttgt     1080
   ctgtccagaa tttttctttg tcagtagagt ttctttttcc accgggctct ttgtcggtta      1140
   gagtggtcaa gtactcaagt ggttgaagtc ttactgattt gatcgatcag cgttgagtcg     1200
   tcttgctagt tcaacatgct aatttgtttc agttattgct tgtattttgg cttattaaag     1260
   tgtctgacct caaacacttt ttatcatgac tatatcttgc tgaaggttta tgcaattgga     1320
   ggggatggaa caatgagagg agctgtggcc atcttcaacg agtttaagcg ccgtggtttg     1380
   aacatttcta ttacagggat cccgaaaact gtggacaatg atatcggcat catagacagg     1440
   tcatttgggt tccaaaccgc agtggagatt gctcagcagg caatcgacgc agcacatgtc     1500
   gaggctgtga gcgccgtgaa tggcattgga cttgtcaaac ttatgggcag gagcacaggc     1560
   cacattgctc ttcatgccac cctgagcagc cgcgatgttg actgctgttt gattcctgag     1620
   gttgatttct atcttgaagg aaaggggggc ctgtttgagt tcttgtatga aaggataaaa      1680
   cagaagggac atgctgttgt cgttgttgct gaaggtgctg gtcaggaatt gattccaagg     1740
   actgacgatc aaaagcggga gcaggacgag tccggcaaca ttgtgttcct tgatgtgggt     1800
   ccctggttaa aatctgagct gggtaaatgg tggaagagag aacacccaag cgagttgttc     1860
   actgtgaagt atatcgatcc cacttacatg atacgagctg ttccagcaaa tgccactgac     1920
   aatctgtact gtacattgtt ggcacattcg gcgatccatg ggatcatggc tgggtacact     1980
   ggcttcgtcc ctggcccgat taatggaaac tatagctaca taccgctgga agatgttgct     2040
   gtggcgaaga acccggtgga tgtgaatgat cacaaatggg catgggttag atcagtcaca     2100
   aaccaaccag atttcatgaa gccaaaatac taagaccaaa agtgctgtta ctggacatgg     2160
   ttgtatgact ttttctccct ggagccttga cacgttaagc ttgatttctt tctatccagt     2220
   ttcttctctt gttatgccga tctatctata agatgttagg tattctgatc tccctggttt     2280
   gtgtttgtgt gcacttgaaa tcgtgttagc aggtattgct gtaaatctgt agtacataaa     2340
   taaatgaagt ggcaggaaat gttgttctcc attggcg                              2377
<210> 147
   <211> 2411
   <212> DNA
   <213> Oryza sativa
<400> 147
   atggttgaaa gtgcaagcat ggtaatttgt gtgattaaac aaatttacac actacgttag       60
   tactctctcc atttcacatt ataagtcgtc ttgtaggagt actaactact ccctccgtcc      120
   caaaatataa gaacttaaag accataaggg atattaggaa cttaaagacc ggaaatgtcc      180
   catccggttc tagattctta tattttgaga cggagagaat aatatcttat aatgtgaaac      240
   agagaaaata cttcttcctt aatgaaacaa ctgtgcaaac tctaaattaa taacaaaaaa 300
   aaagaccgat taatatctga atgaatgatc aaagaacaaa tgtatctgca aatcaaacga 360
   tcatttctgt aacttgcaaa tttcctttgt ccatcatgca ggtgaacgag aactcggaga 420
   atccatactg gaaagcaata ggatacagag tggaagagcc ccgacgtgat cgagcagagt 480
   cgatgccgtc gccgtcgcca tcgccggtat cgcggcggcc actggacaac ggcgtcgtgg 540
   agacgagggc gctgacggac accaccctcc tccggtcgct cgcggcgaag ggcctcgccg 600
   tgaggcccgg cgcgtcggac gagcaccaca cggtgcggtg cgacgccgtc atcgtcggct 660
   ccggctgcgg cggcggcgtg gccgccgcgg tgctcgcgtc cgccgggtac aaggtggtcg 720
   tcgtcgagaa gggcgactac ttcaccaagg aggattacag ctcgatcgag ggcccgtcca 780
   tggagcgcct cttcgagagg ggcggcgtct tctgcacgtc caacgtcacg acgatgatat 840
   tcaccggcgc gacggtcggc ggcgggtcgg cggtgaactg gtcggcgagc atccgcacgc 900
   cggcgggcgt gatgcaggag tggtcgcgcg agcacgggct ggcggtgttc gcgagccccg 960
   ggtacgcgcg ggccatggac gcggtgtgcg agcgcctcgg tgtgaccgac gcgtgccggg 1020
   aggaagggtt ccagaacaag gtggtgcgcc gcgggtgcga cgcgctcggg ctgcgcgccg 1080
   acgccgtgcc gcgcaactcg tcggaggggc acttctgcgg cagctgcaac ttcgggtgcc 1140
   ccaccggcga caagaagggc accgacacga cgtggctcgt cgacgccgtc gagcgcggtg 1200
   cggtcatcct gaccgggtgc aaggccgaac acttcatcgt cgagagcaac ggcggtggcg 1260
   gcggccggag caagaggtgc gtcggcctgg tggcgacgtg catgagcaac ggcatcacca 1320
   agaagctccg cgtcgaggcg aaggtgtcca tctcggcgag cggcgcgctc atgacgccgc 1380
   cgctgctgcg caacagcggg ctcaagaacc gccacatcgg ccggaacctg cacctccacc 1440
   cggtgtccat ggcgtggggc tacttcccgg acaacacgcc ggagccgcac atcccgggga 1500
   agtgctacga gggcggcatc atcaccagca tgcaccgcgt cacggagcgc accatcatcg 1560
   agacgccagc gctcggcccg ggcgccttcg ccgccctggt gccctgggag tccggccgcg 1620
   acatgaagga gcggatgcgc cggtacgcgc gcacggcgca cgcgttcgcg ctggtgcgcg 1680
   accgcggcgc cgggtccgtc gacggcgagg gccgcgtccg ctacgccccg agccgcgacg 1740
   acgccgagga gctccgcgcc ggcctccgcc gcgcgctgcg catcctggtg gccgccggcg 1800
   ccgccgaggt gggcacgcac cgcagcgacg gggcccgcct ccgatgcaag ggcgcgcgcg 1860
   acgcggacgt ggaggcgttc ctcgacgagg tgaccgtgga gaaggggccg atgcactcga 1920
   cgacggacaa gtggtcggtg ctctgctcgg cgcaccagat ggggagctgc cggatgggcg 1980
   cgagcccccg cgacggcgcc gtcgacgtcg ccggcgagag ctgggaggcg gaggggctct 2040
   acgtctgcga cggcagcctg ctcccgacgg cggtgggcgt gaacccgatg atcaccatac 2100
   agtccatcgc ctactgcgtc gccaagggca tagccgactc gatggcacac ggcaaggagc 2160
   agcgctagta aaatcttttt cctcttttgt tcatgcataa attgcaaatt tgcaatgtcc 2220
   ctgcttgtta atcaactgta atagtgatga taaatcacga gcatatttca gcagatgatt 2280
   catatgggaa aataattcta agggatttag agatctgttt agagtctttc agagacgaca 2340
   tgggcctcaa gataaatatg gtaaaggttc aaatggccgg aatgagaagc tgaaatgtct 2400
   gcccgtatta a 2411
<210> 148
   <211> 1278
   <212> DNA
   <213> Oryza sativa
<400> 148
   atggcgccgc acccgctgct gaggggaggg gcgaggcggg ggaggaagta cgcgcacggg 60
   atgcaccccg cgcagatgga ggcgctgcgc gccatgtgcg gcgcgctcat cccgtcgctg 120
   cccgtggacg cggacggcgg cgacggcggg cgccgccccg gcgacaagga cctcgagcgg 180
   ttctacctcg cctccgccgc cgactcctcc atccccgacg aggtcggtcg gtcggtcgat 240
   cggtcgaaca ccccatgcgc gcgagttctt tcttgtttat aatctctaat ctggcggcgg 300
   ccgcggcgac cgcggcgtgg cgtggcgtgc aggtggcgga gctgctggtg acgcgttgca 360
   tatgggaggc ggtggcgctg acgtgggtgg tgctgtgggc gctgagcacg cgggcgggca 420
   cgctgctgct gtgcggccgg gacagcgtcg ccgccgtcga cggcggcggg ttcccgttcg 480
   tgtccgtgcg ccgcttcgcc gacatgccgg cggcgaggcg ggaggcggcg ctgtggcggt 540
   ggagcggcgc gcggtggctc ttcttcccgc tccgcatcgc cttcgccatc gccaagatcc 600
   tctgccacta cgtcttctac tccatggtac gtacgtctcc tcttcctcct cctcctcctc 660
   ctctcgaccg actcacgaat cagaatcacc atgcagcagc acggcatcac ataccccgtg 720
   cttttcaaat ttcaaccaca taaaaatctg acaaatctaa aattctgtaa gaaatcgatc 780
   aattatcgtc aaaatttagc agagatcgaa tttccataca agtatacaac agtcctacac 840
   cgaagcacgc aggtgcacca tgttaacaaa caacagcagg gtttttaatt tcgaaattgg 900
   attttttggc aaggggggac tggaattact gaaatttcgg aaatatcagt aatttcgttt 960
   tttttgccaa aattatttga aattttgact attttgaatg aatttgaata aaatttgatc 1020
   aaattcacaa aaagttgcaa aaaaccaaaa atttcggacg agatttgagc atgctggtgg 1080
   ggggtgaaat caccaaaatt tcaaaccctg aacaacagta caaacaccag ccagtcactc 1140
   gcagcagctg caccgtagac ttcttgttct tggagctacc taggaaccgg tttagagaat 1200
   tttttattta taattcgtct gttttcagca tatgcgtatt ctgcatttgt tcaaattcag 1260
   atactcgtat cagcctaa 1278
<210> 149
   <211> 455
   <212> PRT
   <213> Oryza sativa
<400> 149
   Met Glu Trp Asp Leu Lys Met Pro Pro Ala Ala Ser Trp Glu Leu Ala
   1               5                   10                  15
   Asp Glu Leu Glu Asn Ser Gly Gly Gly Gly Val Pro Ala Ala Val Ser
               20                  25                  30
   Ser Ser Ser Ala Ala Val Gly Gly Gly Val Asn Ala Gly Gly Gly Gly
           35                  40                  45
   Arg Gln Glu Cys Ser Val Asp Leu Lys Leu Gly Gly Leu Gly Glu Phe
       50                  55                  60
   Gly Gly Gly Gly Ala Gln Pro Arg Val Ala Val Ala Gly Glu Pro Ala
   65                  70                  75                  80
   Lys Gly Lys Gly Pro Ala Ala Ala Ala Thr Gly Ala Ala Ala Ala Ala
                   85                  90                  95
   Ser Ser Ala Pro Ala Lys Arg Pro Arg Gly Ala Ala Ala Ala Gly Gln
               100                 105                 110
   Gln Gln Cys Pro Ser Cys Ala Val Asp Gly Cys Lys Glu Asp Leu Ser
           115                 120                 125
   Lys Cys Arg Asp Tyr His Arg Arg His Lys Val Cys Glu Ala His Ser
       130                 135                 140
   Lys Thr Pro Leu Val Val Val Ser Gly Arg Glu Met Arg Phe Cys Gln
   145                 150                 155                 160
   Gln Cys Ser Arg Phe His Leu Leu Gln Glu Phe Asp Glu Ala Lys Arg
                   165                 170                 175
   Ser Cys Arg Lys Arg Leu Asp Gly His Asn Arg Arg Arg Arg Lys Pro
               180                 185                 190
   Gln Pro Asp Pro Met Asn Ser Ala Ser Tyr Leu Ala Ser Gln Gln Gly
           195                 200                 205
   Ala Arg Phe Ser Pro Phe Ala Thr Pro Arg Pro Glu Ala Ser Trp Thr
       210                 215                 220
   Gly Met Ile Lys Thr Glu Glu Ser Pro Tyr Tyr Thr His His Gln Ile
   225                 230                 235                 240
   Pro Leu Gly Ile Ser Ser Arg Gln Gln His Phe Val Gly Ser Thr Ser
                   245                 250                 255
   Asp Gly Gly Arg Arg Phe Pro Phe Leu Gln Glu Gly Glu Ile Ser Phe
               260                 265                 270
   Gly Thr Gly Ala Gly Ala Gly Gly Val Pro Met Asp Gln Ala Ala Ala
           275                 280                 285
   Ala Ala Ala Ala Ser Val Cys Gln Pro Leu Leu Lys Thr Val Ala Pro
       290                 295                 300
   Pro Pro Pro Pro His Gly Gly Gly Gly Ser Gly Gly Gly Lys Met Phe
   305                 310                 315                 320
   Ser Asp Gly Gly Leu Thr Gln Val Leu Asp Ser Asp Cys Ala Leu Ser
                   325                 330                 335
   Leu Leu Ser Ala Pro Ala Asn Ser Thr Ala Ile Asp Val Gly Gly Gly
               340                 345                 350
   Arg Val Val Val Gln Pro Thr Glu His Ile Pro Met Ala Gln Pro Leu
           355                 360                 365
   Ile Ser Gly Leu Gln Phe Gly Gly Gly Gly Gly Ser Ser Ala Trp Phe
       370                 375                 380
   Ala Ala Arg Pro His His Gln Ala Ala Thr Gly Ala Ala Ala Thr Ala
   385                 390                 395                 400
   Val Val Val Ser Thr Ala Gly Phe Ser Cys Pro Val Val Glu Ser Glu
                   405                 410                 415
   Gln Leu Asn Thr Val Leu Ser Ser Asn Asp Asn Glu Met Asn Tyr Asn
               420                 425                 430
   Gly Met Phe His Val Gly Gly Glu Gly Ser Ser Asp Gly Thr Ser Ser
           435                 440                 445
   Ser Leu Pro Phe Ser Trp Gln
       450                 455
<210> 150
   <211> 1368
   <212> DNA
   <213> Oryza sativa
<400> 150
   atggagtggg atctcaagat gccgccggcg gcgagctggg agctagccga cgagctggag 60
   aacagcggcg gcgggggtgt accggcggcg gtatcgtcgt catcggctgc ggttggtggc 120
   ggcgtcaatg cggggggtgg tggcaggcag gagtgctcgg tcgacctcaa gctcggcggg 180
   ttgggggagt tcggcggcgg cggcgcgcag ccgcgggtcg ccgtggcggg cgagccggcc 240
   aaggggaagg ggccagcggc cgccgccacg ggagcagcag cagcagcgtc gtcggcgccg 300
   gcgaagcggc cgcgcggtgc ggcggcggcg gggcagcagc agtgcccgtc gtgcgcggtg 360
   gacgggtgca aggaggacct gagcaagtgc cgcgactacc atcgccggca caaggtgtgc 420
   gaggcccact ccaagacccc cctcgtcgtc gtctccggcc gcgagatgcg cttctgccag 480
   cagtgcagca ggtttcactt gcttcaggag tttgatgagg ccaagcgcag ctgtagaaag 540
   cgactagatg ggcacaaccg tcgccgcagg aagccacagc cagatcccat gaactctgca 600
   agttatcttg caagccaaca aggggcaaga ttctcaccgt tcgcgacgcc gagaccggag 660
   gcaagctgga cagggatgat caaaaccgag gagagcccat actacacgca ccaccaaatc 720
   cctcttggca tcagcagcag gcagcagcat ttcgttggct ccacctctga cggcggccgc 780
   cgcttccctt tcctccagga aggcgagatc agcttcggca ccggcgccgg cgccggcggc 840
   gtgccaatgg atcaggcagc agctgctgct gctgcttcag tgtgccagcc acttctgaag 900
   acggtagctc ctcctcctcc tcctcatggc ggcggcggca gcggcggcgg caagatgttc 960
   tccgatggtg ggttgacaca agtgctcgac tccgattgtg ctctctctct tctgtcagct 1020
   ccggcgaact ccacggccat cgacgtcggc ggtggccggg tggtcgtcca gccgaccgag 1080
   cacatcccca tggcgcagcc tctcatctct ggccttcagt tcggcggcgg cggcggcagc 1140
   tcagcctggt tcgcggcgcg gccgcatcat caggcggcca ccggcgccgc cgccaccgcc 1200
   gtcgtcgtct cgacggccgg tttctcctgc ccggtggtgg agagcgagca gctgaacaca 1260
   gtcctgagct ccaatgacaa tgagatgaac tacaatggga tgtttcacgt cggcggcgaa 1320
   ggctcatcgg atggcacgtc gtcgtctctg ccgttctcat ggcagtag 1368
<210> 151
   <211> 5052
   <212> DNA
   <213> Oryza sativa
<400> 151
   acagctcaag cttacgcggg agctaagctg agctacagcg agcggcggcg gcggccatgg      60
   agtgggatct caagatgccg ccggcggcga gctgggagct agccgacgag ctggagaaca     120
   gcggcggcgg gggtgtaccg gcggcggtat cgtcgtcatc ggctgcggtt ggtggcggcg     180
   tcaatgcggg gggtggtggc aggcaggagt gctcggtcga cctcaagctc ggcgggttgg     240
   gggagttcgg cggcggcggc gcgcagccgc gggtcgccgt ggcgggcgag ccggccaagg     300
   ggaaggggcc agcggccgcc gccacgggag cagcagcagc agcgtcgtcg gcgccggcga     360
   agcggccgcg cggtgcggcg gcggcggggc agcagcagtg cccgtcgtgc gcggtggacg     420
   ggtgcaagga ggacctgagc aagtgccgcg actaccatcg ccggcacaag gtgtgcgagg     480
   cccactccaa gacccccctc gtcgtcgtct ccggccgcga gatgcgcttc tgccagcagt 540
   gcagcaggta accccccccc ccccccccca accattgtct ccttccttcc cgccaaattc 600
   actgcaaaac aaaaaaaaaa tcgtagccca aaacacccca agacgtcatg gcaattcgca 660
   tcaagaactg catatatcaa tttctccact tcttttcagc gtcactgtct ctgatcattc 720
   tctttgctga acaaaagaaa aagaagataa gcaagagttt ttctcttttt tttgctcctt 780
   ttttttttgg ctttgcacaa tctcttcttg cttccagttg caactgacca ttgtgcagta 840
   catgcatctg catctactga ttctaatttc tacgctactt cggatcaaaa ttaattcagt 900
   actgcaaagc acaatttcat tgatccattt catccagcct cggactttgt tcatcatcat 960
   ctatctgtct cttacttcct ttccattggg agcatactat ccggctgtct cgtttcaggg 1020
   acgcacagct ttgcctttaa tggcatgcct tttcagcctt ccctcatgct atcctttagc 1080
   tcggcaactc gtattacccc aaattattac ctctttgctc gcctttagat ttattactat 1140
   catcttttct tttctttttt atatctcttc ttcaccagta gctgcactgt ttttgcactg 1200
   ctcaagagca aagcagctgc tgtagttgtt cagtgtttgt tgcttactga gaaaaaaaag 1260
   tgatagagac agaaaaaaaa gtgagggaga gaaaaaaaaa aaaacagaac tgacgcctga 1320
   atctcatcag ccagagatca cattaggcaa tttaccacca gactgttatg atattatttt 1380
   cagtgtcctc ctgtctgaat atgaccgtct gcttcctcta acaagaacaa taaatcagca 1440
   cctagttcag tactaactaa ttttctcatg aataaataaa taaatatagt cactgtaatt 1500
   agtgacacta ctagcacggt agcacctggt ttagtggtta acaatacttg gttcttgcac 1560
   ttctccctgt cgatgttttt tcgcgtgggg gctagctatc gattgattga ttcctcaact 1620
   atggcatcga aactggaaga acatatgcat actgggacac acaccctgct tgctttctga 1680
   atttctgatt tctcctcaag gcagctggcc taccacatat atctgactga gctgtgctgc 1740
   ttcttgccat gagagctaag ctaccttagc ttagctacta ctaccactta ctacgccgtc 1800
   tgttttggaa gggaaaggca gatgtggatg cccaaaccta gaaagatggt tgtaccactg 1860
   aaagagagag tttgtggatg tgatctgcac taaagcaccc ctgtacaggg aaaggaccat 1920
   gtagccctac tacaagttca ccatttacac ctctgttcct aaggttgggc cacacatata 1980
   tgaagctttt aatgtctcgg tttgttggaa agggttttgc attgccatta caagccagca 2040
   cagtggatac agatagccag ggtgctctct attggagaag aaaaaaaatg gagccctgaa 2100
   caccctgatt ggatctcact attgcatgaa agaatgatga gatttcttgt cttataattt 2160
   ttaaagattt tttttctaaa gtcagtctta gttacattca tttgttatat tccagtttca 2220
   gacttattgg tactaggttc tgtgagatct tttttttttt ttacatcgtt tgagtatcat 2280
   agggtgattc agtaccacct tgacccctgt ttttatcaga gctctaaact tctaacacca 2340
   cttctaactt ttgagctagt cttctaacct tgctgttttc tgaacaaaga tgtatactca 2400
   agattggtca tagatggaga tattctgtga acagaactaa cataatagca ccaaattagt 2460
   cagacatact ctttacaaaa ttactttgga gtttgttgtc cactccttga actagtacaa 2520
   tattgtccta ctgaatgcct tcctgccttt caacttgaaa gttccctatt ttatctgtta 2580
   gttcttttat aaaatgtaac tgcacattgt cagaaggatt tgcatcttat ttcactttgc 2640
   gccagtttta agtaatacat ggtatattgg cataagacca gactctacca ttttttatct 2700
   tgcagagaca tagcaaacaa ctaagtactt tttattgtgg tgtgctcctt tacacagtag 2760
   cacaacttgt aggatgctta tgtgattgtc tcatcaatta ttctctttat ctttaaaaag 2820
   agaatgatac aaaaaatctc tttatctgag aatacacatt acccagtggg gacagtcttt 2880
   caatgatttg attacttcgt cagtgtttgc aaactgggaa gatcattatg ctgctgcatg 2940
   cagactttat aaattaagtg atcttcagag tcagaacaag atgttagctt tctataccta 3000
   tggatccaca tccactgtat tgtggtccat gtacaagtgg ggttaaaata tttttctgcc 3060
   gttgacagaa cttcagttca ataaatttat ctaagatgaa gtatccaagc acggaaagag 3120
   ctaattaact gatgaaattc ctgtggtccc ttgtgttggt atatgagtat tctaagagag 3180
   aatatggaga cagtatatta aattattctg agaatactta tcctgacgtt tctttagtga 3240
   gaactgtggt gcatcgttac aaaacttcag atcatgtttc aggagtattt tatcatgtaa 3300
   gaattttaaa aagacgtaca tcctaggtac agtcatttct taaggtttca tggtactgaa 3360
   tgattaaatt acttcttctg gattgggttt caagcatcat ttggctaatt tcaatgcagt 3420
   taaatgatca taagcttttc tttcttcagg tttcacttgc ttcaggagtt tgatgaggcc 3480
   aagcgcagct gtagaaagcg actagatggg cacaaccgtc gccgcaggaa gccacagcca 3540
   gatcccatga actctgcaag ttatcttgca agccaacaag gtattttctt gtttattatt 3600
   accactctat gatatcgcag ttcatataag attaactggg atatagtcat tcagacttcc 3660
   taactattgt tagactagga aaaaaactat gaaacatgct aatagcatag ataagtcatg 3720
   gtaaaaaaaa agtaaaagaa aatgaaactg tggttaaaaa aaaacgcaaa tattagggaa 3780
   tgacctaata tcaaataatt agaaggagtg aggcttcgaa cccaggtcgt ctagcccatc 3840
   accttttgaa gctagccaga aaacccctgg gcgtttctca gaactgtggt tcagctatga 3900
   ctctgttctt tcaatcctga catcttgtaa catgtaatgc attctagtat acatctaatg 3960
   cattgaacca tatcttatgt actaatttgt gctgatatat caaacatcgc atcaaaattc 4020
   aggggcaaga ttctcaccgt tcgcgacgcc gagaccggag gcaagctgga cagggatgat 4080
   caaaaccgag gagagcccat actacacgca ccaccaaatc cctcttggca tcagcagcag 4140
   gcagcagcat ttcgttggct ccacctctga cggcggccgc cgcttccctt tcctccagga 4200
   aggcgagatc agcttcggca ccggcgccgg cgccggcggc gtgccaatgg atcaggcagc 4260
   agctgctgct gctgcttcag tgtgccagcc acttctgaag acggtagctc ctcctcctcc 4320
   tcctcatggc ggcggcggca gcggcggcgg caagatgttc tccgatggtg ggttgacaca 4380
   agtgctcgac tccgattgtg ctctctctct tctgtcagct ccggcgaact ccacggccat     4440
   cgacgtcggc ggtggccggg tggtcgtcca gccgaccgag cacatcccca tggcgcagcc     4500
   tctcatctct ggccttcagt tcggcggcgg cggcggcagc tcagcctggt tcgcggcgcg     4560
   gccgcatcat caggcggcca ccggcgccgc cgccaccgcc gtcgtcgtct cgacggccgg     4620
   tttctcctgc ccggtggtgg agagcgagca gctgaacaca gtcctgagct ccaatgacaa     4680
   tgagatgaac tacaatggga tgtttcacgt cggcggcgaa ggctcatcgg atggcacgtc     4740
   gtcgtctctg ccgttctcat ggcagtagtt ttttcagtaa ctgtatgttg ctgccttagt     4800
   ttcagtagag ttggttcttc atttcttttc agtgatcaaa ttattgtttc tgttcttttc     4860
   tgccatggta agttcctttt ttttttcttc ttcttgcctt catttgagtt aattacagca     4920
   ttgatttgtg tgaacaaaat tcatcataaa tcagttcctc gcgagatcat tggtctcaac     4980
   atgatggtgc caagtgagaa ctgcagtatt gtgcagtttt cagttttgag tctaagttgt     5040
   ataaacttgc ag                                                         5052

## Description

### Background of the Invention

It is of great interest to identify plant polypeptides that contribute to desired properties, e.g. high yields or improved tolerance to environmental stresses. When introducing their encoding genes into a host plant, such polypeptides impart the desired traits to the host plant.

Database UniProt accession no Q2R3X6 is a protein present in the rice genome for which transgenic plants are known. US patent applications 2007/020621, 2007/118921, 2007/039076 disclose the production of transgenic plants with nucleotide sequences which encode a protein which is identical with SEQ ID NO:103. US patent application 2004/123343 discloses a protein sequences and its corresponding nucleic acid which protein is 94% identical with SEQ ID NO:103. Transgenic plants have been produced.

### Summary

This invention is based on the discovery of a number of rice polypeptides that contribute to various important properties in rice, e.g., seed weight/size and panicle size/density, as well as their encoding polynucleotides.

Described herein is a method of producing a transgenic plant by transforming a host plant with a recombinant DNA construct that expresses in a plant cell a polypeptide containing an amino acid sequence at least 80% identical (e.g., 85%, 90%, 95%, 98%, 99%, or 100%) to an amino acid sequence selected from SEQ ID NOs: 1-49, 99-114, and 149. The recombinant DNA construct can include a nucleotide sequence selected from SEQ ID NOs:50-98, 115-148, 150, and 151, and a promoter sequence functional in a plant cell. The promoter sequence is operably linked to the nucleotide sequence.

The resultant transgenic plant exhibits one or more of the following properties relative to the host plant: (a) improved tolerance to an environmental stress, e.g., drought, cold, heat, salt, low fertilizer intensity, a plant disease, a herbicide, an extreme osmotic condition, a pathogen or pest, (b) elevated plant cell growth, (c) improved or decreased production of galactomannan, lignin, cellulose, flavonoid, or a plant growth regulator, (d) increased yields by modification of photosynthesis, carbohydrate use and/or uptake, nitrogen use and/or uptake, phosphorus use and/or uptake, mineral use and/or uptake, (e) increased yields of seed oil, starch and/or protein, and (f) increased rate of homologous recombination, (g) increased seed size or weight, (h) increased panicle length or density, (i) increased flowering, pollination or fertilization efficiency, and (j) elevated rate of seed development and maturation.

The present invention relates to a method of increasing seed size in a plant, by transforming a host plant or host plant cell with a recombinant DNA construct containing a promoter sequence operably linked to a polynucleotide encoding a polypeptide having an amino acid sequence of at least 80% identical to the sequence of SEQ ID NO:103, the promoter sequence being functional in a cell of the host plant or in the host plant cell.

A transgenic plant is a plant whose genome has been altered by incorporation of foreign genetic material or additional copies of native genetic material, e.g., by transforming or recombination.

The details of one or more aspects are set forth in the description below.

### Detailed Description

Described herein are 66 isolated rice polypeptides (SEQ ID NOs: 1-49, 99-114, and 149), and their functional variants, i.e., a polypeptide having a sequence identity of at least 65% (e.g., 70%, 75%, 80%, 85%, 90%, 95%, 98%, or 99%) to one of SEQ ID NOs:1-49, 99-114, and 149 and possessing the same function as that polypeptide.

The term "isolated polypeptide" used herein refers to a polypeptide substantially free from naturally associated molecules, i.e., the naturally associated molecules constituting at most 20% by dry weight of a preparation containing the
polypeptide. Purity can be measured by any appropriate method, e.g., column chromatography, polyacrylamide gel electrophoresis, and HPLC.

The sequence identity of two amino acid sequences can be determined using the algorism described in Karlin and Altschul, Proc, Natl. Acad. Sci. USA 87:2264-2268,1990, modified as described in Karlin and Altschul, Proc, Natl. Acad. Sci. USA 5873-5877, 1993. Such an algorism is incorporated into the NBLAST and XBLAST programs of Altschul et al., J. Mol. Biol. 215:403-410, 1990. BLAST nucleotide searches are performed with the NBLAST program, score = 100, wordlength = 12, to obtain nucleotide sequences homologous to a nucleic acid molecule of the invention. BLAST protein searches are performed with the XBLAST program, score = 50, wordlength = 3, to obtain amino acid sequences homologous to a reference polypeptide. To obtain gapped alignments for comparison purposes, Gapped BLAST is utilized as described in Altschul et al., Nucleic Acids Res. 25:3389-3402, 1997. When utilizing the BLAST and Gapped BLAST programs, the default parameters of the respective programs (e.g., XBLAST and NBLAST) are used. See
www.ncbi.nlm.nih.gov.

Tables 1 and 2 below list the GenBank accession numbers, amino acid sequences and encoding nucleotide sequences of the 49 rice polypeptides mentioned above. Also listed in Table 1 are functions of these polypeptides.

**Table 1. Rice Polypeptides That Are Involved in Seed Yield or Panicle Formation**

| **Proteins** | **Phenotype** | **TRIM line** | **Gene Locus** | **GOI** | **Protein** | **cDNA** | **GenBank Accession No.** |
|---|---|---|---|---|---|---|---|
| 1 | high seed yield | M0015163 | LOC_Os03g572 90 | cullin | SEQ ID NO:1 | SEQ ID NO:50 | AAT75245 |
| 2 | high seed yield; drought tolerant | M0019261 | LOC_OS_01g61 420 | ring finger protein 5 | SEQ ID NO:2 | SEQ ID NO:51 | BAD73632 |
| 3 | high seed yield; drought tolerant | | LOC_Os01g614 30 | unk zinc finger HIT | SEQ ID NO:3 | SEQ ID NO:52 | NP_19461 1.2 |
| 4 | seed yield | M0025978 | LOC_Os01g527 90 | cytochrome P450 | SEQ ID NO:4 | SEQ ID NO:53 | NP_91675 4 |
| 5 | | | LOC_0s01g5280 0 | cytochrome P450 | SEQ ID NO:5 | SEQ ID NO:54 | BAD86930 |
| 6 | | | LOC_Os01g528 10 | PP2C | SEQ ID NO:6 | SEQ ID NO:55 | NP_56714 5 (ara) |
| 7 | | | LOC_Os01g528 30 | unk | SEQ ID NO:7 | SEQ ID NO:56 | NP_91675 7 (ara) |
| 8 | long panicle | M0038751 | LOC_Os03g641 50 | MatE efflux family | SEQ ID NO:8 | SEQ ID NO:57 | XP_47049 7 |
| 9 | long panicle, big seed | M0046723 | LOC_Os04g581 80 | WD domain Gbeta | SEQ ID NO:9 | SEQ ID NO:58 | XP_47435 8 |
| 10 | | | LOC_Os04g581 90 | dof28 | SEQ ID NO:10 | SEQ ID NO:59 | XP_47435 9 |
| 11 | big seed | M0022658 | LOC_Os02g289 70 | | SEQ ID NO:11 | SEQ ID NO:60 | NP_00104 6872.1 |
| 12 | big seed | M0024255 | LOC_Os07g101 10 | | SEQ ID NO:12 | SEQ ID NO:61 | NP_00104 4695.1 |
| 13 | big seed | M0034189 | LOC_Os04g394 30 ?? | p450, dwarf4 | SEQ ID NO:13 | SEQ ID NO:62 | NP_00105 3047.1 |
| 14 | big seed | M0034642-1 | LOC_Os10g428 20 | DUF221/E RD | SEQ ID NO:14 | SEQ ID NO:63 | NP_00106 5504.1 |
| 15 | big seed | M0039539 | LOC_Os04g556 90 | unk | SEQ ID NO:15 | SEQ ID NO:64 | NP_00105 4091.1 |
| 16 | big seed | M0040296 | LOC_Os07g148 90 | unk | SEQ ID NO:16 | SEQ ID NO:65 | NP_00105 9304.1 |
| 17 | | | LOC_Os07g149 10 | unk | SEQ ID NO:17 | SEQ ID NO:66 | NP_00105 9305.1 |
| 18 | dense panicle | M0033394 | LOC_Os02g562 50 | putative GATA | SEQ ID NO:18 | SEQ ID NO:67 | CT828966, not predicted, NP_00104 8448.1 |
| 19 | dense panicle | M0038155 | LOC_Os02g325 04 | unk | SEQ ID NO:19 | SEQ ID NO:68 | NP_00104 6996.1 |
| 20 | dense panicle | M0039100 | LOC_Os06g508 18 | unk | SEQ ID NO:20 | SEQ ID NO:69 | NP_00105 8607.1 |
| 21 | | | LOC_Os06g508 30 | putative TF bzip | SEQ ID NO:21 | SEQ ID NO:70 | NP_00105 8608.1 |
| 22 | dense panicle | M0039314 | LOC_Os08g419 50 | MADS, MADS7, MADS45 | SEQ ID NO:22 | SEQ ID NO:71 | NP_00106 2335.1 |
| 23 | | | LOC_Os08g419 60 | mads unpredicted AGL72 | SEQ ID NO:23 | SEQ ID NO:72 | NP_00106 2336.1 |
| 24 | dense panicle | M0039419 | LOC_Os02g474 30 | unk not predicted, peptidase M20, | SEQ ID NO:24 | SEQ ID NO:73 | NP_00105 8304.1 |
| 25 | | | LOC_Os02g474 40 | putative syntaxin | SEQ ID NO:25 | SEQ ID NO:74 | NP_00104 7853.1 |
| 26 | dense panicle | M0039485 | LOC_Os12g152 22 | unk | SEQ ID NO:26 | SEQ ID NO:75 | NP_00106 6501.1 |
| 27 | dense panicle | M0043153 | LOC_Os06g462 40 | ARM repeat | SEQ ID NO:27 | SEQ ID NO:76 | |
| 28 | | | LOC_Os06g462 50 | unk, proteophos phoglycan precursor | SEQ ID NO:28 | SEQ ID NO:77 | NP_00105 8344.1 |
| 29 | dense panicle | M0050559 | LOC_Os12g437 20 | put RXW8, DUF221, ERD | SEQ ID NO:29 | SEQ ID NO:78 | NP_00106 7352.1 |
| 30 | | | LOC_Os12g437 30 | unpredicted , cyclin like Fbox | SEQ ID NO:30 | SEQ ID NO:79 | NP_00105 2038.1 |
| 31 | | | LOC_Os12g437 40 | short chain dehydrogen ase | SEQ ID NO:31 | SEQ ID NO:80 | BAF30371 .1 |
| 32 | large grain | M0027918 | LOC_Os10g017 00 | unk expressed, HSR201 | SEQ ID NO:32 | SEQ ID NO:81 | |
| 33 | large grain | M0028590-1 | LOC_Os03g554 30 | 2 proteins in 2 frames ? | SEQ ID NO:33 | SEQ ID NO:82 | |
| 34 | | | | 2 proteins in 2 frames | SEQ ID NO:34 | SEQ ID NO:83 | NP_00105 1361.1 |
| 35 | | | LOC_Os03g554 50 | unk | SEQ ID NO:35 | SEQ ID NO:84 | NP_00105 1362.1 |
| 36 | large grain | M0063563 | LOC_Os08g329 30 | unk | SEQ ID NO:36 | SEQ ID NO:85 | NP_00106 1837.1 |
| 37 | large grain | M0063736 | LOC_Os08g311 30 | unk integral mb prot DUF6 family MtN21 ara like | SEQ ID NO:37 | SEQ ID NO:86 | NP_00106 1761.1 |
| 38 | | | LOC_Os08g311 20 | unk partial | SEQ ID NO:38 | SEQ ID NO:87 | NP_00106 6824.1 |
| 39 | large grain | M0063992 | LOC_Os04g478 90 | Myb-like DNA-binding region, SHAQKYF class | SEQ ID NO:39 | SEQ ID NO:88 | NP_00105 3582.1 |
| 40 | | | LOC_Os04g479 00 | | SEQ ID NO:40 | SEQ ID NO:89 | |
| 41 | large grain | M0064512 | LOC_Os12g019 16 | DS RNA binding | SEQ ID NO:41 | SEQ ID NO:90 | NP_00106 5960.1 |
| 42 | | | LOC_Os12g019 22 | WD like, raptor like | SEQ ID NO:42 | SEQ ID NO:91 | NP_00106 5961.1 |
| 43 | long panicle | M0066298-1 | LOC_Os10g401 40 | VAMP associated protein, sperm protein | SEQ ID NO:43 | SEQ ID NO:92 | NP_00106 5309.1 |
| 44 | large grain | M0067806 | LOC_Os08g015 80 | Disease resistance protein family protein | SEQ ID NO:44 | SEQ ID NO:93 | NP_00106 0794.1 |
| 45 | | | LOC_Os08g015 90 | unk | SEQ ID NO:45 | SEQ ID NO:94 | NP_00106 0795.1 |
| 46 | large grain | M0068164 | LOC_Os03g526 40 | unk protein 95 family | SEQ ID NO:46 | SEQ ID NO:95 | NP_00105 1193.1 |
| 47 | | | LOC_Os03g526 50 | syntaxin 111 like | SEQ ID NO:47 | SEQ ID NO:96 | NP_00105 1194.1 |
| 48 | large grain | M0068730 | LOC_Os03g083 30 | ZIM domain containing protein | SEQ ID NO:48 | SEQ ID NO:97 | NP_00104 9168.1 |
| 49 | large grain | M0069991 | LOC_Os03g149 80 | CTLH, C-terminal to LisH motif domain containing protein, TOPLESS | SEQ ID NO:49 | SEQ ID NO:98 | NP_00104 9587.1 |
| 50 | large grains; drought tolerant | BASF 163 (M0028590 -2) | LOC_Os03g554 60 | | SEQ ID NO:99 | SEQ ID NO:115 | |
| 51 | large grains | BASF 162-1 (M0066298 -2) | LOC_Os03g180 00 | Phosphoino sitide-specific phospholip ase C | SEQ ID NO:100 | SEQ ID NO:116 | |
| 52 | large grains | BASF 162-2 (M0066298 -3) | LOC_Os03g180 10 | Phosphoino sitide-specific phospholip ase C | SEQ ID NO:101 | SEQ ID NO:117 | |
| 53 | large grains | BASF 162-3 (M0066298 -4) | LOC_Os03g180 20 | Rhodanese like protein | SEQ ID NO:102 | SEQ ID NO:118 | |
| 54 | large grains | BASF 148-1 (M0037341 -1) | LOC_Os03g304 30 | | SEQ ID NO:103 | SEQ ID NO:119 | |
| 55 | large grains | BASF 148-2 (M0037341 -2) | LOC_Os03g304 84 | ZOS11-03-C2H2 zinc finger protein | SEQ ID NO:104 | SEQ ID NO:120 | |
| 56 | large grains | BASF 148-3 (M0037341 -3) | LOC_Os03g304 10 | ThiF family domain containing protein | SEQ ID NO:105 | SEQ ID NO:121 | |
| 57 | large grains; taller plant; long panicles | BASF 7-1 (M0034642 -2) | LOC_Os03g488 20 | | SEQ ID NO:106 | SEQ ID NO:122 | |
| 58 | large grains; taller plant; long panicles | BASF 7-2 (M0037341 -3) | LOC_Os03g488 30 | | SEQ ID NO:107 | SEQ ID NO:123 | |
| 59 | large grains | BASF142 | LOC_Os03g129 30 | | SEQ ID NO:108 | SEQ ID NO:124 | |
| 60 | large grains | BASF 143 | LOC_Os03g129 40 | helix-loop-helix DNA-binding domain containing protein | SEQ ID NO:109 | SEQ ID NO:125 | |
| 61 | high seed yield; drought tolerant | BASF 10-1 | LOC_Os08g036 00 | metal ion transporter | SEQ ID NO:110 | SEQ ID NO:126 | |
| 62 | high seed yield; drought tolerant | BASF 10-2 | LOC_Os08g036 10 | LSD1 | SEQ ID NO:111 | SEQ ID NO:127 | |
| 63 | large grains | BASF 12 | LOC_Os04g394 20 | 6-phosphofru ctokinase 2 | SEQ ID NO:112 | SEQ ID NO:128 | |
| 64 | high seed yield | BASF 165-1 | LOC_Os10g335 40 | alcohol oxidase | SEQ ID NO:113 | SEQ ID NO:129 | |
| 65 | high seed yield | BASF 165-2 | LOC_Os10g335 50 | oxidoreduc tase | SEQ ID NO:114 | SEQ ID NO:130 | |
| 66 | high seed yield, large grains | | LOC_Os08g419 40 | Putative glume architecture 1 (SPL16) | SEQ ID NO:149 | SEQ ID NO:150 | |

**Table 2. Amino Acid and Nucleotide Sequences of Rice Polypeptides**

| | |
|---|---|
| SEQ ID NO:1 | |
| SEQ ID NO:2 | |
| SEQ ID NO:3 | |
| SEQ ID NO:4 | |
| SEQ ID NO:5 | |
| SEQ ID NO:6 | MCCSAVAVMKWEALLPNDTFLIVASSDGVFEKVTMQDVCDLMLYVKLGVKQELGSFALTQQNLADYVVDLSL |
| SEQ ID NO:7 | |
| SEQ ID NO:8 | |
| SEQ ID NO:9 | |
| SEQ ID NO:10 | |
| SEQ ID NO:11 | |
| SEQ ID NO:12 | |
| SEQ ID NO:13 | |
| SEQ ID NO:14 | |
| SEQ ID NO:15 | |
| SEQ ID NO:16 | |
| SEQ ID NO:17 | |
| SEQ ID NO:18 | |
| SEQ ID NO:19 | |
| SEQ ID NO:20 | |
| SEQ ID NO:21 | |
| SEQ ID NO:22 | |
| SEQ ID NO:23 | MEGGGRRRKRGKVELRRIEDRTSRQVRFSKRRSGLFKKAYELSVLCDAQVALLVFSPAGRLYEFASSTS |
| SEQ ID NO:24 | |
| SEQ ID NO:25 | |
| SEQ ID NO:26 | |
| SEQ ID NO:27 | |
| SEQ ID NO:28 | |
| SEQ ID NO:29 | |
| SEQ ID NO:30 | |
| SEQ ID NO:31 | |
| SEQ ID NO:32 | |
| SEQ ID NO:33 | |
| SEQ ID NO:34 | |
| SEQ ID NO:35 | |
| SEQ ID NO:36 | |
| SEQ ID NO:37 | |
| SEQ ID NO:38 | |
| SEQ ID NO:39 | |
| SEQ ID NO:40 | |
| SEQ ID NO:41 | |
| SEQ ID NO:42 | |
| SEQ ID NO:43 | |
| SEQ ID NO:44 | |
| SEQ ID NO:45 | |
| SEQ ID NO:46 | |
| SEQ ID NO:47 | |
| SEQ ID NO:48 | |
| SEQ ID NO:49 | |
| SEQ ID NO:50 | |
| SEQ ID NO:51 | |
| SEQ ID NO:52 | |
| SEQ ID NO:53 | |
| SEQ ID NO:54 | |
| SEQ ID NO:55 | |
| SEQ ID NO:56 | |
| SEQ ID NO:57 | |
| SEQ ID NO:58 | |
| | |
| SEQ ID NO:59 | |
| SEQ ID NO:60 | |
| SEQ ID NO:61 | |
| SEQ ID NO:62 | |
| SEQ ID NO:63 | |
| | |
| SEQ ID NO:64 | |
| SEQ ID NO:65 | |
| SEQ ID NO:66 | |
| SEQ ID NO:67 | |
| SEQ ID NO:68 | |
| SEQ ID NO:69 | |
| SEQ ID NO:70 | |
| SEQ ID NO:71 | |
| SEQ ID NO:72 | |
| SEQ ID NO:73 | |
| SEQ ID NO:74 | |
| SEQ ID NO:75 | |
| SEQ ID NO:76 | |
| | |
| SEQ ID NO:77 | |
| SEQ ID NO:78 | |
| | |
| SEQ ID NO:79 | |
| SEQ ID NO:80 | |
| SEQ ID NO:81 | |
| SEQ ID NO:82 | |
| | |
| SEQ ID NO:83 | |
| SEQ ID NO:84 | |
| SEQ ID NO:85 | |
| SEQ ID NO:86 | |
| SEQ ID NO:87 | |
| SEQ ID NO:88 | |
| | |
| SEQ ID NO:89 | |
| SEQ ID NO:90 | |
| SEQ ID NO:91 | |
| | |
| SEQ ID NO:92 | |
| SEQ ID NO:93 | |
| | |
| SEQ ID NO:94 | |
| SEQ ID NO:95 | |
| SEQ ID NO:96 | |
| SEQ ID NO:97 | |
| SEQ ID | |
| NO:98 | |
| SEQ ID NO:99 | |
| SEQ ID NO:100 | |
| SEQ ID NO:101 | |
| SEQ ID NO:102 | |
| SEQ ID NO:103 | |
| SEQ ID NO:104 | |
| SEQ ID NO:105 | |
| SEQ ID NO:106 | |
| SEQ ID NO:107 | |
| SEQ ID NO:108 | |
| SEQ ID NO:109 | |
| SEQ ID NO:110 | |
| SEQ ID NO:111 | |
| SEQ ID NO:112 | |
| SEQ ID NO:113 | |
| | |
| SEQ ID NO:114 | |
| SEQ ID NO:115 | |
| SEQ ID NO:116 | |
| SEQ ID NO:117 | |
| SEQ ID NO:118 | |
| SEQ ID NO:119 | |
| | |
| SEQ ID NO:120 | |
| SEQ ID NO:121 | |
| SEQ ID NO:122 | |
| SEQ ID NO:123 | |
| SEQ ID NO:124 | |
| | |
| SEQ ID NO:125 | |
| SEQ ID NO:126 | |
| SEQ ID NO:127 | |
| SEQ ID NO:128 | |
| SEQ ID NO:129 | |
| SEQ ID NO:130 | |
| SEQ ID NO:149 | |
| SEQ ID NO:150 | |
| | |

**Table 3. Genomic Sequences Encoding Certain Rice Polypeptides**

| Protein | Genomic Sequence |
|---|---|
| 2 | |
| 3 | |
| 50 | |
| 51 | |
| 52 | |
| | |
| 53 | |
| 54 | |
| 55 | |
| | |
| 56 | |
| | |
| 57 | |
| | |
| 58 | |
| | |
| 59 | |
| | |
| 60 | |
| | |
| 61 | |
| | |
| 62 | |
| 63 | |
| 64 | |
| 65 | |
| 66 | |
| | |

The above-described polypeptides are involved in one or more important biological properties in plants, e.g., rice seed yields, panicle formation, and/or tolerance to environmental stresses. Such polypeptides can be produced in transgenic plants to provide plants having improved phenotypic properties or improved response to stressful environmental conditions.

Also described herein is an isolated nucleic acid that includes a polynucleotide (e.g., SEQ ID NOs: 50-98, 115-148, 150, and 151) encoding any of the polypeptides described above. An isolated nucleic acid refers to a nucleic acid the structure of which is not identical to that of any naturally occurring nucleic acid or to that of any fragment of a naturally occurring genomic nucleic acid. The term therefore covers, for example, (a) a DNA which has the sequence of part of a naturally occurring genomic DNA molecule but is not flanked by both of the coding sequences that flank that part of the molecule in the genome of the organism in which it naturally occurs; (b) a nucleic acid incorporated into a vector or into the genomic DNA of a prokaryote or eukaryote in a manner such that the resulting molecule is not identical to any naturally occurring vector or genomic DNA; (c) a separate molecule such as a cDNA, a genomic fragment, a fragment produced by polymerase chain reaction (PCR), or a restriction fragment; and (d) a recombinant nucleotide sequence that is part of a hybrid gene, i.e., a gene encoding a fusion protein. In one example, the just-described isolated nucleic acid is a portion of a recombinant DNA construct, which preferably is an expression vector containing a promoter region operably linked to the just-mentioned nucleic acid. Upon introducing into a plant cell, this DNA construct expresses a polypeptide encoded by the polynucleotide.

The expression vector described above can be used to generate transgenic plants to provide for increased expression of the polypeptides also described above. As a result of such biotechnological applications, plants, particularly crop plants, having improved properties are obtained. Crop plants of interest in the present invention include, but are not limited to soy, cotton, canola, maize, wheat, sunflower, sorghum, alfalfa, barley, millet, rice, tobacco, fruit and vegetable crops, and turf grass.

Any of the recombinant DNA constructs described above can be used to transform a host cell, e.g., an E. coli., a yeast, an insect, a plant, or a mammalian cell. The DNA construct and the transformed host cell can be used for producing a polypeptide described herein.

The just-described DNA construct and a host plant cell transformed with the DNA construct can also be used for generating a transgenic plant containing the recombinant DNA construct described above to provide for increased or decreased expression of the polypeptides described herein. To generate a transgenic plant, one can (1) introduce into a plant cell a recombinant nucleic acid encoding one just- described heterologous polypeptide; (2) expressing the polypeptide in the cell, and (3) cultivating the cell to generate a plant. As a result of such biotechnological applications, plants, in particular, crop plants having one or more of the improved properties described below are obtained. Exemplary crop plants include, but are not limited to, soy, cotton, canola, maize, wheat, sunflower, sorghum, alfalfa, barley, millet, rice, tobacco, fruit and vegetable crops, and turf grass. The crop plants, when transformed with any of the DNA construct described herein have improved yields resulting from one or more of the following mechanisms:
(1) Improving utilization of key biochemical compounds, such as nitrogen, phosphorous, mineral, and carbohydrate. For example, a polypeptide of interest may improve nitrogen flow, sensing, uptake, storage and/or transport. Examples of such polypeptide include those involved in aspartate and glutamate biosynthesis,in aspartate and glutamate transport, associated with the TOR (Target of Rapamycin) pathway, nitrate transporters, ammonium transporters, chlorate transporters, or involved in tetrapyrrole biosynthesis. In another example, a polypeptide of interest effects on carbohydrate metabolism, for example by increasing sucrose production and/or transport, e.g., a polypeptide that plays a role in sucrose or starch metabolism, in carbon assimilation or carbohydrate transport (e.g., sucrose transporters or glucose/hexose transporters). Such a polypeptide can also be an enzyme involved in glycolysis/gluconeogenesis, the pentose phosphate cycle, or raffinose biosynthesis, or one that is involved in glucose signaling, such as SNF1 complex proteins. In yet another example, a polypeptide of interest, e.g., a phosphatase or phosphate transporter, is capable of increasing phosphorus uptake, transport or utilization.
(2) Improving responses to environmental stresses, such as cold, heat, drought, salt, pest/pathogen, or herbicide. Polypeptides useful for improved stress tolerance under a variety of stress conditions include polypeptides involved in gene regulation, such as serine/threonine-protein kinases, MAP kinases, MAP kinase kinases, and MAP kinase kinase kinases; polypeptides that act as receptors for signal transduction and regulation, such as receptor protein kinases; intracellular signaling proteins, such as protein phosphatases, GTP binding proteins, and phospholipid signaling proteins; polypeptides involved in arginine biosynthesis; polypeptides involved in ATP metabolism, including for example ATPase, adenylate transporters, and polypeptides involved in ATP synthesis and transport; polypeptides involved in glycine betaine, jasmonic acid, flavonoid or steroid biosynthesis; and hemoglobin. Enhanced or reduced activity of such polypeptides in transgenic plants will provide changes in the ability of a plant to respond to a variety of environmental stresses, such as those mentioned above.
   For example, polypeptides that improve plant tolerance to cold or freezing temperatures include those involved in biosynthesis of trehalose or raffinose, those encoded by cold induced genes, fatty acyl desaturases and others involved in glycerolipid or membrane lipid biosynthesis, which find use in modification of membrane fatty acid composition, alternative oxidase, calcium-dependent protein kinases, LEA proteins and uncoupling protein. Exemplary polypeptides capable of improving plant tolerance to heat include polypeptides involved in biosynthesis of trehalose, glycerolipid biosynthesis, or membrane lipid metabolism (for altering membrane fatty acid composition), e.g., heat shock proteins or mitochondrial NDK. Polypeptides that increase plant tolerance to extreme osmotic conditions are those that play roles in proline biosynthesis, and polypeptides that increase plant tolerance to drought are those involved in biosynthesis of trehalose, wax, LEA proteins or invertase, e.g., aquaporins. As to pest/pathogen tolerance, any of the following polypeptides contributes to this feature: proteases, a polypeptide involved in anthocyanin biosynthesis, a polypeptide involved in cell wall metabolism (e.g., cellulases, glucosidases, pectin methylesterase, pectinase, polygalacturonase, chitinase, chitosanase, or cellulose synthase), a polypeptide involved in biosynthesis of terpenoids or indole for production of bioactive metabolites to provide defense against herbivorous insects. Polypeptides contributing to plant herbicides tolerance include those involved in the shikimate pathway, which are of interest for providing glyphosate tolerant plants. Such polypeptides include polypeptides involved in biosynthesis of chorismate, phenylalanine, tyrosine and tryptophan.
(3) Modifying plant growth rate or cell cycle. Such polypeptides include those that encode cell cycle enzymes and regulators of the cell cycle pathway, e.g., cyclins and EIF5alpha pathway proteins, polypeptides involved in polyamine metabolism, polypeptides which act as regulators of the cell cycle pathway, including cyclin-dependent kinases (CDKs), CDK-activating kinases, CDK-inhibitors, Rb and Rb-binding proteins, and transcription factors that activate genes involved in cell proliferation and division, such as the E2F family of transcription factors, proteins involved in degradation of cyclins, such as cullins, and plant homologs of tumor suppressor polypeptides. They also include those involved in the biosynthesis of plant growth hormones, such as gibberellins, cytokinins, auxins, ethylene and abscisic acid, and other proteins involved in the activity and/or transport of such polypeptides, including for example, cytokinin oxidase, cytokinin/purine permeases, F-box proteins, G-proteins and phytosulfokines. These polypeptides are useful for manipulating growth rate in plants to provide early vigor and accelerated maturation leading to improved yield. Improvements in quality traits, such as seed oil content, may also be o obtained by expression of cell cycle enzymes and cell cycle regulators.
(4) Modifying photosynthesis pathway. Polypeptides useful for increasing the rate of photosynthesis include phytochrome, photosystem I and II proteins, electron carriers, ATP synthase, NADH dehydrogenase and cytochrome oxidase.
(5) Regulating seed/panicle formation and size/weight. Such polypeptides increase seed protein quantity/quality (e.g., polypeptides involved in the metabolism of amino acids in plants, and polypeptides involved in biosynthesis of methionine/cysteine and lysine, amino acid transporters, amino acid efflux carriers, seed storage proteins, proteases, and polypeptides involved in phytic acid metabolism), increase seed oil quantity and/or quality (e.g., polypeptides involved in fatty acid and glycerolipid biosynthesis, beta-oxidation enzymes, enzymes involved in biosynthesis of nutritional compounds, such as carotenoids and tocopherols, and polypeptides that increase embryo size or number or thickness of aleurone).
(6) Regulating homologous recombination. Increasing the rate of homologous recombination in plants is useful for accelerating the introgression of transgenes into breeding varieties by backcrossing, and to enhance the conventional breeding process by allowing rare recombinants between closely linked genes in phase repulsion to be identified more easily. Polypeptides useful for expression in plants to provide increased homologous recombination include polypeptides involved in mitosis and/or meiosis, including for example, resolvases and polypeptide members of the RAD52 epistasis group. The polypeptides described herein may also play roles in imparting improved disease resistance or increased reserve polysaccharides for use in food, pharmaceutical, cosmetic, paper and paint industries by improving production of galactomannans,to a transgenic plant carrying genes encoding such. They may also modify flavonoid/isoflavonoid metabolism in plants (e.g., cinnamate-4-hydroxylase, chalcone synthase), or affect lignin biosynthesis. Enhanced or reduced activity of such polypeptides in transgenic plants will provide changes in the quantity and/or speed of flavonoid metabolism in plants, improve disease resistance by enhancing synthesis of protective secondary metabolites or improving signaling pathways governing disease resistance, or increase plants' resistance to lodging and for increasing the usefulness of plant materials as biofuels.

In addition to the mechanisms set forth above, the polypeptides described herein may also affect flowering, pollination or fertilization efficiency or improve plant growth under undesirable conditions, e.g., low fertilizer concentration or environmental stresses. Table 1 above lists the phenotypes that the polypeptides described herein contribute to. Other functions of these polypeptides can be determined by comparison of the amino acid sequence of the novel polypeptides to amino acid sequences of known polypeptides. A variety of homology based search algorithms are available to compare a query sequence to a protein database, including for example, BLAST, FASTA, and Smith-Waterman. In the present application, BLASTX and BLASTP algorithms are used to provide protein function information.

There is also described a transgenic plant produced by the method described above.

## Claims

1. A method of increasing seed size in a plant by transforming a host plant or host plant cell with a recombinant DNA construct containing a promoter sequence operably linked to a polynucleotide encoding a polypeptide having an amino acid sequence at least 80% identical to the sequence of SEQ ID NO:103, the promoter sequence being functional in a cell of the host plant or in the host plant cell.

2. The method of claim 1, wherein the polynucleotide encodes a polypeptide having the amino acid sequence of SEQ ID NO:103.

3. The method of claim 1, wherein the polynucleotide has the nucleotide sequence of SEQ ID NO:119.

4. The method of claim 1, wherein the host plant is a crop.

## Patentansprüche

1. Ein Verfahren zum Steigern der Samengröße in einer Pflanze durch Transformieren einer Wirtspflanze oder einer Wirtspflanzenzelle mit einem rekombinanten DNA Konstrukt enthaltend eine Promotorsequenz funktionsfähig verknüpft an ein Polynukleotid kodierend ein Polypeptid mit einer Aminosäuresequenz mindestens 80% identisch zu der Sequenz von SEQ ID NO:103, wobei die Promotorsequenz funktional in einer Zelle der Wirtspflanze oder in der Wirtspflanzenzelle ist.

2. Das Verfahren nach Anspruch 1, wobei das Polynukleotid ein Polypeptid kodiert mit der Aminosäuresequenz nach SEQ ID NO:103.

3. Das Verfahren nach Anspruch 1, wobei das Polynukleotid die Nukleotidsequenz von SEQ ID NO:119 hat.

4. Das Verfahren nach Anspruch 1, wobei die Wirtspflanze ein Getreide ist.

## Revendications

1. Méthode d'augmentation de la taille des graines dans une plante par transformation d'une plante hôte ou d'une cellule végétale hôte avec une construction d'ADN recombinant contenant une séquence promoteur liée de façon fonctionnelle à un polynucléotide codant pour un polypeptide ayant une séquence d'acides aminés au moins identique à 80 % à la séquence de SEQ ID NO : 103, la séquence promoteur étant fonctionnelle dans une cellule de la plante hôte ou dans la cellule végétale hôte.

2. Méthode selon la revendication 1, dans laquelle le polynucléotide code pour un polypeptide ayant la séquence d'acides aminés de SEQ ID NO : 103.

3. Méthode selon la revendication 1, dans laquelle le polynucléotide a la séquence nucléotidique de SEQ ID NO : 119.

4. Méthode selon la revendication 1, dans laquelle la plante hôte est une culture.
